# EUROPEAN PATENT SPECIFICATION

(11) **EP 2 508 585 B1**
(45) Date of publication and mention of the grant of the patent: **29.06.2016**
(21) Application number: 11852216.8
(22) Date of filing: 29.04.2011
(51) Int. Cl.: C09K 11/06, H01L 51/54, C09B 57/00, H01L 51/00, H01L 51/50

(54) **COMPOUND FOR ORGANIC OPTOELECTRONIC DEVICE, ORGANIC LIGHT EMITTING DIODE INCLUDING THE SAME, AND DISPLAY DEVICE INCLUDING ORGANIC LIGHT EMITTING DIODE**
VERBINDUNG FÜR ORGANISCHE OPTOELEKTRONISCHE VORRICHTUNG, ORGANISCHE LICHTEMITTIERENDE DIODE DAMIT UND ANZEIGEVORRICHTUNG MIT ORGANISCHER LICHTEMITTIERENDER DIODE
COMPOSÉ POUR DISPOSITIF OPTOÉLECTRONIQUE ORGANIQUE, DIODE ÉLECTROLUMINESCENTE ORGANIQUE LE CONTENANT ET DISPOSITIF D'AFFICHAGE UTILISANT LA DIODE ÉLECTROLUMINESCENTE ORGANIQUE

(30) Priority: 31.12.2010 KR 20100140563
(43) Date of publication of application: 10.10.2012
(73) Proprietor: Cheil Industries Inc., Kumi-city, Kyungsangbuk-do 730-030 (KR)
(72) Inventor: KANG, Dong-Min, Uiwang-si Gyeonggi-do 437-711 (KR); KANG, Myeong-Soon, Uiwang-si Gyeonggi-do 437-711 (KR); KIM, Nam-Soo, Uiwang-si Gyeonggi-do 437-711 (KR); SHIN, Chang-Ju, Uiwang-si Gyeonggi-do 437-711 (KR); LEE, Nam-Heon, Uiwang-si Gyeonggi-do 437-711 (KR); JUNG, Ho-Kuk, Uiwang-si Gyeonggi-do 437-711 (KR); CHAE, Mi-Young, Uiwang-si Gyeonggi-do 437-711 (KR)
(74) Representative: Michalski Hüttermann & Partner Patentanwälte mbB
(86) International application number: PCT/KR2011/003224
(87) International publication number: WO 2012/091225

(56) References cited:
- EP-A1- 1 783 189
- WO-A1-2004/017137
- WO-A1-2006/021982
- WO-A1-2006/039982
- WO-A1-2009/100925
- WERNER HERZ ET AL: "The Dimer of 1,3-Diphenyl-1,3-butadiene 1,2", THE JOURNAL OF ORGANIC CHEMISTRY, vol. 23, no. 11, 1 November 1958 (1958-11-01), pages 1646-1653, XP055134268, ISSN: 0022-3263, DOI: 10.1021/jo01105a017
- R Belcher ET AL: "[l958] New Substituted Naphthylamines and Naphthidines", Journal of the chemical society, 3 March 1958 (1958-03-03), pages 3243-3248, XP055134521, DOI: 10.1039/JR9580003243 Retrieved from the Internet: URL:http://pubs.rsc.org/en/content/article pdf/1958/jr/jr9580003243 [retrieved on 2014-08-13]
- DATABASE REGISTRY [Online] CHEMICAL ABSTRACTS SERVICE, COLUMBUS, OHIO, US; "STN file registry for compound with CAS No. 121964-61-2", XP055134517,
- Otto Kruber ET AL: "Zur Kenntnis des Stinkohlenteer-Anthracenöls", Chemische Berichte, vol. 85, no. 4, 3 April 1952 (1952-04-03), pages 327-333, XP055159627, ISSN: 0009-2940, DOI: 10.1002/cber.19520850410

## Description

### CROSS-REFERENCE TO RELATED APPLICATION

This application claims priority to and the benefit of Korean Patent Application No. 10-2010-0140563 filed in the Korean Intellectual Property Office on December 31, 2010.

### BACKGROUND OF THE INVENTION

### (a) Field of the Invention

A compound for an organic optoelectronic device being capable of providing an organic optoelectronic device having excellent life-span, efficiency, electrochemical stability, and thermal stability, and an organic optoelectronic device including the same are related.

### (b) Description of the Related Art

An organic optoelectronic device is, in a broad sense, a device for transforming photo-energy to electrical energy, or conversely, a device for transforming electrical energy to photo-energy.

An organic optoelectronic device may be classified as follows in accordance with its driving principles. A first organic optoelectronic device is an electronic device driven as follows: excitons are generated in an organic material layer by photons from an external light source; the excitons are separated into electrons and holes; and the electrons and holes are transferred to different electrodes as a current source (voltage source).

A second organic optoelectronic device is an electronic device driven as follows: a voltage or a current is applied to at least two electrodes to inject holes and/or electrons into an organic material semiconductor positioned at an interface of the electrodes, and the device is driven by the injected electrons and holes.

Examples of an organic optoelectronic device includes an organic photoelectric device, an organic solar cell, an organic photo conductor drum, and an organic transistor, and it requires a hole injecting or transporting material, an electron injecting or transporting material, or a light emitting material.

Particularly, an organic light emitting diode (OLED) has recently drawn attention due to an increase in demand for flat panel displays. In general, organic light emission refers to transformation of electrical energy to photo-energy.

Such an organic light emitting diode transforms electrical energy into light by applying current to an organic light emitting material. It has a structure in which a functional organic material layer is interposed between an anode and a cathode. The organic material layer includes a multi-layer including different materials, for example a hole injection layer (HIL), a hole transport layer (HTL), an emission layer, an electron transport layer (ETL), and an electron injection layer (EIL), in order to improve efficiency and stability of an organic photoelectric device.

In such an organic light emitting diode, when a voltage is applied between an anode and a cathode, holes from the anode and electrons from the cathode are injected to an organic material layer and recombined to generate excitons having high energy. The generated excitons generate light having certain wavelengths while shifting to a ground state.

Recently, it has become known that a phosphorescent light emitting material can be used for a light emitting material of an organic light emitting diode in addition to the fluorescent light emitting material. Such a phosphorescent material emits lights by transiting the electrons from a ground state to an exited state, non-radiance transiting of a singlet exciton to a triplet exciton through intersystem crossing, and transiting a triplet exciton to a ground state to emit light.

As described above, in an organic light emitting diode, an organic material layer includes a light emitting material and a charge transport material, for example a hole injection material, a hole transport material, an electron transport material, an electron injection material, and so on.

The light emitting material is classified as blue, green, and red light emitting materials according to emitted colors, and yellow and orange light emitting materials to emit colors approaching natural colors.

When one material is used as a light emitting material, a maximum light emitting wavelength is shifted to a long wavelength or color purity decreases because of interactions between molecules, or device efficiency decreases because of a light emitting quenching effect. Therefore, a host/dopant system is included as a light emitting material in order to improve color purity and increase luminous efficiency and stability through energy transfer.

In order to implement excellent performance of an organic light emitting diode, a material constituting an organic material layer, for example a hole injection material, a hole transport material, a light emitting material, an electron transport material, an electron injection material, and a light emitting material such as a host and/or a dopant, should be stable and have good efficiency. However, development of an organic material layer forming material for an organic light emitting diode has thus far not been satisfactory and thus there is a need for a novel material. This material development is also required for other organic optoelectronic devices.

A low molecular organic light emitting diode is manufactured as a thin film in a vacuum deposition method, and can have good efficiency and life-span performance. A polymer organic light emitting diode is manufactured in an Inkjet or spin coating method and has an advantage of low initial cost and being large-sized.

Both low molecular organic light emitting and polymer organic light emitting diodes have advantages of being self-light emitting and ultrathin, and having a high speed response, a wide viewing angle, high image quality, durability, a large driving temperature range, and the like, and therefore it is highlighted as the next generation display. In particular, they have good visibility due to the self-light emitting characteristic compared with a conventional LCD (liquid crystal display) and have an advantage of decreasing thickness and weight of LCD by up to a third, because they do not need a backlight.

In addition, since they have a response speed that is 1000 times faster per microsecond unit than an LCD, they can realize a perfect motion picture without an after-image. Therefore, recently it may be as an optimal display in compliance with multimedia generation, and based on these advantages, they have been remarkably developed to have 80 times the efficiency and more than 100 times the life-span since they were first developed in the late 1980s. Recently, these diodes have been used in displays that are rapidly becoming larger, such as for a 40-inch organic light emitting diode panel.

These displays must simultaneously have improved luminous efficiency and life-span in order to be larger. In order to increase the luminous efficiency, smooth combination between holes and electrons in an emission layer is needed. However, since an organic material in general has slower electron mobility than hole mobility, electron injection from a cathode and mobility using efficient electron transport layer (ETL) are to be heightened and transfer of a hole is to be inhibited, in order to realize efficient recombination of a hole and an electron in an emission layer.

In addition, the device may have a decreased life-span since the material therein may be crystallized due to Joule heat generated when it is driven. Therefore, there is a strong need for an organic compound having excellent electron injection and mobility and high thermal stability.

### SUMMARY OF THE INVENTION

A compound for an organic optoelectronic device that may act as a light emitting, or electron injection and transporting, and also as a light emitting host along with an appropriate dopant is provided.

An organic light emitting diode having excellent life-span, efficiency, a driving voltage, electrochemical stability, and thermal stability is provided.

Phenanthren and Diquinoxaline derivatives are described in Herz et al., Journal of Organic Chemistry, 1958, 1646-1653, Belcher et al. Journal of the Chemical Society, 1958, 3243, under the CAS No. 121964-61-2 and in the EP 1 783 189.

According to one aspect of the present invention, a compound for an organic optoelectronic device represented by the following Chemical Formula 2 is provided.

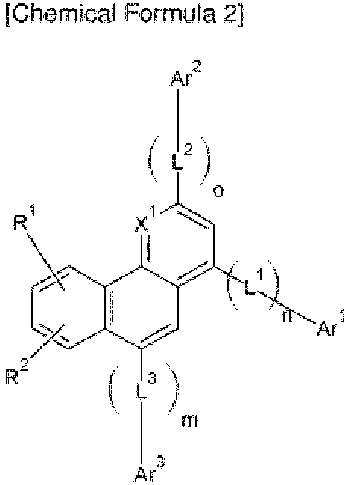

In Chemical Formula 2, X¹ is -N-, R¹ and R² are the same or different and independently hydrogen, deuterium, a substituted or unsubstituted C1 to C20 alkyl group, a substituted or unsubstituted C6 to C30 aryl group, a substituted or unsubstituted C3 to C30 heteroaryl group, or a combination thereof, Ar' to Ar³ are the same or different and independently substituted or unsubstituted C6 to C30 aryl group; or a substituted or unsubstituted C3 to C30 heteroaryl group, L¹ to L³ are the same or different and independently a single bond, a substituted or unsubstituted C2 to C6 alkenyl group, a substituted or unsubstituted C2 to C6 alkynyl group, a substituted or unsubstituted C6 to C30 arylene group, a substituted or unsubstituted C3 to C30 heteroarylene group, or a combination thereof, and n, m or o are the same or different and independently 0 or 1.

At least one of Ar¹ or Ar² may be a substituted or unsubstituted C3 to C30 heteroaryl group.

Ar¹ may be a substituted or unsubstituted C3 to C30 heteroaryl group, and Ar² and Ar³ may be a substituted or unsubstituted C6 to C30 aryl group.

Ar² may be a substituted or unsubstituted C3 to C30 heteroaryl group, and Ar¹ and Ar³ may be a substituted or unsubstituted C6 to C30 aryl group.

The substituted or unsubstituted C3 to C30 heteroaryl group may be a substituted or unsubstituted imidazolyl group, a substituted or unsubstituted triazolyl group, a substituted or unsubstituted tetrazolyl group, a substituted or unsubstituted carbazolyl group, a substituted or unsubstituted oxadiazolyl group, a substituted or unsubstituted oxatriazolyl group, a substituted or unsubstituted thiatriazolyl group, a substituted or unsubstituted benzimidazolyl group, a substituted or unsubstituted benzotriazolyl group, a substituted or unsubstituted pyridinyl group, a substituted or unsubstituted pyrimidinyl group, a substituted or unsubstituted triazinyl group, a substituted or unsubstituted pyrazinyl group, a substituted or unsubstituted pyridazinyl group, a substituted or unsubstituted purinyl group, a substituted or unsubstituted quinolinyl group, a substituted or unsubstituted isoquinolinyl group, a substituted or unsubstituted phthalazinyl group, a substituted or unsubstituted naphthpyridinyl group, a substituted or unsubstituted quinoxalinyl group, a substituted or unsubstituted quinazolinyl group, a substituted or unsubstituted acridinyl group, a substituted or unsubstituted phenanthrolinyl group, a substituted or unsubstituted phenazinyl group, or a combination thereof.

The substituted or unsubstituted C6 to C30 aryl group may be a substituted or unsubstituted phenyl group, a substituted or unsubstituted naphthyl group, a substituted or unsubstituted triperylenyl group, a substituted or unsubstituted fluorenyl group, a substituted or unsubstituted spirofluorenyl group, a substituted or unsubstituted biphenyl group, a substituted or unsubstituted terphenyl group, a substituted or unsubstituted pyrenyl group, a substituted or unsubstituted perylenyl group, a substituted or unsubstituted phenanthrenyl group, a substituted or unsubstituted anthracenyl group, or a combination thereof.

The compound for an organic optoelectronic device may be represented by one of the following Chemical Formulae A1 to A126.

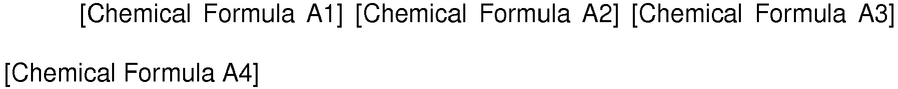

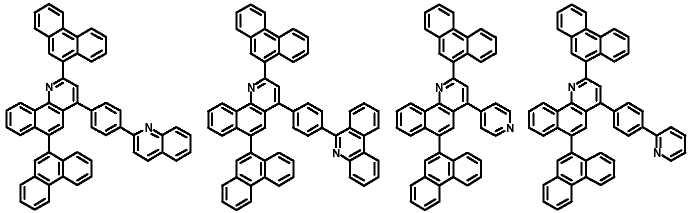

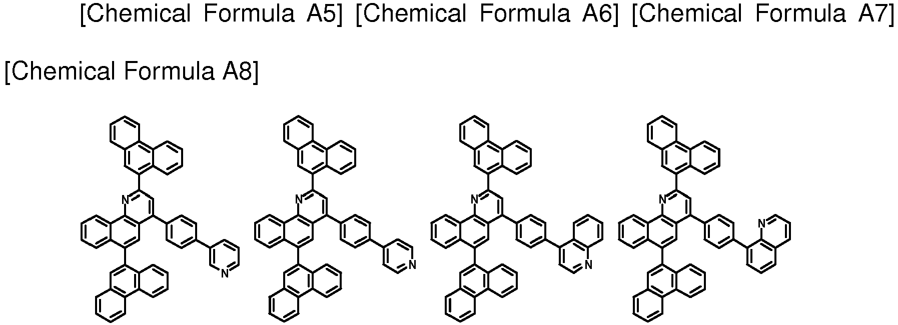

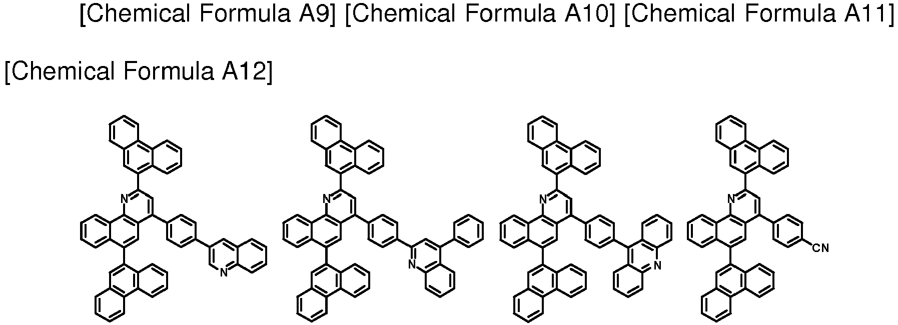

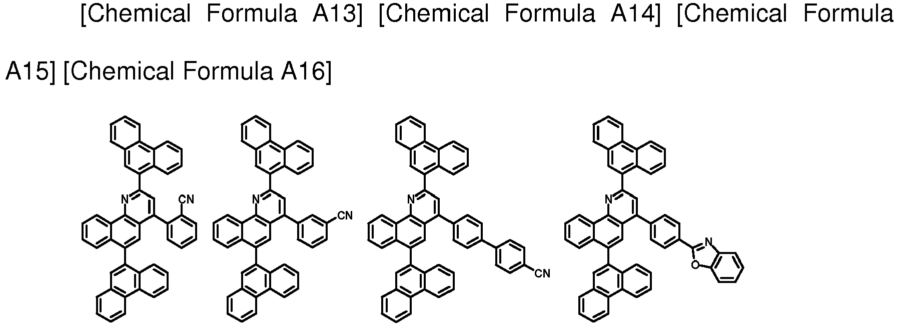

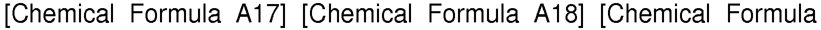

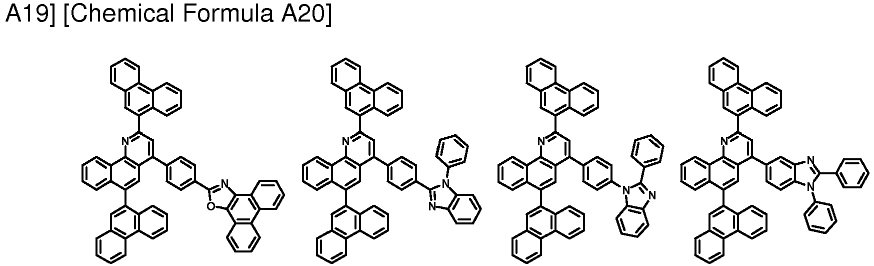

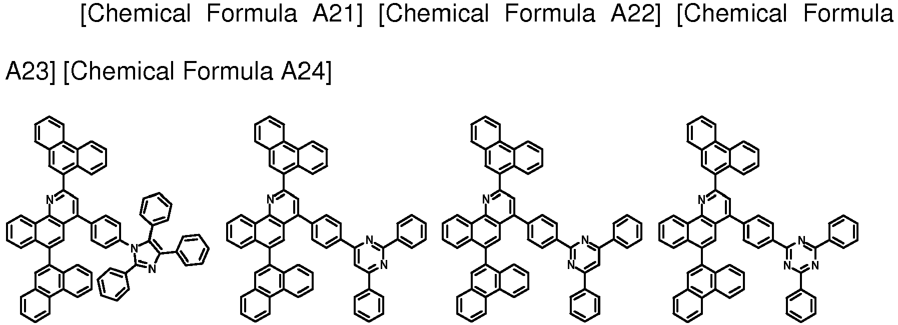

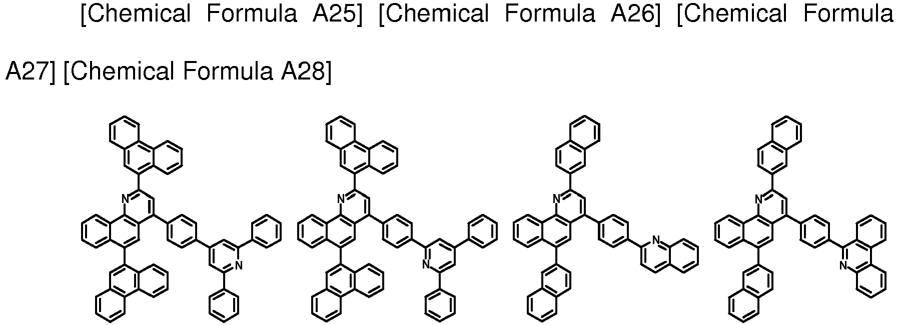

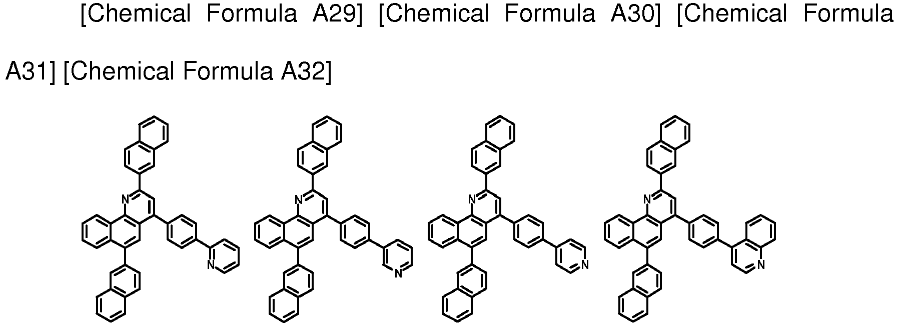

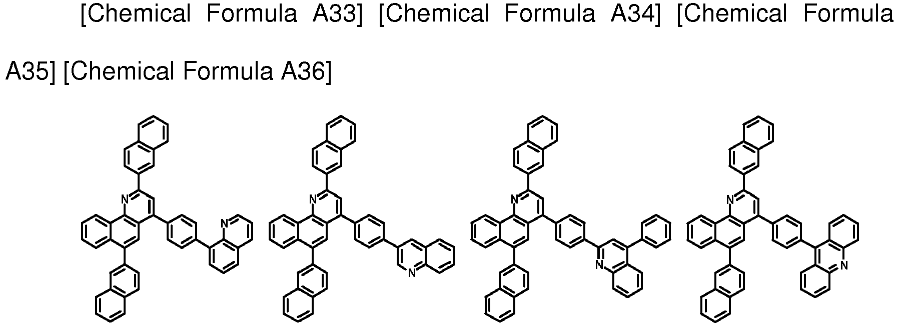

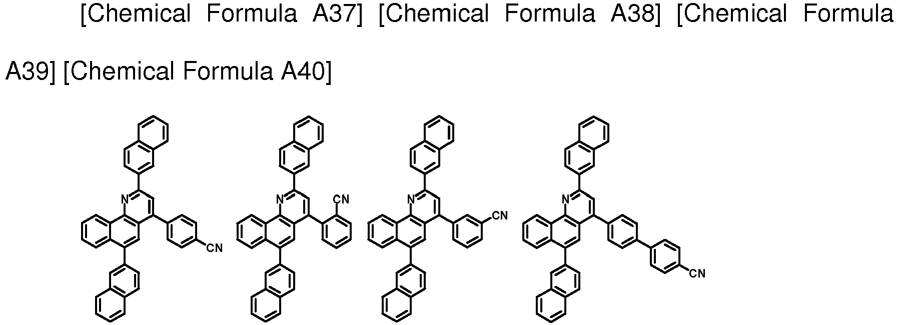

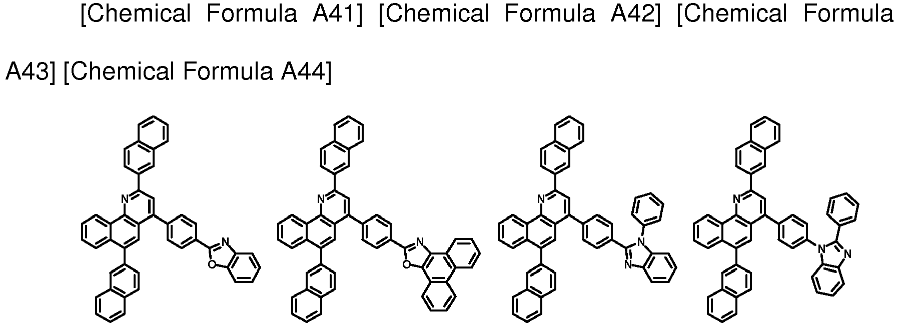

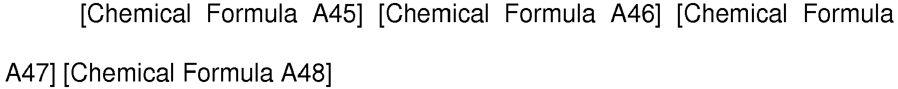

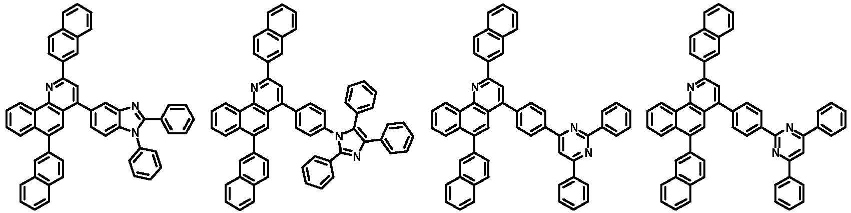

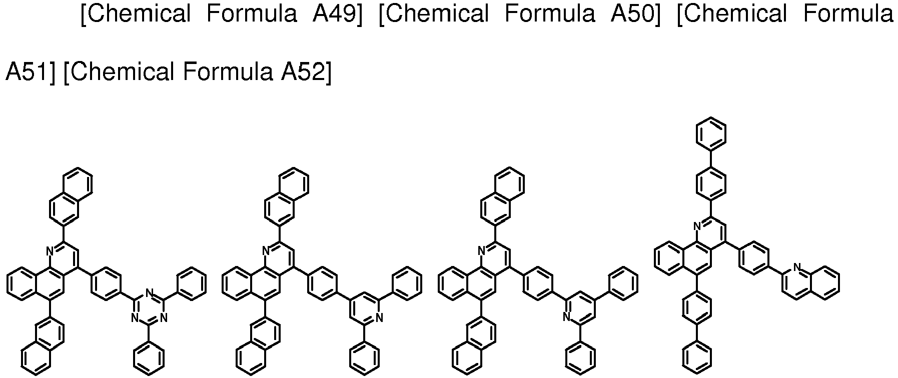

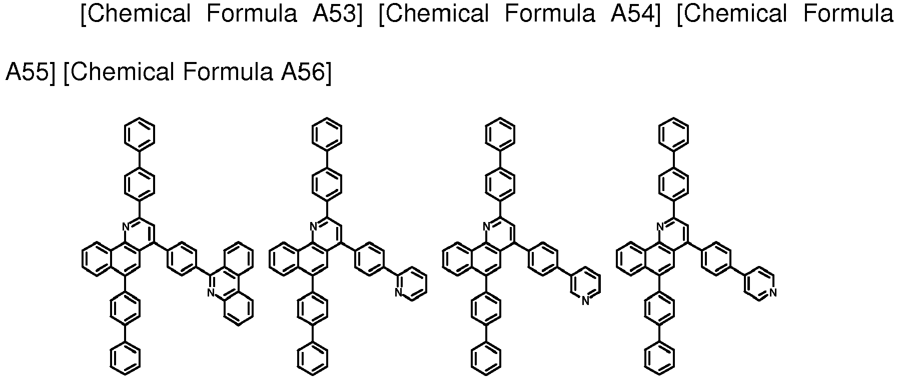

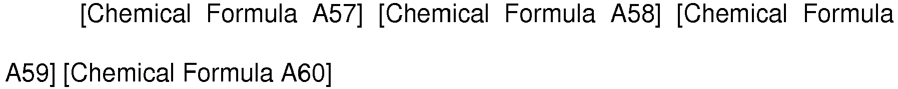

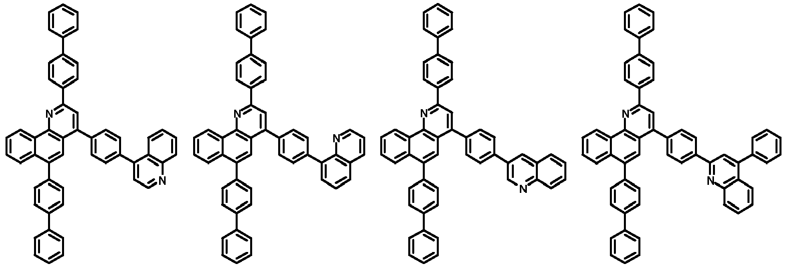

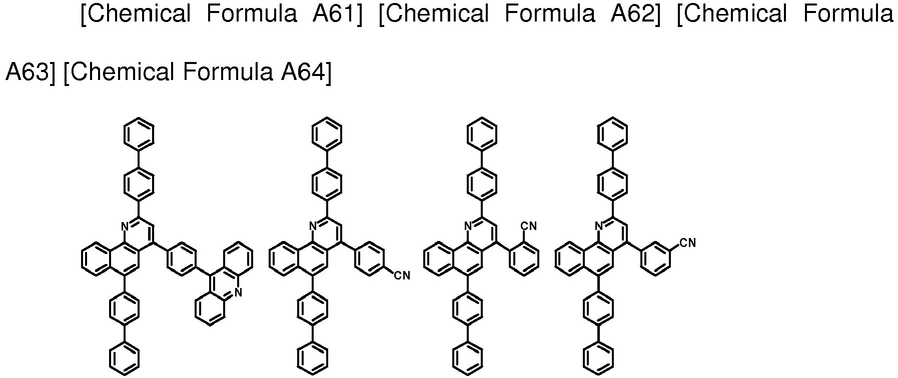

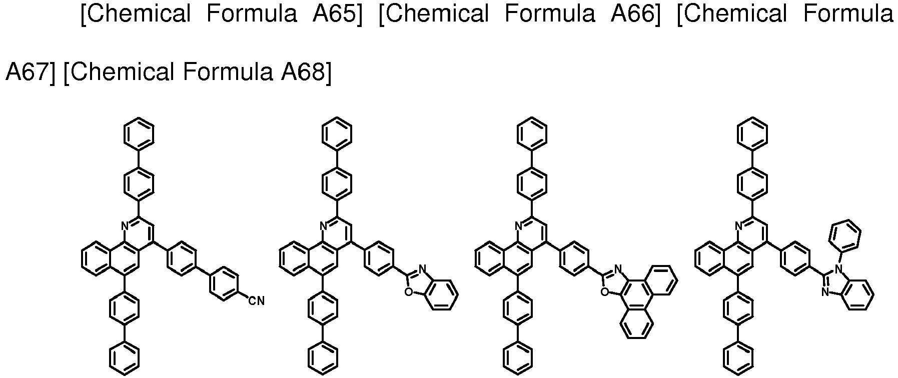

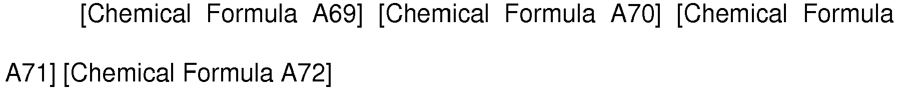

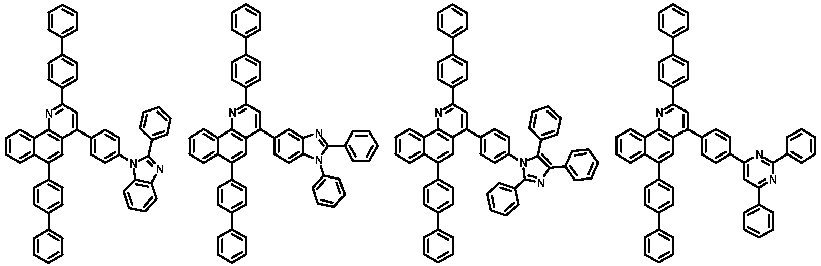

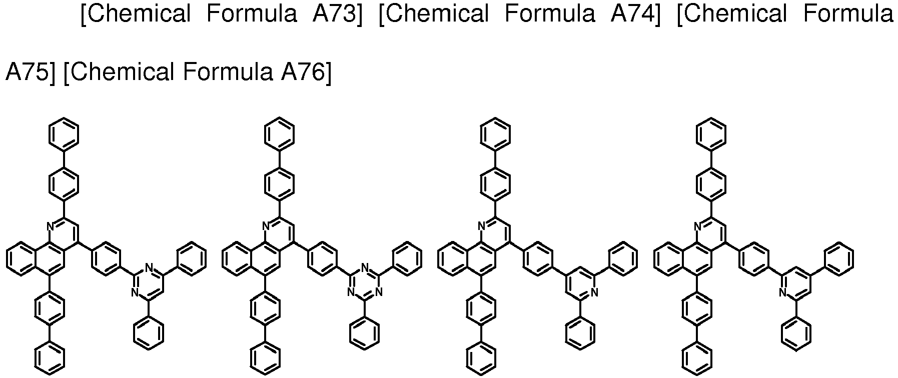

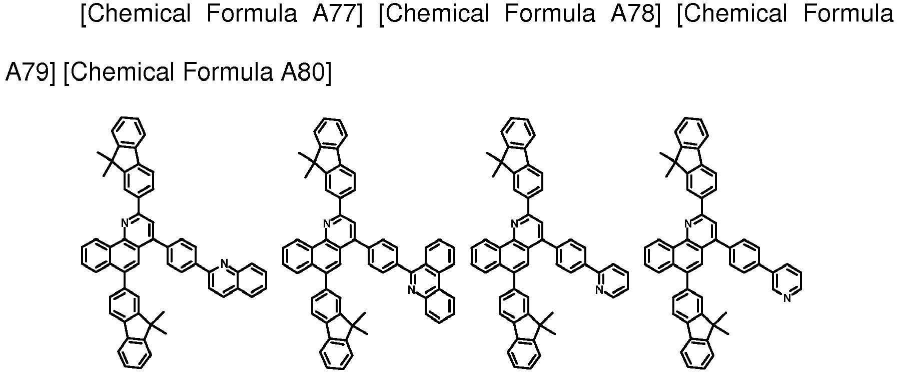

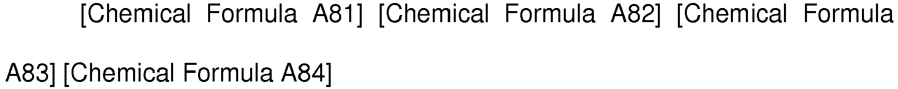

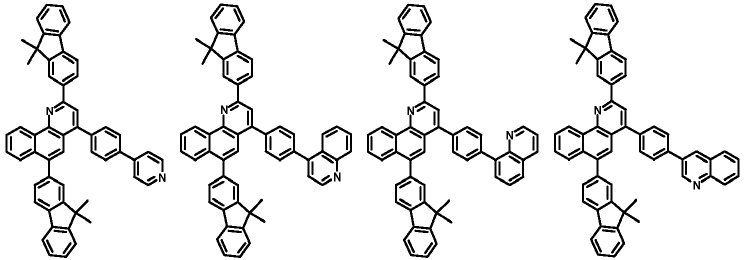

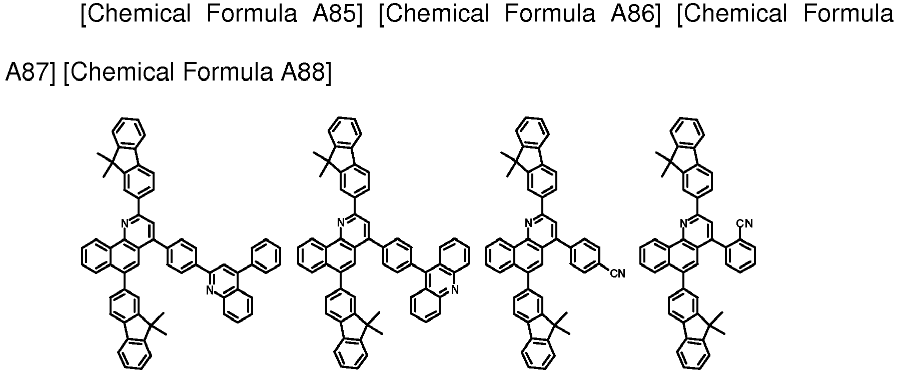

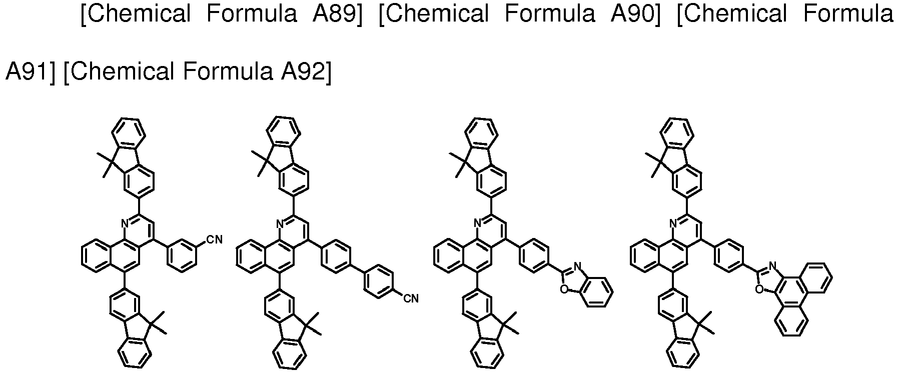

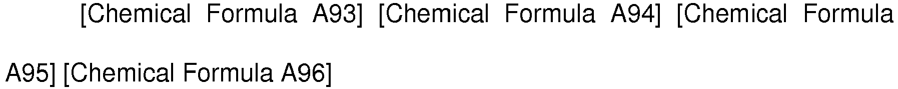

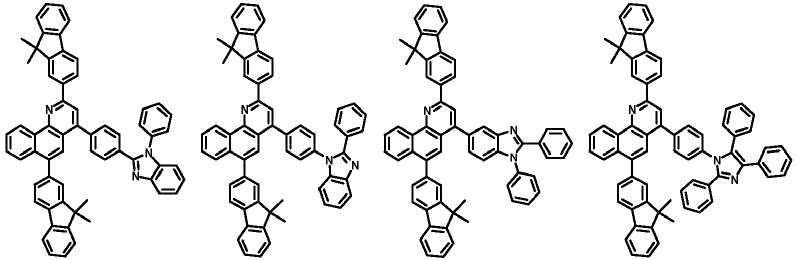

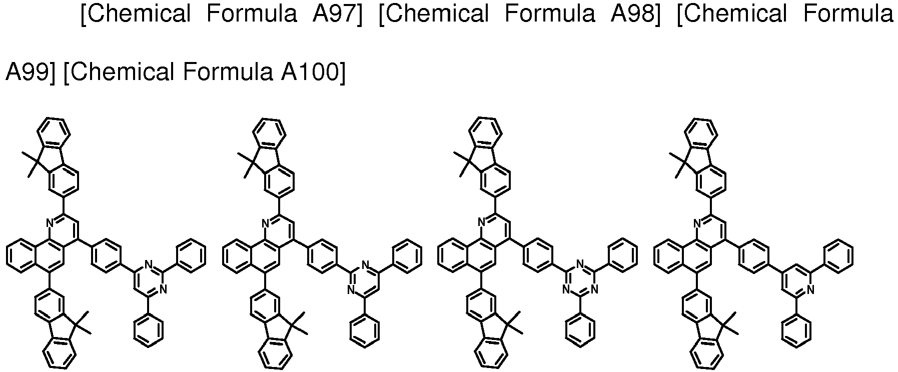

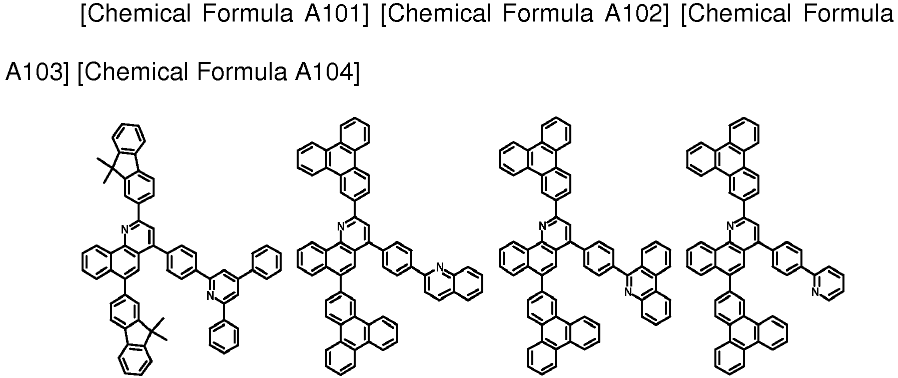

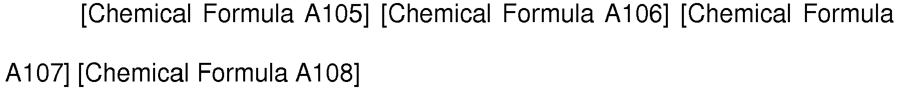

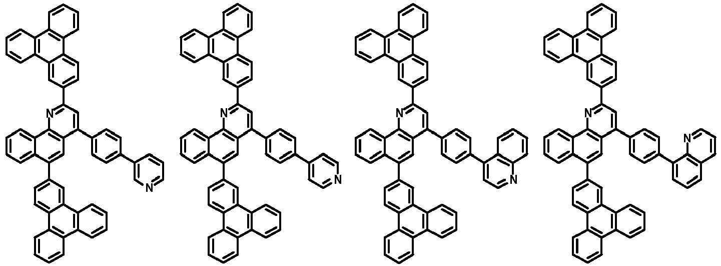

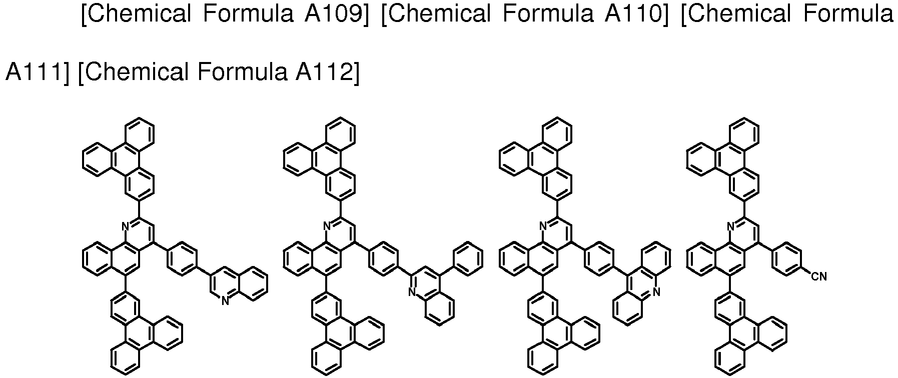

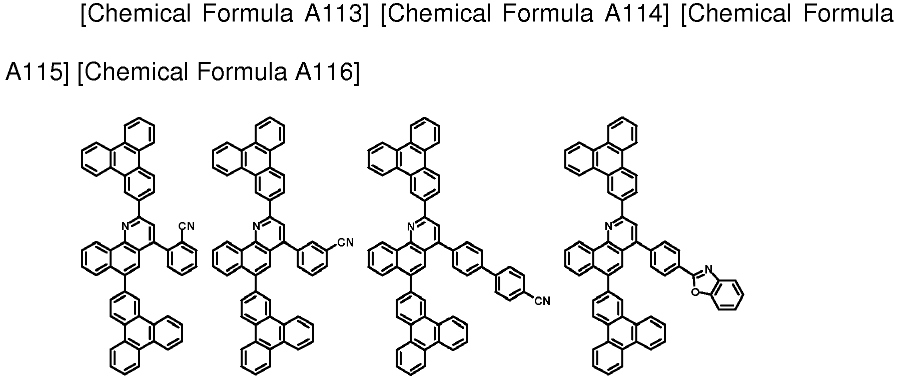

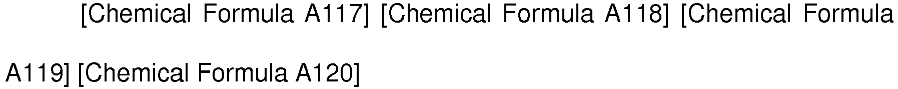

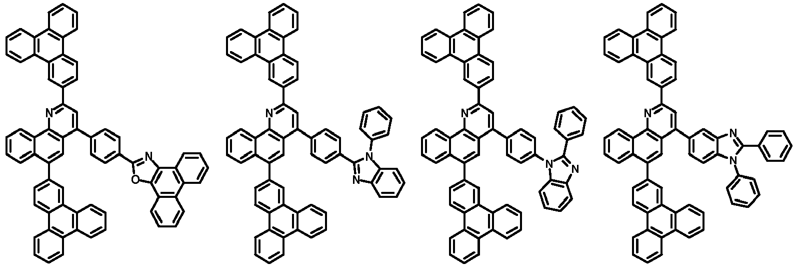

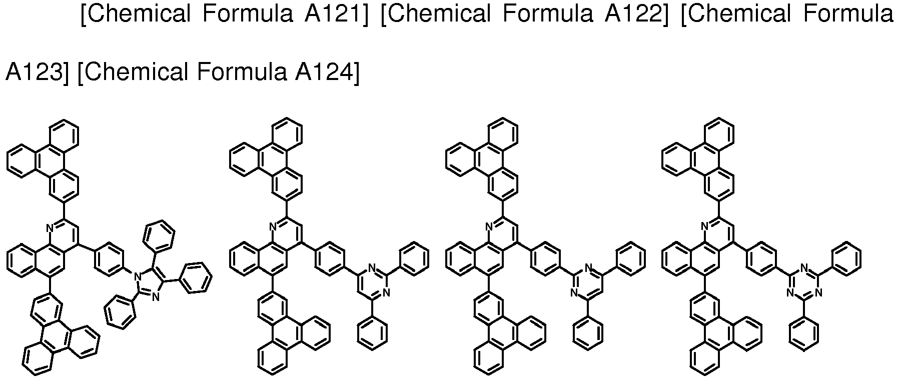

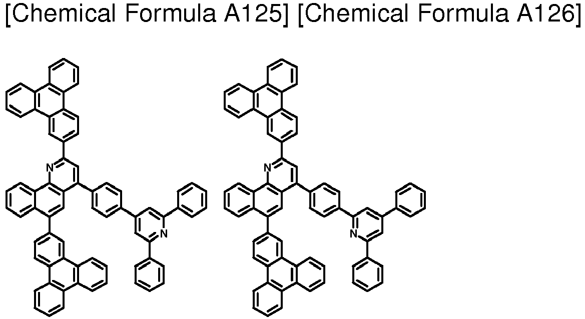

The compound for an organic optoelectronic device may be represented by one of the following Chemical Formulae B1 to B125.

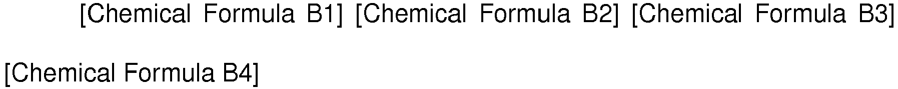

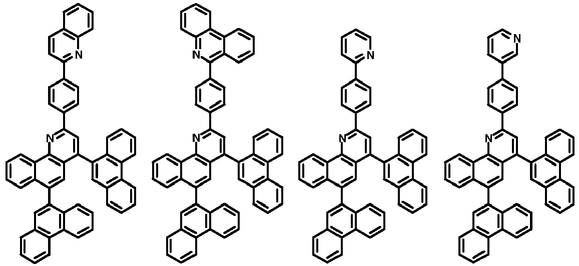

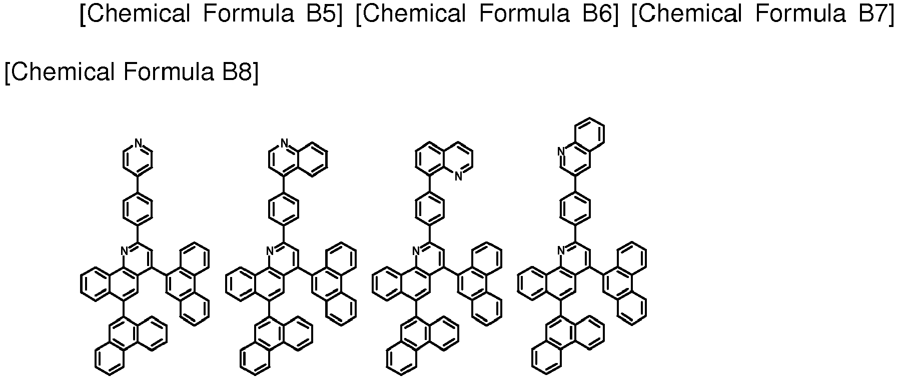

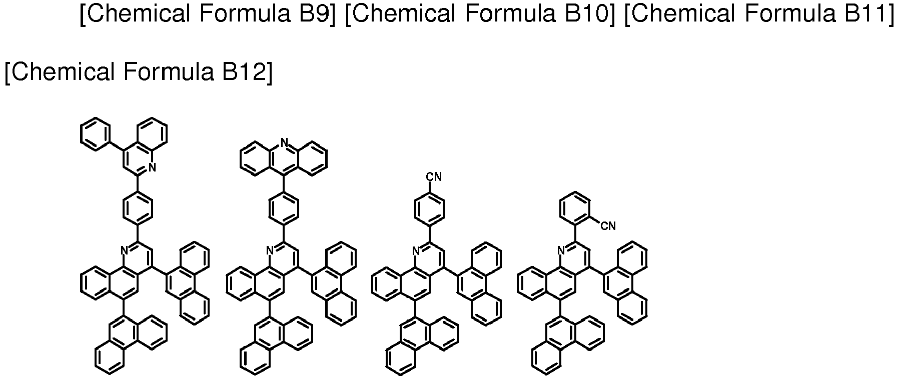

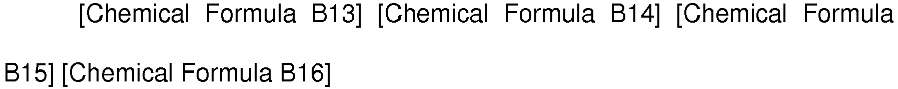

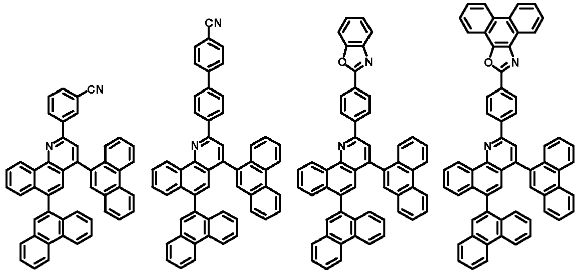

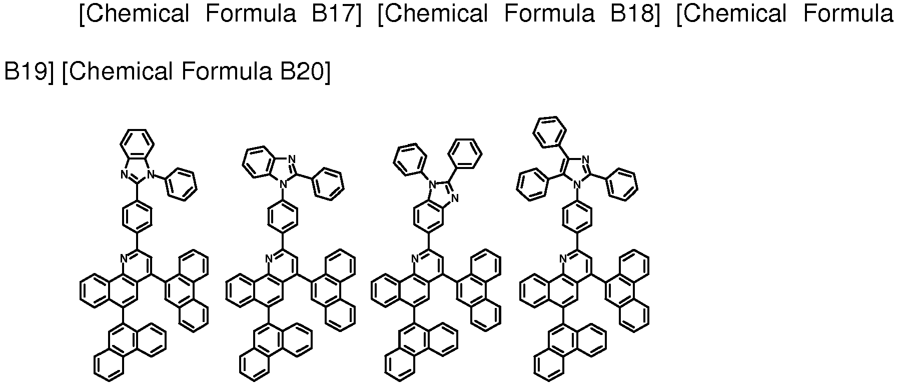

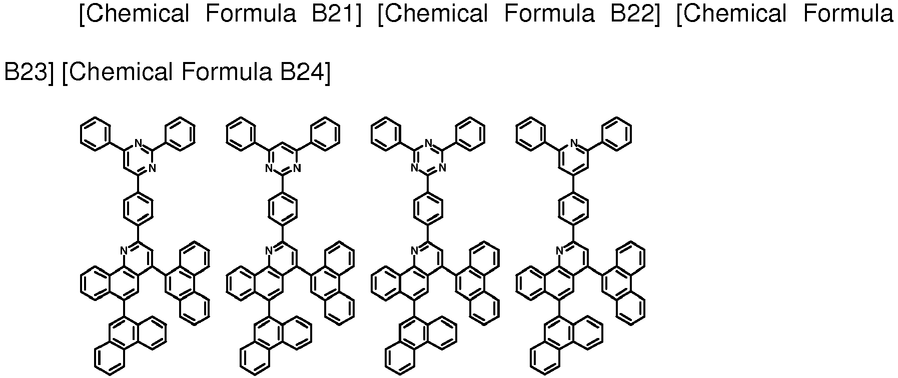

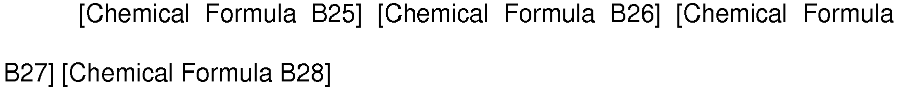

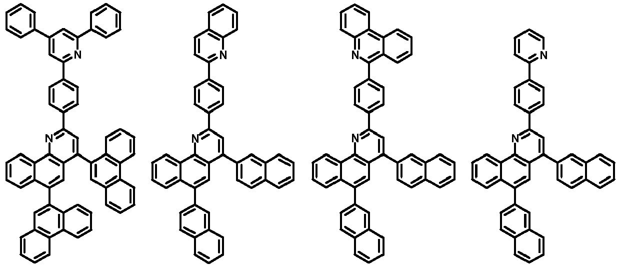

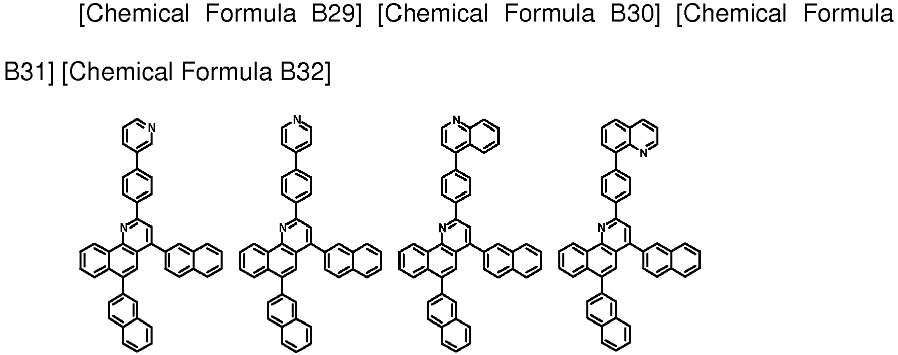

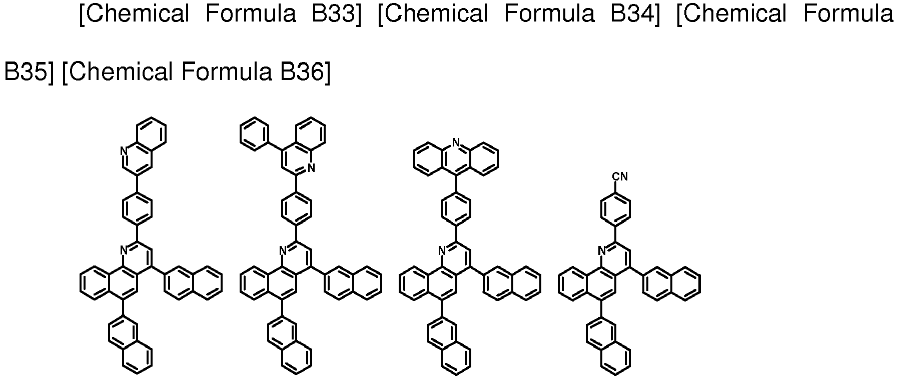

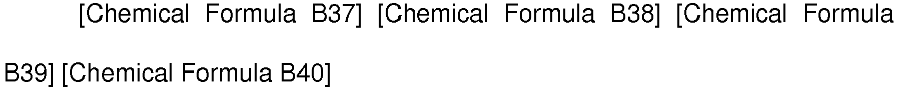

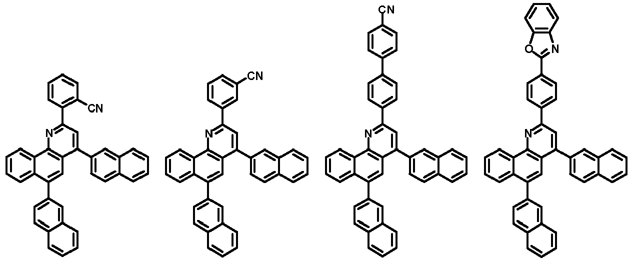

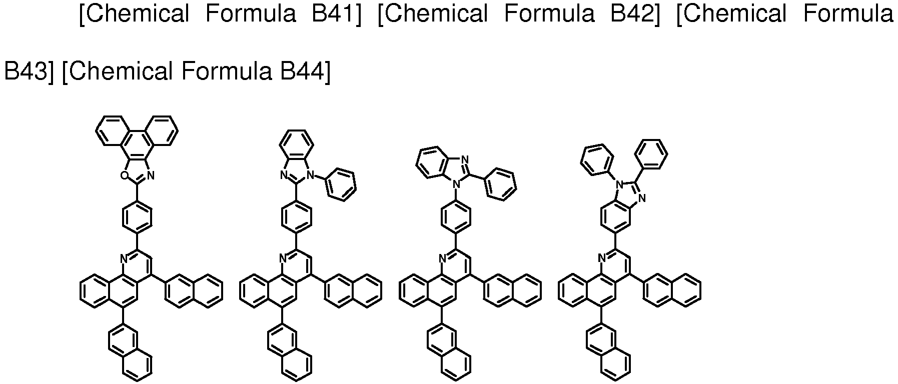

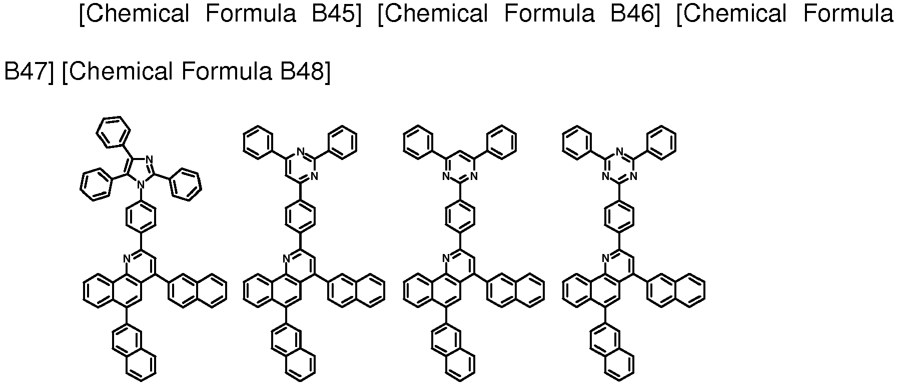

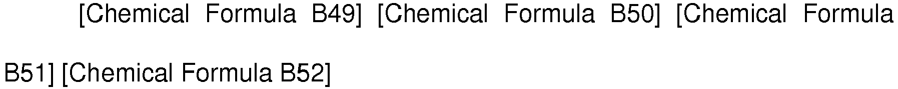

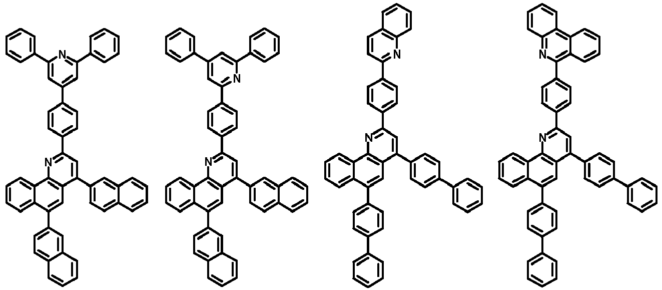

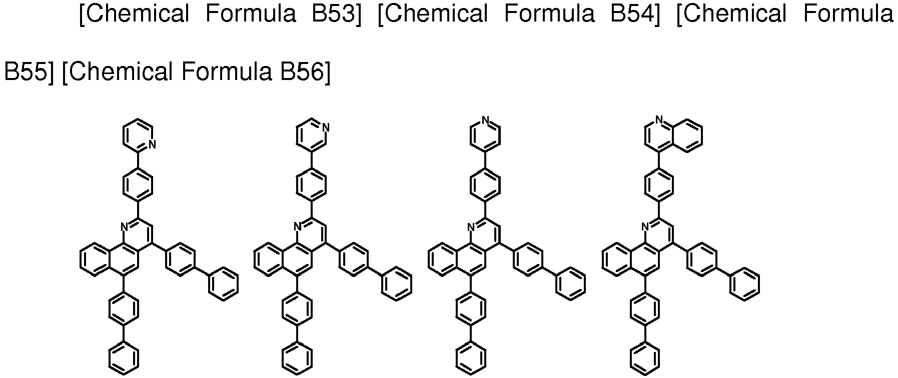

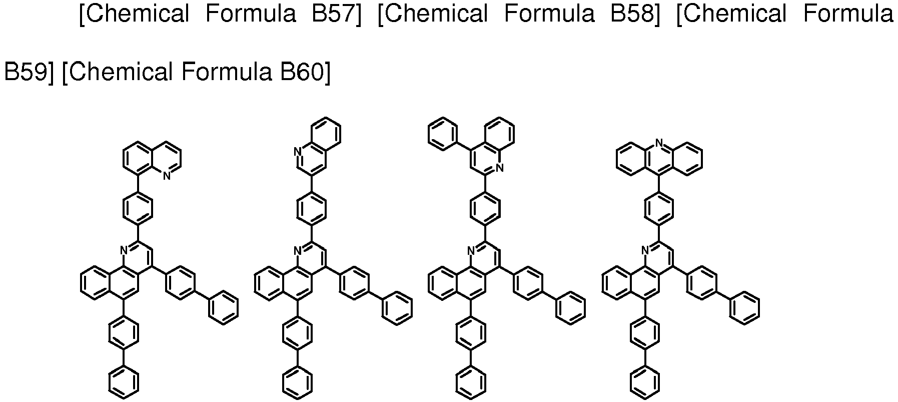

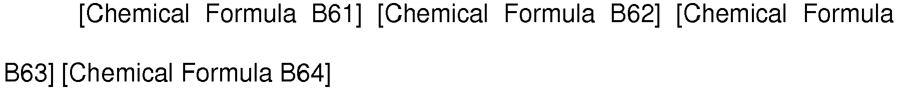

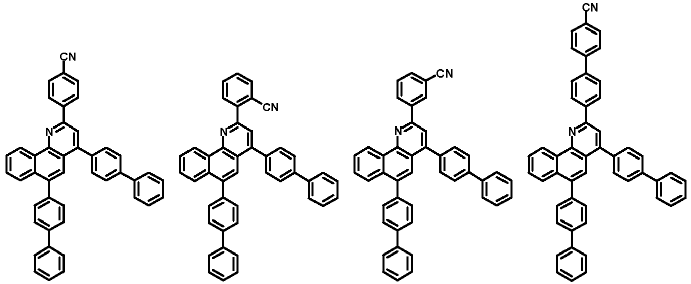

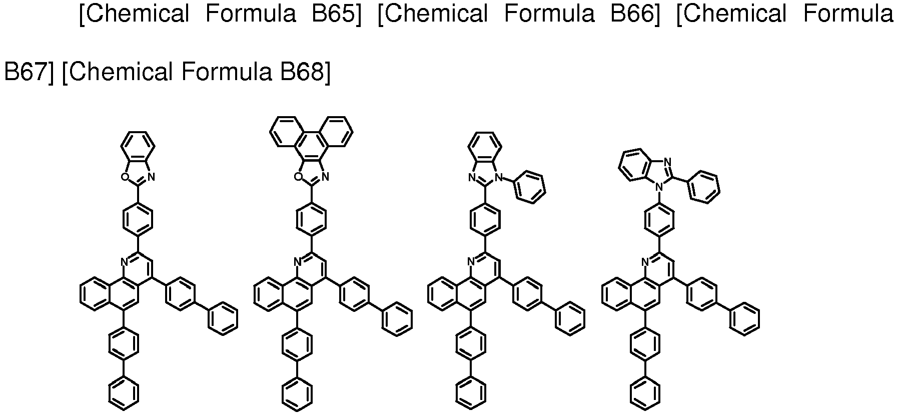

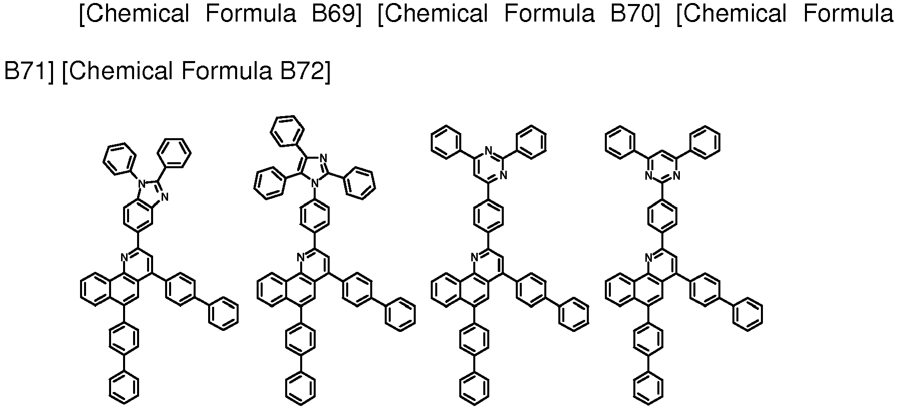

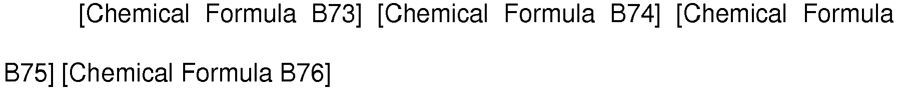

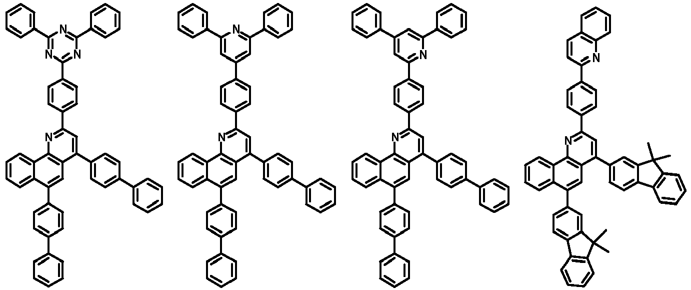

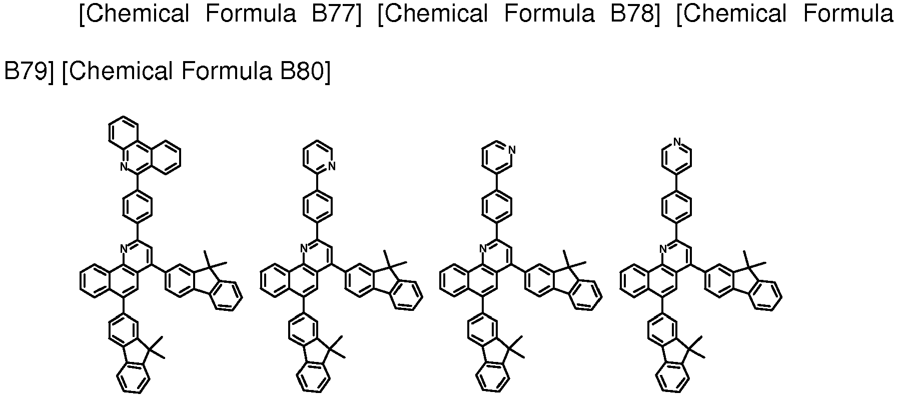

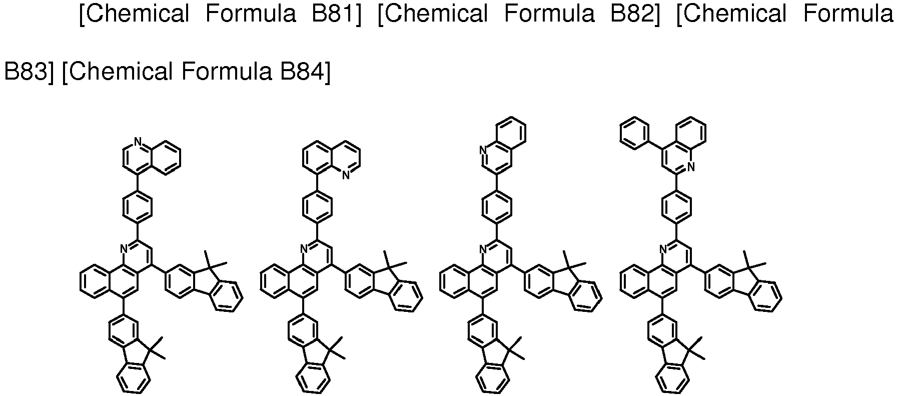

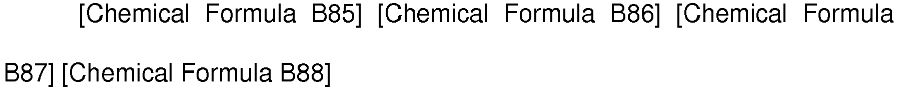

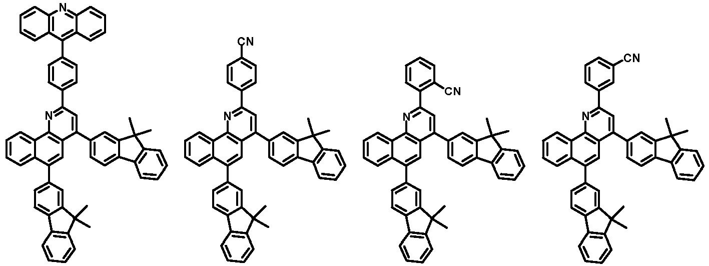

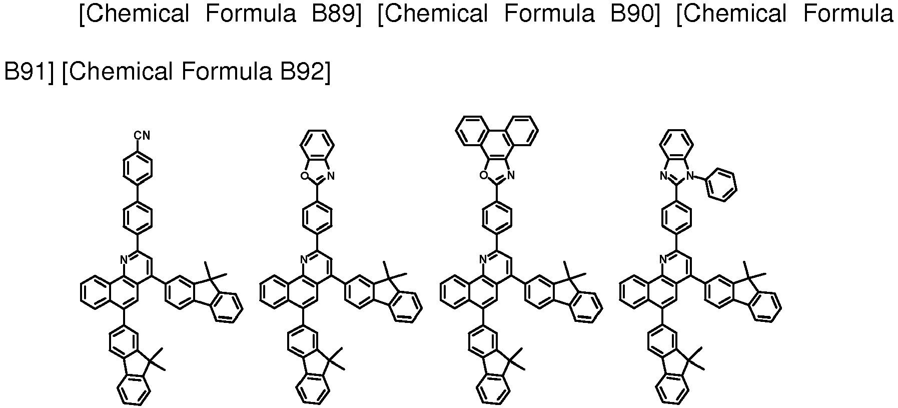

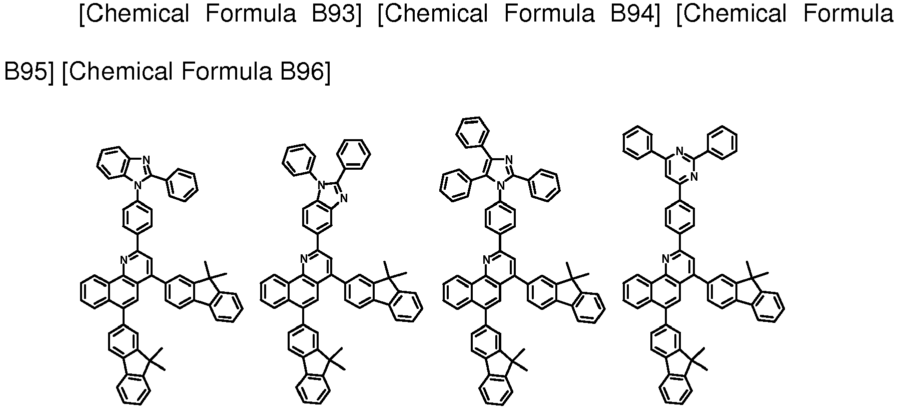

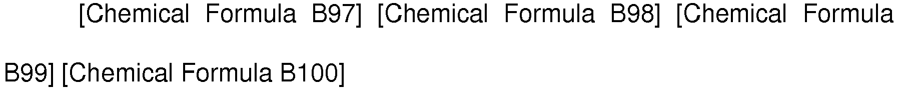

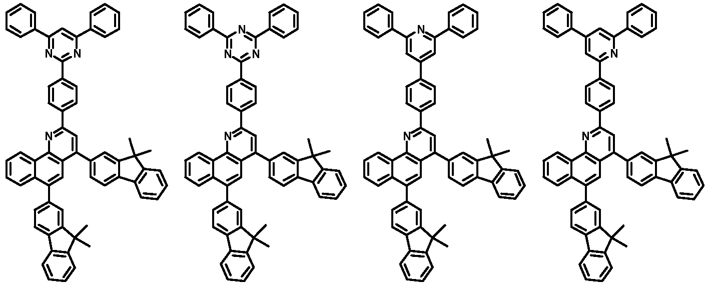

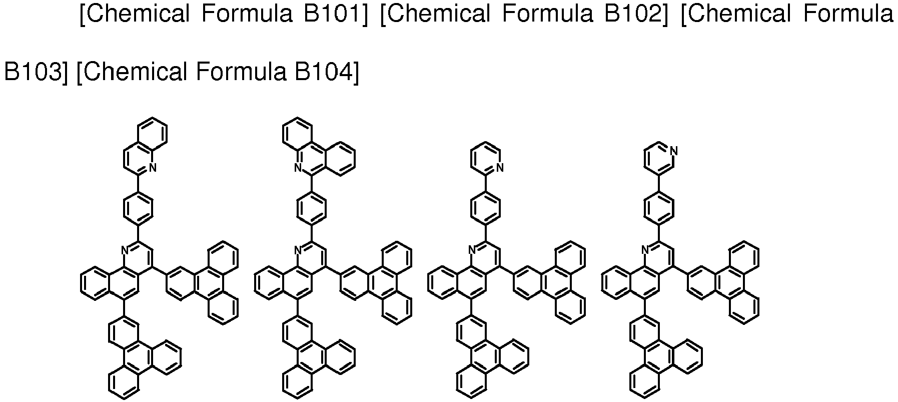

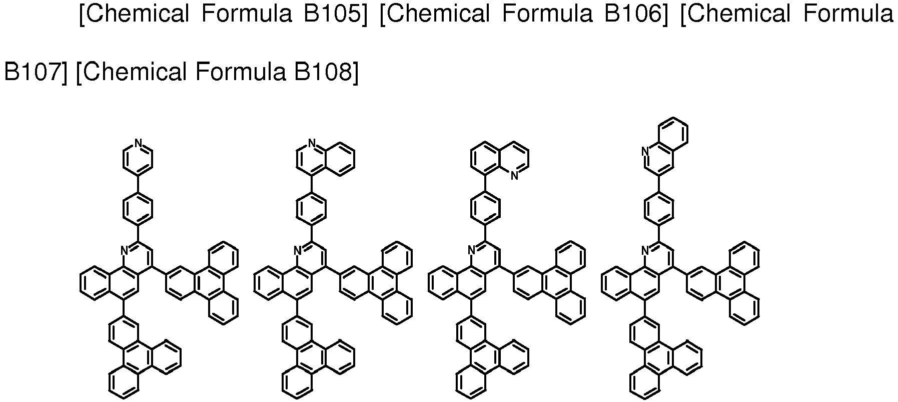

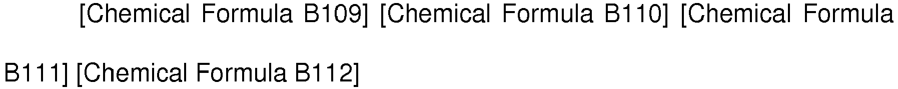

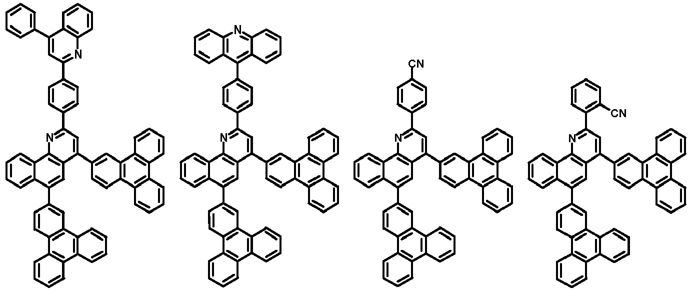

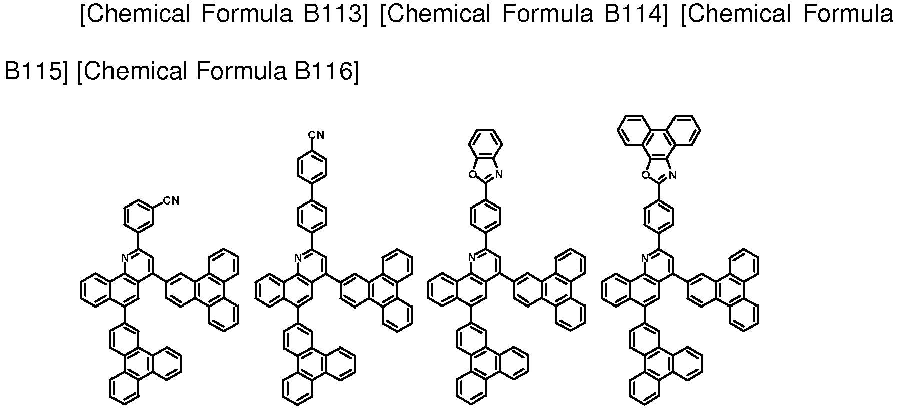

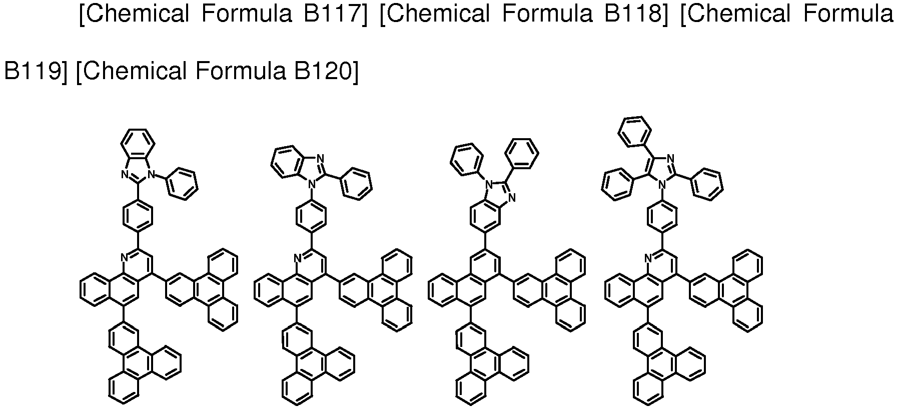

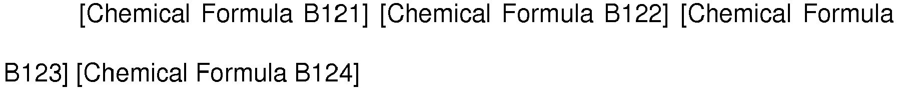

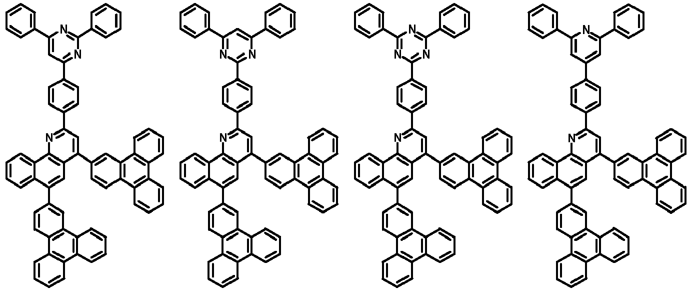

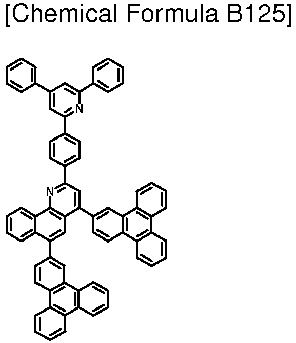

The compound for an organic optoelectronic device may be represented by one of the following Chemical Formulae C1 to C127.

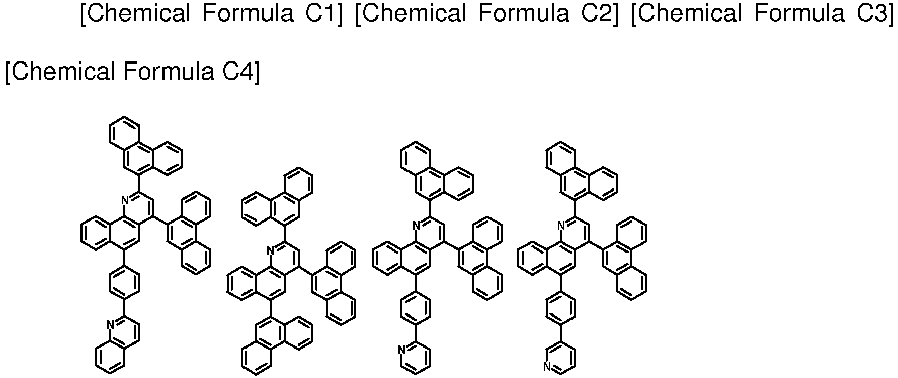

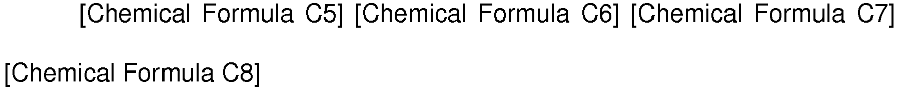

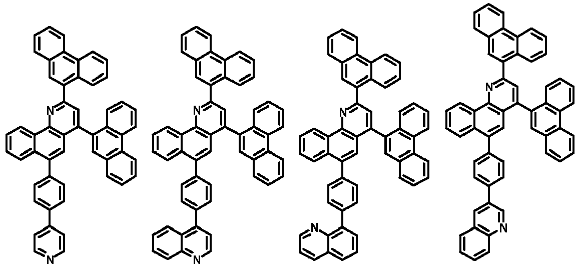

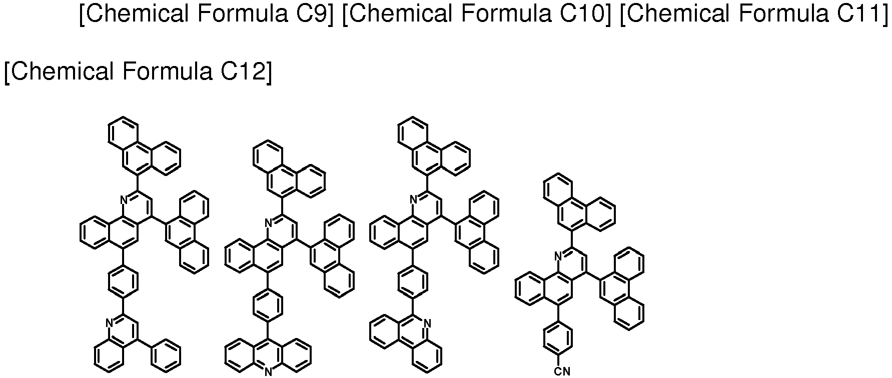

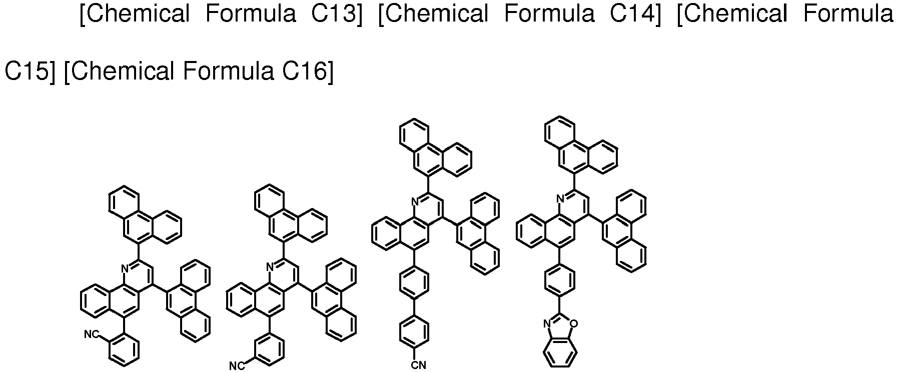

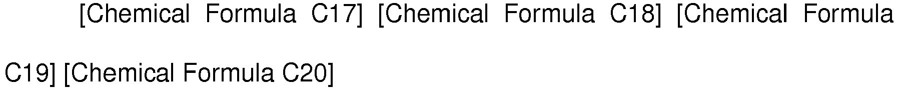

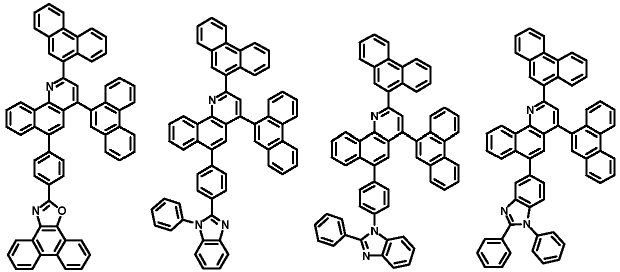

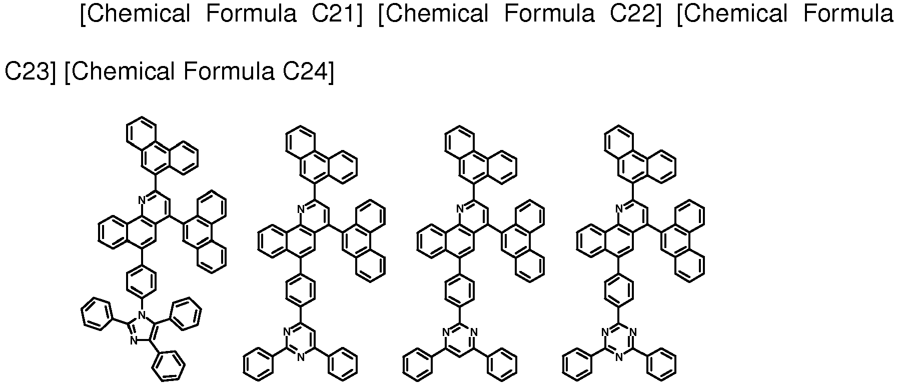

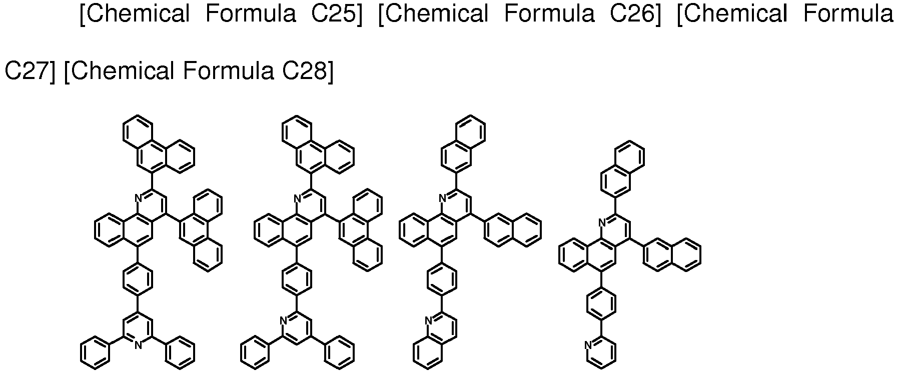

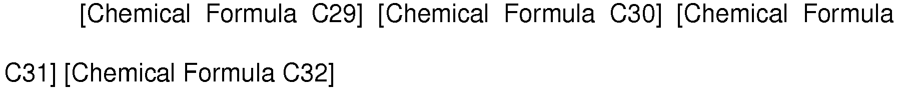

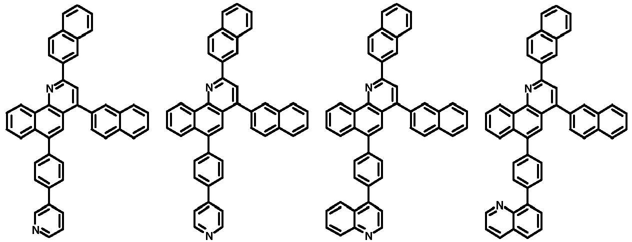

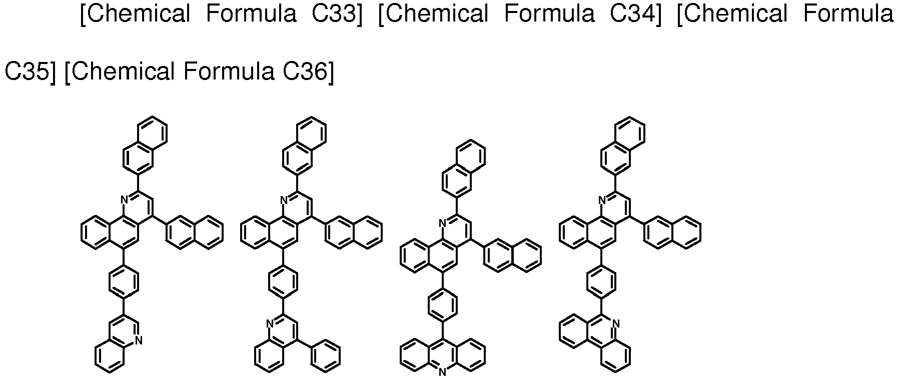

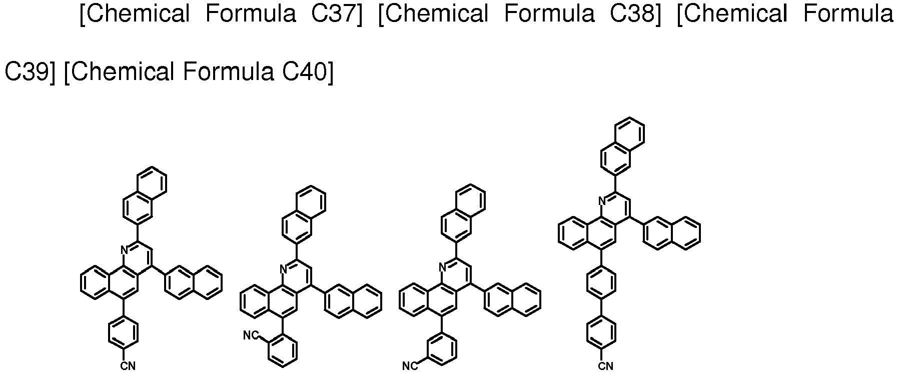

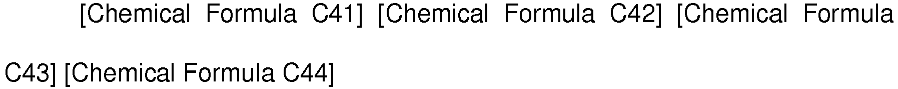

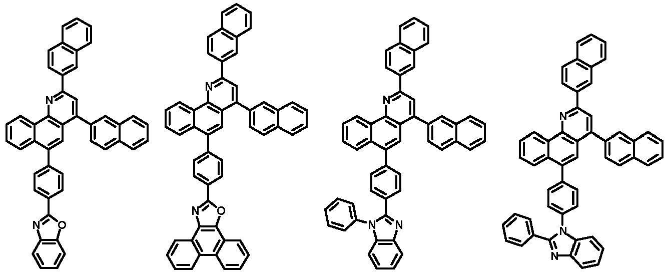

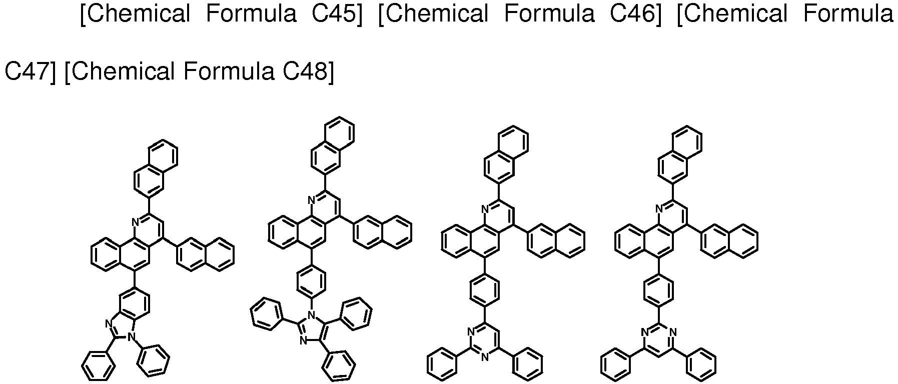

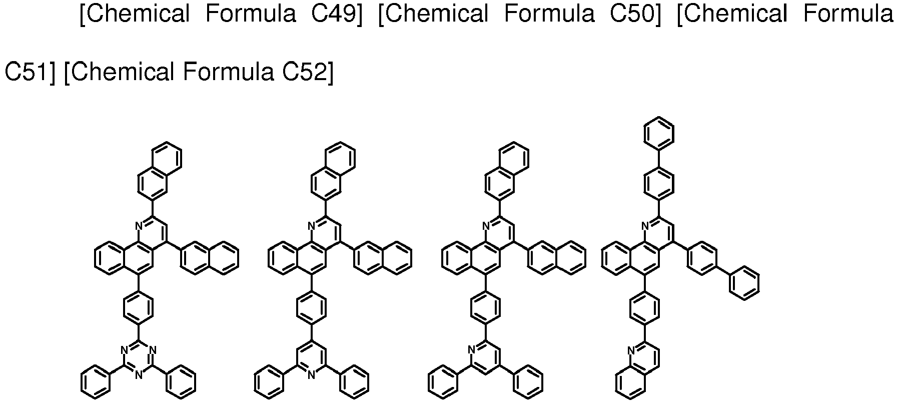

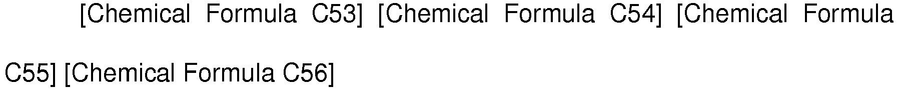

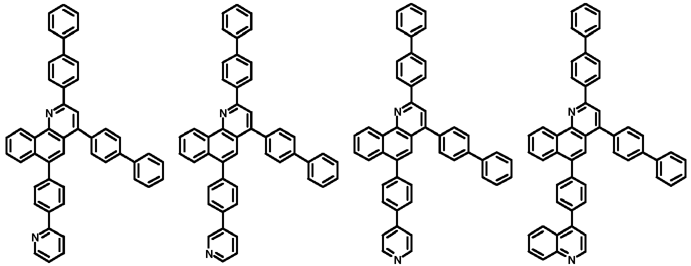

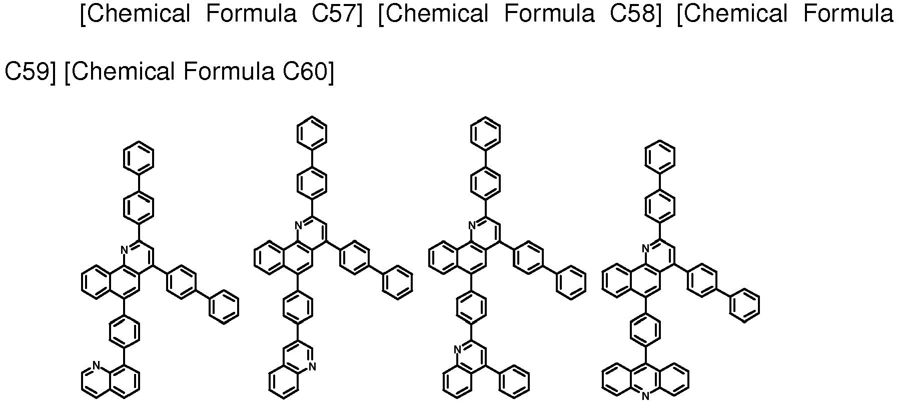

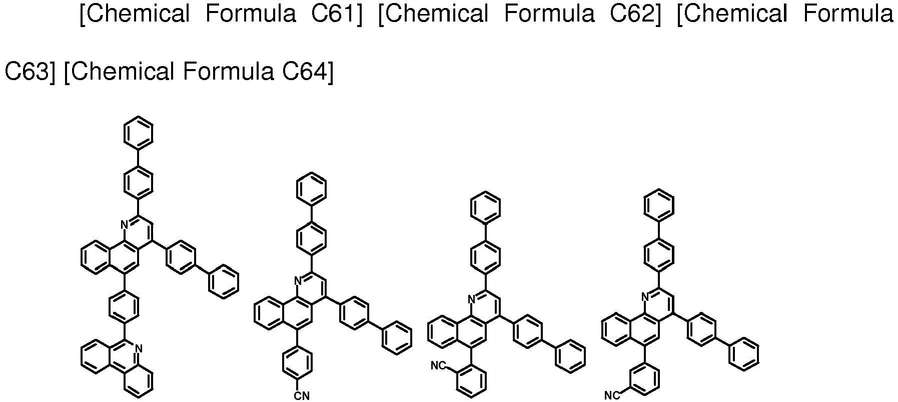

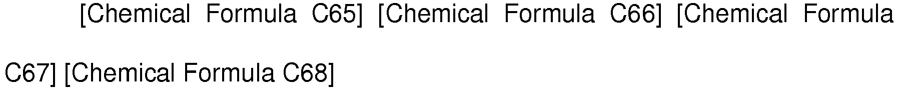

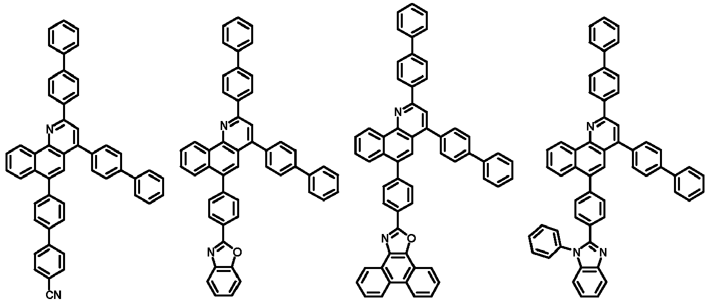

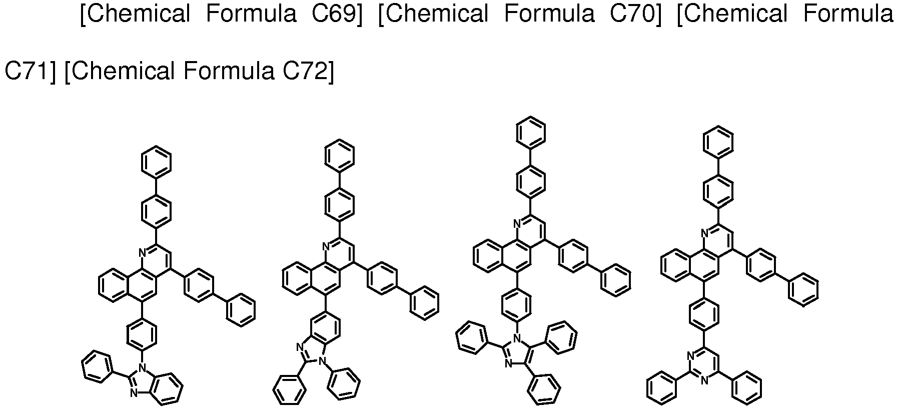

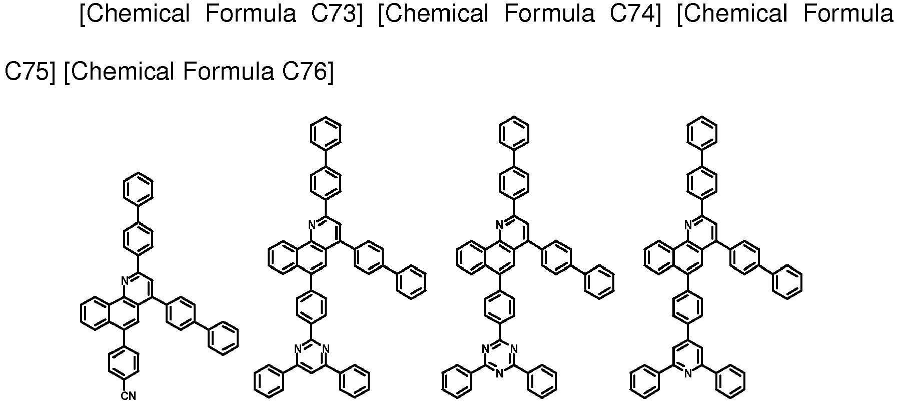

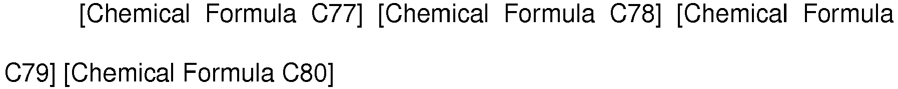

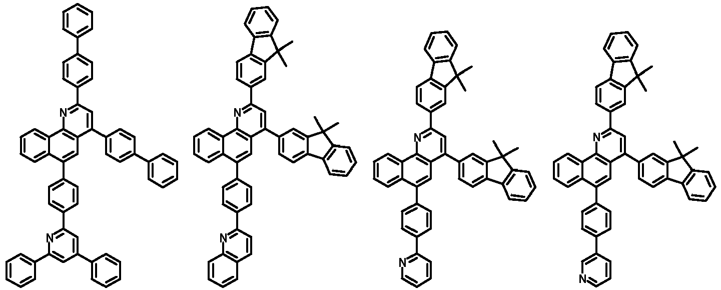

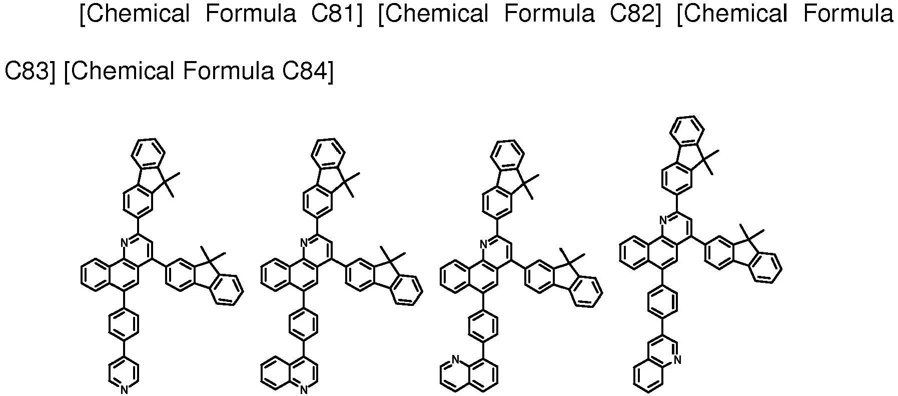

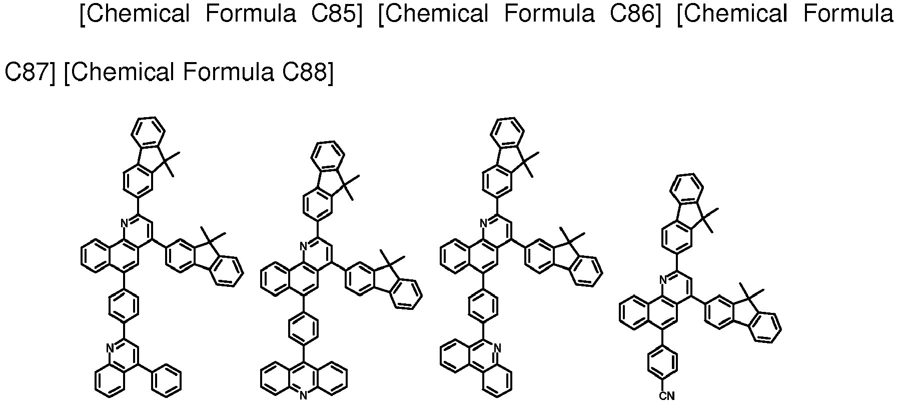

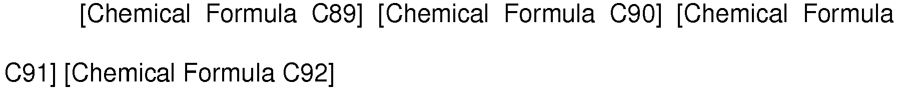

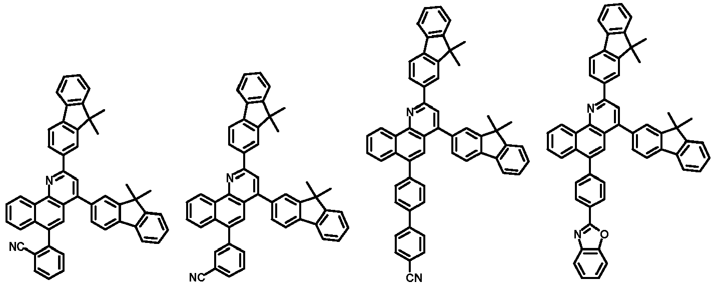

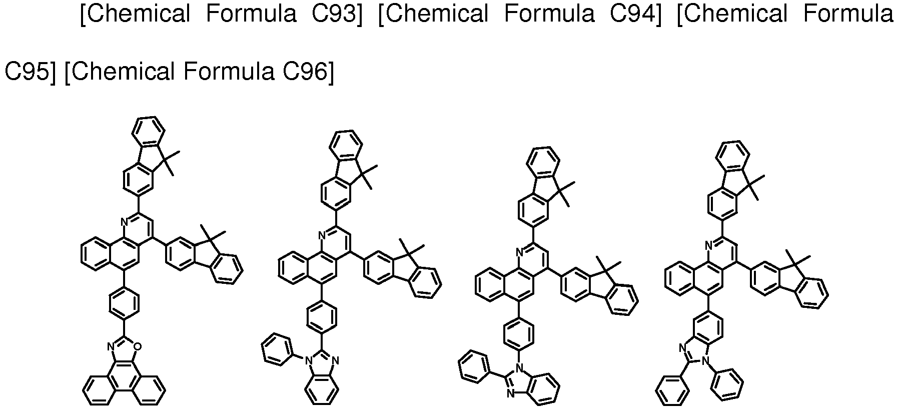

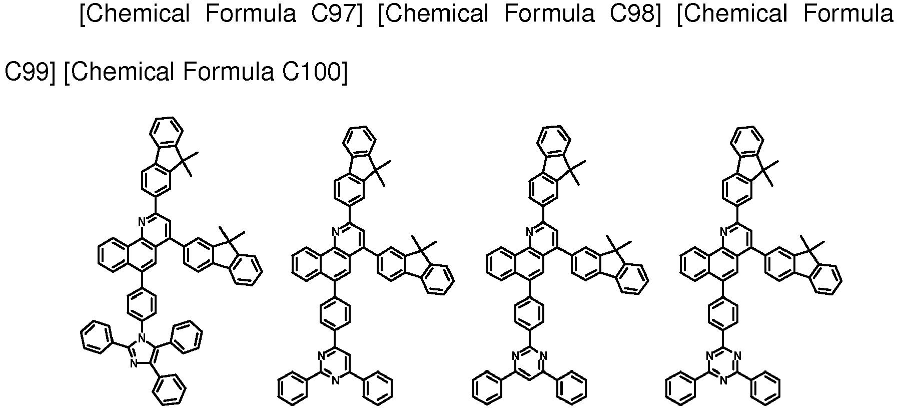

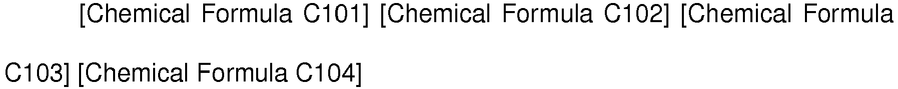

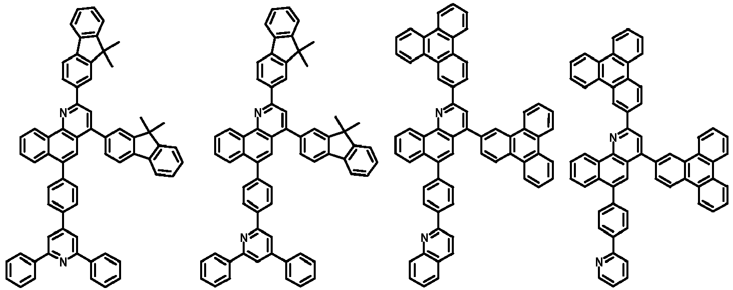

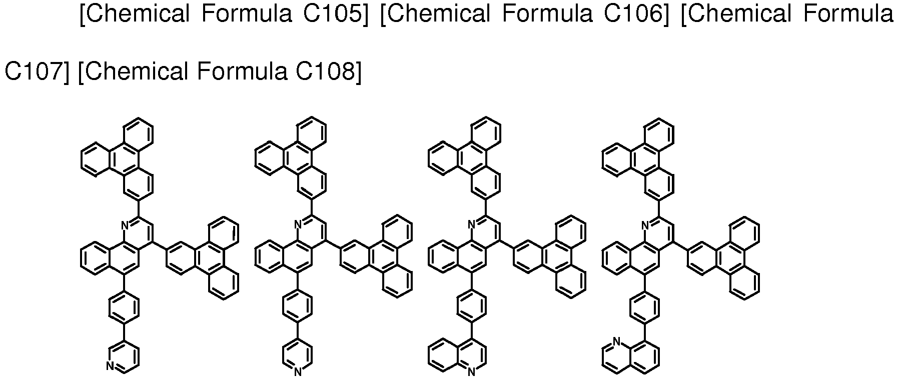

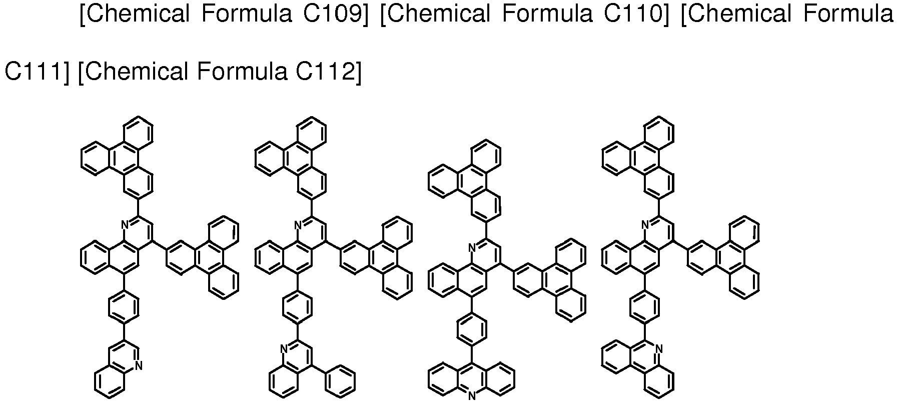

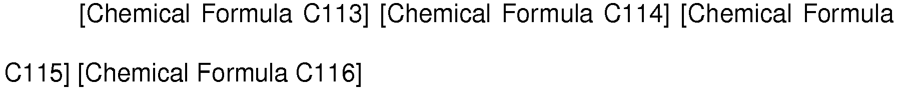

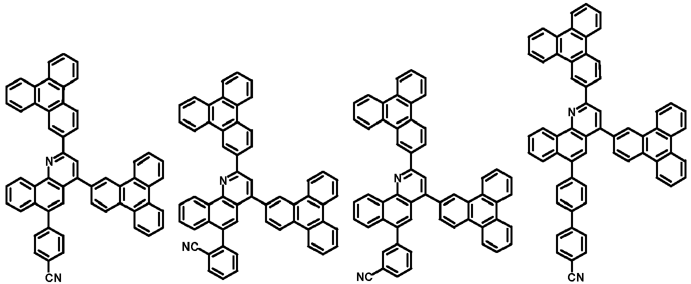

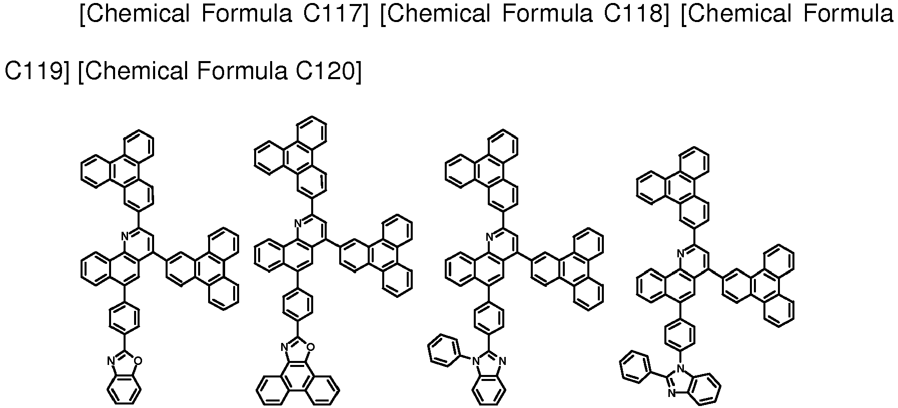

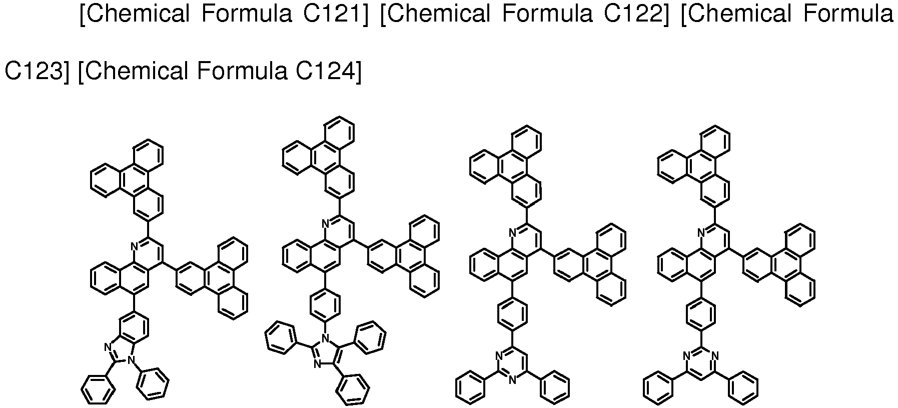

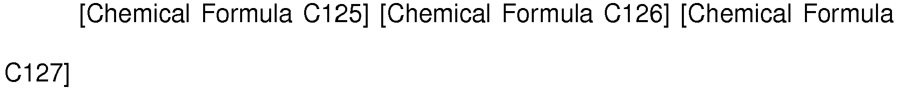

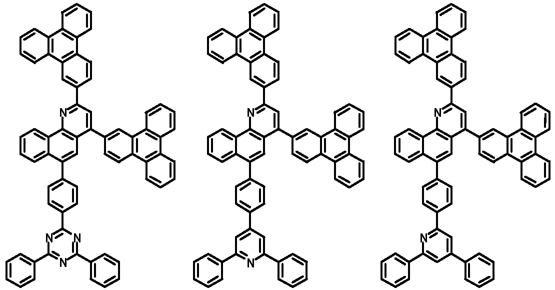

The organic optoelectronic device may be selected from the group consisting of an organic photoelectric device, an organic light emitting diode, an organic solar cell, an organic transistor, an organic photo conductor drum, and an organic memory device.

According to another aspect of the present invention, an organic light emitting diode including an anode, a cathode, and at least one or more organic thin layer between the anode and the cathode is provided. At least one of the organic thin layer includes the compound for an organic optoelectronic deviceof the present invention described above.

The organic thin layer may be selected from the group consisting of an emission layer, a hole transport layer (HTL), a hole injection layer (HIL), an electron transport layer (ETL), an electron injection layer (EIL), a hole blocking layer, and a combination thereof.

The compound for an organic optoelectronic device may be included in an electron transport layer (ETL) or an electron injection layer (EIL).

The compound for an organic optoelectronic device may be included in an emission layer.

The compound for an organic optoelectronic device may be used as a phosphorescent or fluorescent host material in an emission layer.

The compound for an organic optoelectronic device may be used as a fluorescent blue dopant material in an emission layer.

According to another aspect of the present invention, a display device including the organic light emitting diode is provided.

An organic optoelectronic device having excellent electrochemical and thermal stability and life-span characteristics, and high luminous efficiency at a low driving voltage may be provided.

### BRIEF DESCRIPTION OF THE DRAWINGS

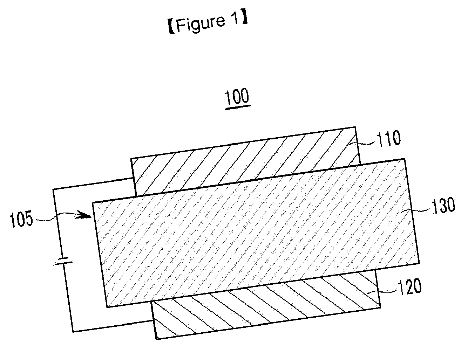

FIGS. 1 to 5 are cross-sectional views showing organic optoelectronic devices according to various embodiments of the present invention including compound for an organic optoelectronic device according to one embodiment of the present invention.

### DETAILED DESCRIPTION

Exemplary embodiments of the present invention will hereinafter be described in detail. However, these embodiments are only exemplary, and the present invention is not limited thereto but rather is defined by the scope of the appended claims.

As used herein, when specific definition is not otherwise provided, the term "substituted" refers to one substituted with a C1 to C30 alkyl group, a C1 to C10 alkylsilyl group, a C3 to C30 cycloalkyl group, a C6 to C30 aryl group, a C1 to C10 alkoxy group, a fluoro group, a C1 to C10 trifluoro alkyl group such as trifluoromethyl group, or a cyano group.

As used herein, when specific definition is not otherwise provided, the term "hetero" refers to one including 1 to 3 hetero atoms selected from the group consisting of N, O, S, and P, and remaining carbons in one functional group.

As used herein, when a definition is not otherwise provided, the term "combination thereof" refers to at least two substituents bound to each other by a linker, or at least two substituents condensed to each other.

As used herein, when a definition is not otherwise provided, the term "alkyl" refers to an aliphatic hydrocarbon group. The alkyl group may be a "saturated alkyl group" that does not include a double bond or a triple bond.

The alkyl group may be an "unsaturated alkyl group" including at least one alkenyl group or alkynyl group. Regardless of being saturated or unsaturated, the alkyl may be branched, linear, or cyclic.

The alkyl group may be a C1 to C20 alkyl group. The alkyl group may be a C1 to C10 medium-sized alkyl group. The alkyl group may be a C1 to C6 lower alkyl group.

For example, a C1 to C4 alkyl group may have 1 to 4 carbon atoms and may be selected from the group consisting of methyl, ethyl, propyl, iso-propyl, n-butyl, iso-butyl, sec-butyl, and t-butyl.

Examples of an alkyl group may be selected from the group consisting of a methyl group, an ethyl group, a propyl group, an isopropyl group, a butyl group, an isobutyl group, a t-butyl group, a pentyl group, a hexyl group, an ethenyl group, a propenyl group, a butenyl group, a cyclopropyl group, a cyclobutyl group, a cyclopentyl group, a cyclohexyl group, and the like.

The term "aromatic group" may refer a functional group including a cyclic structure where all elements have p-orbitals which form conjugation. Specific examples include an aryl group and a heteroaryl group.

The term "aryl" may refer to a monocyclic or fused ring-containing polycyclic (i.e., rings sharing adjacent pairs of carbon atoms) groups.

The "heteroaryl group" may refer to one including 1 to 3 heteroatoms selected from the group consisting of N, 0, S, and P in an aryl group, and remaining carbons.

The term "spiro structure" refers to a cyclic structure having a contact point of one carbon. Further, the spiro structure may be used as a compound including the spiro structure or a substituent including the spiro structure.

According to one embodiment of the present invention, a compound for an organic optoelectronic device represented by the following Chemical Formula 2 is provided.

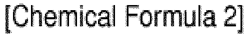

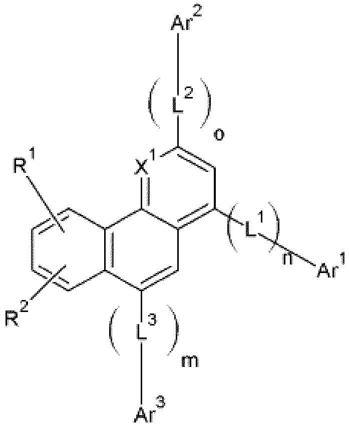

In Chemical Formula 2, X¹ is -N-, R¹ and R² are the same or different and independently hydrogen, deuterium, a substituted or unsubstituted C1 to C20 alkyl group, a substituted or unsubstituted C6 to C30 aryl group, a substituted or unsubstituted C3 to C30 heteroaryl group, or a combination thereof, Ar¹ to Ar³ are the same or different and independently substituted or unsubstituted C6 to C30 aryl group; or a substituted or unsubstituted C3 to C30 heteroaryl group, L¹ to L³ are the same or different and independently a single bond, a substituted or unsubstituted C2 to C6 alkenyl group, a substituted or unsubstituted C2 to C6 alkynyl group, a substituted or unsubstituted C6 to C30 arylene group, a substituted or unsubstituted C3 to C30 heteroarylene group, or a combination thereof, and n, m or o are the same or different and independently 0 or 1.

In the case of the structure represented by Chemical Formula 2, it has merits of being easily synthesized, preventing being easily crystallized as an asymmetric structure in a device, and having high thermal stability due to a bulk core.

The fused ring core may include at least one of nitrogen atom, for example, may include two or one of nitrogen atom.

That is to say, X¹ may be N.

The compound may control the characteristics of entire compound by introducing appropriate substituent to a core structure having excellent electron characteristics.

The compound for an organic optoelectronic device may have various energy band gaps by introducing the various other substituents to the core part and the substituent substituted in the core part. Thereby, the compound may be applied to an electron injection layer (EIL) and transport layer or also applied to an emission layer.

By applying the compound having an appropriate energy level according to the substituent of the compound to the organic photoelectric device, the electron transport property is enforced to provide excellent effects on the efficiency and the driving voltage, and electrochemical and thermal stability are so excellent to improve the life-span characteristics during driving an organic photoelectric device.

The electron characteristic refers to characteristic that electron formed in the negative electrode is easily injected into the emission layer and transported in the emission layer due to conductive characteristics according to LUMO level.

In an opposite concept, the hole characteristic refers to characteristic that hole formed in the positive electrode is easily injected into the emission layer and transported in the emission layer due to conductive characteristic according to HOMO level.

Ar¹ to Ar³ are the same or different and may be independently a substituted or unsubstituted C6 to C30 aryl group; or a substituted or unsubstituted C3 to C30 heteroaryl group.

The asymmetric structure having bipolar characteristics may be provided by appropriately combining the substituent, and the asymmetric structure having bipolar characteristics may improve the electron transport property, so it is anticipated to improve the luminous efficiency and performance of device using the same.

The substituted or unsubstituted C3 to C30 heteroaryl group may be, for example a substituted or unsubstituted imidazolyl group, a substituted or unsubstituted triazolyl group, a substituted or unsubstituted tetrazolyl group, a substituted or unsubstituted carbazolyl group, a substituted or unsubstituted oxadiazolyl group, a substituted or unsubstituted oxatriazolyl group, a substituted or unsubstituted thiatriazolyl group, a substituted or unsubstituted benzimidazolyl group, a substituted or unsubstituted benzotriazolyl group, a substituted or unsubstituted pyridinyl group, a substituted or unsubstituted pyrimidinyl group, a substituted or unsubstituted triazinyl group, a substituted or unsubstituted pyrazinyl group, a substituted or unsubstituted pyridazinyl group, a substituted or unsubstituted purinyl group, a substituted or unsubstituted quinolinyl group, a substituted or unsubstituted isoquinolinyl group, a substituted or unsubstituted phthalazinyl group, a substituted or unsubstituted naphpyridinyl group, a substituted or unsubstituted quinoxalinyl group, a substituted or unsubstituted quinazolinyl group, a substituted or unsubstituted acridinyl group, a substituted or unsubstituted phenanthrolinyl group, a substituted or unsubstituted phenazinyl group, and the like. A combination thereof may be also included.

The substituted or unsubstituted C6 to C30 aryl group may be, for example a substituted or unsubstituted phenyl group, a substituted or unsubstituted naphthyl group, a substituted or unsubstituted triperylenyl group, a substituted or unsubstituted fluorenyl group, a substituted or unsubstituted spirofluorenyl group, a substituted or unsubstituted biphenyl group, a substituted or unsubstituted terphenyl group, a substituted or unsubstituted pyrenyl group, a substituted or unsubstituted perylenyl group, a substituted or unsubstituted phenanthrenyl group, a substituted or unsubstituted anthracenyl group, and the like. A combination thereof may be also included.

At least one of Ar¹ or Ar² may be a substituted or unsubstituted C3 to C30 heteroaryl group. In this case, the electron characteristic of entire compound may be further enforced by the electron characteristics of general heteroaryl groups.

Ar¹ may be a substituted or unsubstituted C3 to C30 heteroaryl group, and Ar² and Ar³ may be a substituted or unsubstituted C6 to C30 aryl group. When having the structure, the molecule polarity may be controlled to improve electron injection and transport capability.

Ar² may be a substituted or unsubstituted C3 to C30 heteroaryl group, and Ar¹ and Ar³ may be a substituted or unsubstituted C6 to C30 aryl group. By polarizing the molecular polarity when having the structure, the property of injecting and transporting electron may be improved.

By appropriately combining the substituent, the compound may have the structure of excellent thermal stability and excellent resistance to oxidation.

L¹ to L³ may be the same or different and may be, for example independently substituted or unsubstituted ethenylene, a substituted or unsubstituted ethynylene, a substituted or unsubstituted phenylene, a substituted or unsubstituted biphenylene, a substituted or unsubstituted naphthalene, a substituted or unsubstituted pyridinylene, a substituted or unsubstituted pyrimidinylene, a substituted or unsubstituted triazinylene, and the like.

Since the substituent has π-bond, the substituent increases triplet energy bandgap by controlling the total π-conjugation length of compound, so as to be very usefully applied to the emission layer of organic photoelectric device as phosphorescent host. But since n, m, or o may be independently 0, the linking groups such as L¹ to L³ may be not present.

R¹ and R² are the same or different and independently hydrogen, deuterium, a substituted or unsubstituted C1 to C20 alkyl group, a substituted or unsubstituted C6 to C30 aryl group, a substituted or unsubstituted C3 to C30 heteroaryl group, or a combination thereof.

The entire compound may have a bulk structure by controlling the substituents, so the crystallinity may be decreased. When the crystallinity of entire compound is decreased, the life-span of organic photoelectric device using the same may be prolonged.

The compound for an organic optoelectronic device may be represented by one of the following Chemical Formulae A1 to A126. However, the present invention is not limited to the following compounds.

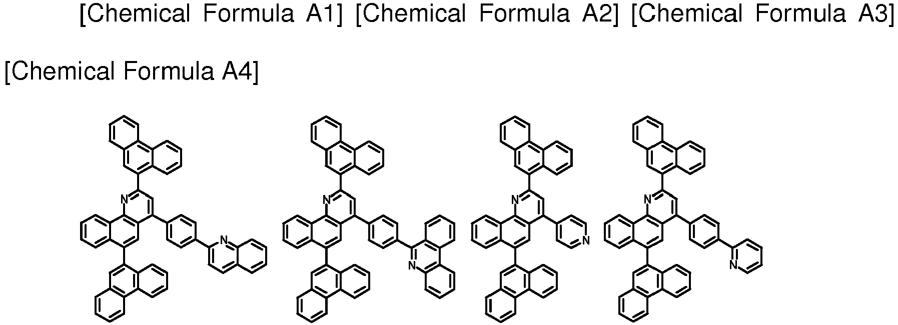

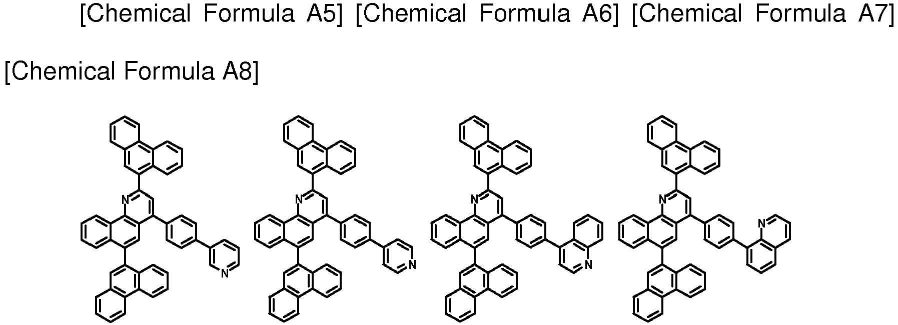

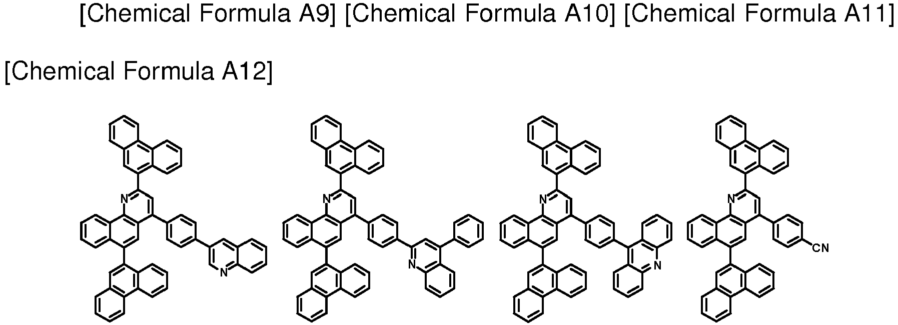

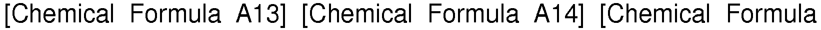

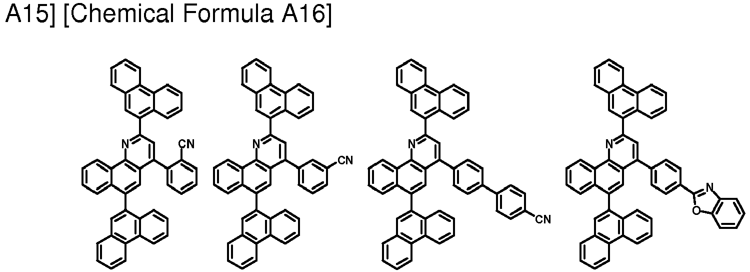

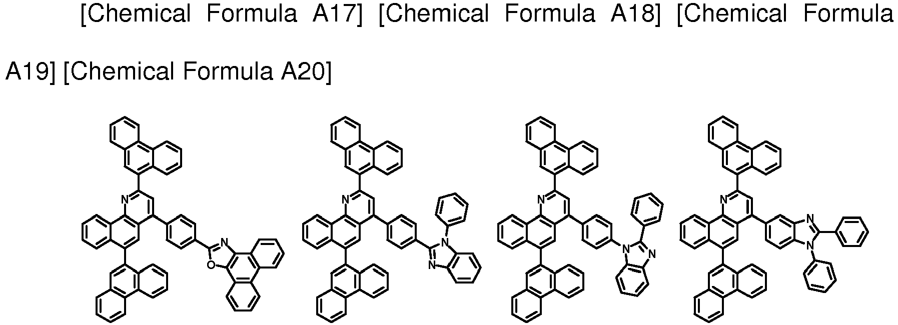

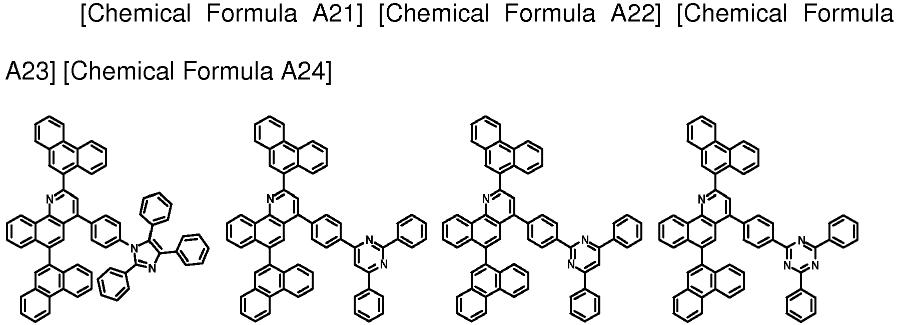

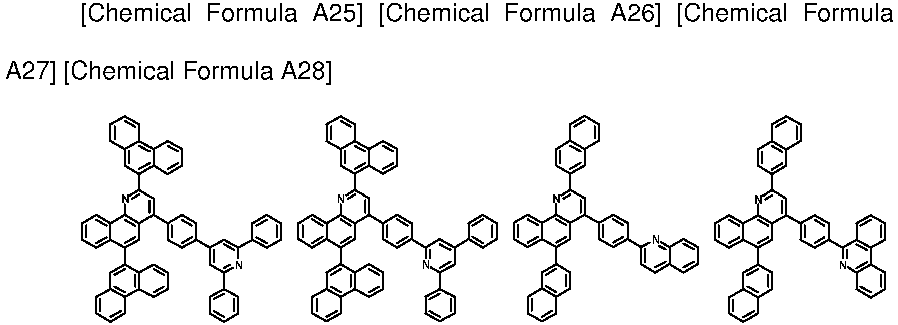

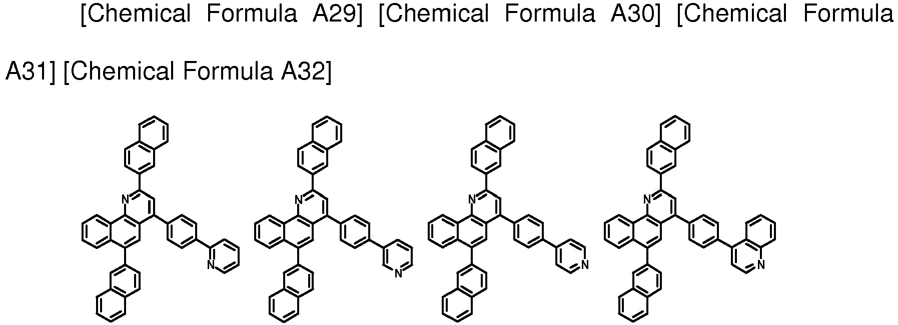

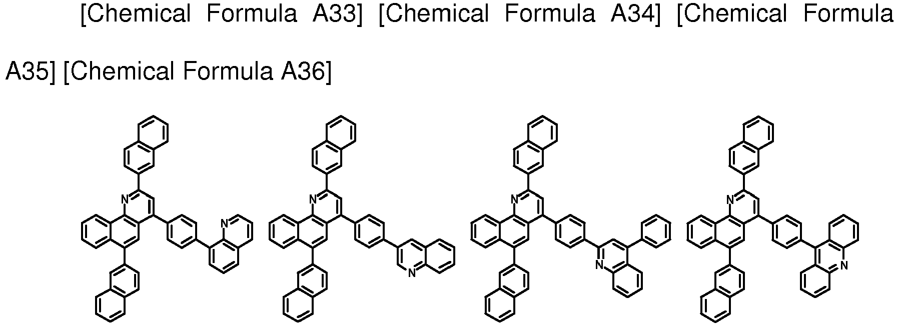

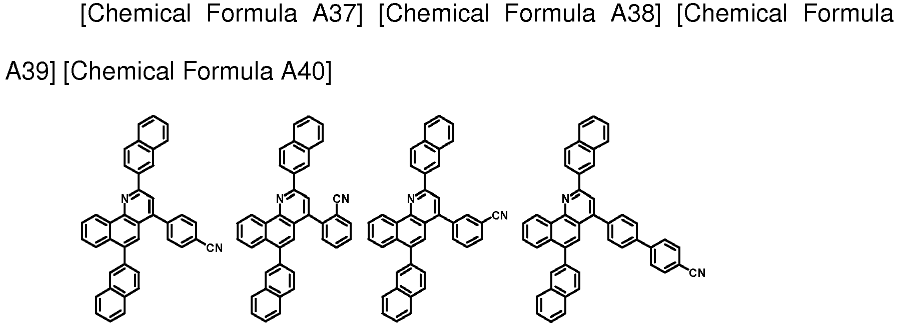

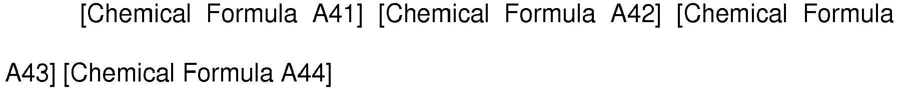

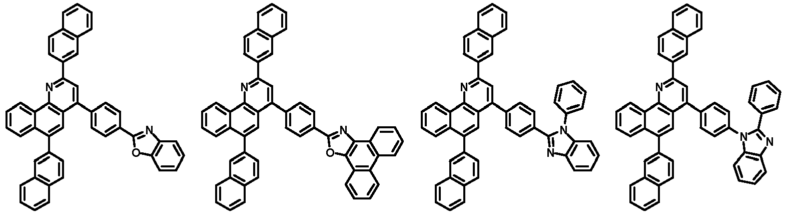

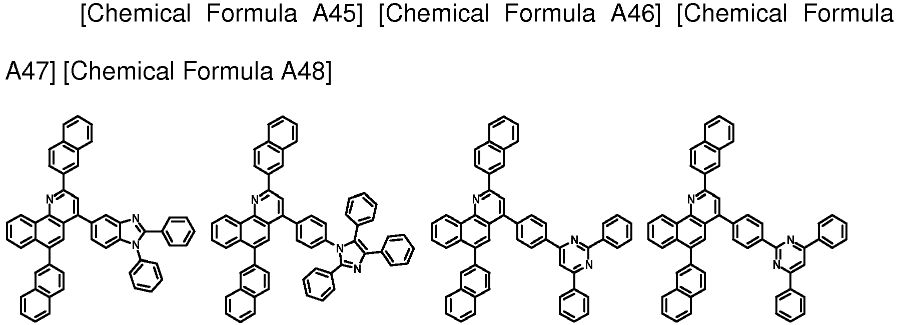

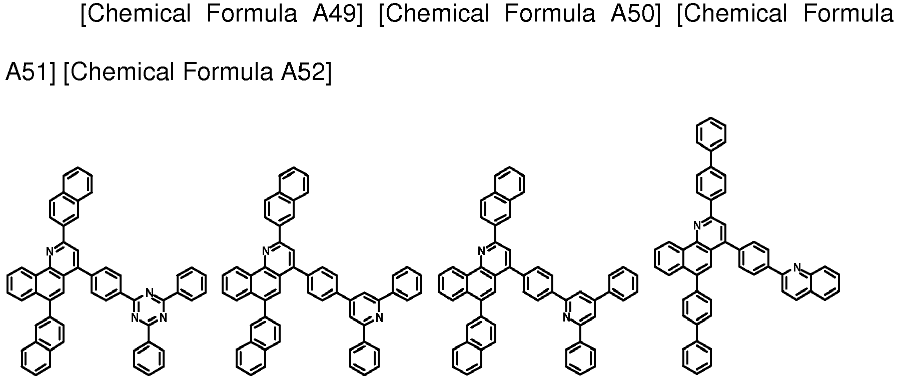

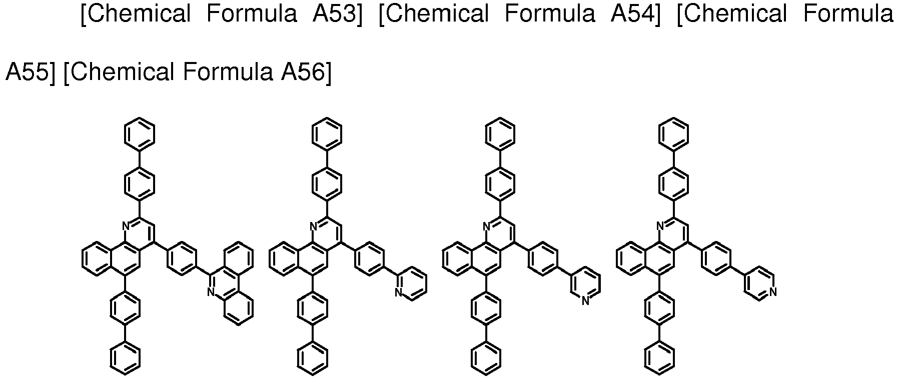

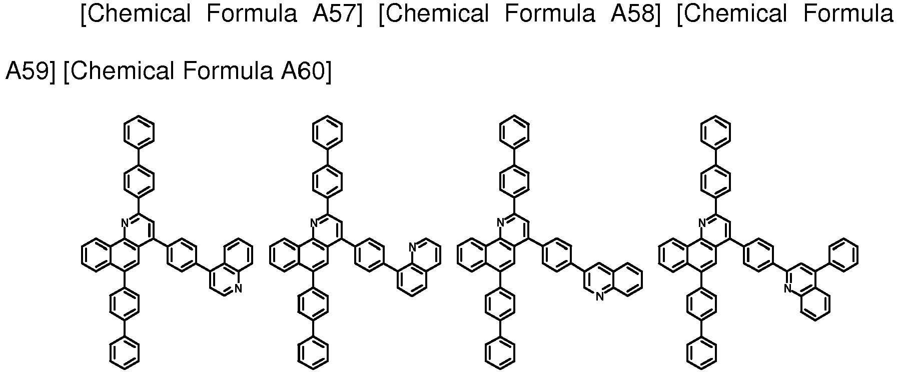

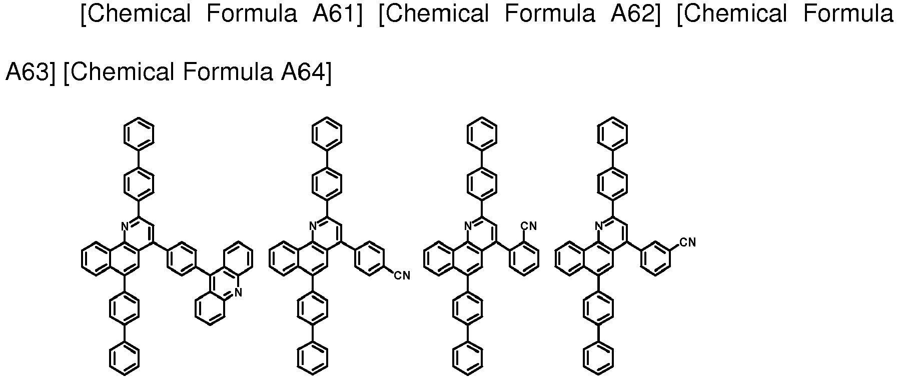

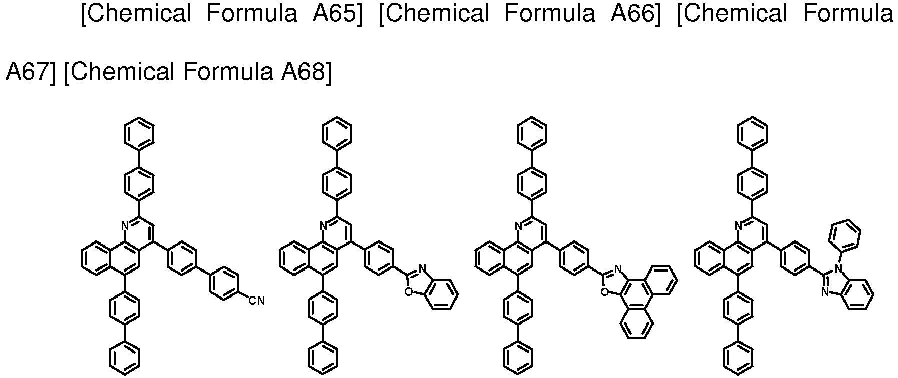

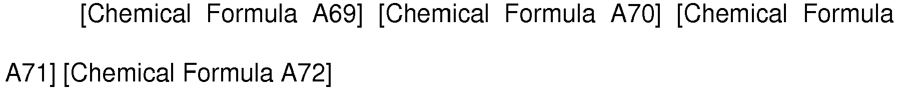

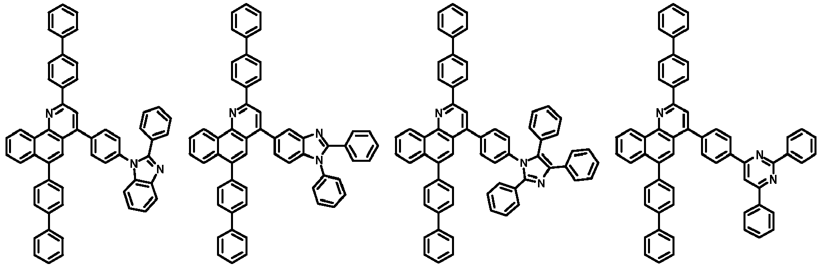

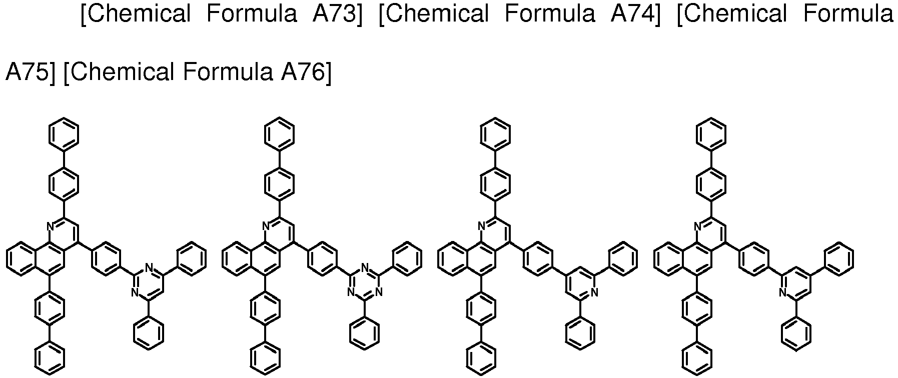

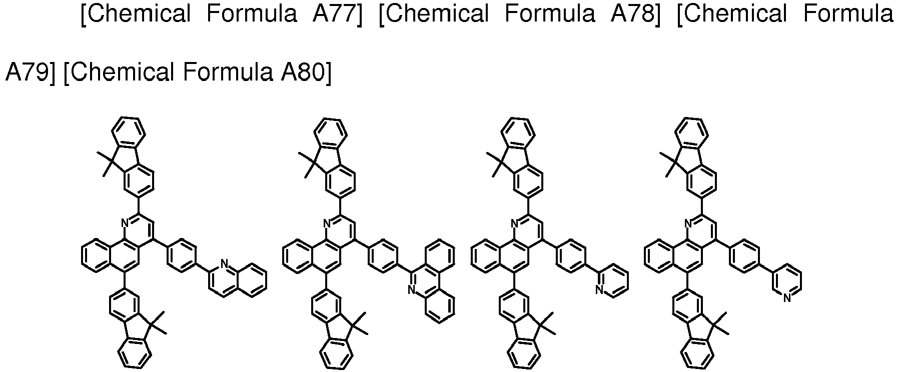

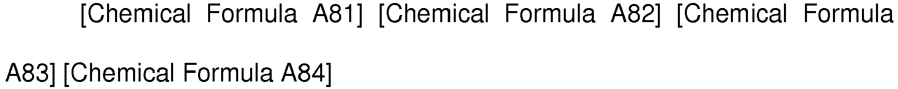

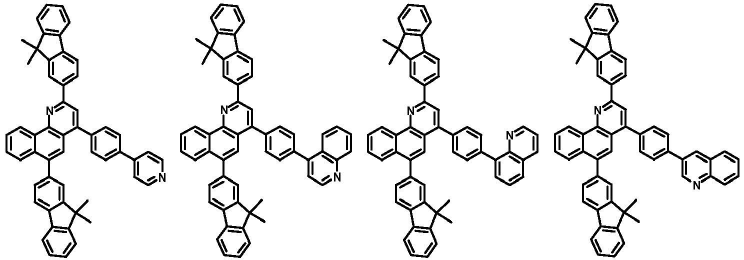

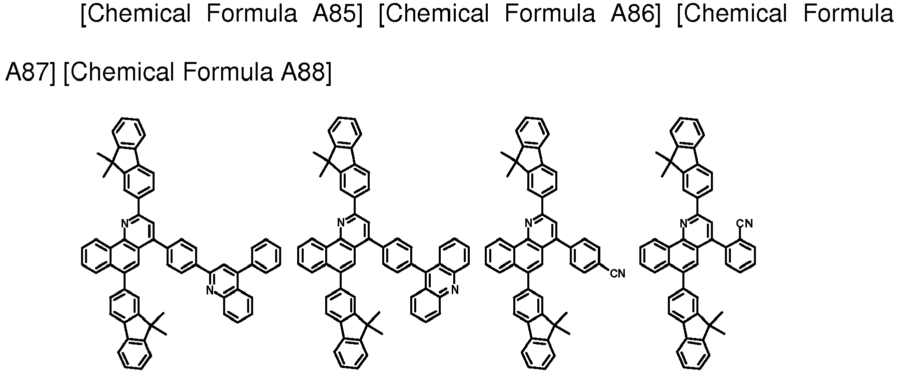

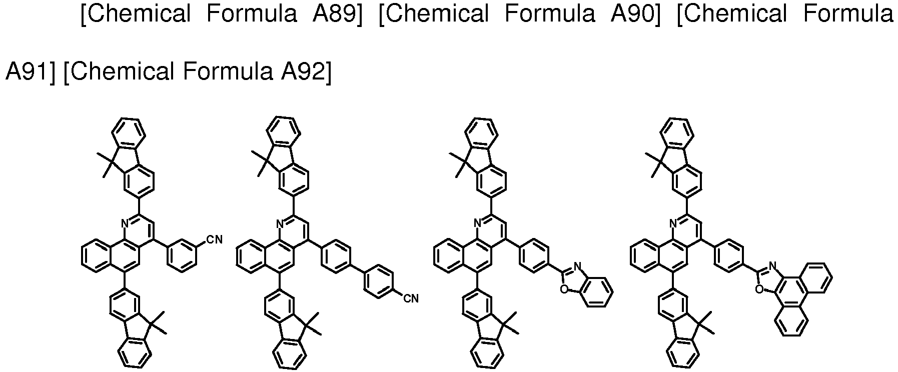

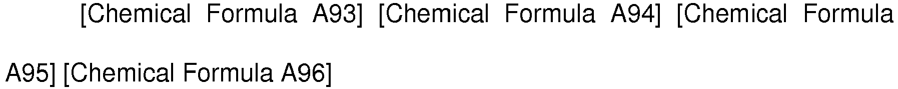

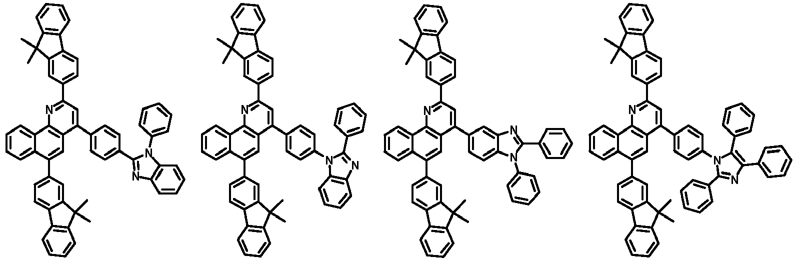

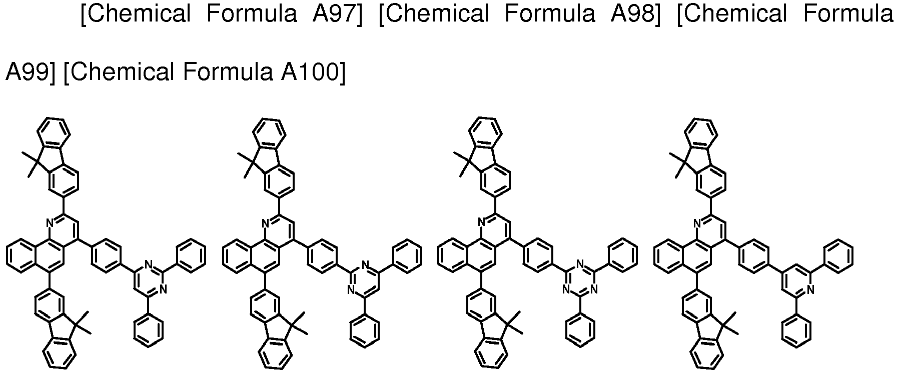

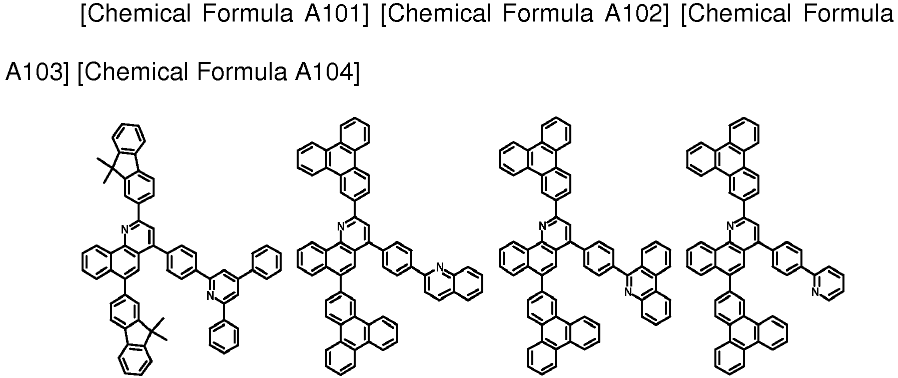

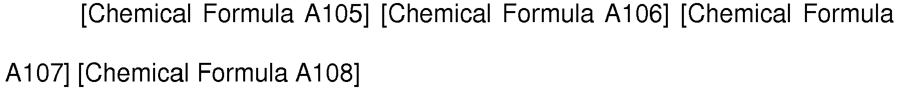

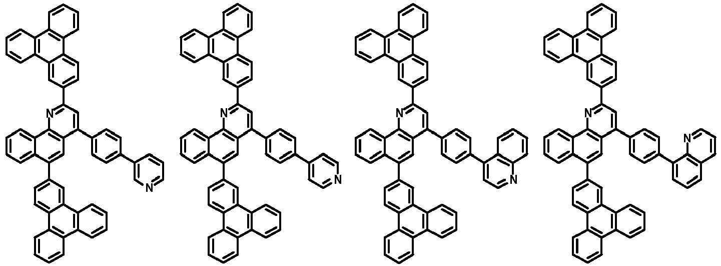

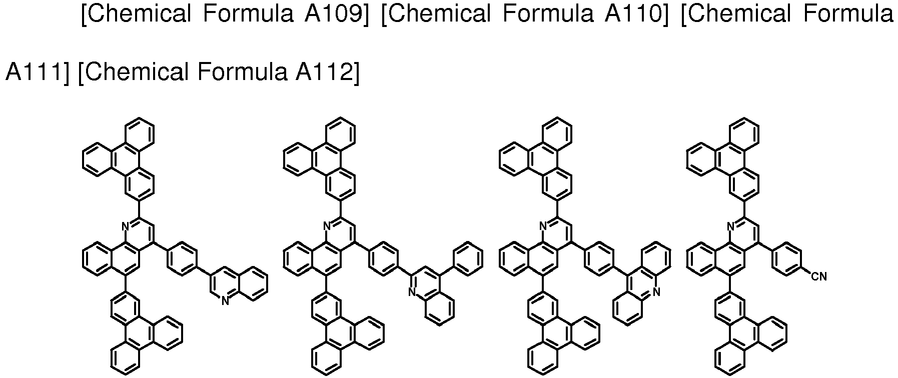

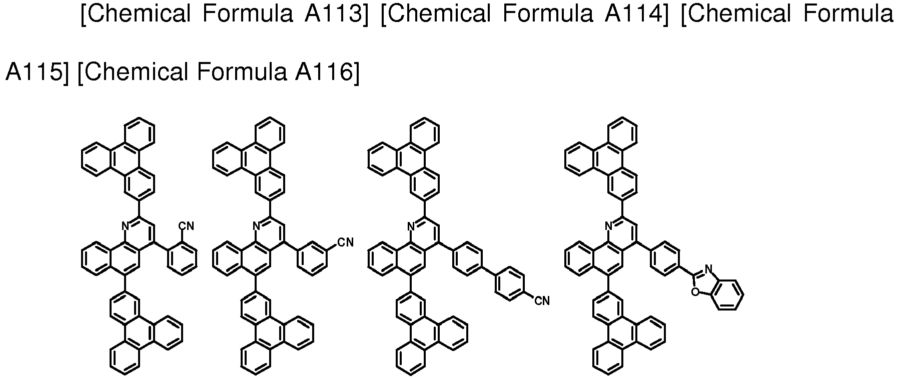

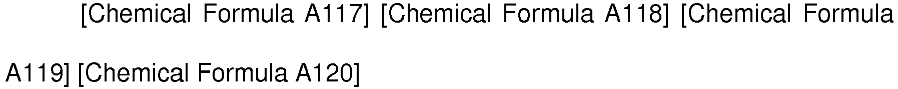

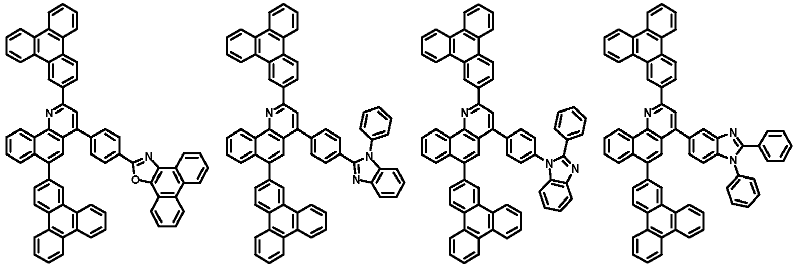

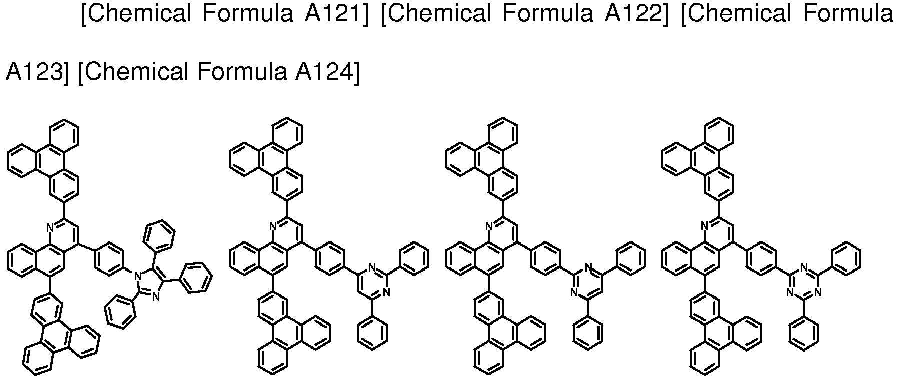

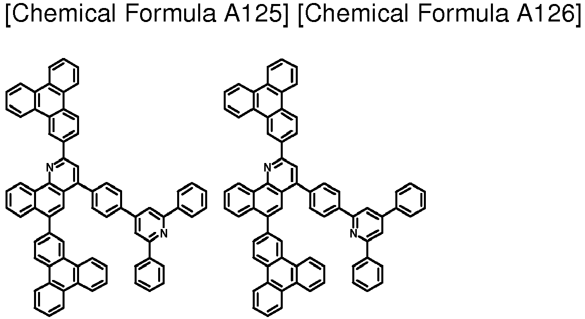

The compound for an organic optoelectronic device may be represented by one of the following Chemical Formulae B1 to B125. However, the present invention is not limited to the following compounds.

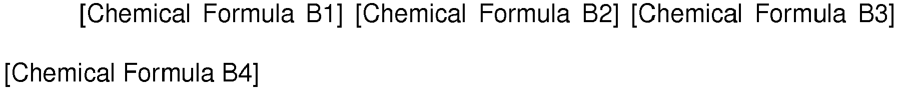

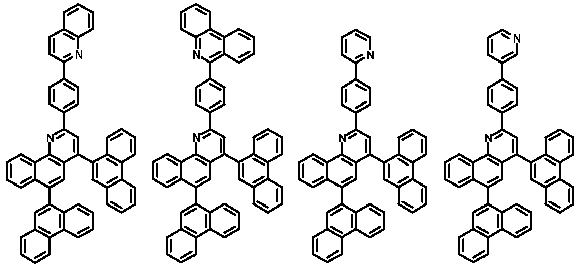

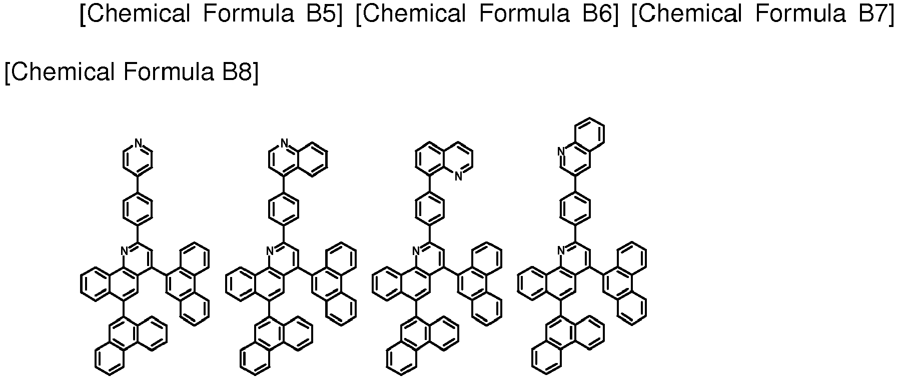

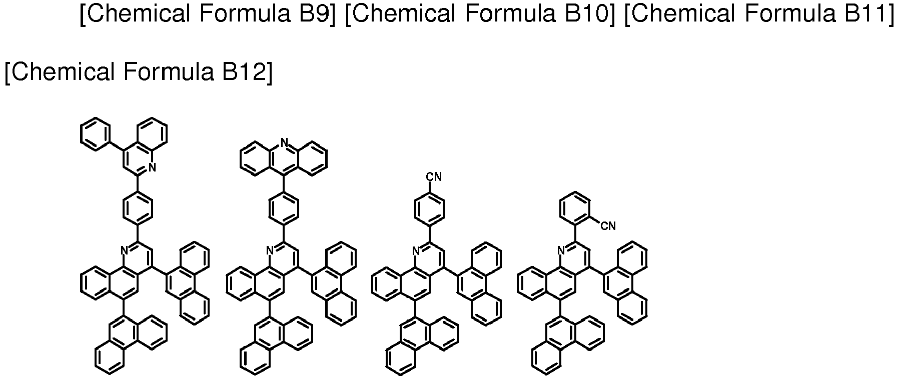

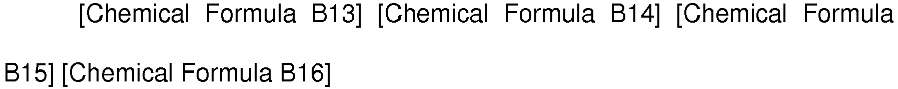

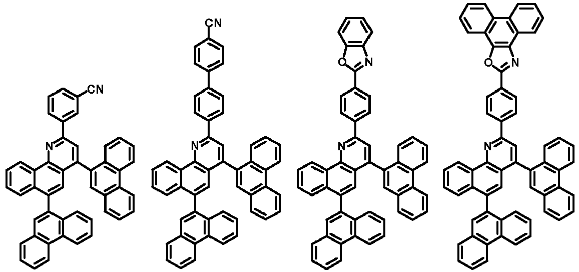

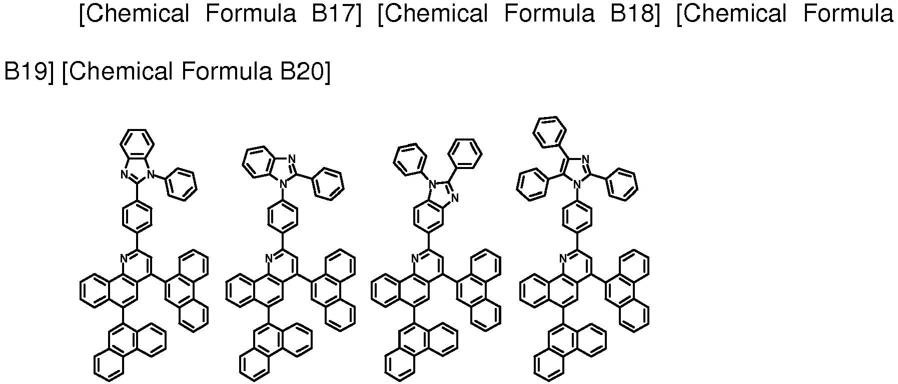

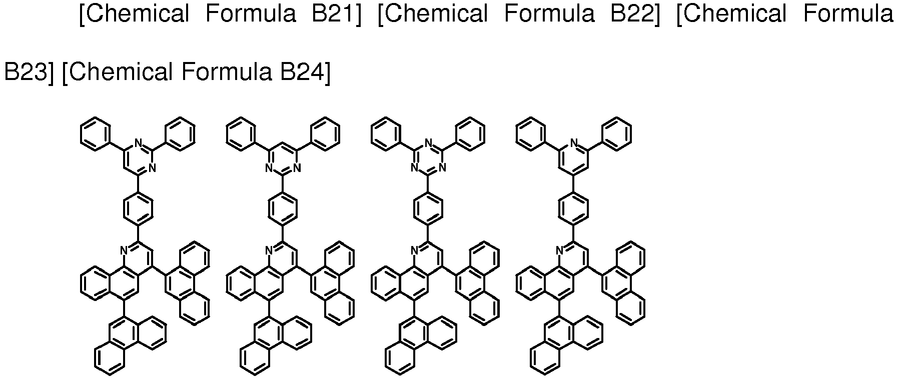

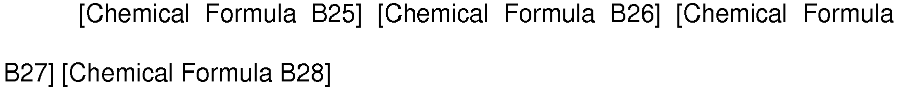

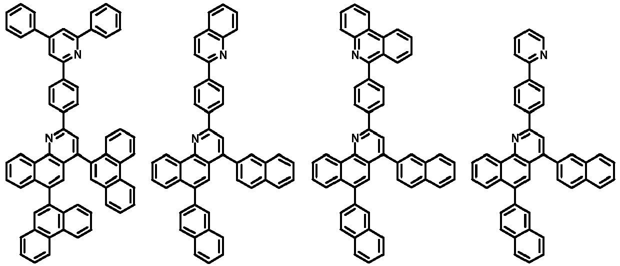

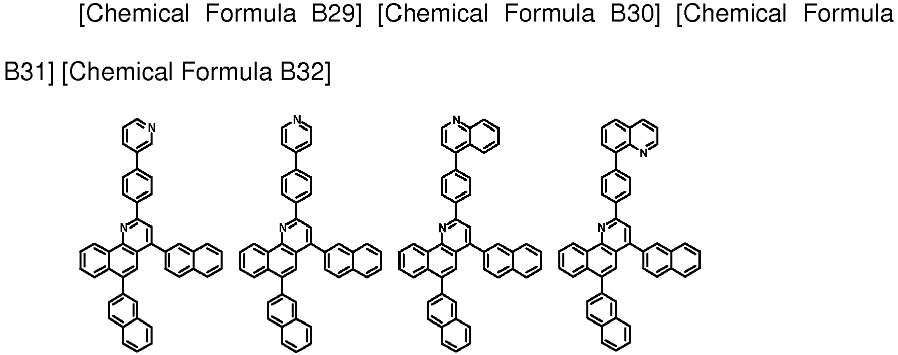

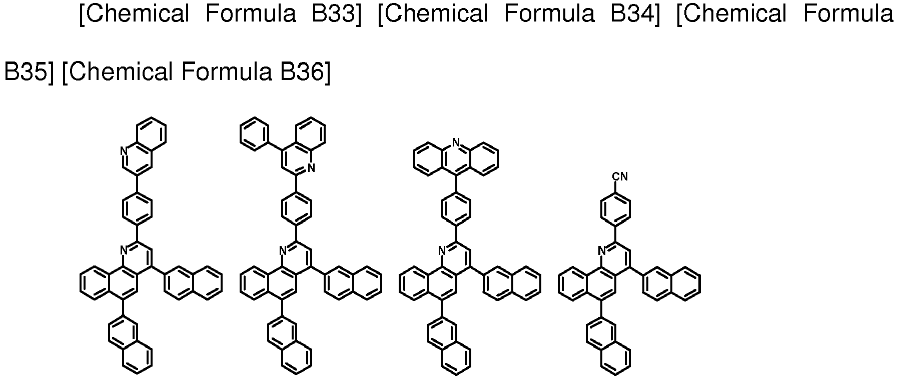

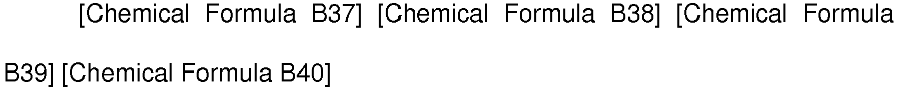

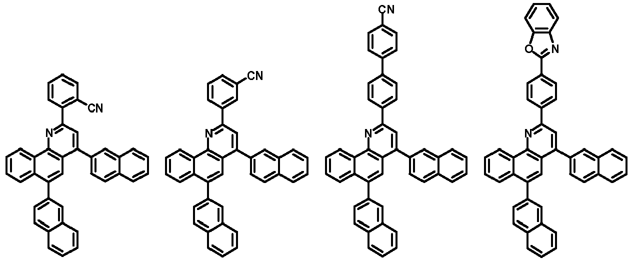

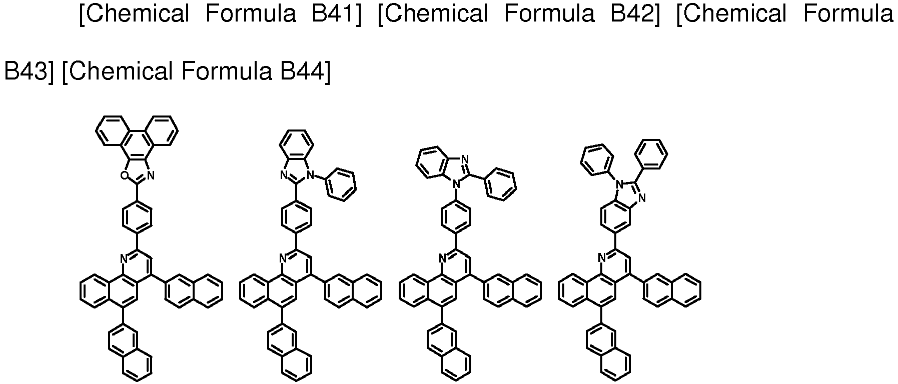

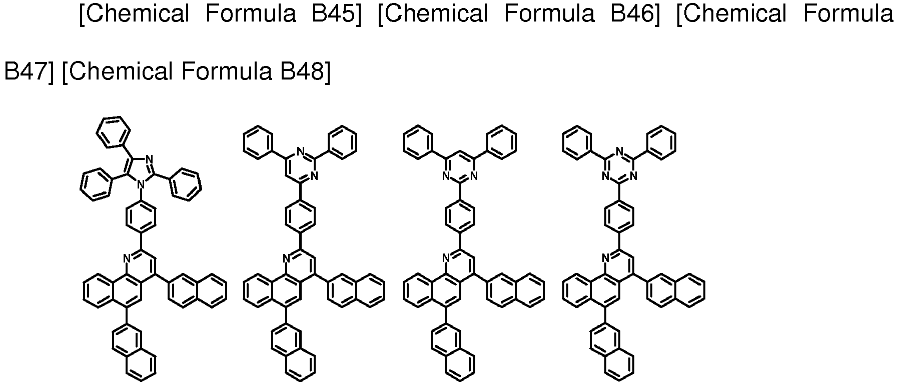

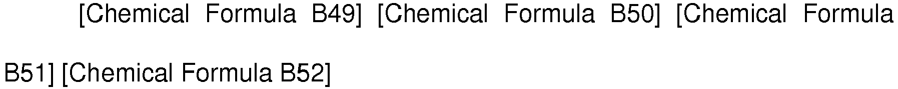

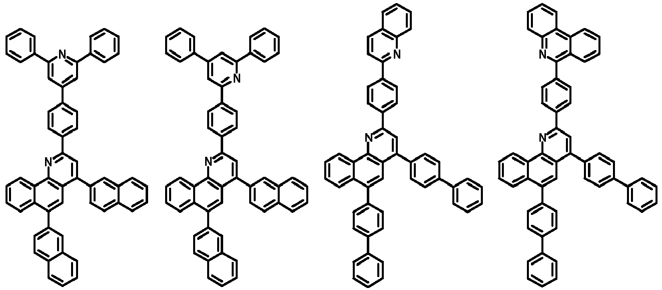

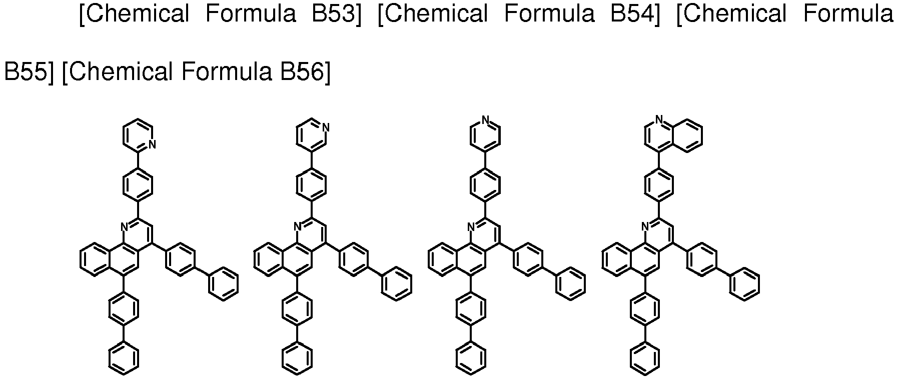

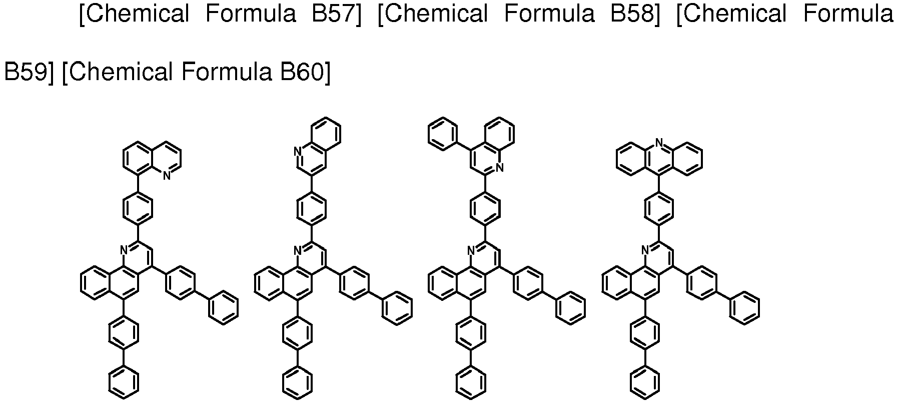

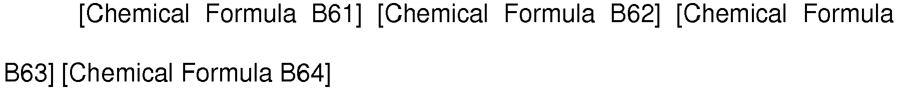

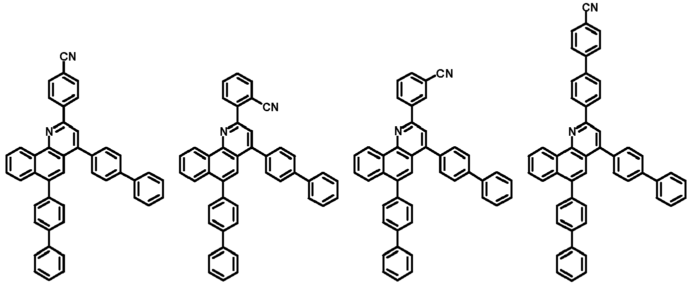

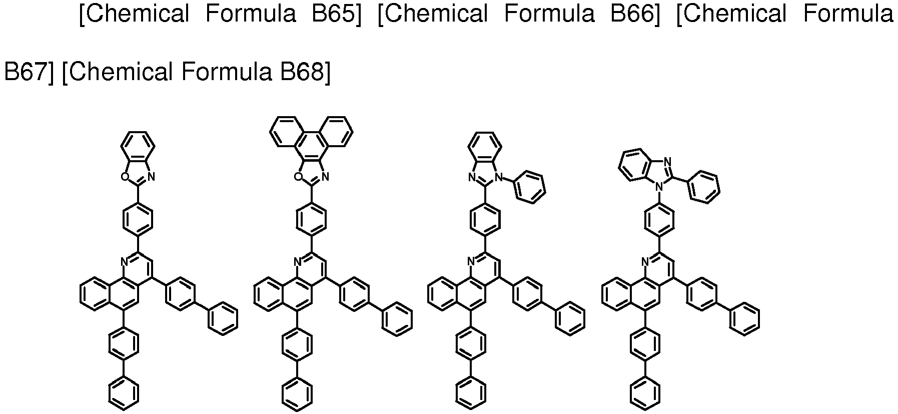

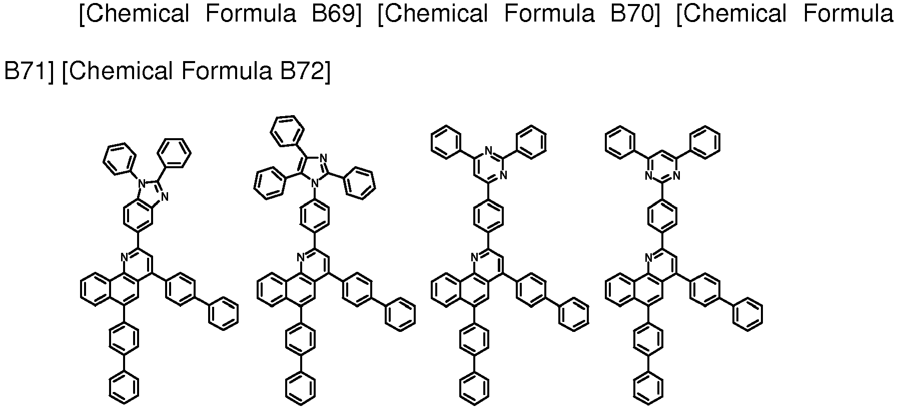

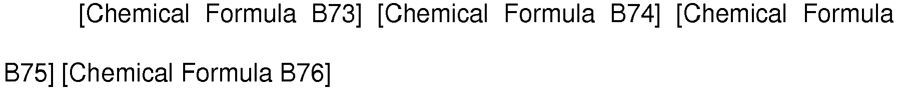

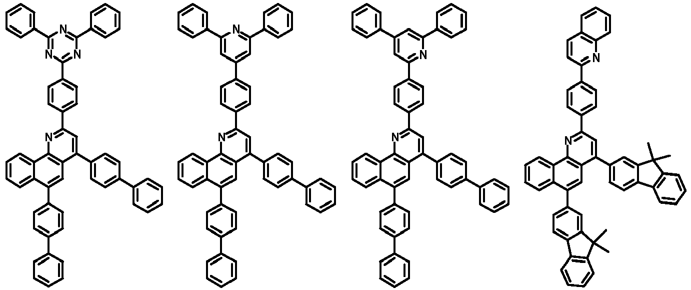

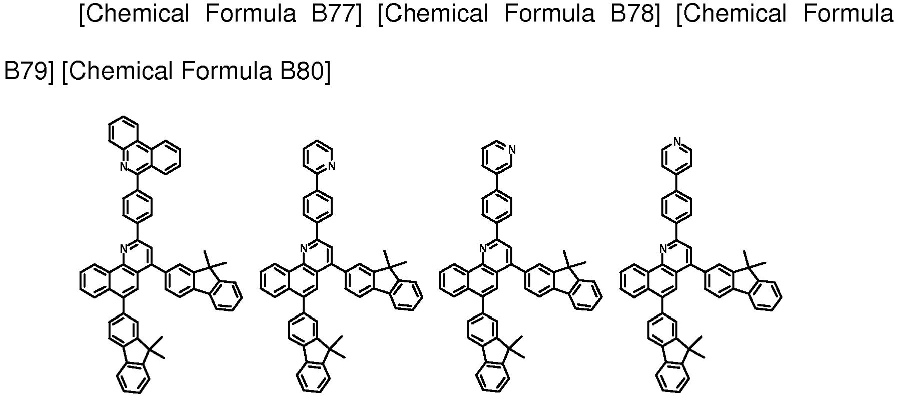

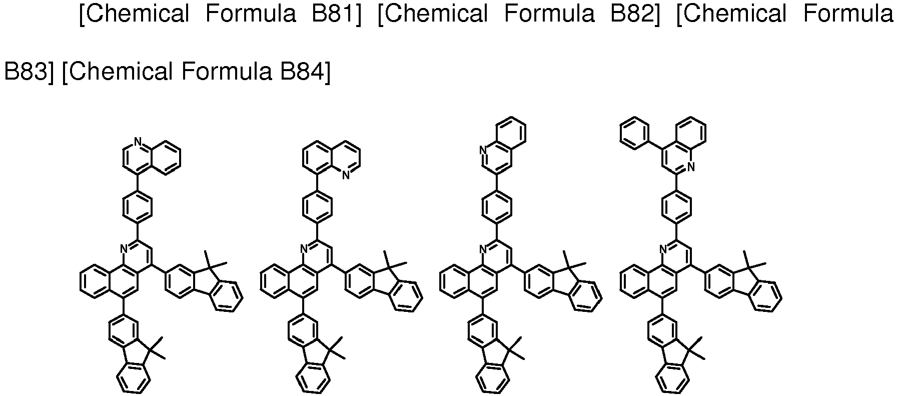

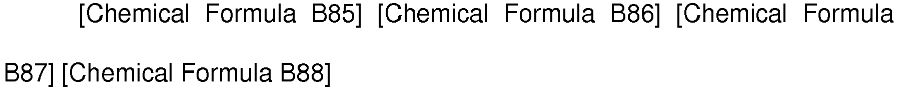

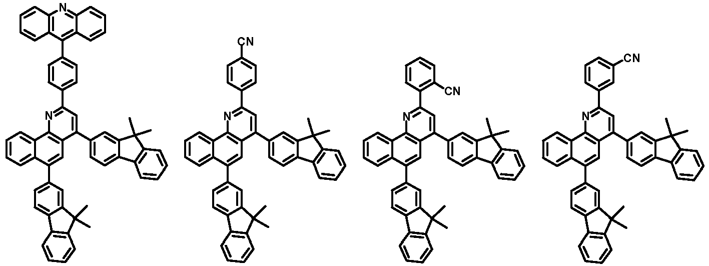

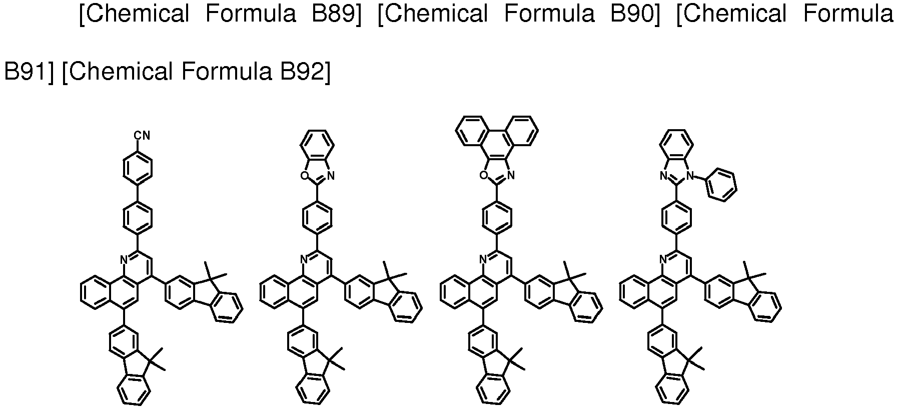

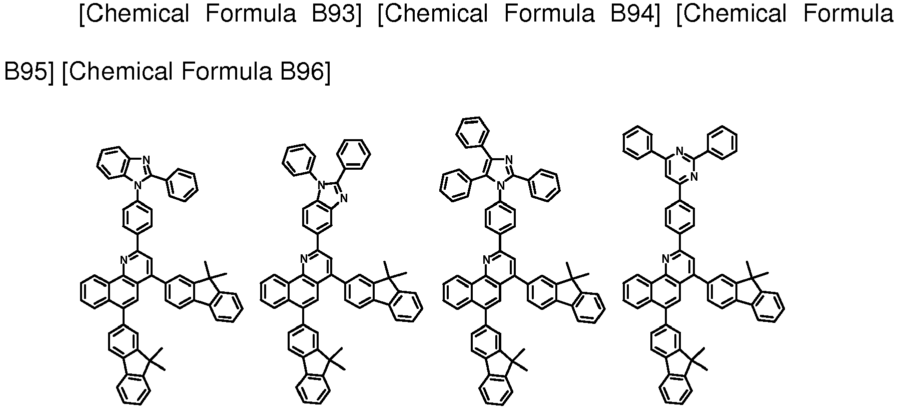

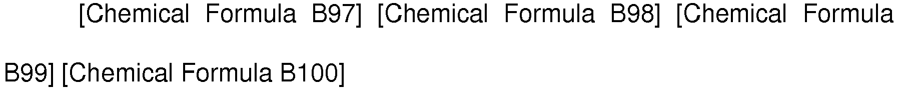

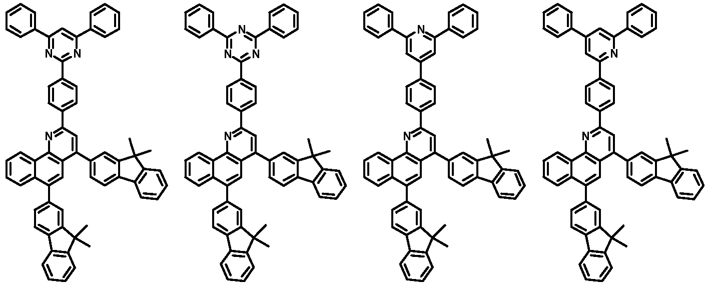

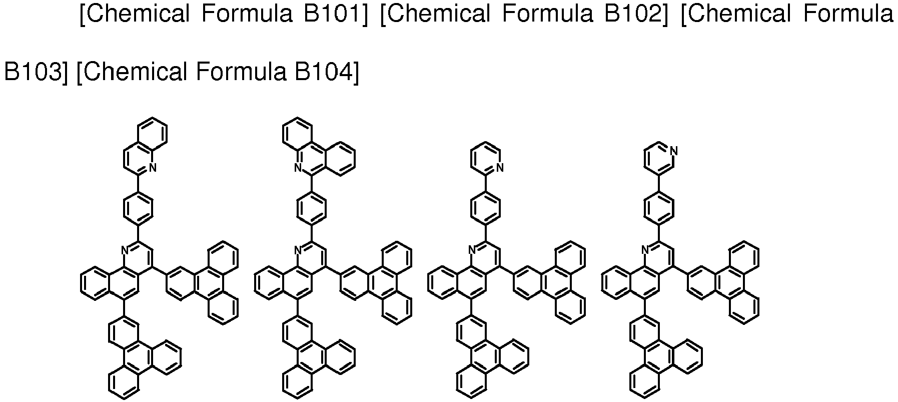

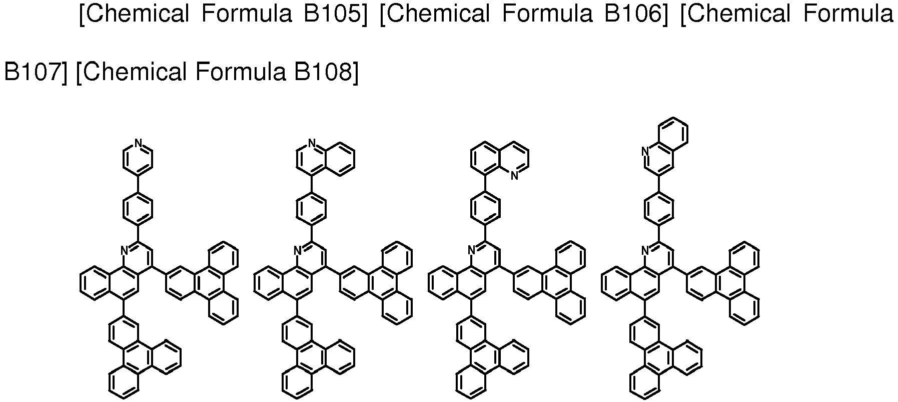

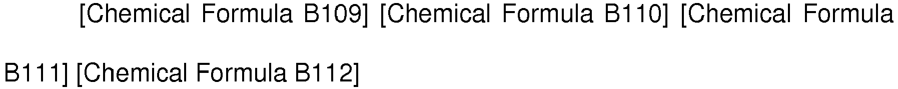

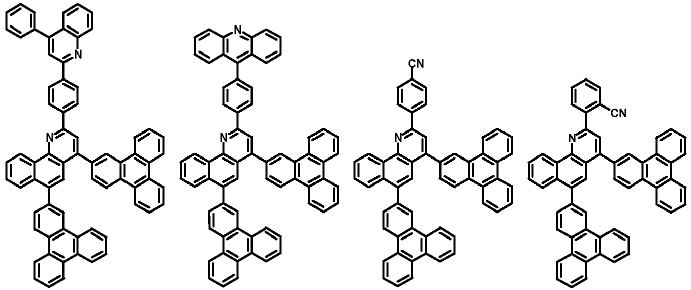

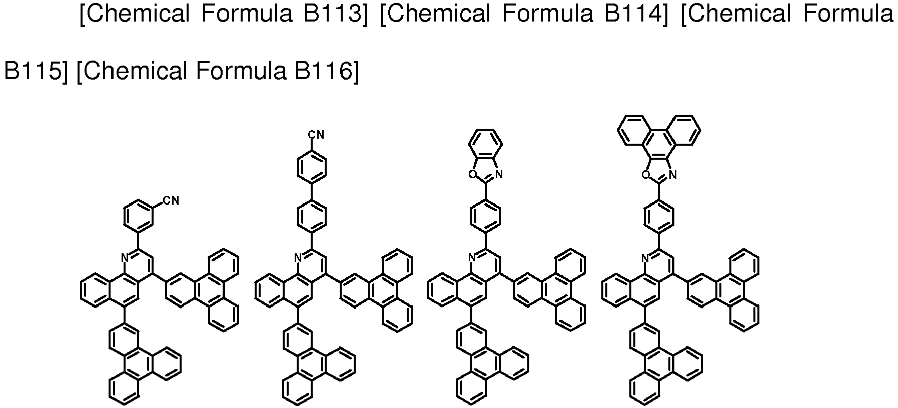

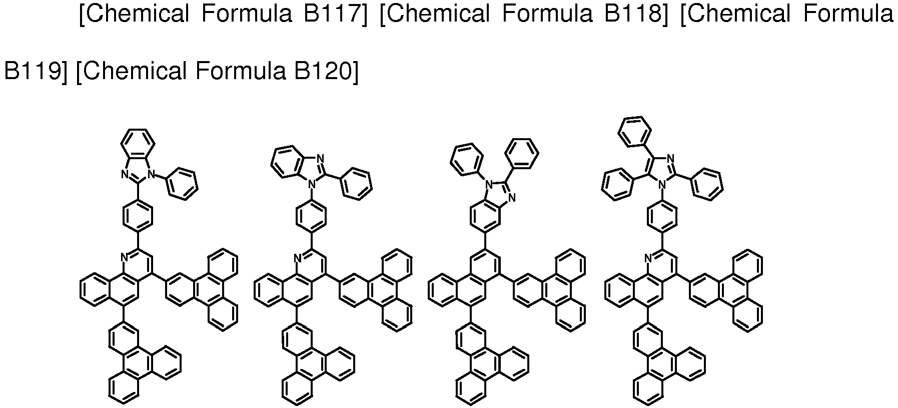

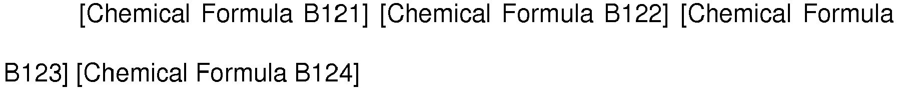

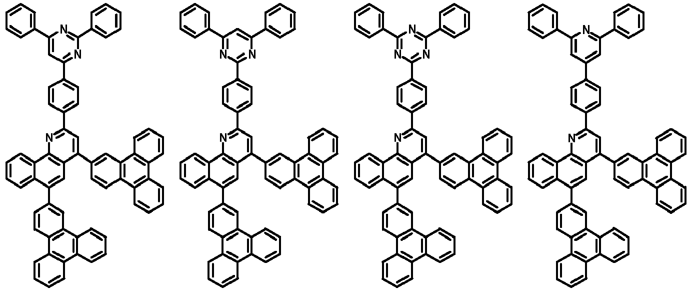

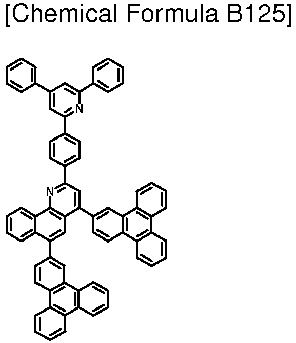

The compound for an organic optoelectronic device may be represented by one of the following Chemical Formulae C1 to C127. However, the present invention is not limited to the following compounds.

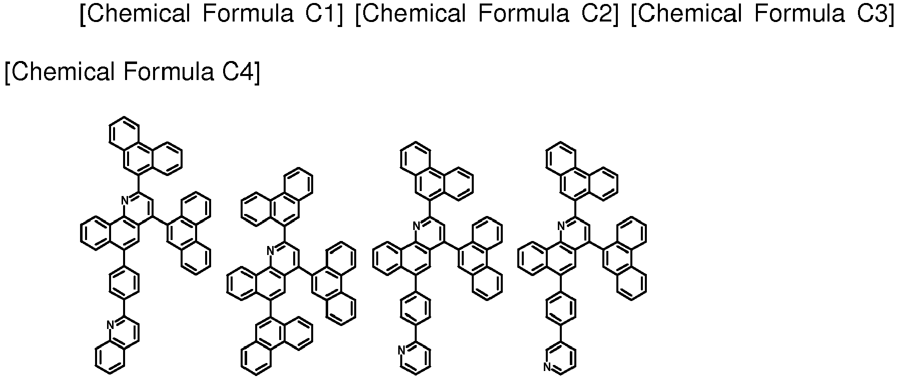

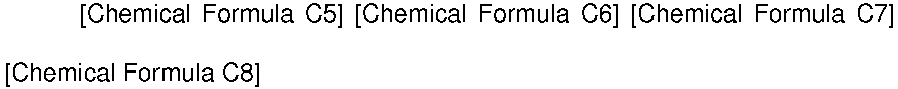

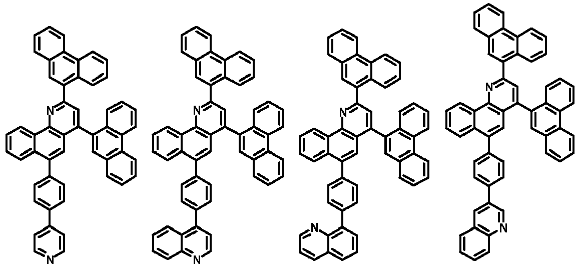

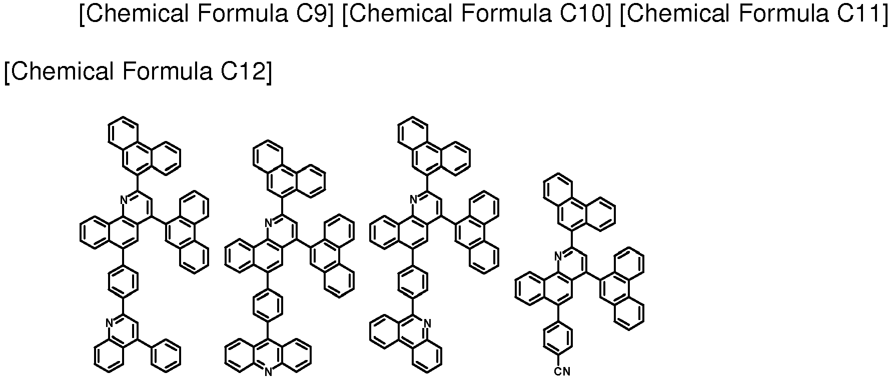

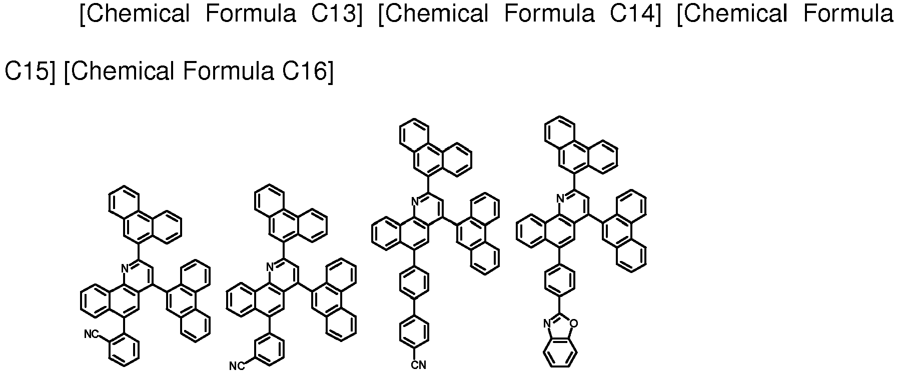

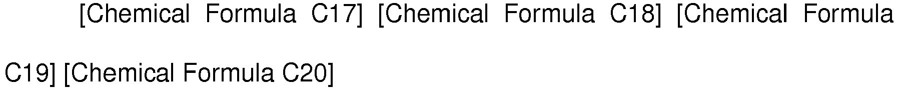

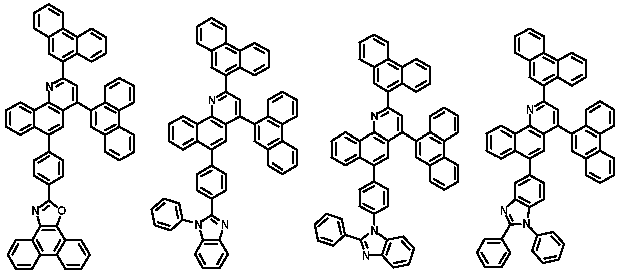

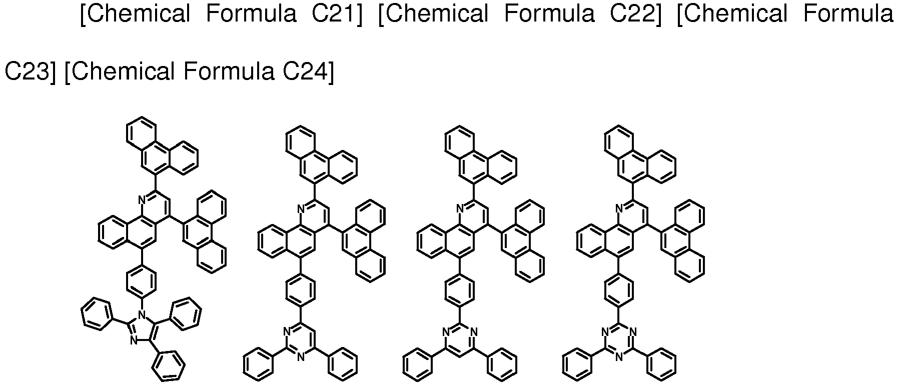

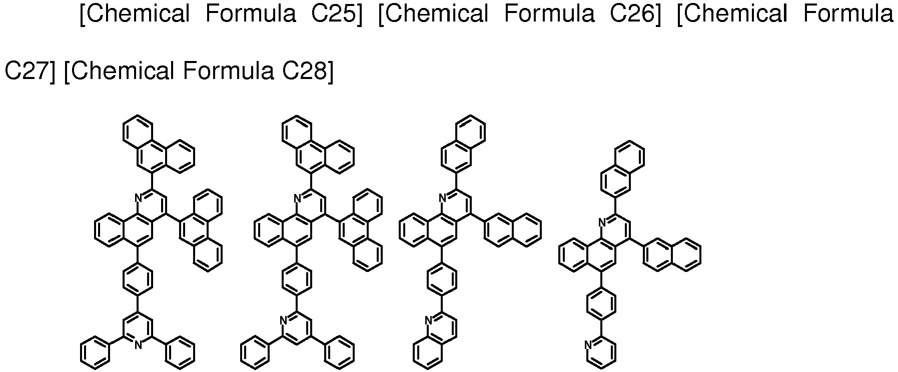

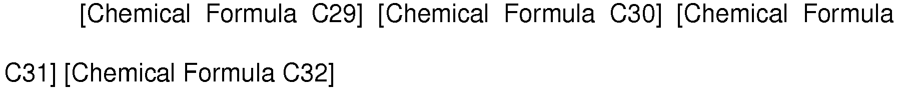

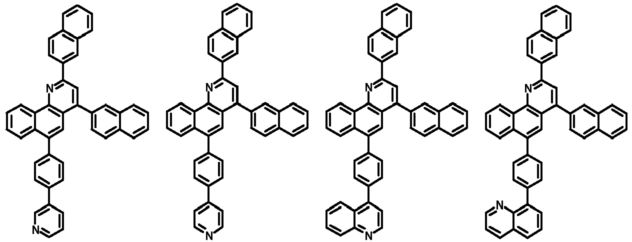

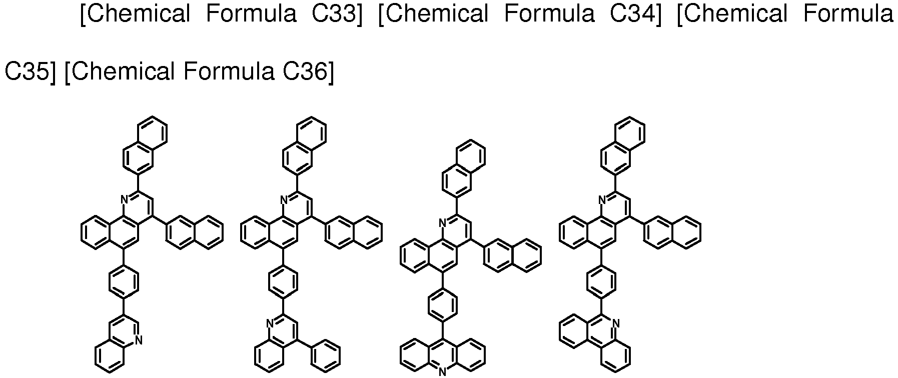

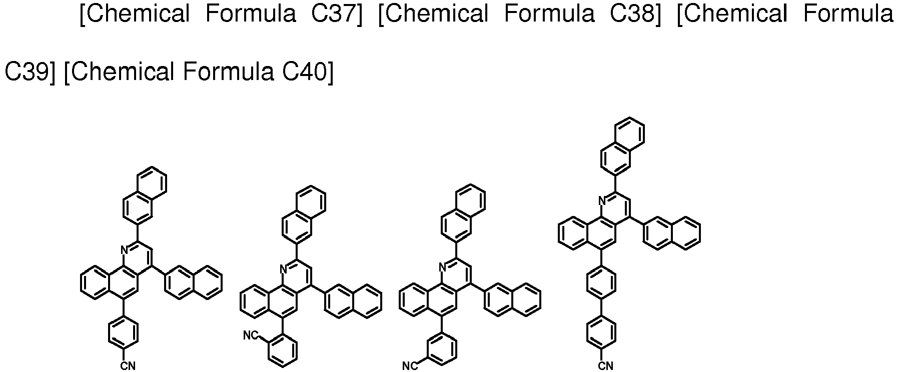

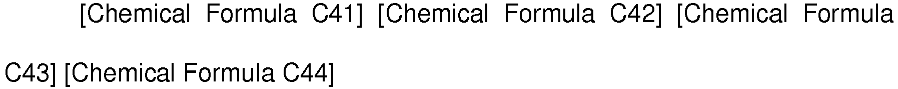

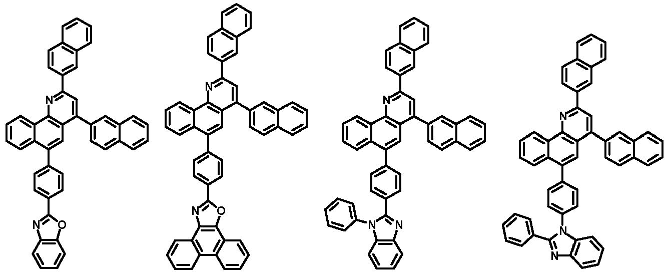

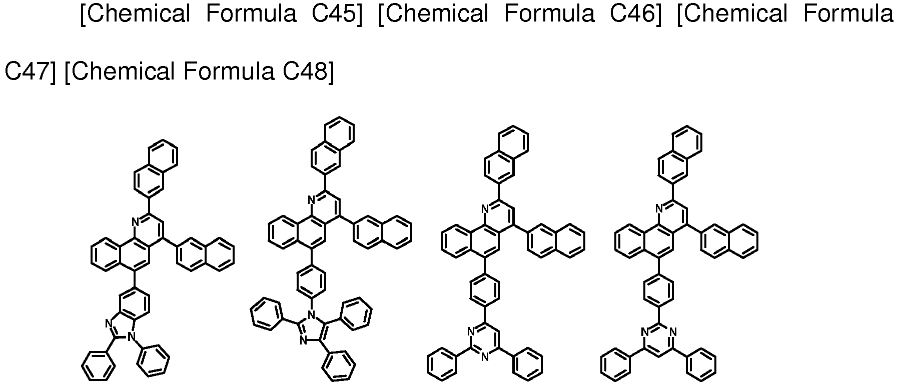

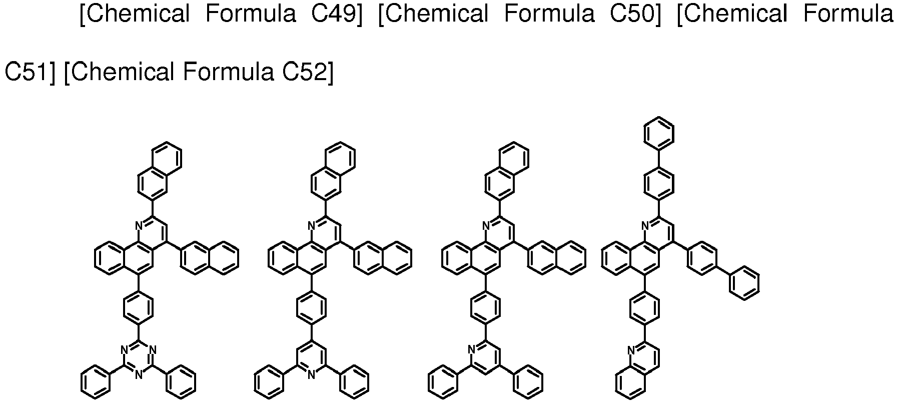

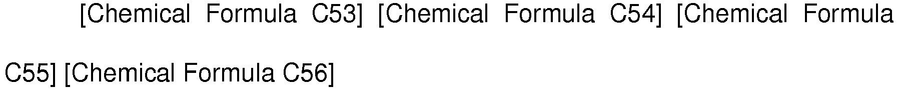

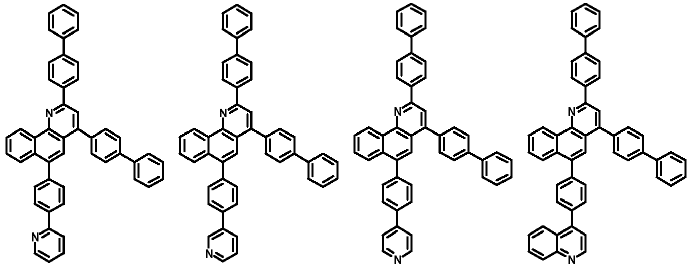

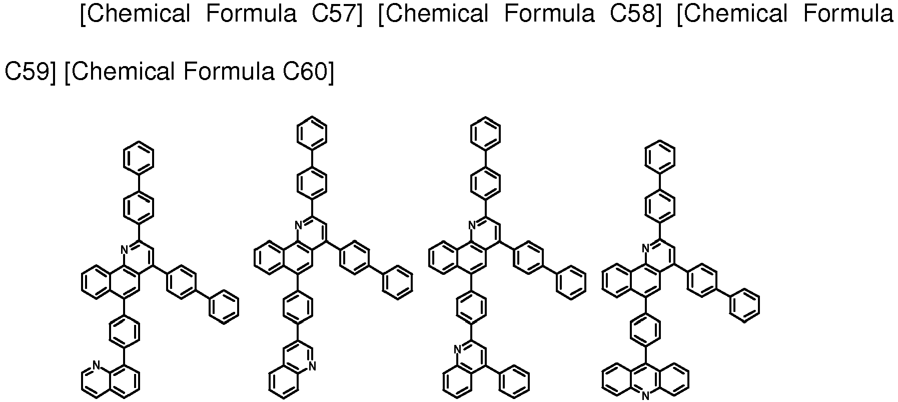

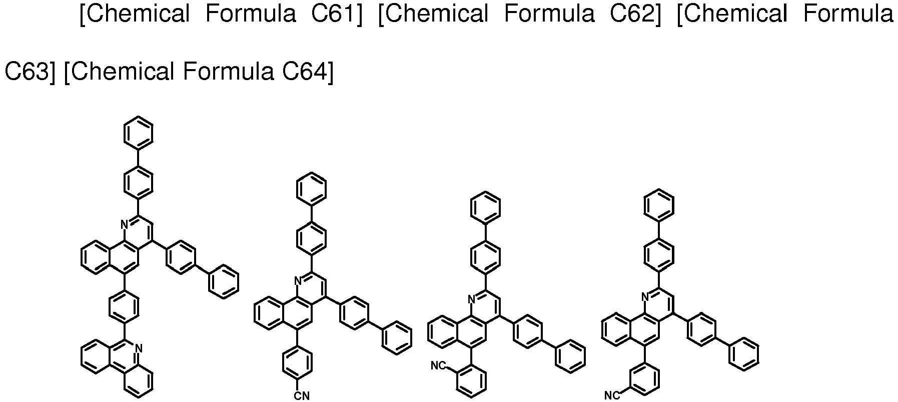

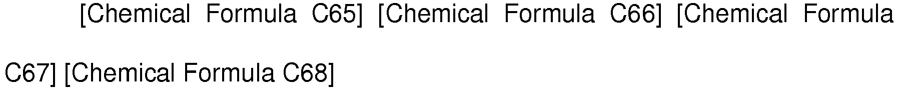

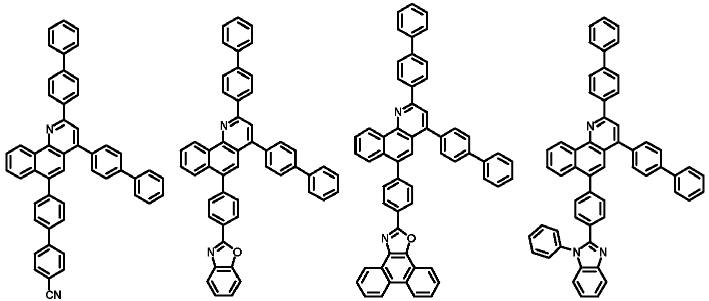

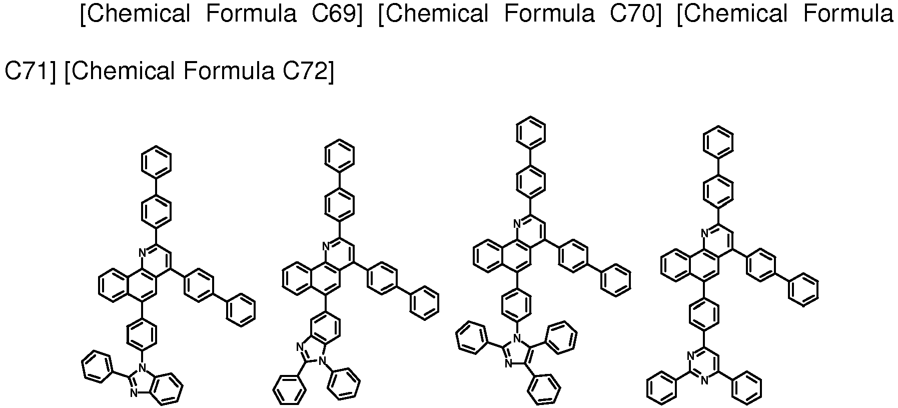

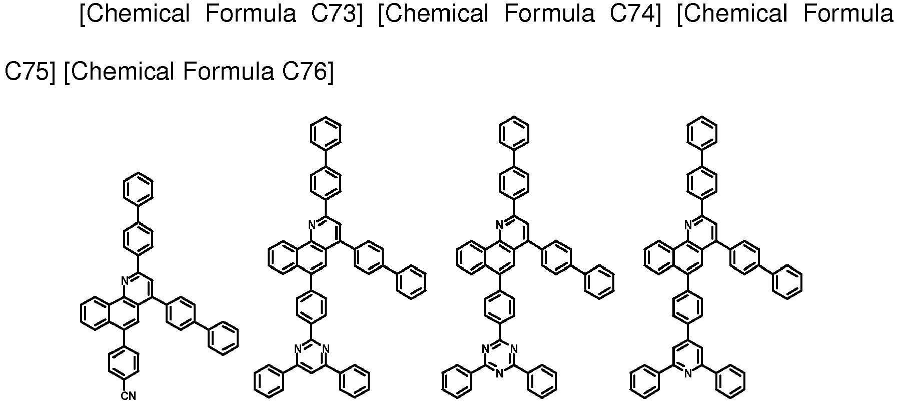

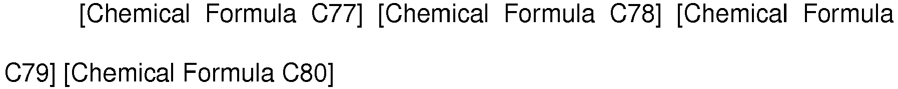

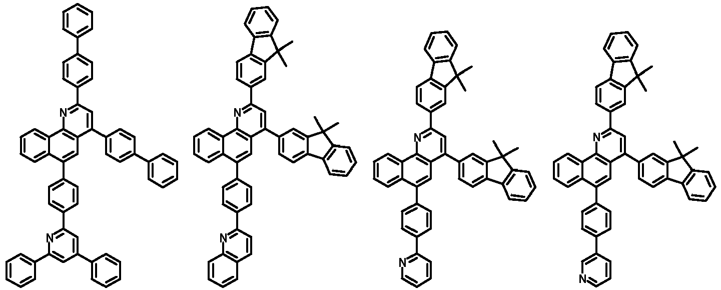

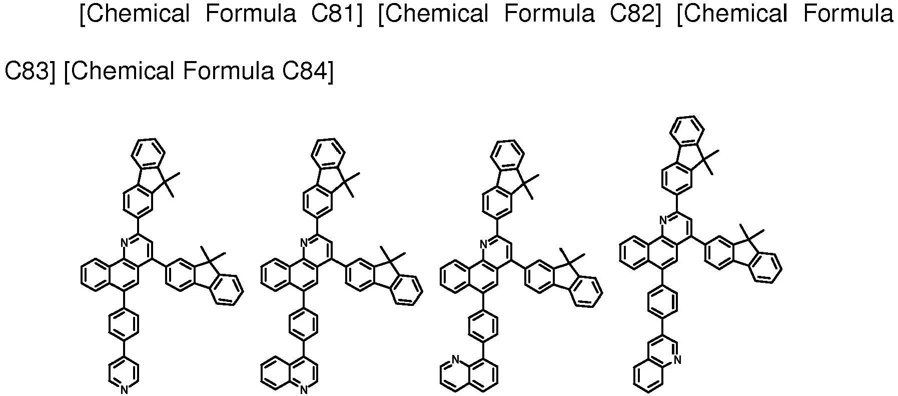

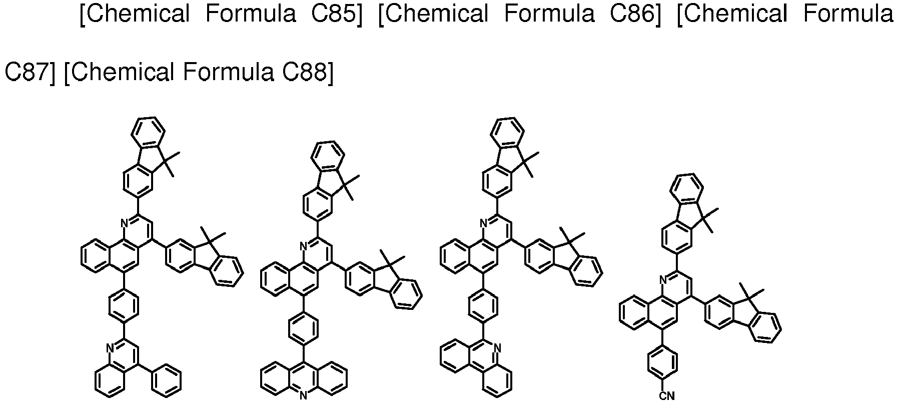

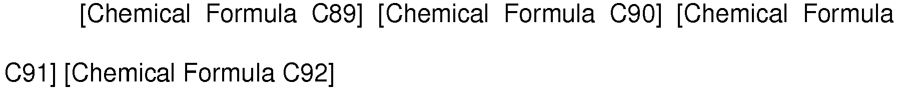

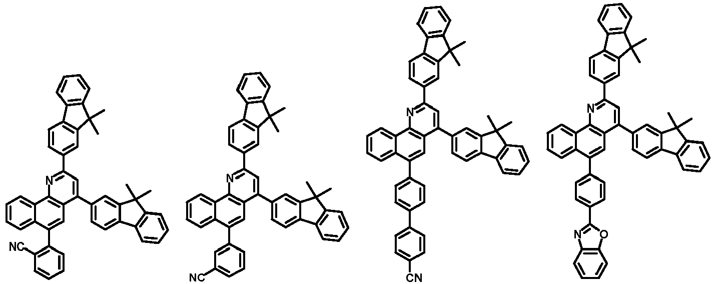

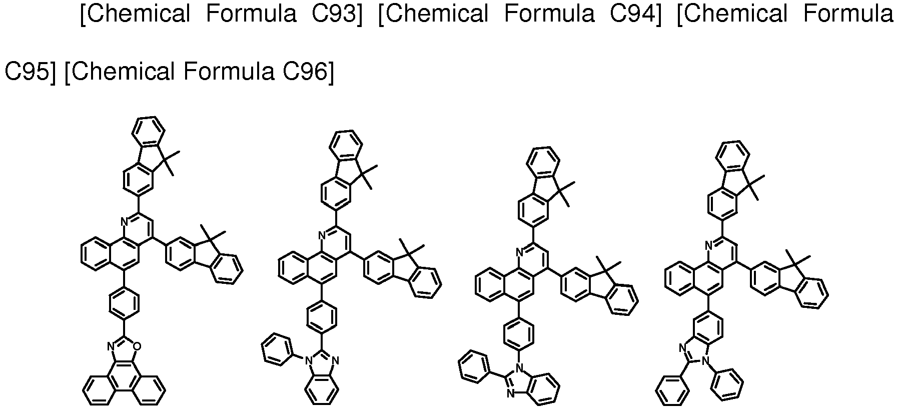

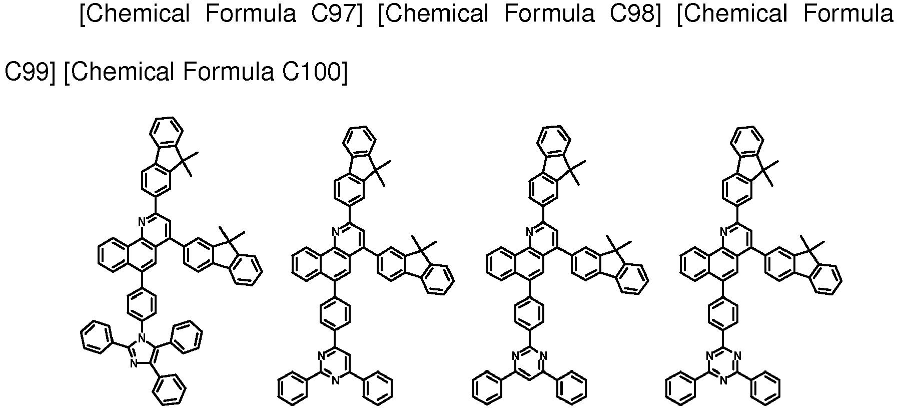

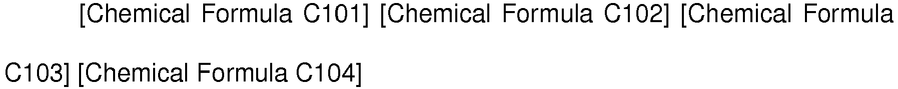

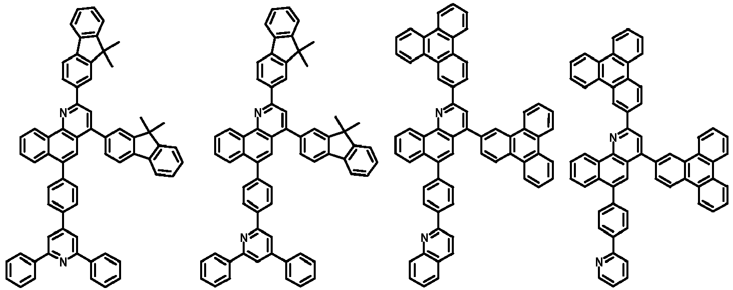

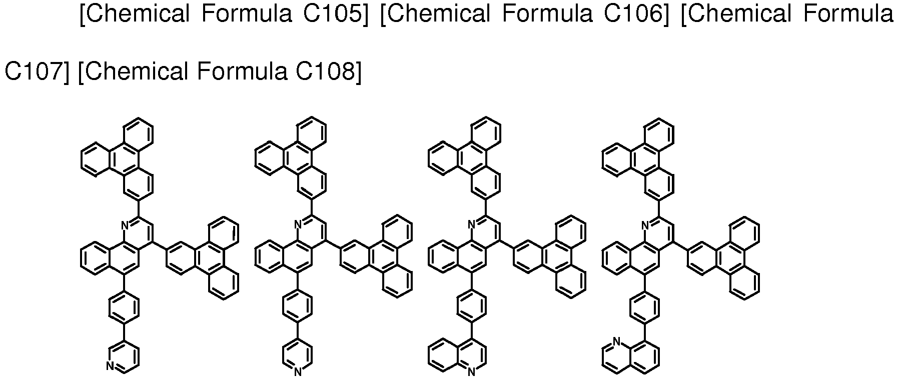

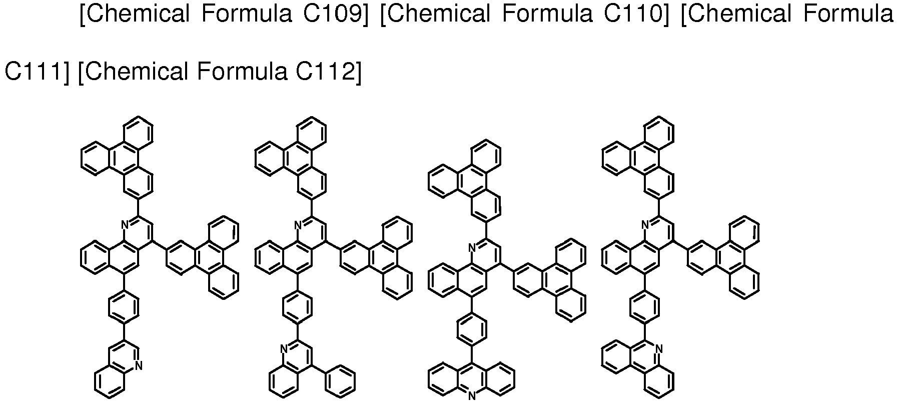

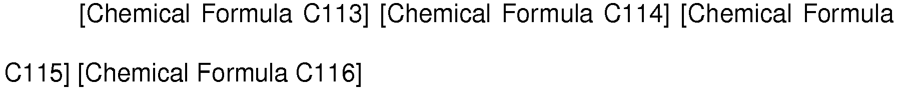

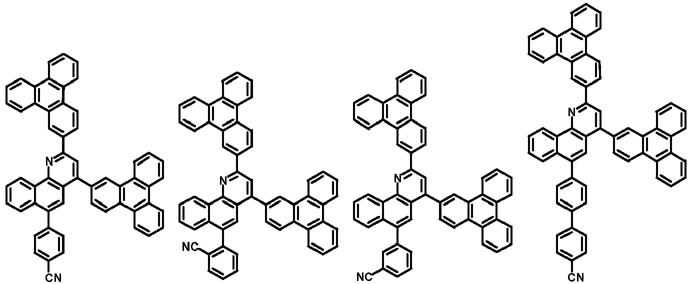

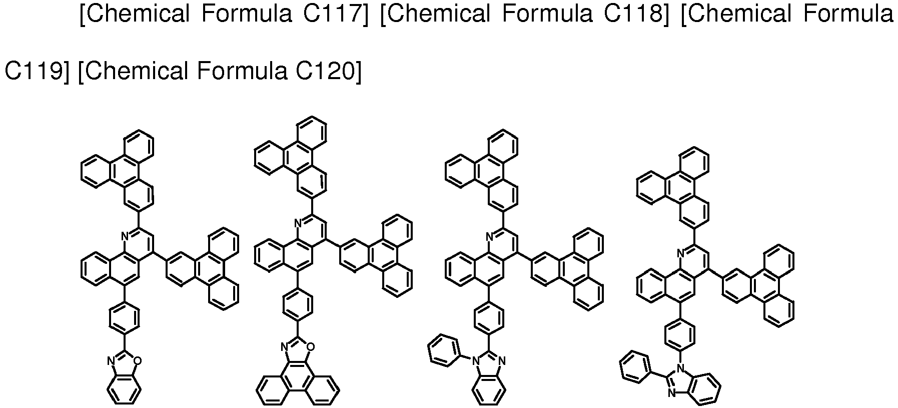

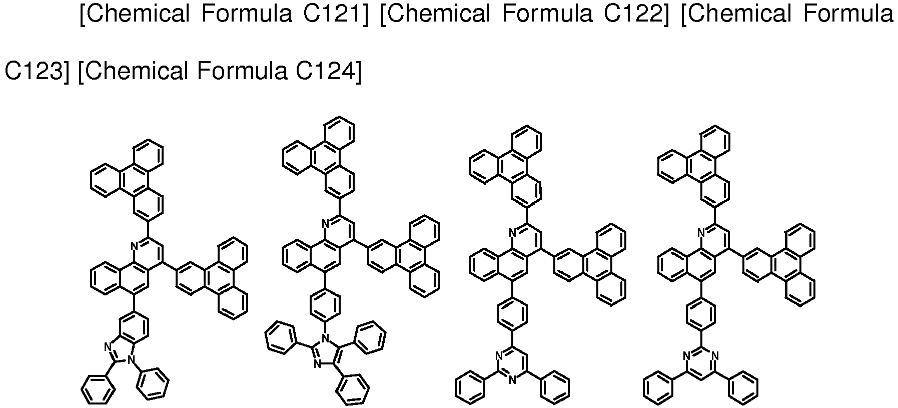

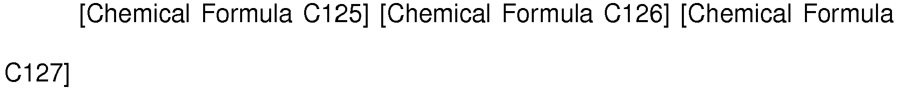

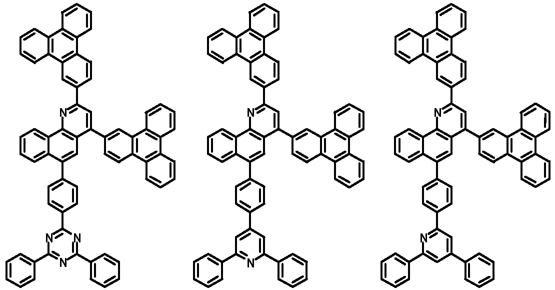

The compound for an organic optoelectronic device including the above compounds has a glass transition temperature of 150°C or higher and a thermal decomposition temperature of 400°C or higher, indicating improved thermal stability. Thereby, it is possible to produce an organic optoelectronic device having a high efficiency.

The compound for an organic optoelectronic device including the above compounds may play a role for emitting light or injecting and/or transporting electrons, and also act as a light emitting host with an appropriate dopantln other words, the compound for an organic optoelectronic device may be used as a phosphorescent or fluorescent host material, a blue light emitting dopant material, or an electron transporting material.

Since the compound for an organic optoelectronic device according to one embodiment is used for an organic thin layer, it may improve the life-span characteristic, efficiency characteristic, electrochemical stability, and thermal stability of an organic photoelectric device and decrease the driving voltage.

Therefore, according to another embodiment, an organic optoelectronic device is provided that includes the compound for an organic optoelectronic device. The organic optoelectronic device may include an organic photoelectric device, an organic light emitting diode, an organic solar cell, an organic transistor, an organic photo conductor drum, an organic memory device, and the like. For example, the compound for an organic optoelectronic device according to one embodiment may be included in an electrode or an electrode buffer layer in the organic solar cell to improve the quantum efficiency, and it may be used as an electrode material for a gate, a source-drain electrode, or the like in the organic transistor.

Hereinafter, an organic light emitting diode will be described in detail.

According to another embodiment of the present invention provides an organic light emitting diode including an anode, a cathode, and at least one or more organic thin layer between the anode and the cathode, and at least one of the organic thin layer may include the compound for an organic optoelectronic device of the present invention.

The organic thin layer that may include the compound for an organic optoelectronic device may include a layer selected from the group consisting of an emission layer, a hole transport layer (HTL), a hole injection layer (HIL), an electron transport layer (ETL), an electron injection layer (EIL), a hole blocking layer, and a combination thereof. The at least one layer includes the compound for an organic optoelectronic device according to one embodiment. Particularly, the compound for an organic optoelectronic device according to one embodiment may be included in an electron transport layer (ETL) or an electron injection layer (EIL). In addition, when the compound for an organic optoelectronic device is included in the emission layer, the compound for an organic optoelectronic device may be included as a phosphorescent or fluorescent host, and particularly, as a fluorescent blue dopant material.

FIGS. 1 to 5 are cross-sectional views showing organic photoelectric devices including the compound for an organic optoelectronic device according to one embodiment of the present invention.

Referring to FIGS. 1 to 5, organic photoelectric devices 100, 200, 300, 400, and 500 according to one embodiment include at least one organic thin layer 105 interposed between an anode 120 and a cathode 110.

The anode 120 includes an anode material laving a large work function to help hole injection into an organic thin layer. The anode material includes: a metal such as nickel, platinum, vanadium, chromium, copper, zinc, and gold, or alloys thereof; a metal oxide such as zinc oxide, indium oxide, indium tin oxide (ITO), and indium zinc oxide (IZO); a combined metal and oxide such as ZnO:Al or SnO₂:Sb; or a conductive polymer such as poly(3-methylthiophene), poly[3,4-(ethylene-1,2-dioxy)thiophene] (PEDT), polypyrrole, and polyaniline, but is not limited thereto. It is preferable to include a transparent electrode including indium tin oxide (ITO) as an anode.

The cathode 110 includes a cathode material having a small work function to help electron injection into an organic thin layer. The cathode material includes: a metal such as magnesium, calcium, sodium, potassium, titanium, indium, yttrium, lithium, gadolinium, aluminum, silver, tin, and lead, or alloys thereof; or a multi-layered material such as LiF/Al, Liq/Al, LiO₂/Al, LiF/Ca, LiF/Al, and BaF₂/Ca, but is not limited thereto. It is preferable to include a metal electrode including aluminum as a cathode.

Referring to FIG. 1, the organic photoelectric device 100 includes an organic thin layer 105 including only an emission layer 130.

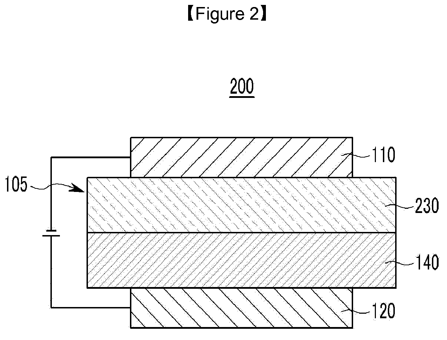

Referring to FIG. 2, a double-layered organic photoelectric device 200 includes an organic thin layer 105 including an emission layer 230 including an electron transport layer (ETL), and a hole transport layer (HTL) 140. As shown in FIG. 2, the organic thin layer 105 includes a double layer of the emission layer 230 and hole transport layer (HTL) 140. The emission layer 130 also functions as an electron transport layer (ETL), and the hole transport layer (HTL) 140 layer has an excellent binding property with a transparent electrode such as ITO or an excellent hole transporting property.

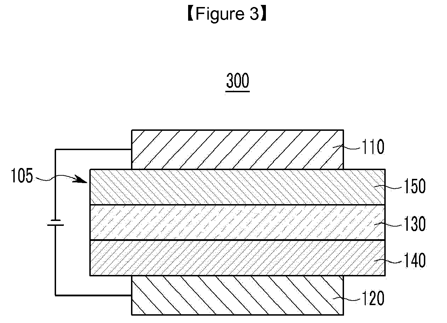

Referring to FIG. 3, a three-layered organic photoelectric device 300 includes an organic thin layer 105 including an electron transport layer (ETL) 150, an emission layer 130, and a hole transport layer (HTL) 140. The emission layer 130 is independently installed, and layers having an excellent electron transporting property or an excellent hole transporting property are separately stacked.

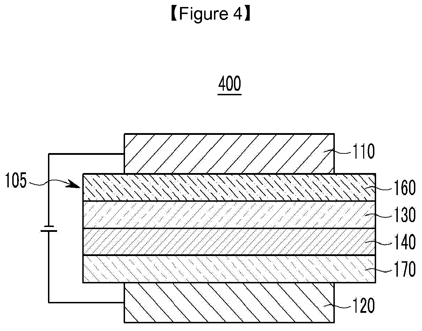

As shown in FIG. 4, a four-layered organic photoelectric device 400 includes an organic thin layer 105 including an electron injection layer (EIL) 160, an emission layer 130, a hole transport layer (HTL) 140, and a hole injection layer (HIL) 170 for adherence with the cathode of ITO.

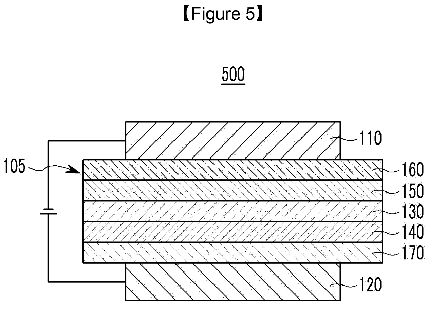

As shown in FIG. 5, a five layered organic photoelectric device 500 includes an organic thin layer 105 including an electron transport layer (ETL) 150, an emission layer 130, a hole transport layer (HTL) 140, and a hole injection layer (HIL) 170, and further includes an electron injection layer (EIL) 160 to achieve a low voltage.

In FIGS. 1 to 5, the organic thin layer 105 including at least one selected from the group consisting of an electron transport layer (ETL) 150, an electron injection layer (EIL) 160, emission layers 130 and 230, a hole transport layer (HTL) 140, a hole injection layer (HIL) 170, and combinations thereof includes a compound for an organic optoelectronic device. The compound for an organic optoelectronic device may be used for an electron transport layer (ETL) 150 including the electron transport layer (ETL) 150 or electron injection layer (EIL) 160. When it is used for the electron transport layer (ETL), it is possible to provide an organic photoelectric device having a more simple structure because it does not require an additional hole blocking layer (not shown).

Furthermore, when the compound for an organic optoelectronic device is included in the emission layers 130 and 230, the material for the organic photoelectric device may be included as a phosphorescent or fluorescent host or a fluorescent blue dopant.

The organic light emitting diode may be fabricated by: forming an anode on a substrate; forming an organic thin layer in accordance with a dry coating method such as evaporation, sputtering, plasma plating, and ion plating or a wet coating method such as spin coating, dipping, and flow coating; and providing a cathode thereon.

Another embodiment of the present invention provides a display device including the organic photoelectric device according to the above embodiment.

Hereinafter, the embodiments are illustrated in more detail with reference to examples. However, the following are exemplary embodiments and are not limiting.

### (Preparation of compound for an organic optoelectronic device)

### Example 1: Synthesis of compound represented by Chemical Formula A1

As an example of the compound for an organic optoelectronic device, the compound represented by the above Chemical Formula A1 was synthesized through 4 step processes in accordance with the following Reaction Scheme 1.

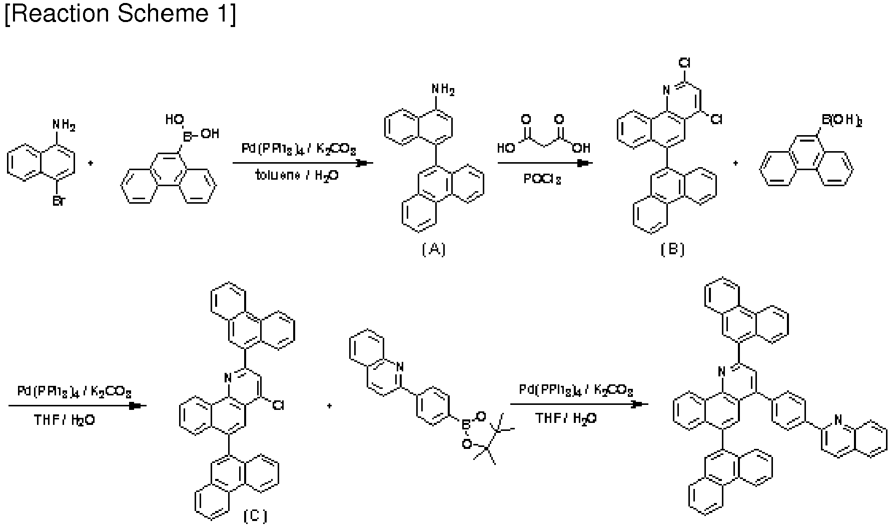

### First step: Synthesis of intermediate product (A)

25.0 g (112.6 mmol) of 1-amino-4-bromonaphthalene, 30.0 g (135.1 mmol) of 9-phenanthrene boronic acid, and 3.3 g (2.8 mmol) of tetrakis(triphenylphosphine)palladium [Pd(PPh₃)₄] were dissolved in 750 mL of a toluene solvent and added with a solution that 31.1 g (225.1 mmol) of potassium carbonate (K₂CO₃) was dissolved in 250 ml of water, and then they were reacted at 85 °C for 12 hours. The water layer of obtained reactant was removed, and the solvent was removed under reduced pressure, and it was rinsed with water and methanol. The obtained solid mixture was separated by a column and dried to provide a yellow solid of an intermediate product (A) in 31.0 g (yield: 86%).

### Second step: Synthesis of intermediate product (B)

20.0 g (62.6 mmol) of the intermediate product (A), and 9.8 g (93.9 mmol) of malonic acid were dissolved in a 58 mL of phosphorus oxychloride (POCl₃) solvent and reacted at 140 °C for 4 hours. The obtained reactanttion products were poured into ice water and filtered for the formed solid and rinsed with water and sodium hydrogen carbonate saturated aqueous solution. The obtained solid mixture was rinsed with methanol and dried to provide a pale yellow solid of an intermediate product (B) in 13.0 g (yield: 49%).

### Third step: Synthesis of intermediate product (C)

14.0 g (33.0 mmol) of intermediate product (B), 8.1 g (36.3 mmol) of 9-phenanthrene boronic acid, and 1.2 g (1.0 mmol) of tetrakis(triphenylphosphine)palladium [Pd(PPh₃)₄] were dissolved in 280 mL of a tetrahydrofuran (THF) solvent and added with a solution that 9.1 g (66.0 mmol) of potassium carbonate (K₂CO₃) was dissolved in 140 ml of water, and then they were reacted at 80°C for 12 hours. The solvent of obtained reactantion products was removed under a reduced pressure, and it was rinsed with water and methanol. The residue was recrystallized with toluene, and the precipitated crystal was separated by a filter and rinsed with toluene and dried to provide a white solid of an intermediate compound (C) in 14.9 g (yield: 51%).

### Fourth step: Synthesis of compound represented by Chemical Formula A1

10.0 g (17.7 mmol) of intermediate product (C), 7.0 g (21.2 mmol) of 8-(4-(4,4,5,5-tetramethyl-1,3,2-dioxaborolan-2-yl)phenyl)quinoline, and 0.6 g (0.5 mmol) of tetrakis(triphenylphosphine)palladium [Pd(PPh₃)₄] were dissolved in 200 mL of a tetrahydrofuran (THF) solvent and added with a solution that 4.9g (35.3 mmol) of potassium carbonate (K₂CO₃) was dissolved in 100 ml of water, and then they were reacted at 90 °C for 12 hours. The solvent of obtained reactants was removed under a reduced pressure, and it was rinsed with water and methanol. The residue was recrystallized with toluene, and the precipitated crystal was separated by a filter and rinsed with toluene and dried to provide a white solid of a compound in 11.0 g (yield: 85%). (calculation value: 734.88, measurement value: MS[M+1] 735.18)

### Example 2: Synthesis of compound represented by Chemical Formula B1

As an example of the compound for an organic optoelectronic device, the compound represented by the above Chemical Formula B1 was synthesized through 2 step processes in accordance with the following Reaction Scheme 2.

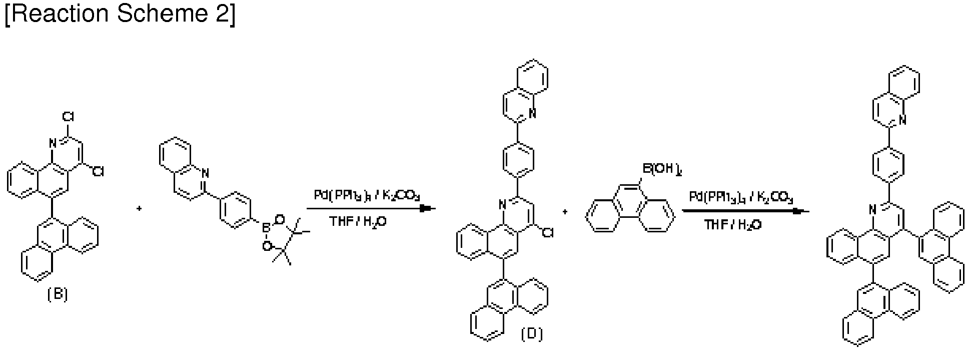

### First step: Synthesis of intermediate product (D)

5.2 g (12.3 mmol) of the intermediate product (B), 4.5 g (13.5 mmol) of 8-(4-(4,4,5,5-tetramethyl-1,3,2-dioxaborolan-2-yl)phenyl)quinoline, and 0.4 g (0.4 mmol) of tetrakis(triphenylphosphine)palladium [Pd(PPh₃)₄] were dissolved in a 100 mL of a tetrahydrofuran (THF) solvent and added with a solution that 3.4 g (24.5 mmol) of potassium carbonate (K₂CO₃) was dissolved in 50 ml of water, and then they were reacted at 80°C for 12 hours. The solvent of obtained reactants was removed under a reduced pressure, and it was rinsed with water and methanol. The residue was recrystallized with toluene, and the precipitated crystal was separated by a filter and rinsed with toluene and dried to provide a white solid of an intermediate product (C) in 5.0 g (yield: 69%).

### Second step: Synthesis of compound represented by Chemical Formula B1

5.0 g (8.4 mmol) of intermediate product (D), 2.3 g (10.1 mmol) of 9-phenanthrene boroic acid, and 0.3 g (0.3 mmol) of tetrakis(triphenylphosphine)palladium [Pd(PPh₃)₄] were dissolved in 100 mL of a tetrahydrofuran (THF) solvent and added with a solution that 2.3g (16.9 mmol) of potassium carbonate (K₂CO₃) was dissolved in 50 ml of water, and then they were reacted at 90 °C for 12 hours. The solvent of obtained reactants was removed under a reduced pressure, and it was rinsed with water and methanol. The residue was recrystallized with toluene, and the precipitated crystal was separated by a filter and rinsed with toluene and dried to provide a white solid of a compound in 4.2 g (yield: 68%). (calculation value: 734.88, measurement value: MS[M+1] 735.18)

### Example 3: Synthesis of compound represented by Chemical Formula C1

As an example of the compound for an organic optoelectronic device, the compound represented by the above Chemical Formula C1 was synthesized through 3 step processes in accordance with the following Reaction Scheme 3.

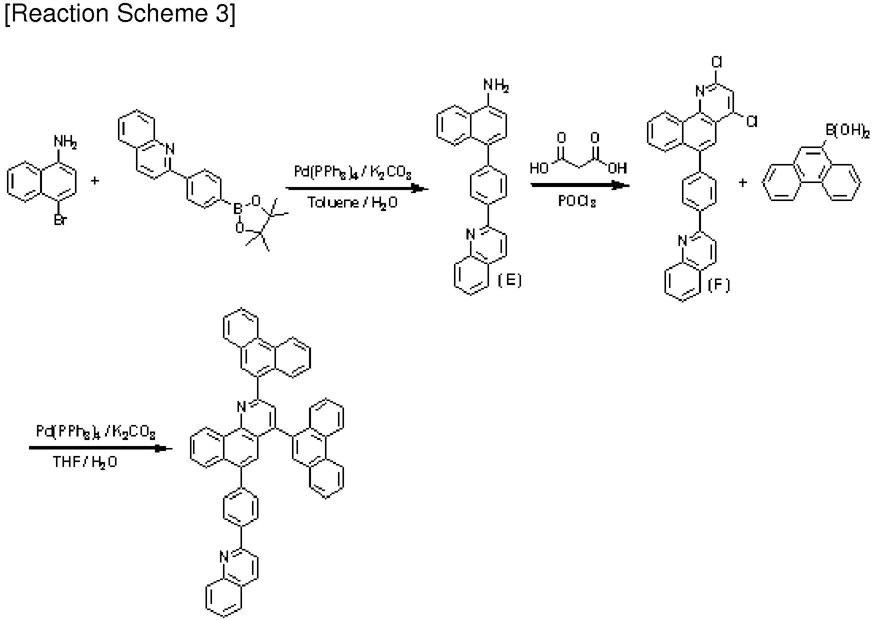

### First step: Synthesis of intermediate product (E)

15.0 g (67.5 mmol) of 1-amino-4-bromonaphthalene, 24.6 g (74.3 mmol) of 8-(4-(4,4,5,5-tetramethyl-1,3,2-dioxaborolan-2-yl)phenyl)quinoline, and 2.0 g (1.7 mmol) of tetrakis(triphenylphosphine)palladium [Pd(PPh₃)₄] were dissolved in 450 mL of a toluene solvent and added with a solution that 18.7 g (135.1 mmol) of potassium carbonate (K₂CO₃) was dissolved in 150 ml of water, and then they were reacted at 85 °C for 12 hours. The water layer of obtained reactant was removed, and the solvent was removed under a reduced pressure, and it was rinsed with water and methanol. The obtained solid mixture was separated by a column and dried to provide a yellow solid of an intermediate product (E) in 15.5 g (yield: 66%).

### Second step: Synthesis of intermediate product (F)

15.5 g (44.7 mmol) of intermediate product (E), and 7.0 g (67.1 mmol) of malonic acid were dissolved in 41 mL of phosphorus oxychloride (POCl₃) solvent and reacted at 140 °C for 4 hours. The obtained reactant was poured into ice water and filtered for the formed solid and rinsed with sodium hydrogen carbonate saturated aqueous solution. The obtained solid mixture was rinsed with methanol and dried to provide a pale yellow solid of an intermediate product (F) in 5.0 g (yield: 25%).

### Third step: Synthesis of compound represented by Chemical Formula C1

2.2 g (4.9 mmol) of intermediate product (F), 2.4 g (10.7 mmol) of 9-phenanthrene boronic acid, and 0.3 g (0.2 mmol) of tetrakis(triphenylphosphine)palladium [Pd(PPh₃)₄] were dissolved in 60 mL of a tetrahydrofuran (THF) solvent and added with a solution that 2.7g (19.5 mmol) of potassium carbonate (K₂CO₃) was dissolved in 20 ml of water, and then they were reacted at 90 °C for 12 hours. The solvent of obtained reactants was removed under reduced pressure, and it was rinsed with water and methanol. The residue was recrystallized with toluene, and the precipitated crystal was separated by a filter and rinsed with toluene and dried to provide a white solid of a compound in 2.8 g (yield: 78%). (calculation value: 734.88, measurement value: MS[M+1] 735.18)

### Example 4: Synthesis of compound represented by Chemical Formula A2

As an example of the compound for an organic optoelectronic device, the compound represented by the above Chemical Formula A2 was synthesized in accordance with the following Reaction Scheme 4. 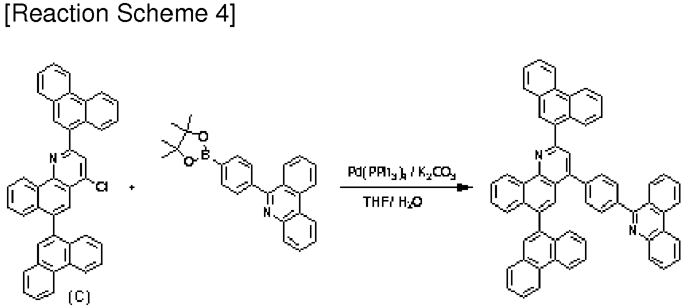 10 g (17.7 mmol) of intermediate product (C), 8.1 g (21.2 mmol) of 6-(4-(4,4,5,5-tetramethyl-1,3,2-dioxaborolan-2-yl)phenyl)phenantridine, and 0.6 g (0.5 mmol) of tetrakis(triphenylphosphine)palladium [Pd(PPh₃)₄] were dissolved in 200 mL of a tetrahydrofuran (THF) solvent and added with a solution that 4.9g (35.3 mmol) of potassium carbonate (K₂CO₃) was dissolved in 100 ml of water, and then they were reacted at 90 °C for 12 hours. The solvent of obtained reactants was removed under reduced pressure, and it was rinsed with water and methanol. The residue was recrystallized with toluene, and the precipitated crystal was separated by a filter and rinsed with toluene and dried to provide a white solid of a compound in 11.0 g (yield: 79%). (calculation value: 784.94, measurement value: MS[M+1] 785.29)

### Example 5: Synthesis of compound represented by Chemical Formula B2

As an example of the compound for an organic optoelectronic device, the compound represented by the above Chemical Formula B2 was synthesized in accordance with the following Reaction Scheme 5.

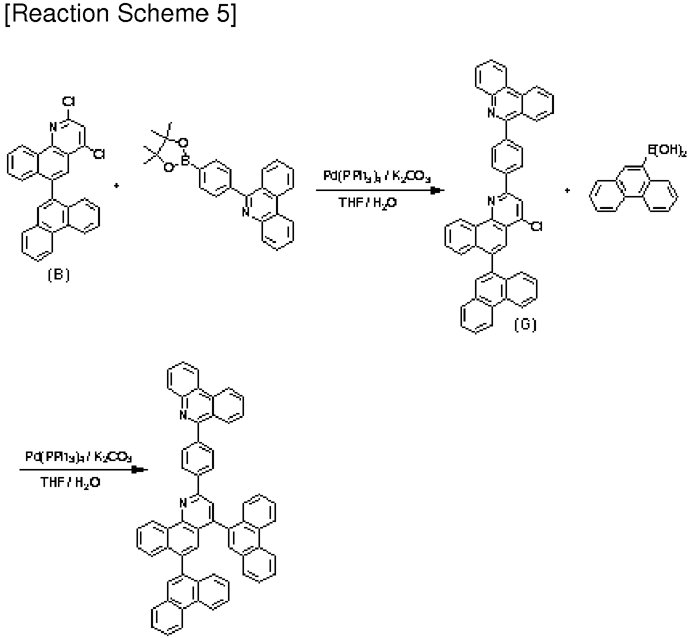

### First step: Synthesis of intermediate product (G)

11.0 g (25.9 mmol) of intermediate product (B), 10.9 g (28.5 mmol) of 6-(4-(4,4,5,5-tetramethyl-1,3,2-dioxaborolan-2-yl)phenyl)phenantridine, and 0.9 g (0.8 mmol) of tetrakis(triphenylphosphine)palladium [Pd(PPh₃)₄] were dissolved in 220 ml of a tetrahydrofuran (THF) solvent and added with a solution that 7.2 g (51.9 mmol) of potassium carbonate (K₂CO₃) was added into 110 ml of water, and then they were reacted at 80 °C for 12 hours. The water layer of obtained reactant was removed, and the solvent was removed under reduced pressure, and it was rinsed with water and methanol. The residue was recrystallized with toluene, and the precipitated crystal was separated by a filter and rinsed with toluene and dried to provide a pale yellow solid of intermediate product (G) in 13.69 g (yield: 82%).

### Second step: Synthesis of compound represented by Chemical Formula B2

13.0 g (20.2 mmol) of intermediate product (G), 5.4 g (24.3 mmol) of 9-phenanthrene boronic acid, and 0.7 g (0.6 mmol) of tetrakis(triphenylphosphine)palladium [Pd(PPh₃)₄] were dissolved with a solvent of 390 mL of toluene and 260 mL of tetrahydrofuran (THF) and added with a solution that 5.6 g (40.4 mmol) of potassium carbonate (K₂CO₃) was dissolved in 20 mL of water, and then they were reacted at 90 °C for 12 hours. The solvent of obtained reactants was removed under reduced pressure, and it was rinsed with water and methanol. The residue was recrystallized with toluene, and the precipitated crystal was separated by a filter and rinsed with toluene and dried to provide a white solid of a compound in 13.1 g (yield: 83%). (calculation value: 784.94, measurement value: MS[M+1] 785.29)

### Example 6: Synthesis of compound represented by Chemical Formula A3

As an example of the compound for an organic optoelectronic device, the compound represented by the above Chemical Formula A3 was synthesized through one step process in accordance with the following Reaction Scheme 6.

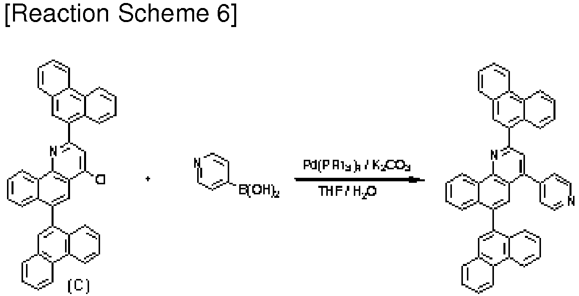

16.0 g (28.3 mmol) of intermediate product (C), 4.2 g (33.9 mmol) of 4-pyridine boronic acid, and 1.0 g (0.9 mmol) of tetrakis(triphenylphosphine)palladium [Pd(PPh₃)₄] were dissolved in 320 mL of a tetrahydrofuran (THF) solvent and added with a solution that 7.8 g (56.5 mmol) of potassium carbonate (K₂CO₃) was dissolved in 160 ml of water, and then they were reacted at 90 °C for 12 hours. The solvent of obtained reactants was removed under reduced pressure, and it was rinsed with water and methanol. The residue was recrystallized with toluene, and the precipitated crystal was separated by a filter and rinsed with toluene and dried to provide a white solid of a compound in 13.0 g (yield: 75%). (calculation value: 608.73, measurement value: MS[M+1] 609.23)

### Example 7: Synthesis of compound represented by Chemical Formula C2

As an example of the compound for an organic optoelectronic device, the compound represented by the above Chemical Formula C2 was synthesized through two step processes in accordance with the following Reaction Scheme 7.

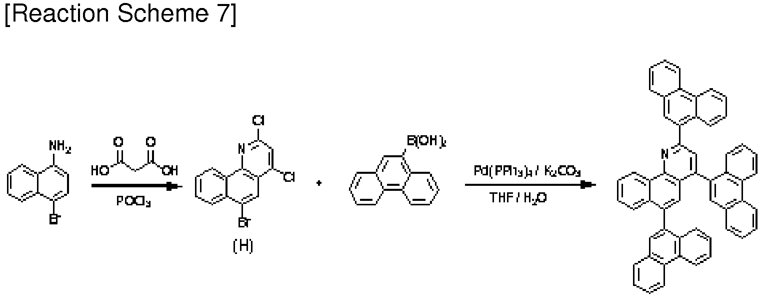

### First step: Synthesis of intermediate product (H)

50.0 g (225.1 mmol) of 1-amino-4-bromonaphthalene, and 35.1 g (337.7 mmol) of malonic acid were dissolved in 345 ml of phosphorus oxychloride (POCl₃) and reacted at 140 °C for 4 hours. The obtained reactant was poured into ice water and filtered for the formed solid and rinsed with sodium hydrogen carbonate saturated aqueous solution. The obtained solid mixture was rinsed with methanol and dried to provide a pale yellow solid of an intermediate product (H) in 16.6 g (yield: 23%).

### Second step: Synthesis of compound represented by Chemical Formula C2

8.0 g (24.5 mmol) of intermediate product (H), 19.6 g (88.1 mmol) of 9-phenanthrene boronic acid, and 2.1 g (1.8 mmol) of tetrakis(triphenylphosphine)palladium [Pd(PPh₃)₄] were dissolved in 240 mL of tetrahydrofuran (THF) and added with a solution that 20.3g (146.8 mmol) of potassium carbonate (K₂CO₃) was dissolved in 120 ml of water, and then they were reacted at 90 °C for 12 hours. The solvent of obtained reactants was removed under reduced pressure, and it was rinsed with water and methanol. The residue was recrystallized with toluene, and the precipitated crystal was separated by a filter and rinsed with toluene and dried to provide a white solid of a compound in 12.0 g (yield: 69%). (calculation value: 707.86, measurement value: MS[M+1] 708.26)

### Example 8: Synthesis of compound represented by Chemical Formula C3

As an example of the compound for an organic optoelectronic device, the compound represented by the above Chemical Formula C3 was synthesized through three step processes in accordance with the following Reaction Scheme 8.

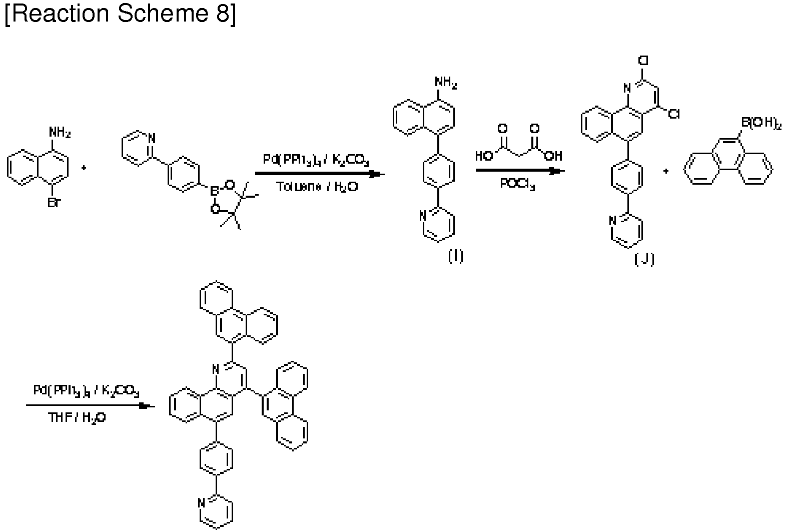

### First step: Synthesis of intermediate product (I)

30.0 g (135.1 mmol) of 1-amino-4-bromonaphthalene, 41.8 g (148.6 mmol) of 2-(4-(4,4,5,5-tetramethyl-1,3,2-dioxaborolan-2-yl)phenyl)pyridine, and 3.9 g (3.4 mmol) of tetrakis(triphenylphosphine)palladium [Pd(PPh₃)₄] were dissolved in a 900 ml of solvent and added with a solution that 37.3 g (270.2 mmol) of potassium carbonate (K₂CO₃) was dissolved in 300 ml of water, and then they were reacted at 85 °C for 12 hours. The water layer of obtained reactant was removed, and the solvent was removed under reduced pressure, and it was rinsed with water and methanol. The obtained solid mixture was separated by a column and dried to provide a yellow solid of an intermediate product (I) in 24.9 g (yield: 62%).

### Second step: Synthesis of intermediate product (J)

24.9 g (84.1 mmol) of intermediate product (I), and 13.1 g (126.2 mmol) of malonic acid were dissolved in 38 mL of phosphorus oxychloride (POCl₃) solvent and reacted at 140 °C for 4 hours. The obtained reactant was poured into ice water and filtered for the formed solid and rinsed with sodium hydrogen carbonate saturated aqueous solution. The obtained solid mixture was rinsed with methanol and dried to provide a pale yellow solid of an intermediate product (J) in 5.6 g (yield: 17%).

### Third step: Synthesis of compound represented by Chemical Formula C3

5.5 g (13.7 mmol) of intermediate product (J), 6.7 g (30.2 mmol) of 9-phenanthrene boronic acid, and 0.8 g (0.1 mmol) of tetrakis(triphenylphosphine)palladium [Pd(PPh₃)₄] were dissolved in 110 mL of a tetrahydrofuran (THF) solvent and added with a solution that 7.6g (54.8 mmol) of potassium carbonate (K₂CO₃) was dissolved in 55 ml of water, and then they were reacted at 90 °C for 12 hours. The solvent of obtained reactants was removed under reduced pressure, and it was rinsed with water and methanol. The residue was recrystallized with toluene, and the precipitated crystal was separated by a filter and rinsed with toluene and dried to provide a white solid of a compound in 6.0 g (yield: 64%). (calculation value: 684.82, measurement value: MS[M+1] 685.25)

### Example 9: Synthesis of compound represented by Chemical Formula A4

As an example of the compound for an organic optoelectronic device, the compound represented by the above Chemical Formula A4 was synthesized in accordance with the following Reaction Scheme 9.

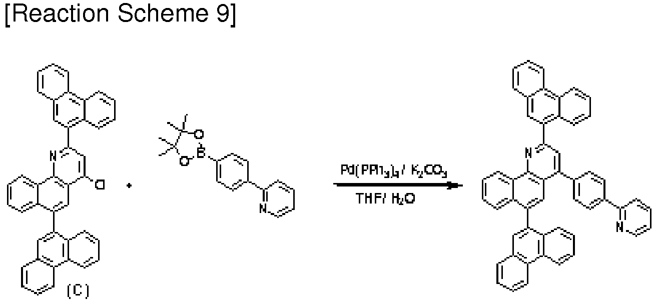

14.9 g (26.3 mmol) of intermediate product (C), 8.9 g (31.6 mmol) of 6-(4-(4,4,5,5-tetramethyl-1,3,2-dioxaborolan-2-yl)phenyl)pyridine, and 0.9 g (0.8 mmol) of tetrakis(triphenylphosphine)palladium [Pd(PPh₃)₄] were dissolved in 300 mL of a tetrahydrofuran (THF) solvent and added with a solution that 7.3 g (52.6 mmol) of potassium carbonate (K₂CO₃) was dissolved in 150 ml of water, and then they were reacted at 90 °C for 12 hours. The solvent of obtained reactants was removed under reduced pressure, and it was rinsed with water and methanol. The residue was recrystallized with toluene, and the precipitated crystal was separated by a filter and rinsed with toluene and dried to provide a white solid of a compound in 13.9 g (yield: 77%). (calculation value: 684.82, measurement value: MS[M+1] 685.25)

### Example 10: Synthesis of compound represented by Chemical Formula B3

As an example of the compound for an organic optoelectronic device, the compound represented by the above Chemical Formula B3 was synthesized through two step processes in accordance with the following Reaction Scheme 10.

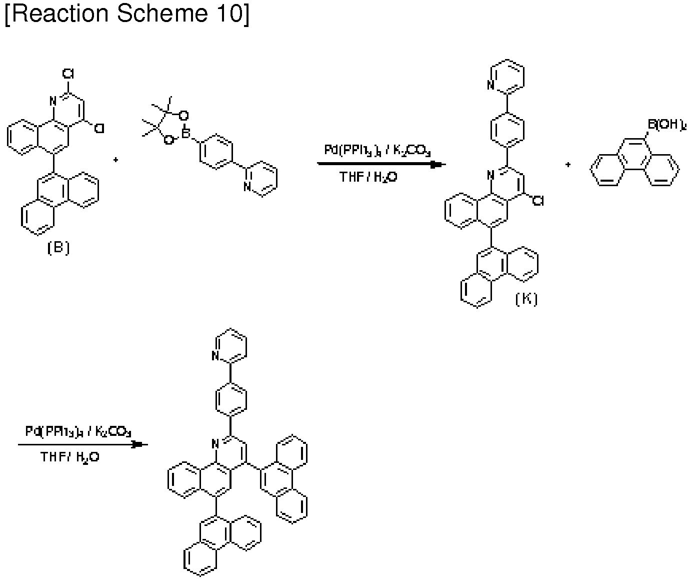

### First step: Synthesis of intermediate product (K)

14.0 g (32.9 mmol) of intermediate product (B), 10.2 g (36.3 mmol) of 6-(4-(4,4,5,5-tetramethyl-1,3,2-dioxaborolan-2-yl)phenyl)pyridine, and 1.1 g (1.0 mmol) of tetrakis(triphenylphosphine)palladium [Pd(PPh₃)₄] were dissolved in 280 ml of a tetrahydrofuran (THF) solvent and added with a solution that 9.1 g (66.0 mmol) of potassium carbonate (K₂CO₃) was dissolved in 140 ml of water, and then they were reacted at 80 °C for 12 hours. The water layer of obtained reactant was removed, and the solvent was removed under reduced pressure, and it was rinsed with water and methanol. The residue was recrystallized with toluene, and the precipitated crystal was separated by a filter and rinsed with toluene and dried to provide a pale yellow solid of intermediate product (K) in 9.7 g (yield: 54%).

### Second step: Synthesis of compound represented by Chemical Formula B3

9.7 g (17.8 mmol) of intermediate product (K), 5.4 g (21.4 mmol) of 9-phenanthrene boronic acid, and 0.6 g (0.5 mmol) of tetrakis(triphenylphosphine)palladium [Pd(PPh₃)₄] were dissolved in 380 mL of a tetrahydrofuran (THF) solvent and added with a solution that 4.9 g (35.6 mmol) of potassium carbonate (K₂CO₃) was dissolved in 95 mL of water, and then they were reacted at 90 °C for 12 hours. The solvent of obtained reactants was removed under reduced pressure, and it was rinsed with water and methanol. The residue was recrystallized with toluene, and the precipitated crystal was separated by a filter and rinsed with toluene and dried to provide a white solid of a compound in 10.0 g (yield: 82%). (calculation value: 684.82, measurement value: MS[M+1] 685.25)

### (Fabrication of organic light emitting diode)

### Example 11

As an anode, ITO having a thickness of 1000 A was used and as a cathode, aluminum (Al) having a thickness of 1000 Å was used.

Specifically, organic light emitting diodes were fabricated as follows: an ITO glass substrate having sheet resistance of 15Ω/cm² was cut to a size of 50mm x 50mm x 0.7mm and ultrasonic wave cleaning the same in acetone, isopropylalcohol and pure water fo 5 minutes each, and UV ozone cleaning the same for 30 minutes to provide an anode.

N1,N1'-(biphenyl-4,4'-diyl)bis(N1-(naphthalen-2-yl)-N4,N4-diphenylbenzene-1,4-diamine) was deposited on the glass substrate to be 10nm thick, and N,N'-di(1-naphthyl)-N,N'-diphenylbenzidine was sequentially deposited to form a 40 nm-thick hole injection layer (HIL).

4 wt% of N,N,N',N'-tetrakis (3,4-dimethylphenyl)chrysene-6,12-diamine and 96 wt% of 9-(3-(naphthalen-1-yl)phenyl)-10-(naphthalen-2-yl)anthracene were deposited to provide a 25 nm-thick emission layer.

Subsequentially, the compound synthesized in Example 1 was deposited to provide a 30 nm-thick electron transport layer (ETL).

Liq was vacuum-deposited on the electron transport layer (ETL) to provide a 0.5 nm-thick electron injection layer (EIL), and Al was vacuum-deposited to form a 100 nm-thick Liq/Al electrode.

### Example 12

An organic light emitting diode was fabricated in accordance with the same procedure as in Example 11, except that the compound synthesized in Example 3 was used for an electron transport layer (ETL) instead of using the compound synthesized from Example 1.

### Example 13

An organic light emitting diode was fabricated in accordance with the same procedure as in Example 11, except that the compound synthesized in Example 5 was used for an electron transport layer (ETL) instead of using the compound synthesized from Example 1.

### Example 14

An organic light emitting diode was fabricated in accordance with the same procedure as in Example 11, except that the compound synthesized in Example 7 was used for an electron transport layer (ETL) instead of using the compound synthesized from Example 1.

### Example 15

An organic light emitting diode was fabricated in accordance with the same procedure as in Example 11, except that the compound synthesized in Example 8 was used for an electron transport layer (ETL) instead of using the compound synthesized from Example 1.

### Example 16

An organic light emitting diode was fabricated in accordance with the same procedure as in Example 11, except that the compound synthesized in Example 9 was used for an electron transport layer (ETL) instead of using the compound synthesized from Example 1.

### Example 17

An organic light emitting diode was fabricated in accordance with the same procedure as in Example 11, except that the compound synthesized in Example 10 was used for an electron transport layer (ETL) instead of using the compound synthesized from Example 1.

### Example 18

An organic light emitting diode was fabricated in accordance with the same procedure as in Example 11, except that the compound synthesized in Example 1 and Liq at 1:1 (a ratio of weight) were deposited for an electron transport layer (ETL).

### Example 19

An organic light emitting diode was fabricated in accordance with the same procedure as in Example 11, except that the compound synthesized in Example 3 and Liq at 1:1 were deposited for an electron transport layer (ETL).

### Example 20

An organic light emitting diode was fabricated in accordance with the same procedure as in Example 11, except that the compound synthesized in Example 5 and Liq at 1:1 were deposited for an electron transport layer (ETL).

### Example 21

An organic light emitting diode was fabricated in accordance with the same procedure as in Example 11, except that the compound synthesized in Example 7 and Liq at 1:1 were deposited for an electron transport layer (ETL).

### Example 22

An organic light emitting diode was fabricated in accordance with the same procedure as in Example 11, except that the compound synthesized in Example 8 and Liq at 1:1 were deposited for an electron transport layer (ETL).

### Example 23

An organic light emitting diode was fabricated in accordance with the same procedure as in Example 11, except that the compound synthesized in Example 9 and Liq at 1:1 were deposited for an electron transport layer (ETL).

### Example 24

An organic light emitting diode was fabricated in accordance with the same procedure as in Example 11, except that the compound synthesized in Example 10 and Liq at 1:1 were deposited for an electron transport layer (ETL).

### Comparative Example 1

An organic light emitting diode was fabricated in accordance with the same procedure as in Example 11, except that the compound represented by the following Chemical Formula 3 was used for an electron transport layer (ETL) instead of using the compound synthesized from Example 1.

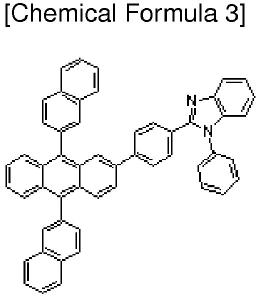

### Comparative Example 2

An organic light emitting diode was fabricated in accordance with the same procedure as in Example 18, except that the compound represented by the above Chemical Formula 3 was used for an electron transport layer (ETL) instead of using the compound synthesized from Example 1.

### (Measurement of performance of organic light emitting diode)

### Experimental Examples

Each organic light emitting diode according to the Example 13, 15, 16, 17, 20, and 23, and Comparative Example 1 and 2 was measured for current density change depending upon the voltage, luminance change, and luminous efficiency. Spcific measurement methods were as follows and the results are shown in the following Table 1.

### (1) Measurement of current density change depending on voltage change

The fabricated organic light emitting diodes were measured for current value flowing in the unit device while increasing the voltage from 0V to 10V using a current-voltage meter (Keithley 2400), and the measured current value was divided by area to provide the result.

### (2) Measurement of luminance change depending on voltage change

The fabricated organic light emitting diodes were measured for luminance while increasing the voltage form 0V to 10V using a luminance meter (Minolta Cs-1000A).

### (3) Measurement of Luminous efficiency

Current efficiency (cd/A) and electric power efficiency (Im/W) at the same luminance (1000cd/m²) were calculated by using luminance and current density from the item (1) and (2) and voltage.

**Table 1**

| | luminance at 500 cd/m² | | | | |
|---|---|---|---|---|---|
| | Driving voltage | Luminous efficiency | Electric power efficiency | CIE chromaticity | |
| | (V) | (cd/A) | (Im/W) | X | y |
| Example 13 | 4.4 | 7.4 | 5.3 | 0.14 | 0.05 |
| Example 15 | 3.9 | 5.4 | 4.3 | 0.14 | 0.05 |
| Example 16 | 4.5 | 7.6 | 5.4 | 0.14 | 0.05 |
| Example 17 | 4.2 | 6.2 | 4.6 | 0.14 | 0.05 |
| **Comparative Example 1** | **5.1** | **3.7** | **2.3** | **0.14** | **0.05** |
| Example 20 | 3.8 | 7.5 | 6.2 | 0.14 | 0.04 |
| Example 23 | 3.8 | 8.2 | 6.9 | 0.14 | 0.05 |
| **Comparative Example 2** | **4.2** | **5.4** | **4.1** | **0.14** | **0.05** |

As shown in Table 1, it is confirmed that the organic light emitting diodes according to Examples 13, 15, 16, and 17 had lower driving voltages and more excellent luminous efficiency and electric power efficiency than those of Comparative Example 1.
In addition, it is also confirmed that the organic light emitting diodes according to Examples 20 and 23 had lower driving voltage and more excellent luminous efficiency and electric power efficiency than those of Comparative Example 2.
While this invention has been described in connection with what is presently considered to be practical exemplary embodiments, the aforementioned embodiments should be understood to be exemplary but not limiting the present invention in any way.

### <Description of symbols>

100: organic photoelectric device 110: cathode 120: anode 105: organic thin layer 130: emission layer 140: hole transport layer (HTL) 150: electron transport layer (ETL) 160: electron injection layer (EIL) 170: hole injection layer (HIL) 230: emission layer + electron transport layer (ETL)

## Claims

1. A compound for an organic optoelectronic device represented by the following Chemical Formula 2: 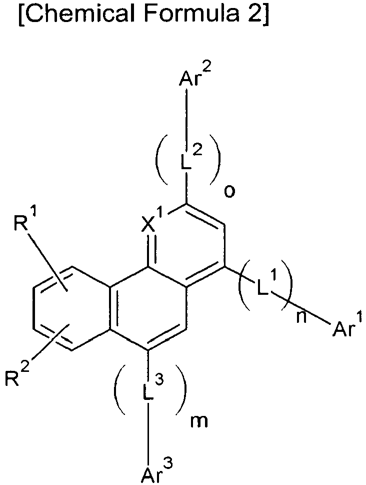 whereby in Chemical Formula 2,
X¹ is -N-
R¹ and R² are the same or different and independently hydrogen, deuterium, a substituted or unsubstituted C1 to C20 alkyl group, a substituted or unsubstituted C6 to C30 aryl group, a substituted or unsubstituted C3 to C30 heteroaryl group, or a combination thereof,
Ar¹ to Ar³ are the same or different and independently substituted or unsubstituted C6 to C30 aryl group; or a substituted or unsubstituted C3 to C30 heteroaryl group,
L¹ to L³ are the same or different and independently a single bond, a substituted or unsubstituted C2 to C6 alkenyl group, a substituted or unsubstituted C2 to C6 alkynyl group, a substituted or unsubstituted C6 to C30 arylene group, a substituted or unsubstituted C3 to C30 heteroarylene group, or a combination thereof, and
n, m or o are the same or different and independently 0 or 1.

2. The compound for an organic optoelectronic device of claim 1, wherein at least one of Ar¹ or Ar² may be a substituted or unsubstituted C3 to C30 heteroaryl group.

3. The compound for an organic optoelectronic device of claim 1, wherein Ar¹ is a substituted or unsubstituted C3 to C30 heteroaryl group, and
Ar² and Ar³ are a substituted or unsubstituted C6 to C30 aryl group

4. The compound for an organic optoelectronic device of claim 1, wherein Ar² is a substituted or unsubstituted C3 to C30 heteroaryl group, and
Ar¹ and Ar³ are a substituted or unsubstituted C6 to C30 aryl group.

5. The compound for an organic optoelectronic device of claim 1, wherein the substituted or unsubstituted C3 to C30 heteroaryl group is a substituted or unsubstituted imidazolyl group, a substituted or unsubstituted triazolyl group, a substituted or unsubstituted tetrazolyl group, a substituted or unsubstituted carbazolyl group, a substituted or unsubstituted oxadiazolyl group, a substituted or unsubstituted oxatriazolyl group, a substituted or unsubstituted thiatriazolyl group, a substituted or unsubstituted benzimidazolyl group, a substituted or unsubstituted benzotriazolyl group, a substituted or unsubstituted pyridinyl group, a substituted or unsubstituted pyrimidinyl group, a substituted or unsubstituted triazinyl group, a substituted or unsubstituted pyrazinyl group, a substituted or unsubstituted pyridazinyl group, a substituted or unsubstituted purinyl group, a substituted or unsubstituted quinolinyl group, a substituted or unsubstituted isoquinolinyl group, a substituted or unsubstituted phthalazinyl group, a substituted or unsubstituted naphpyridinyl group, a substituted or unsubstituted quinoxalinyl group, a substituted or unsubstituted quinazolinyl group, a substituted or unsubstituted acridinyl group, a substituted or unsubstituted phenanthrolinyl group, a substituted or unsubstituted phenazinyl group, or a combination thereof.

6. The compound for an organic optoelectronic device of claim 1, wherein the substituted or unsubstituted C6 to C30 aryl group is a substituted or unsubstituted phenyl group, a substituted or unsubstituted naphthyl group, a substituted or unsubstituted triperylenyl group, a substituted or unsubstituted fluorenyl group, a substituted or unsubstituted spirofluorenyl group, a substituted or unsubstituted biphenyl group, a substituted or unsubstituted terphenyl group, a substituted or unsubstituted pyrenyl group, a substituted or unsubstituted perylenyl group, a substituted or unsubstituted phenanthrenyl group, a substituted or unsubstituted anthracenyl group, or a combination thereof.

7. A compound for an organic optoelectronic device of Claims 1 represented by the following Chemical Formulae A1 to A126:

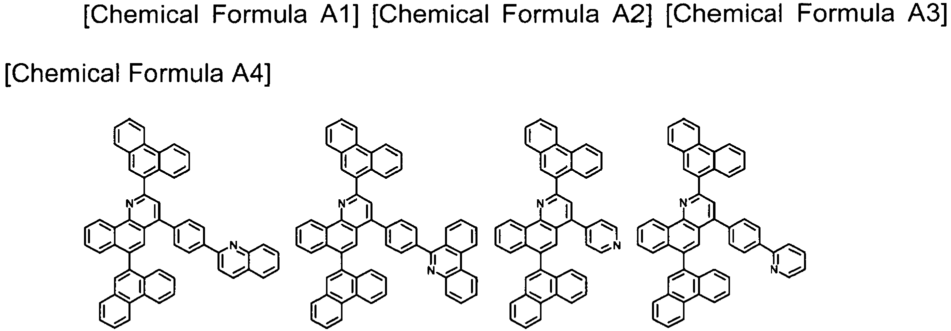

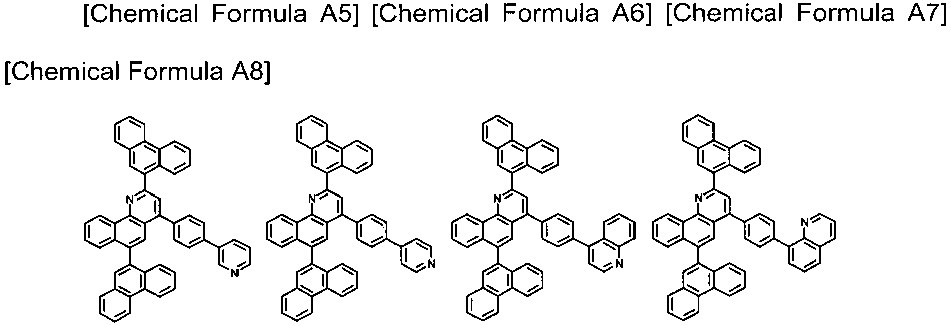

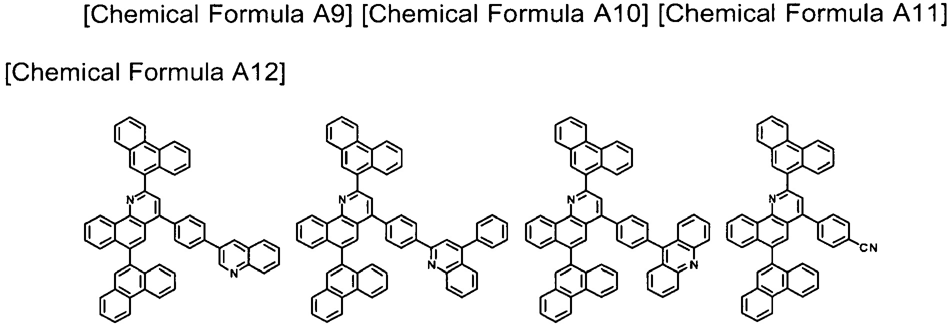

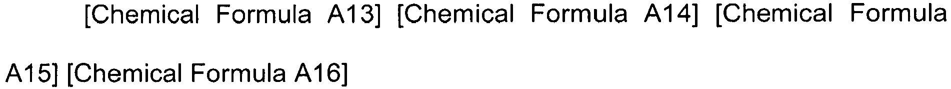

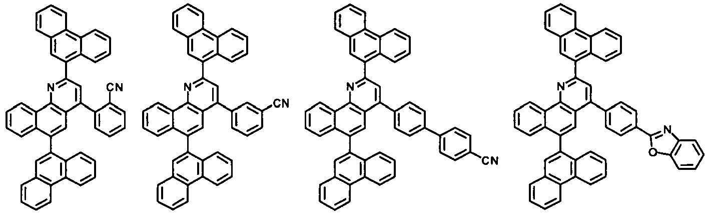

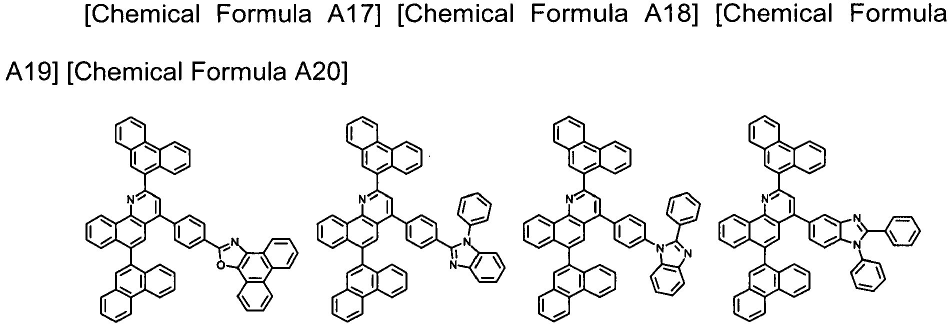

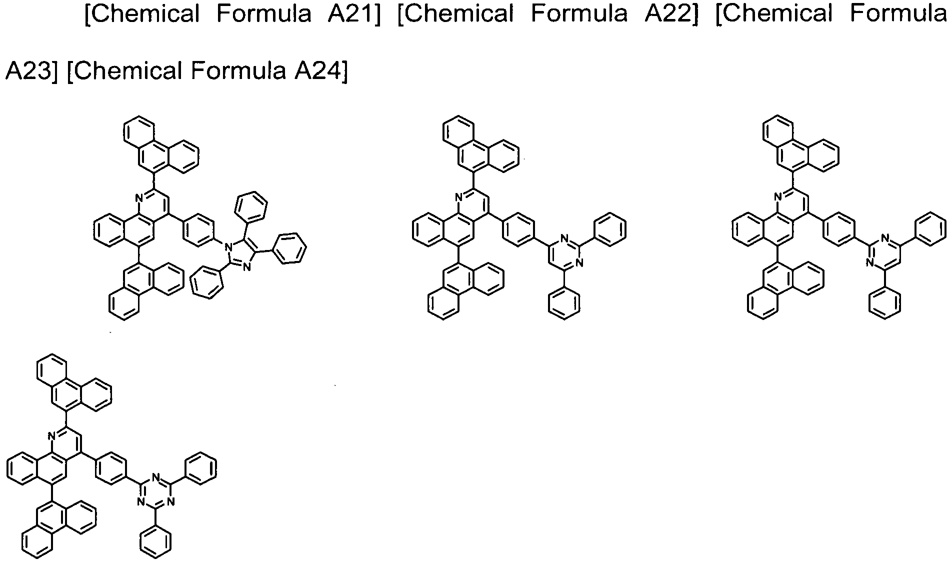

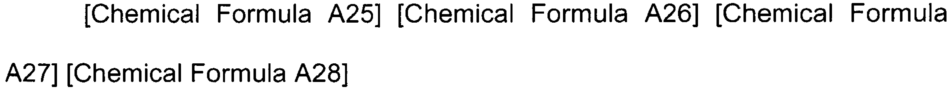

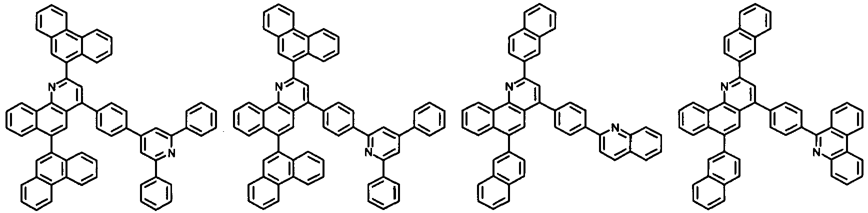

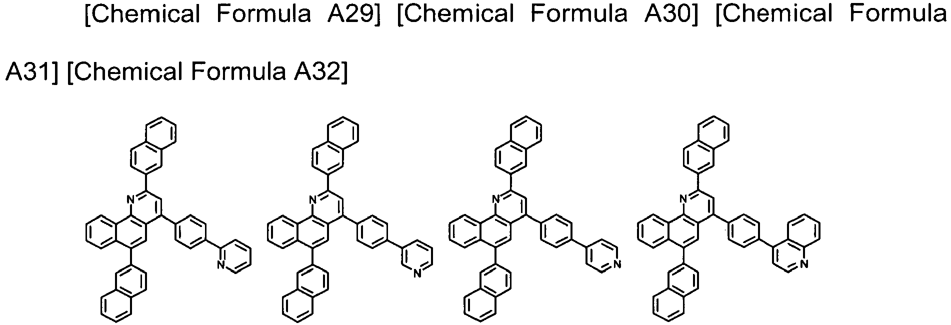

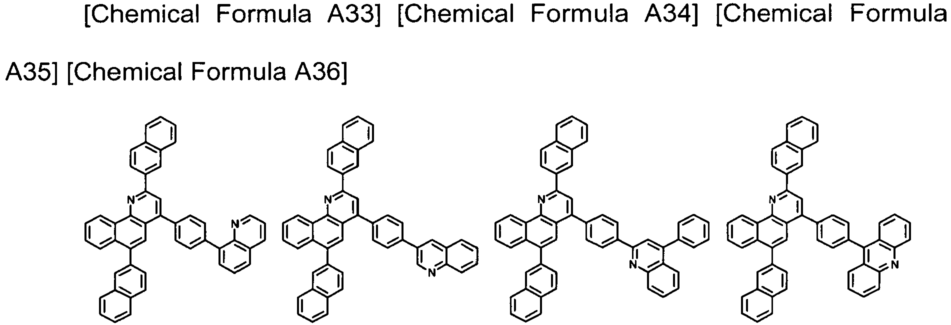

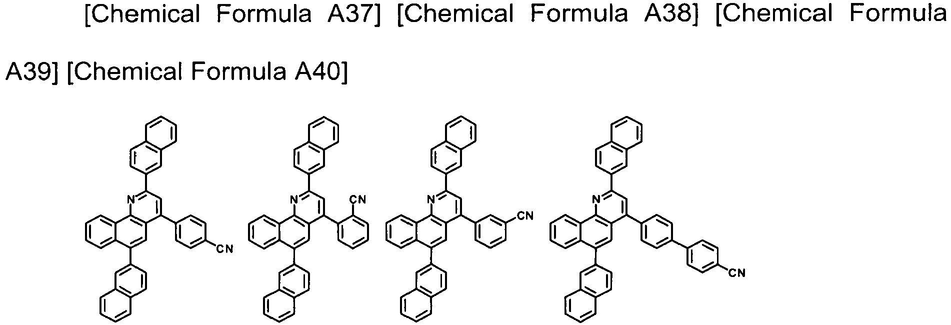

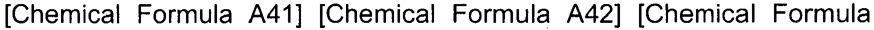

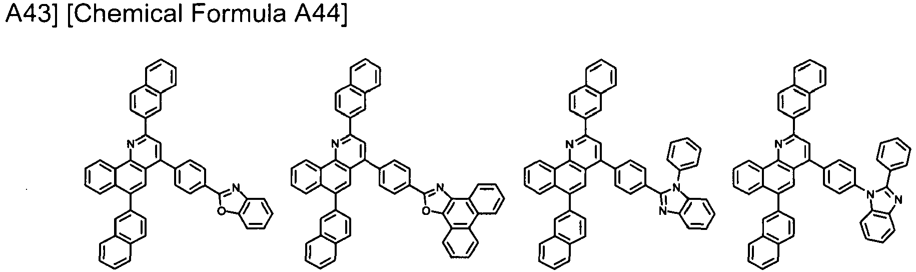

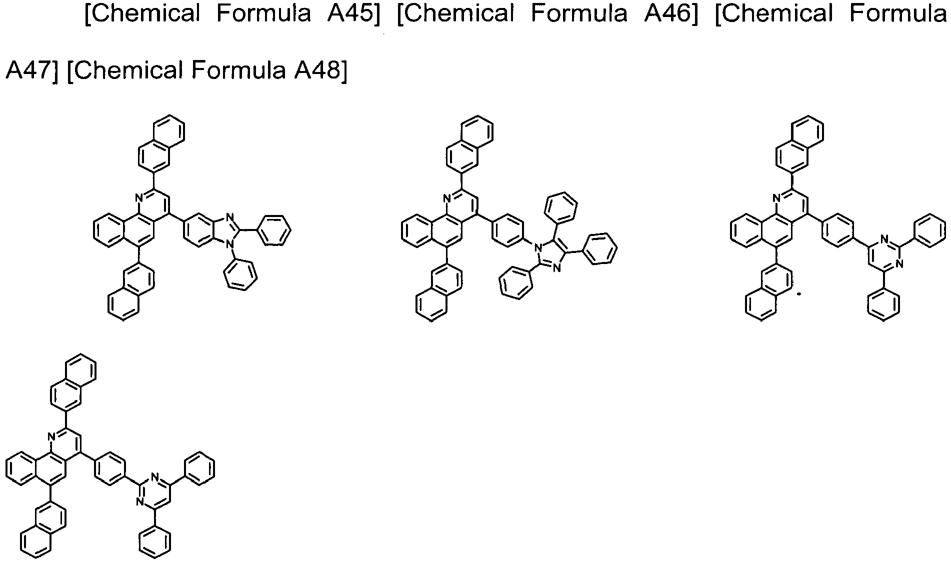

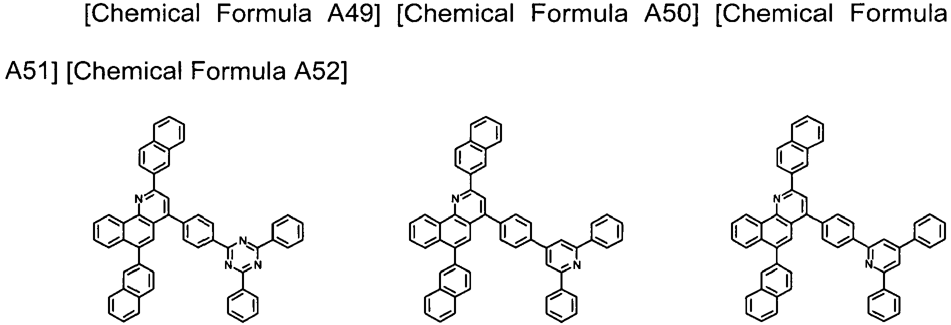

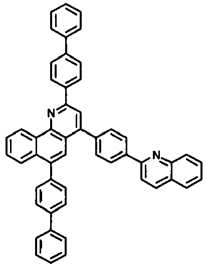

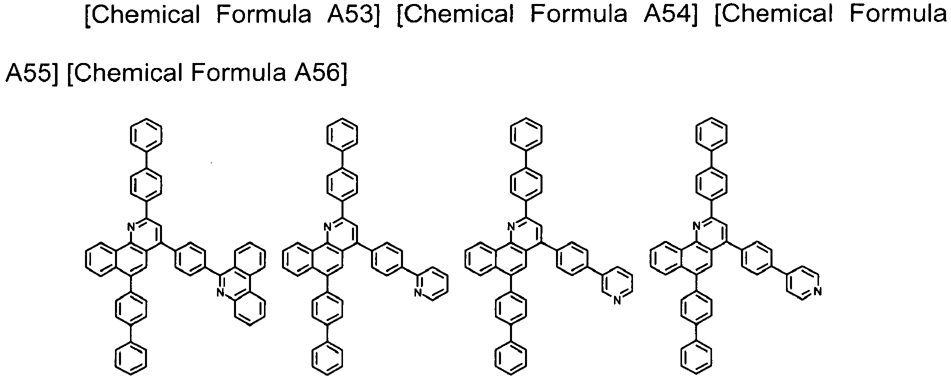

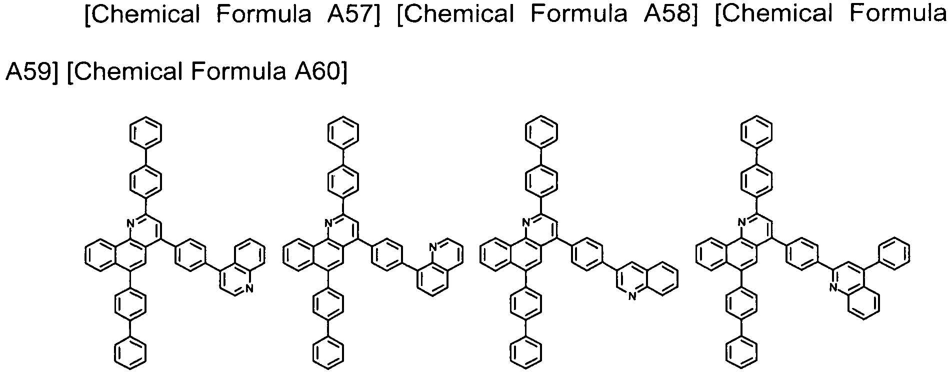

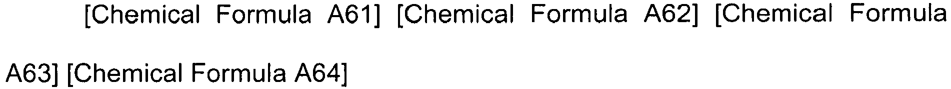

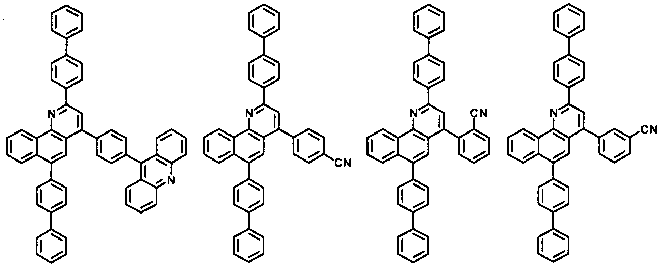

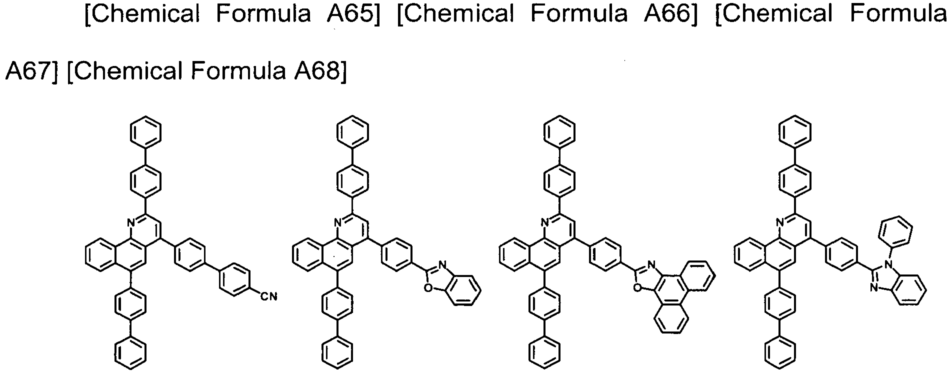

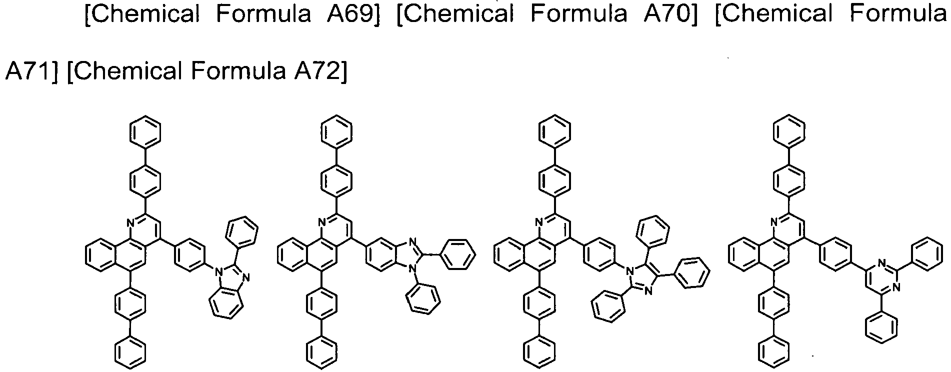

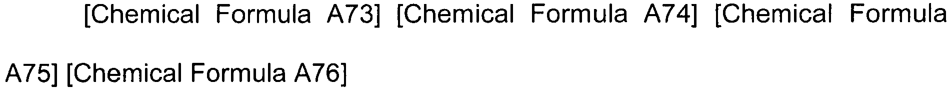

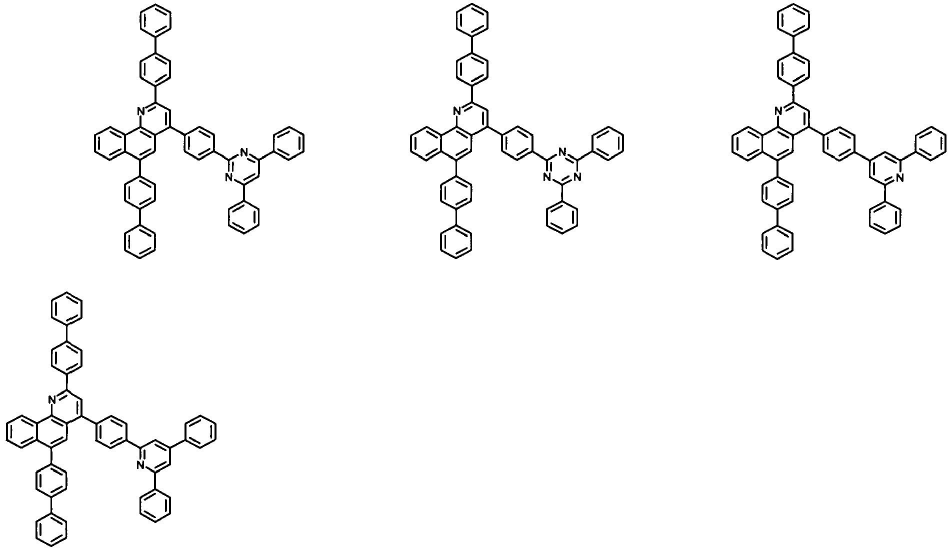

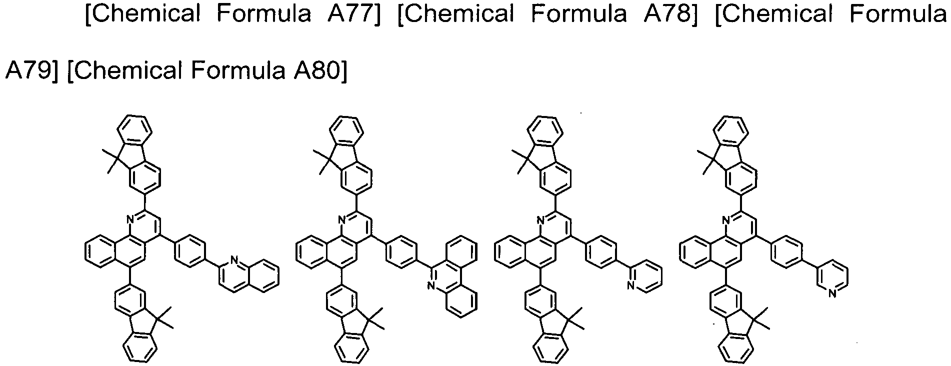

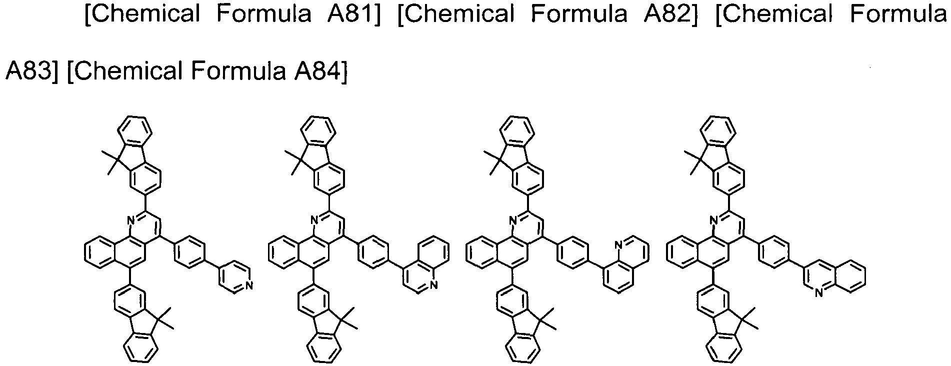

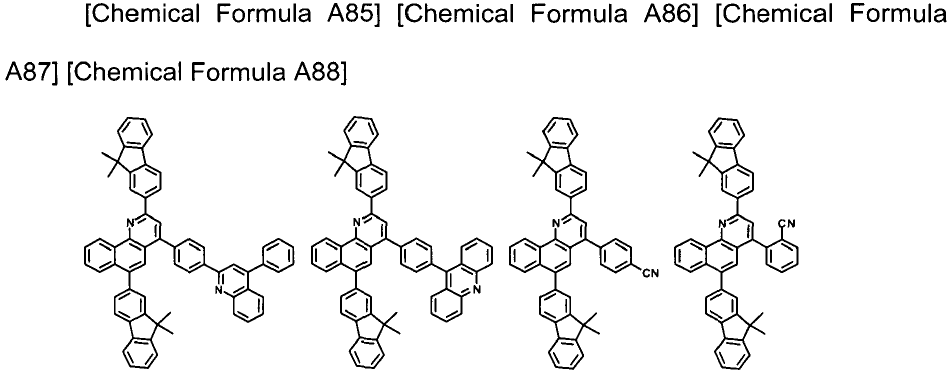

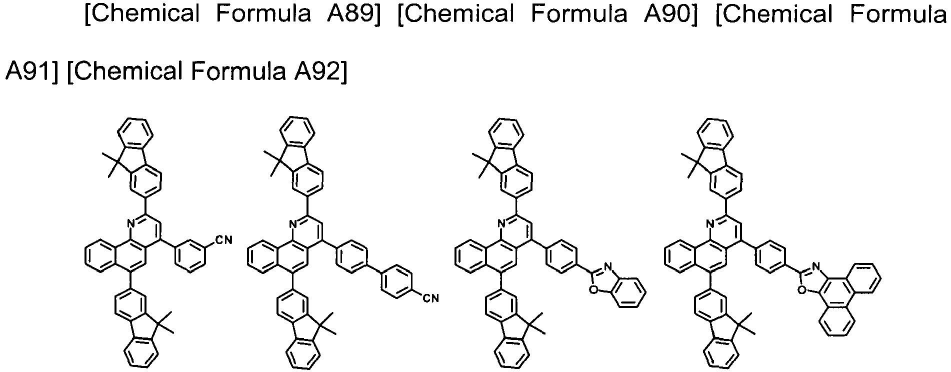

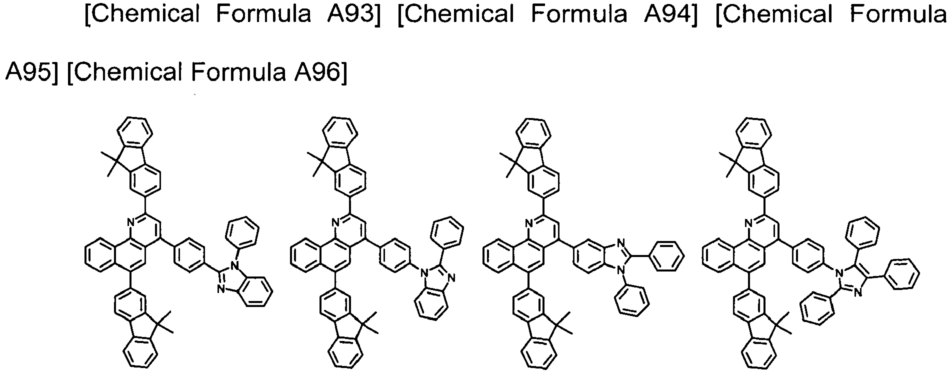

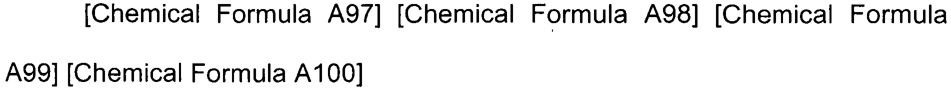

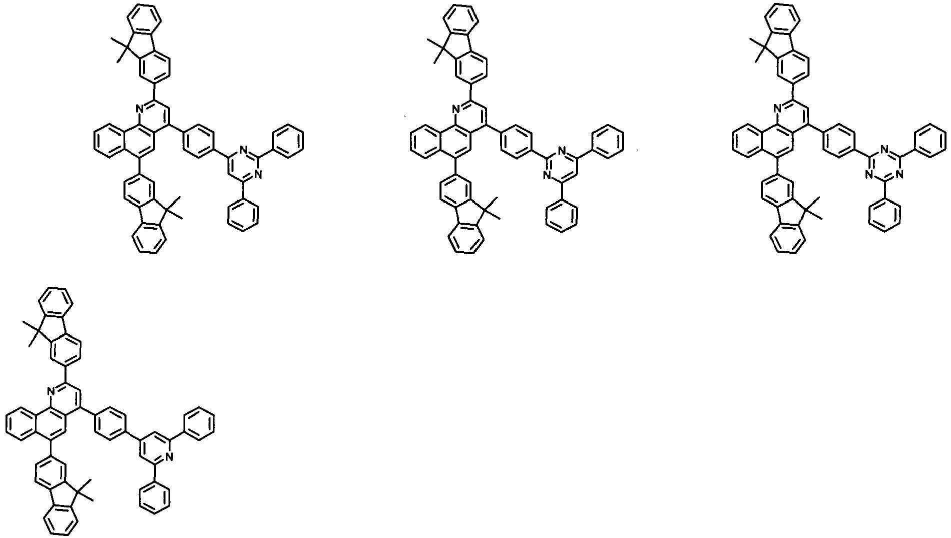

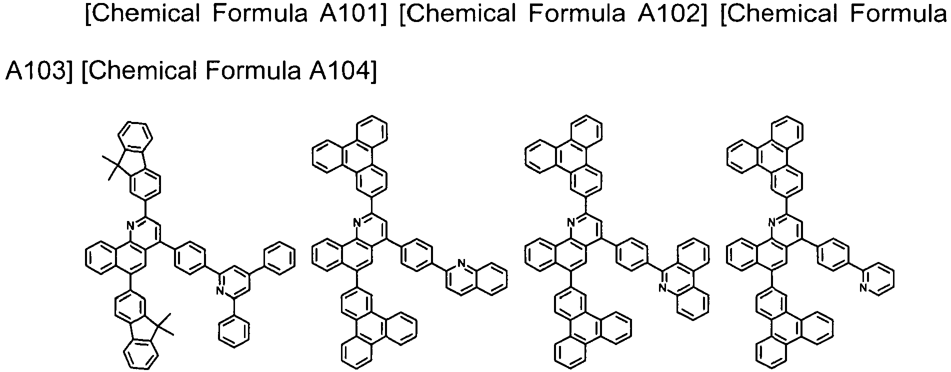

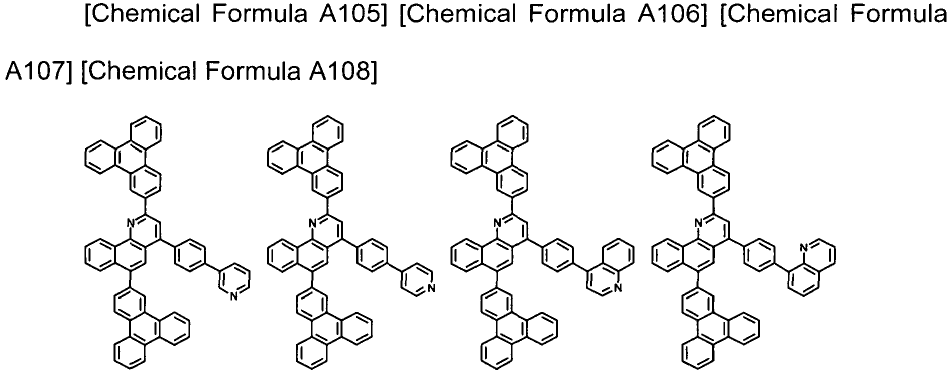

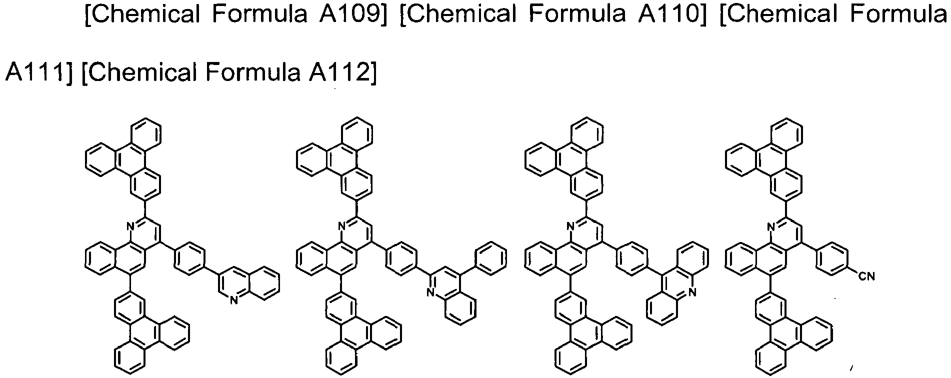

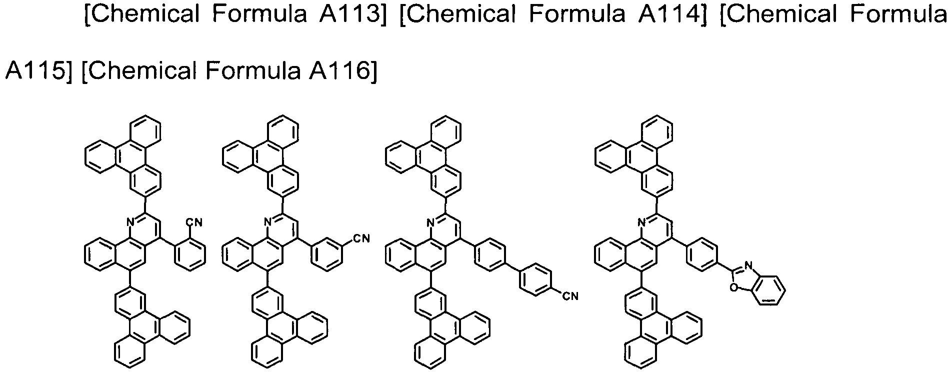

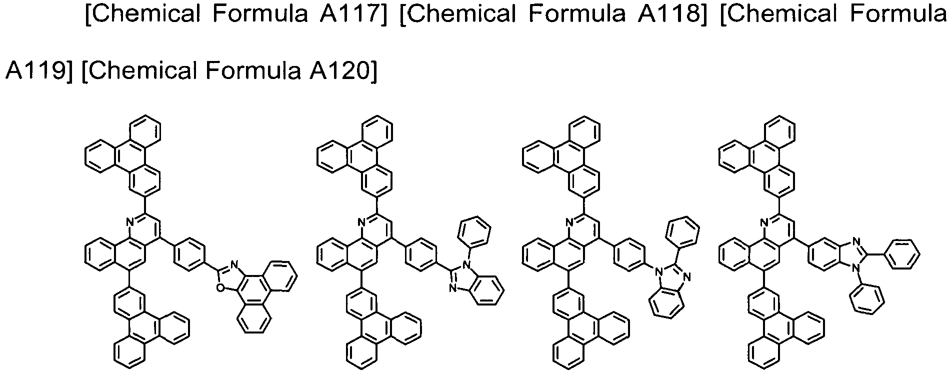

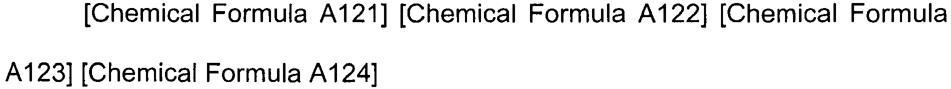

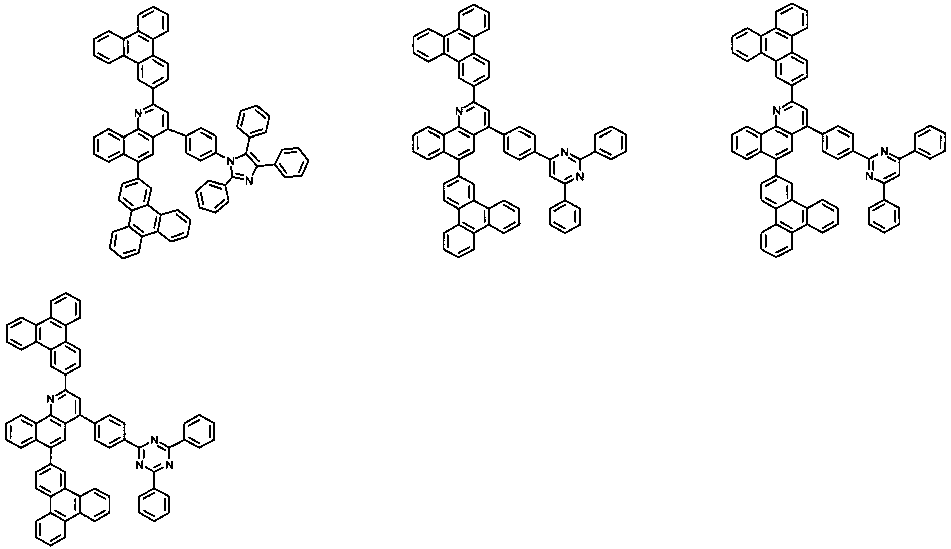

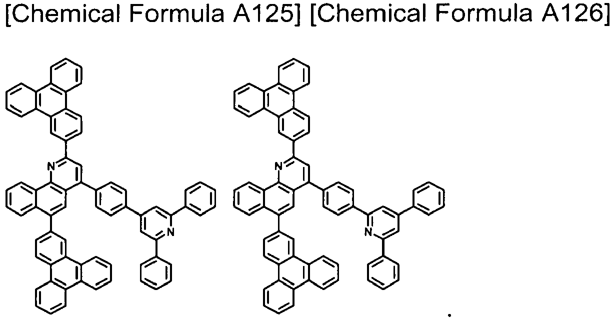

8. A compound for an organic optoelectronic device of claim 1 represented by the following Chemical Formulae B1 to B125:

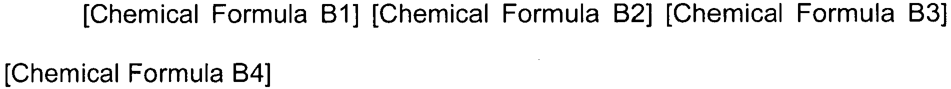

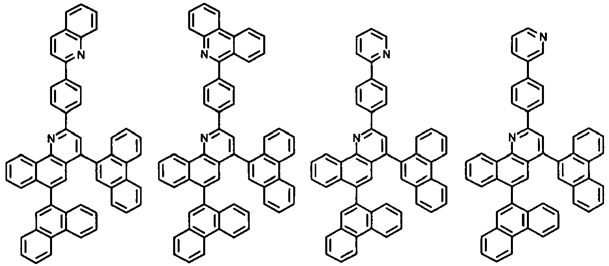

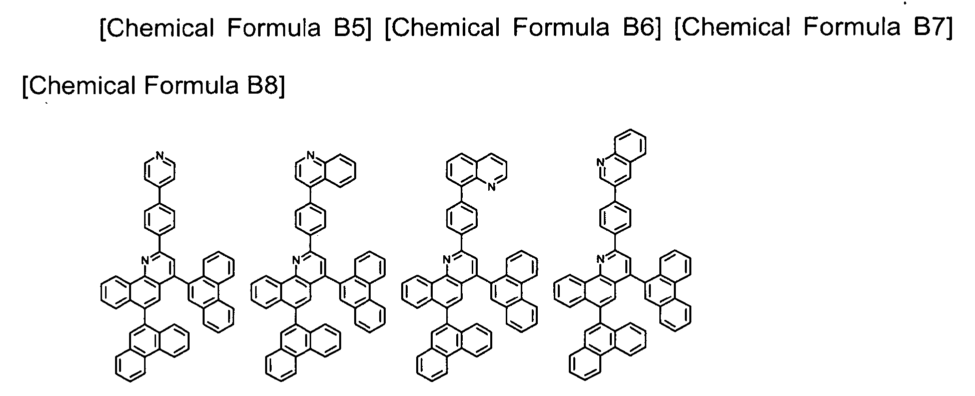

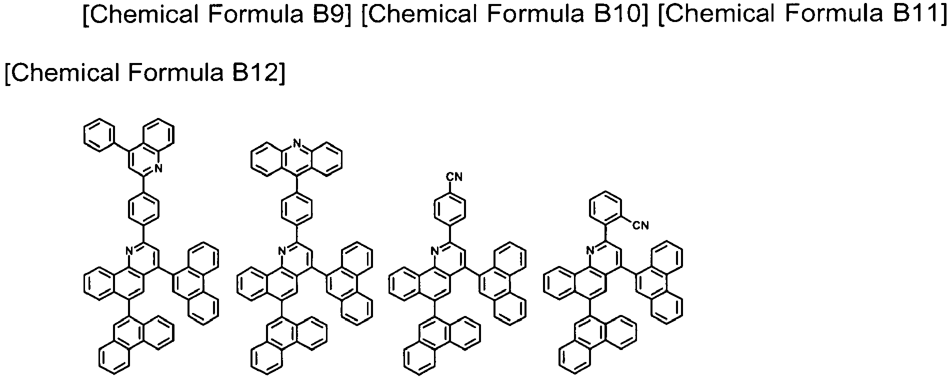

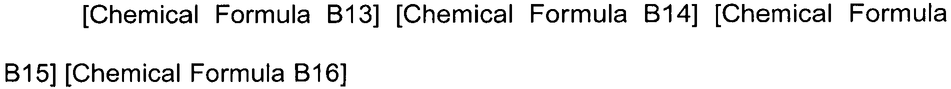

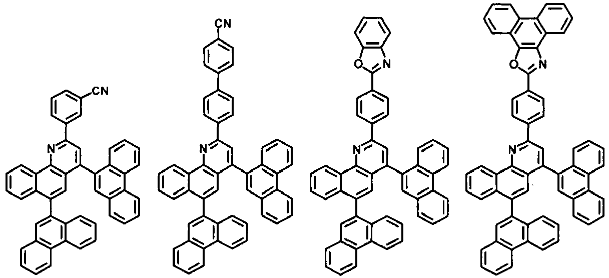

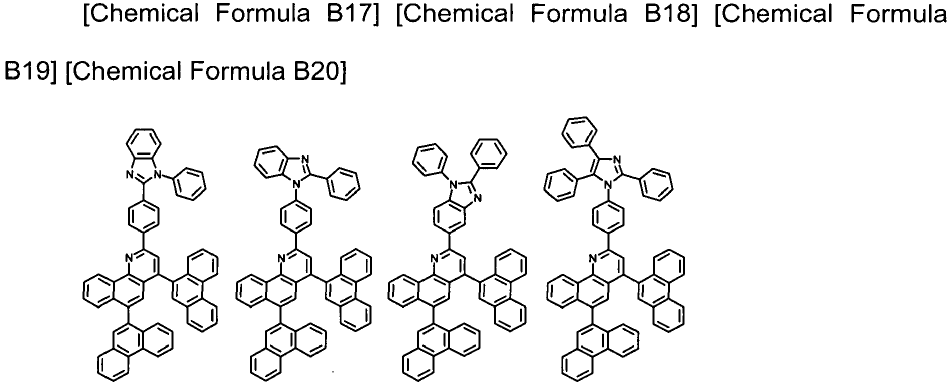

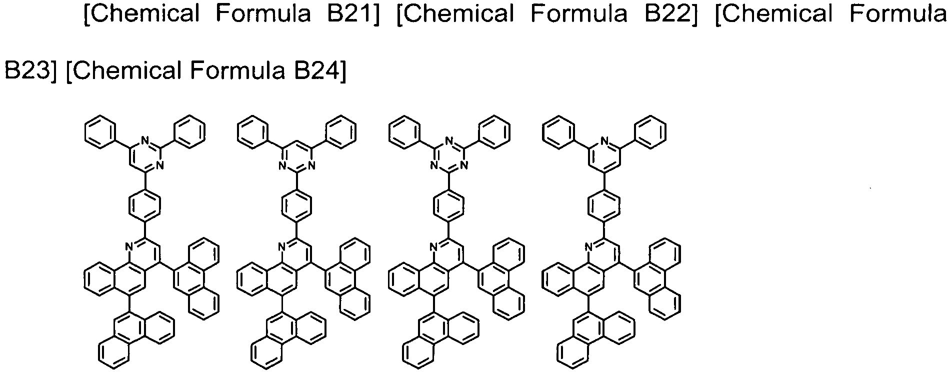

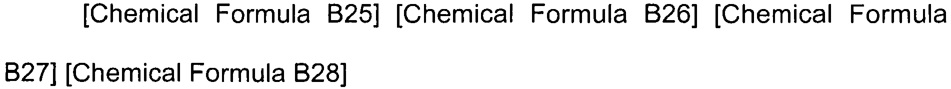

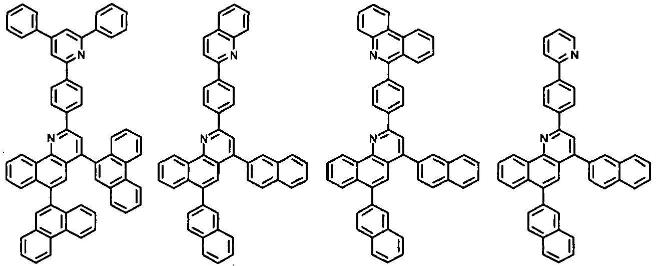

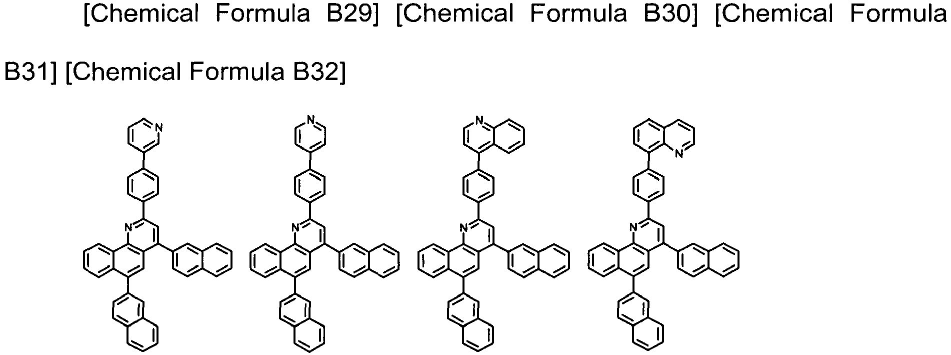

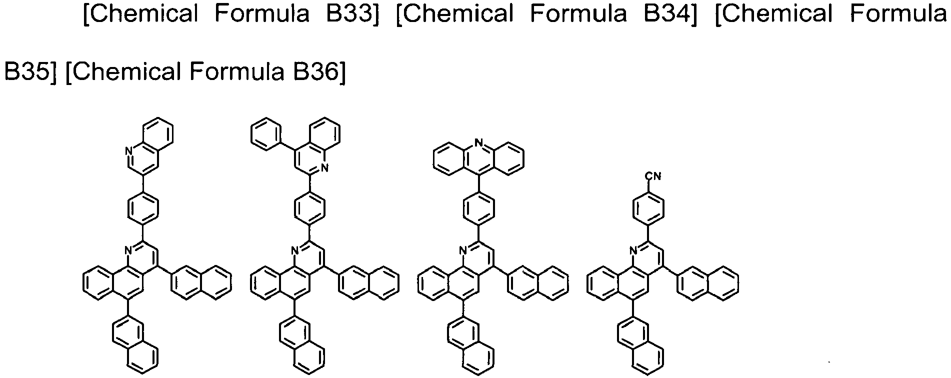

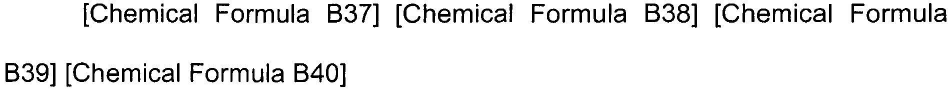

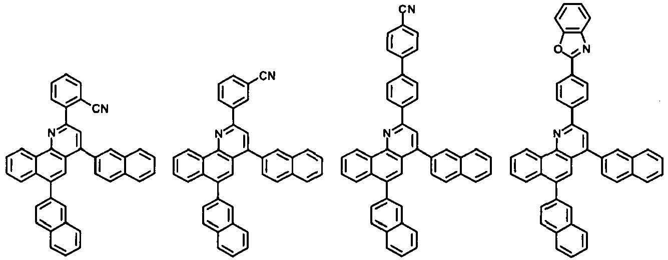

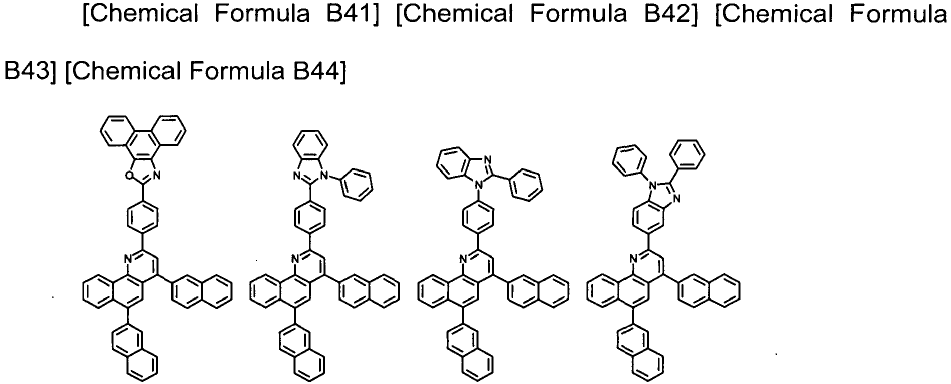

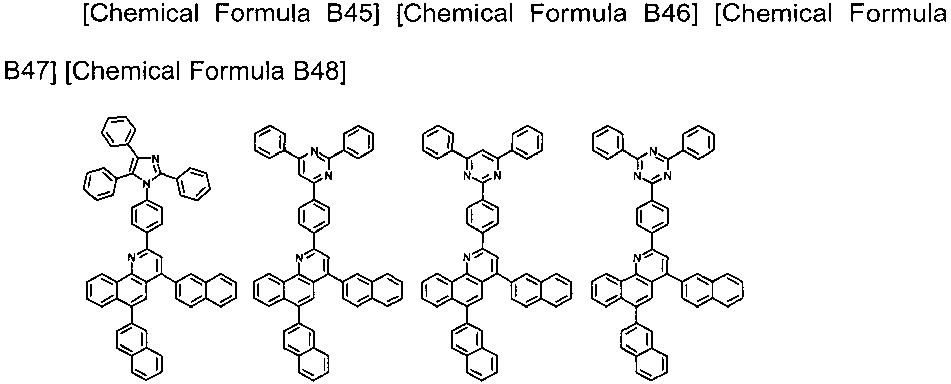

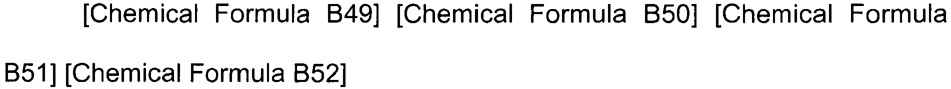

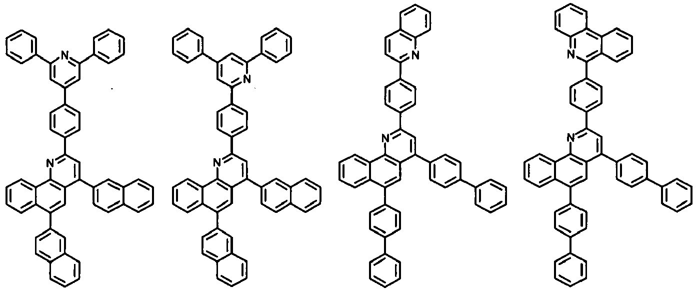

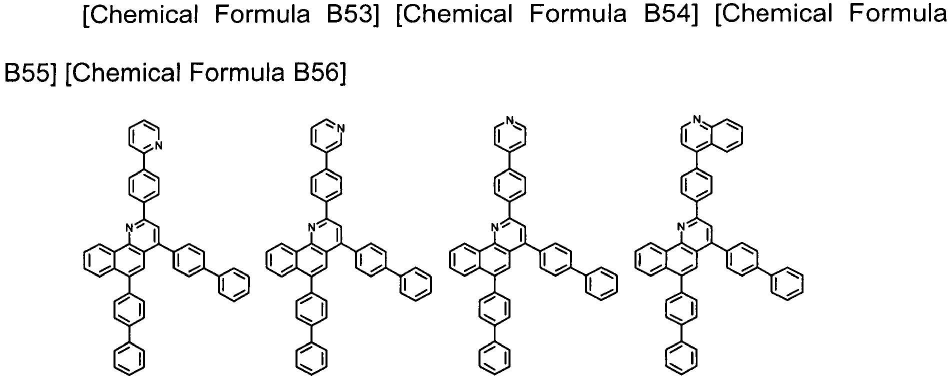

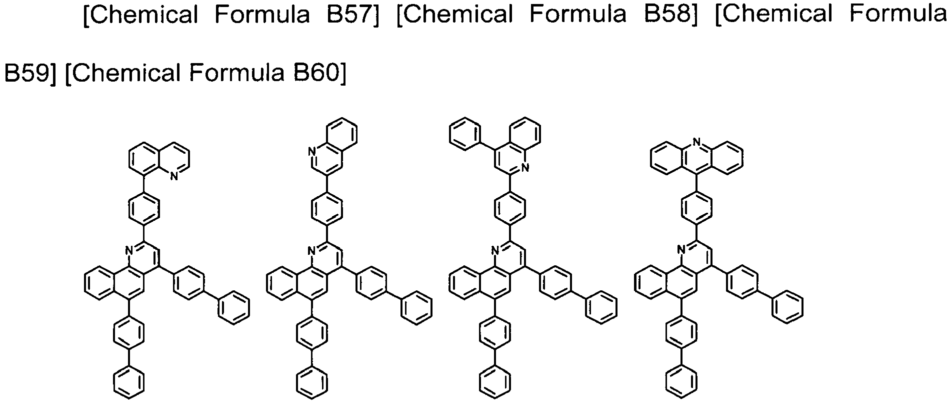

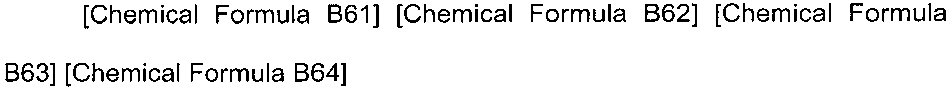

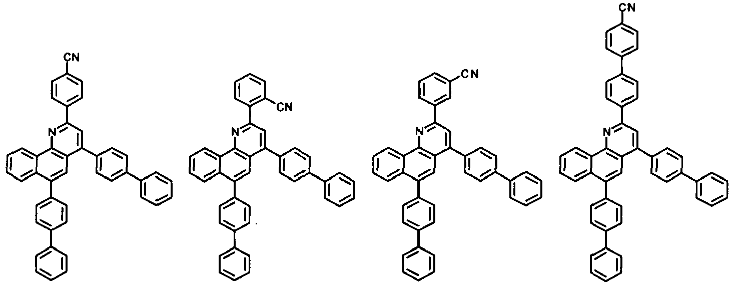

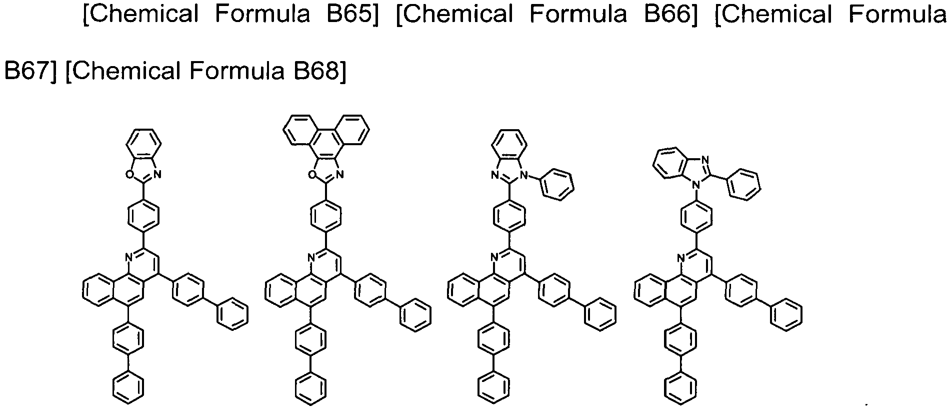

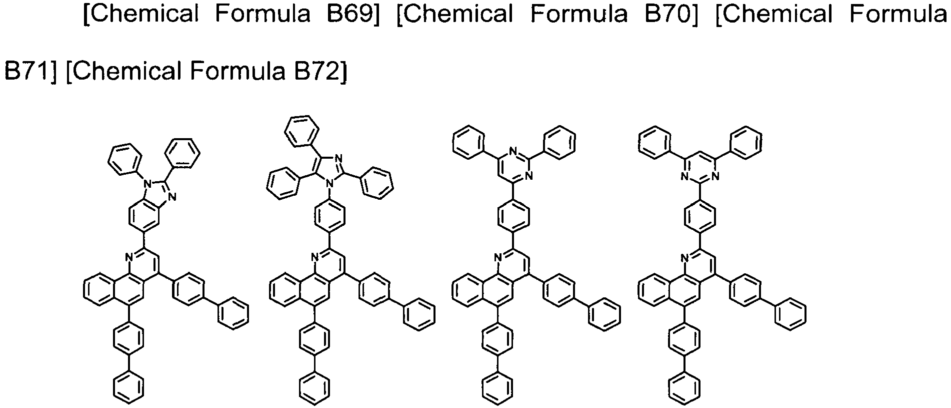

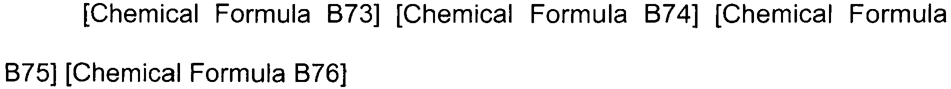

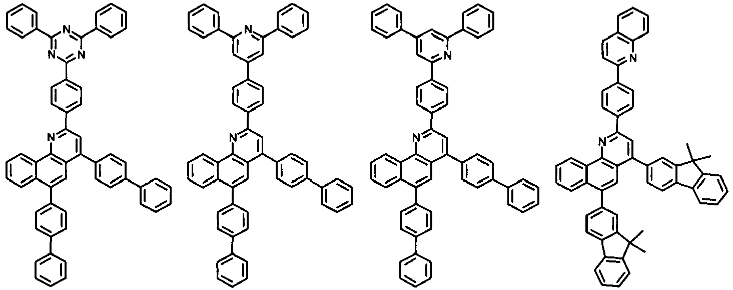

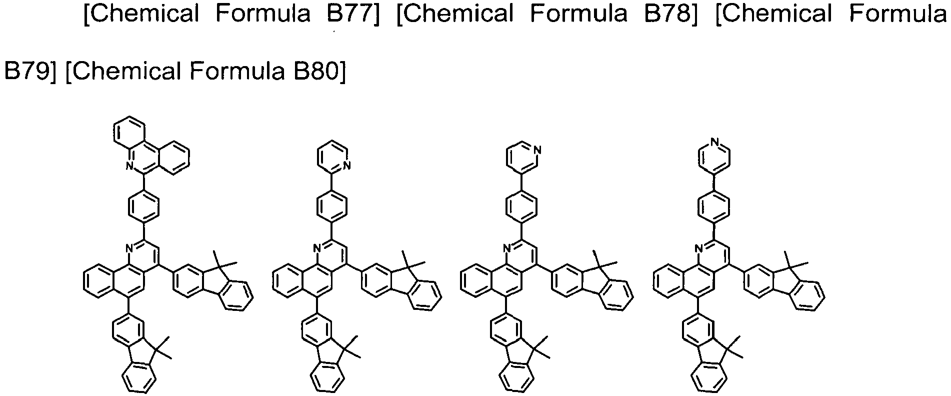

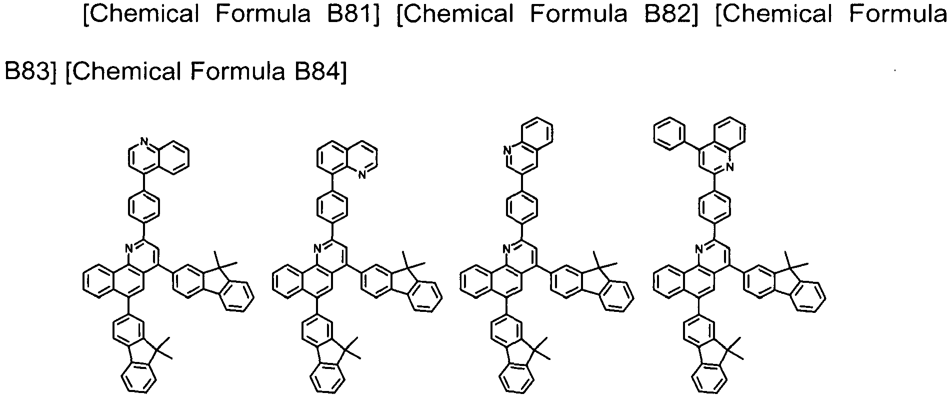

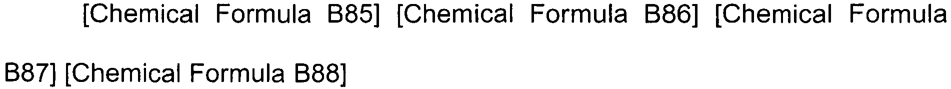

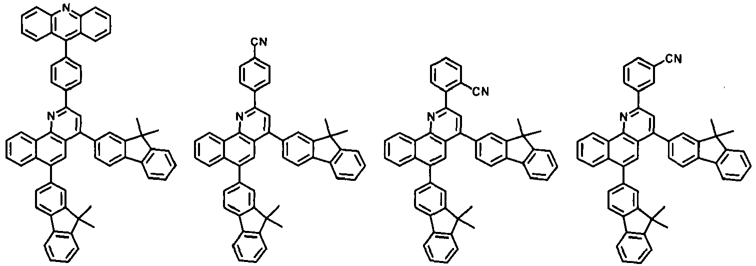

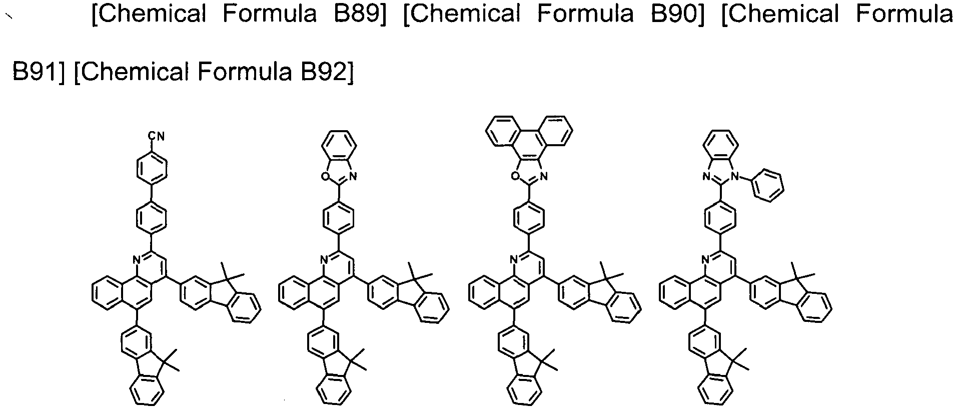

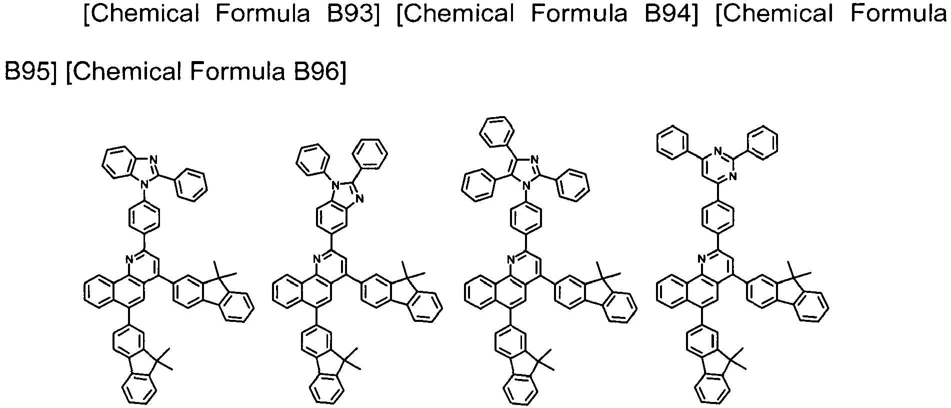

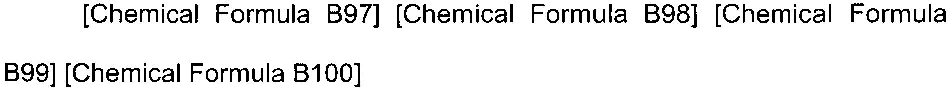

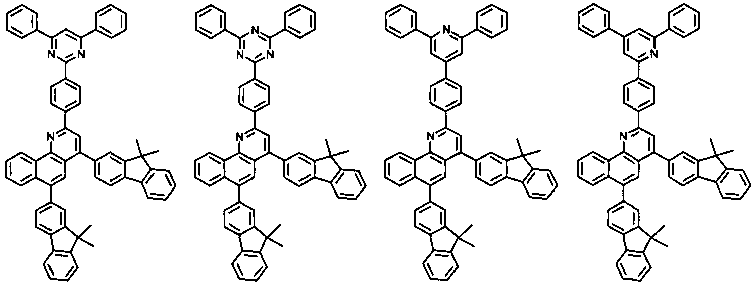

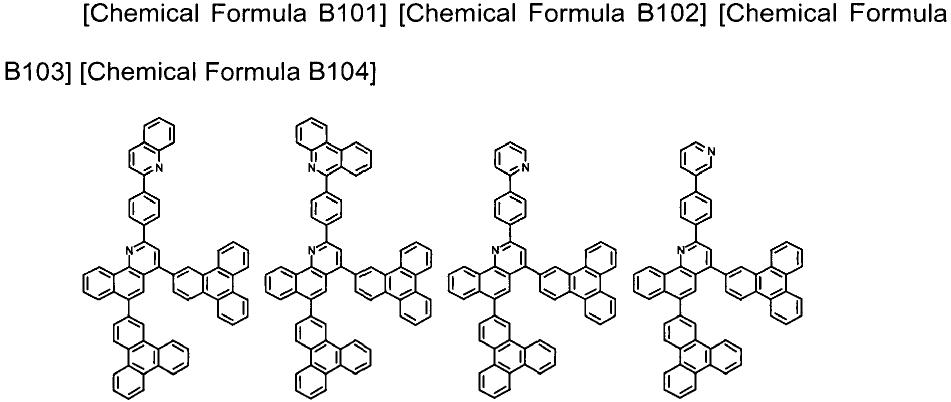

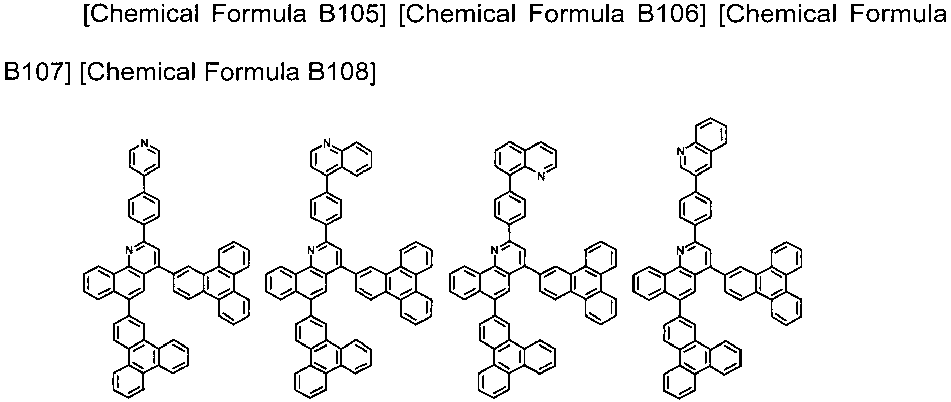

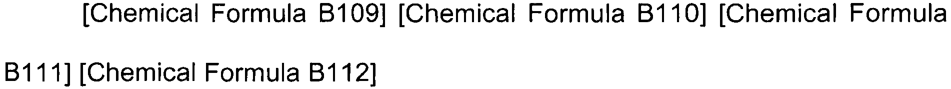

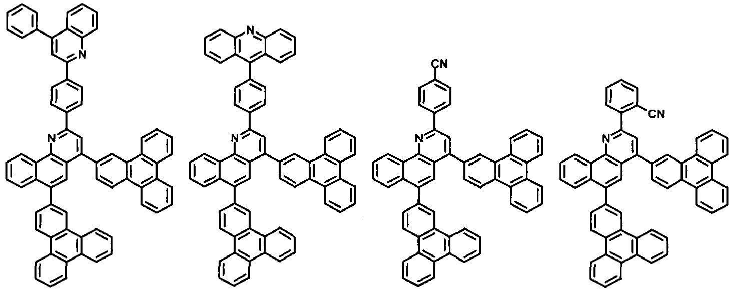

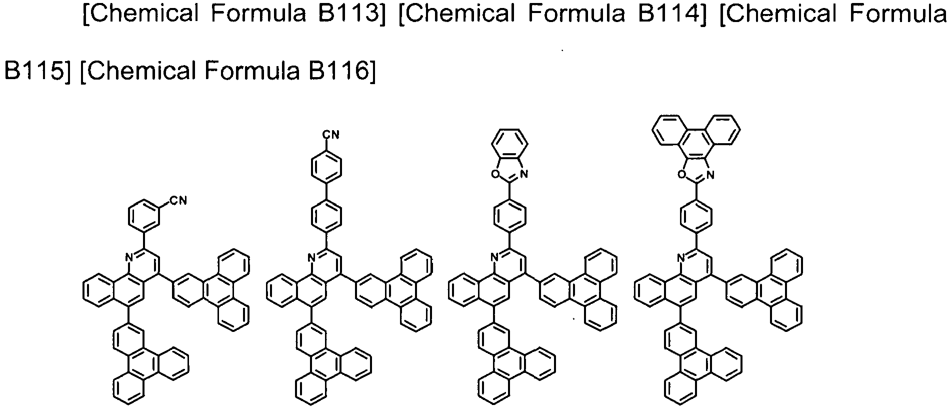

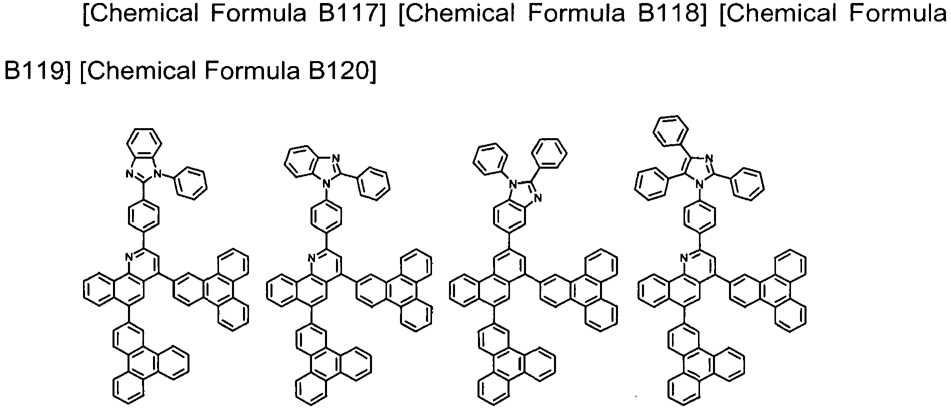

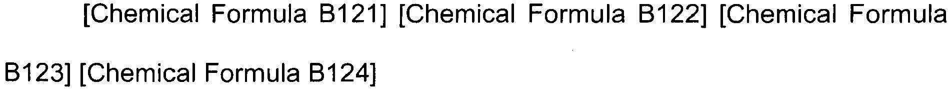

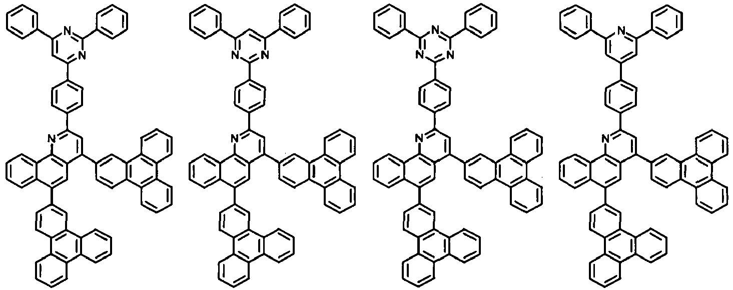

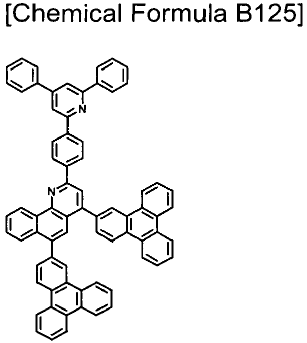

9. A compound for an organic optoelectronic device of claim 1 represented by one of the following Chemical Formulae C1 to C127:

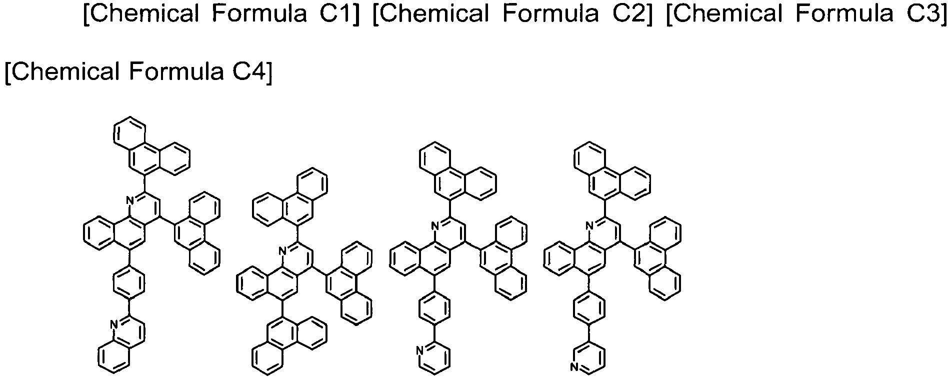

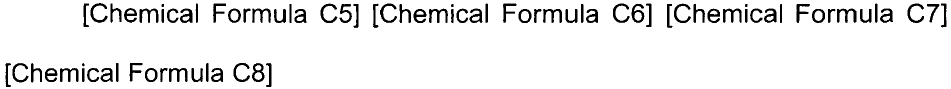

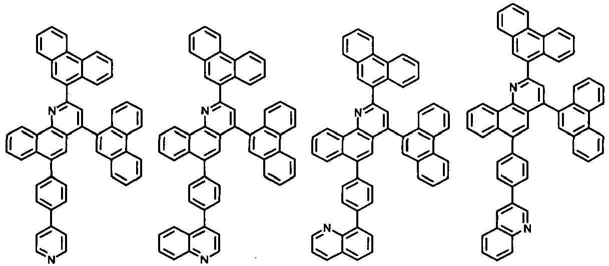

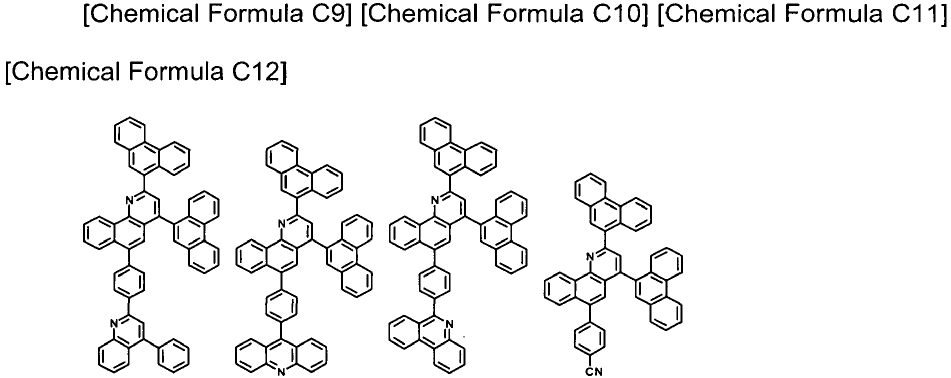

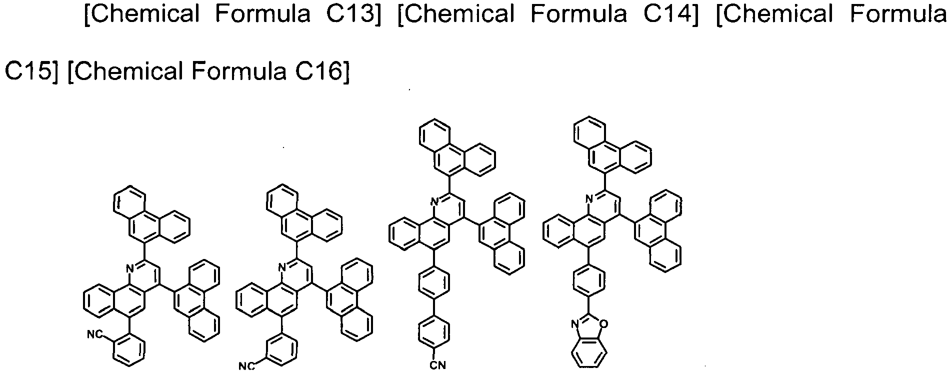

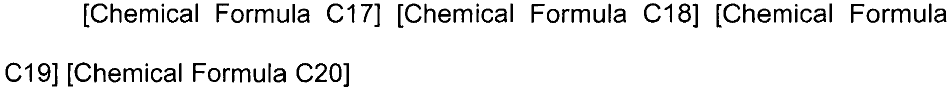

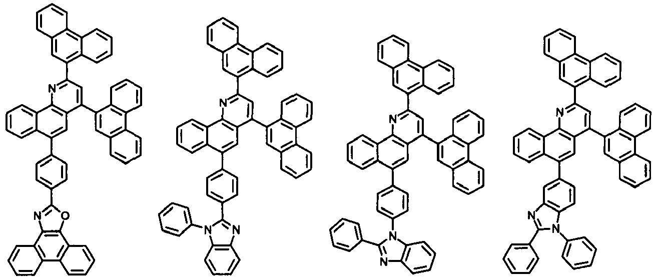

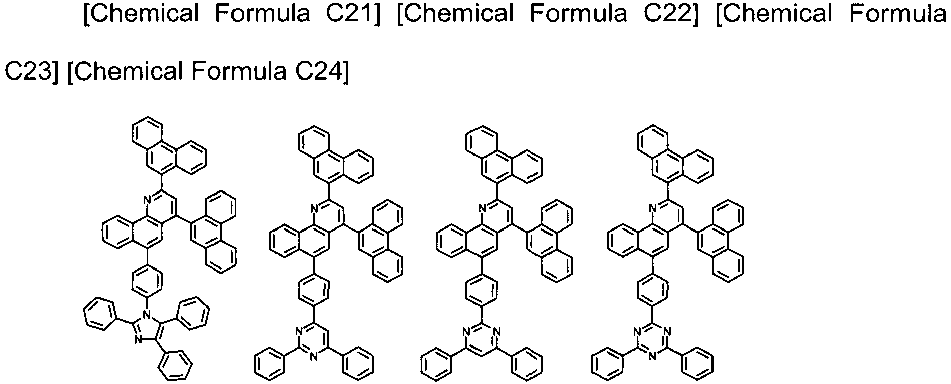

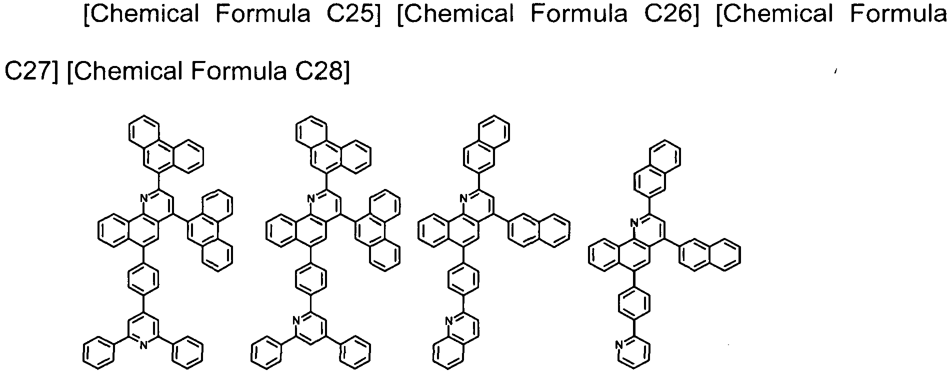

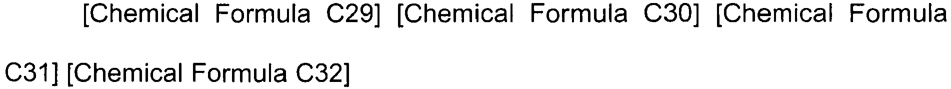

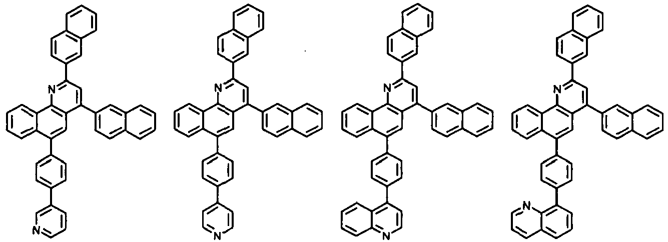

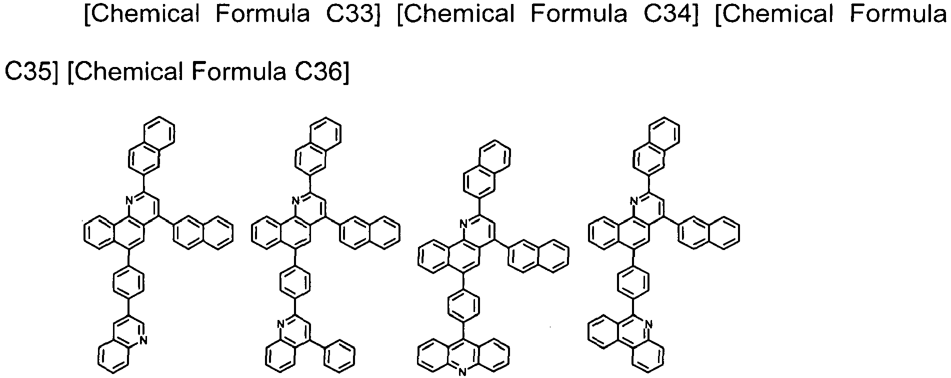

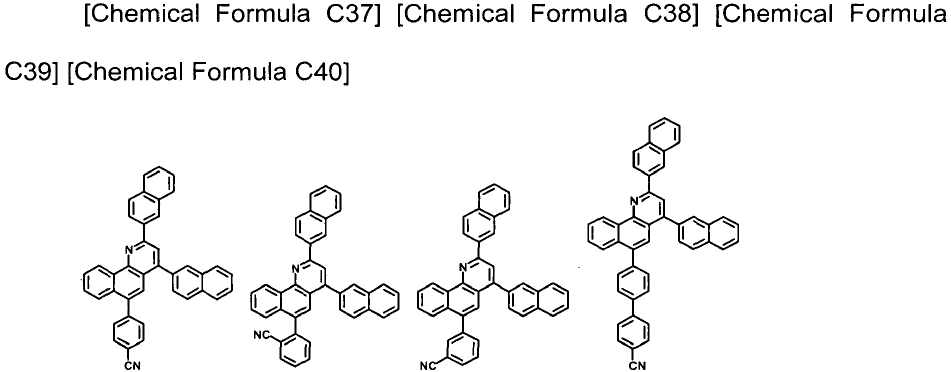

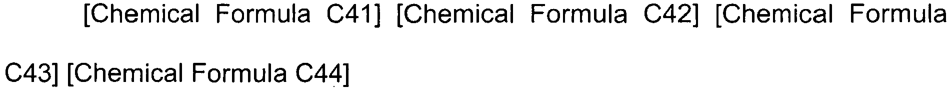

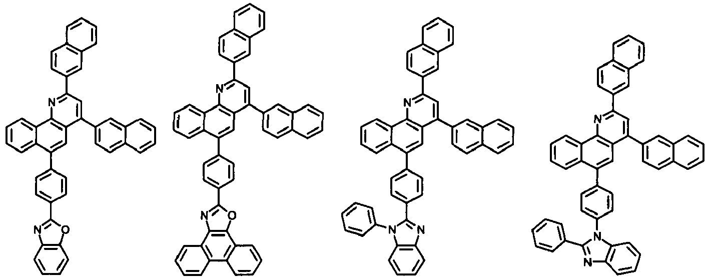

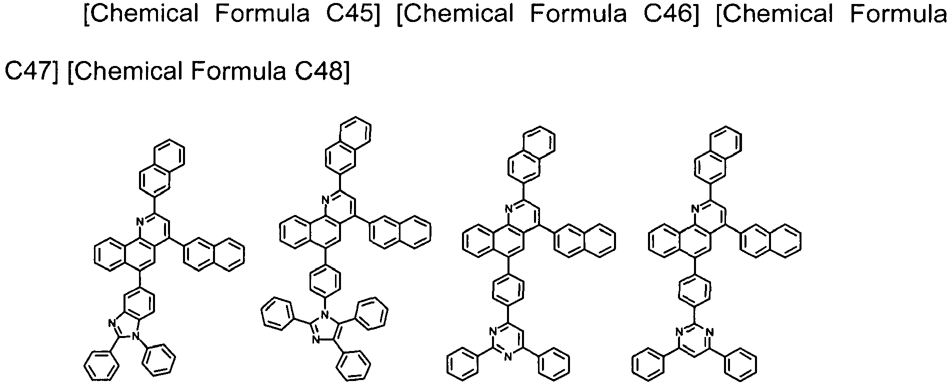

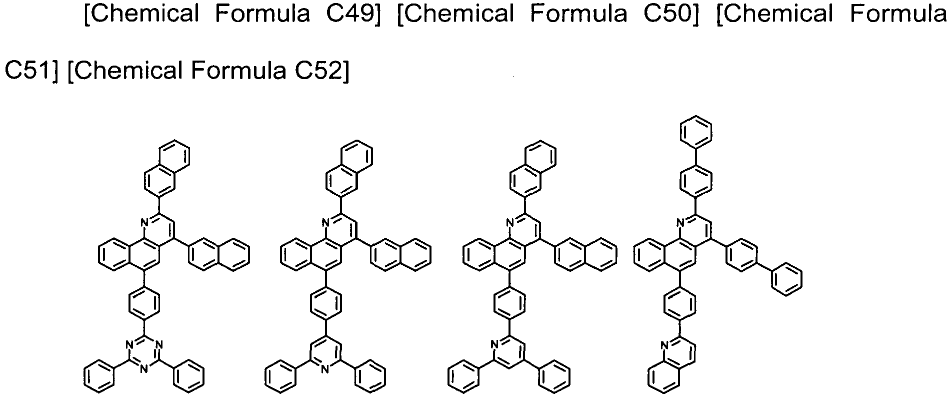

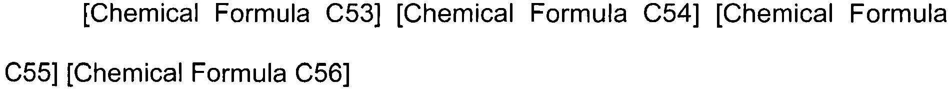

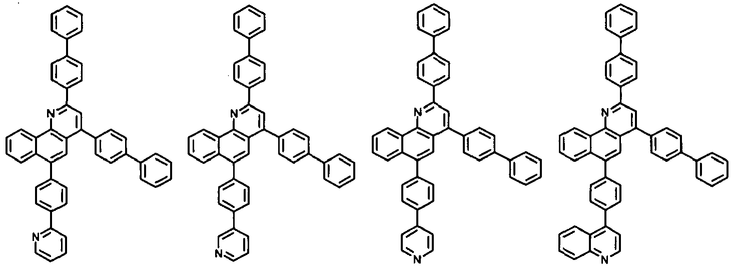

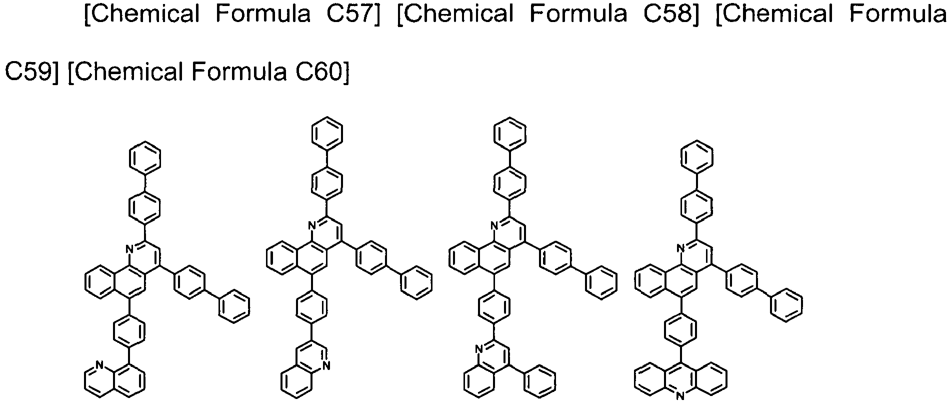

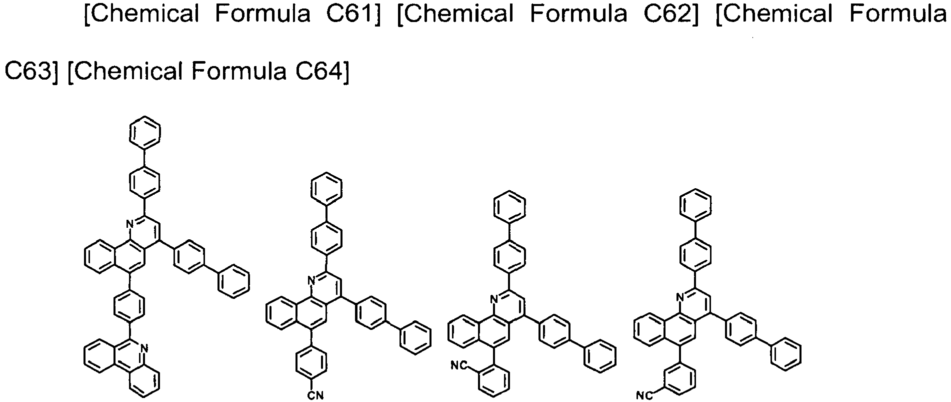

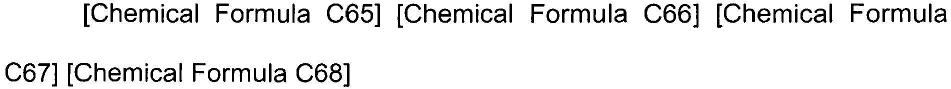

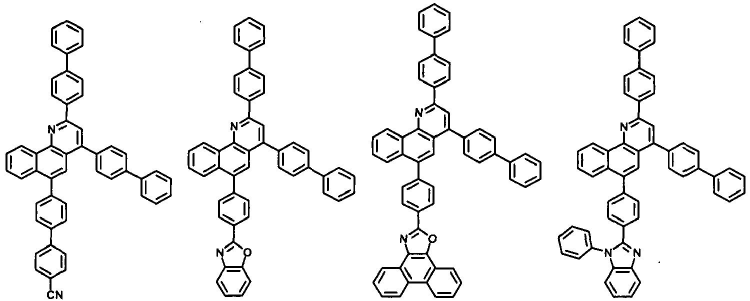

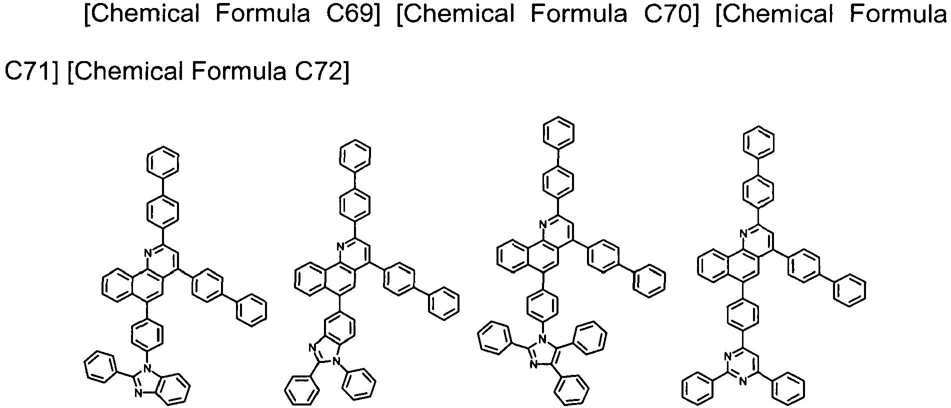

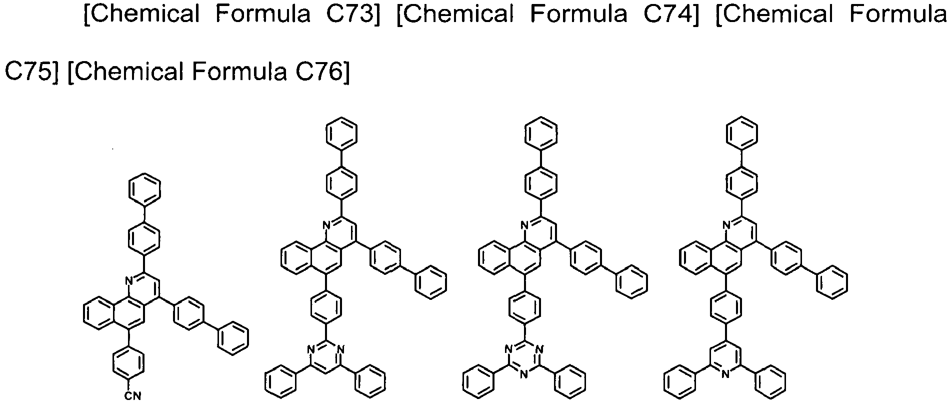

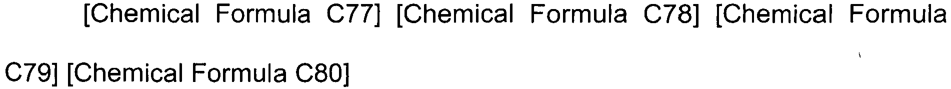

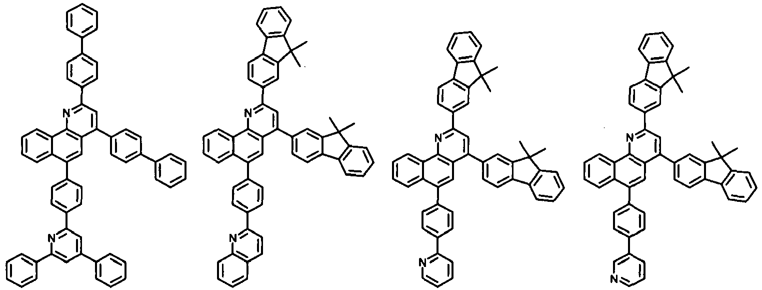

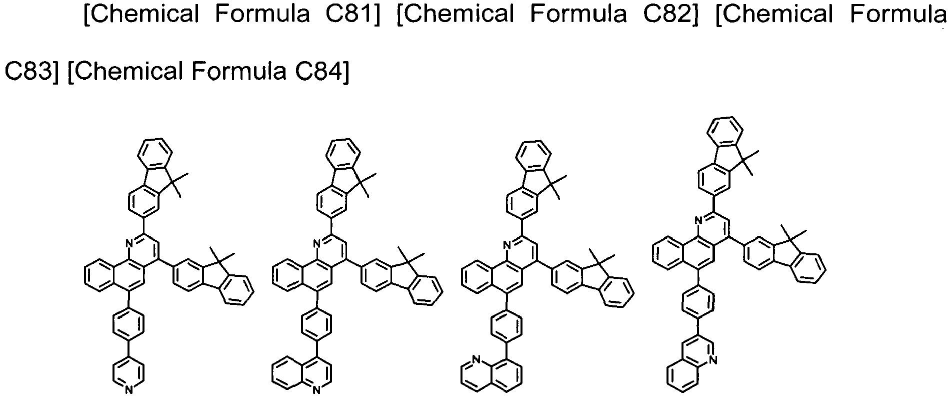

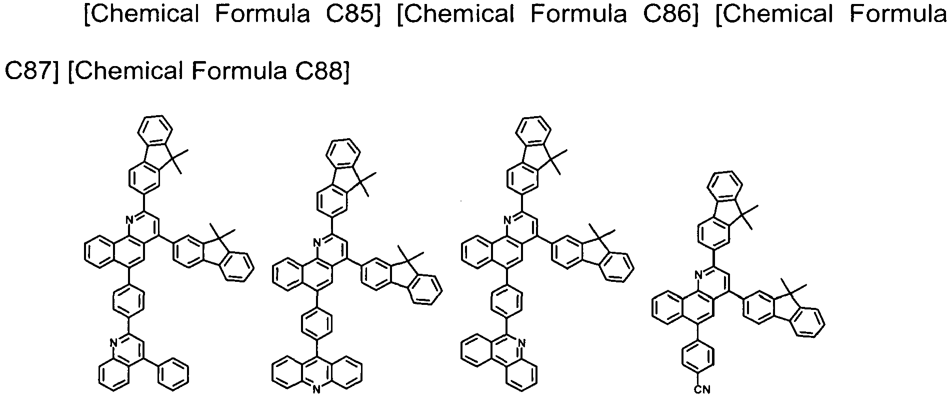

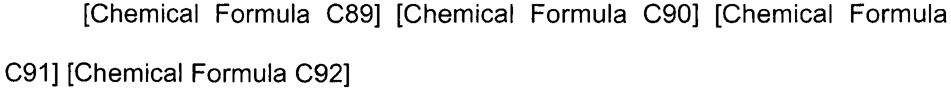

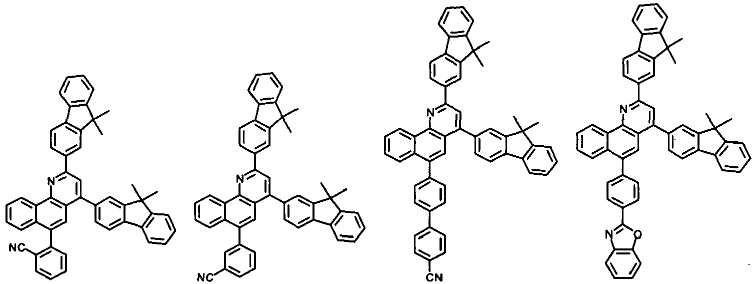

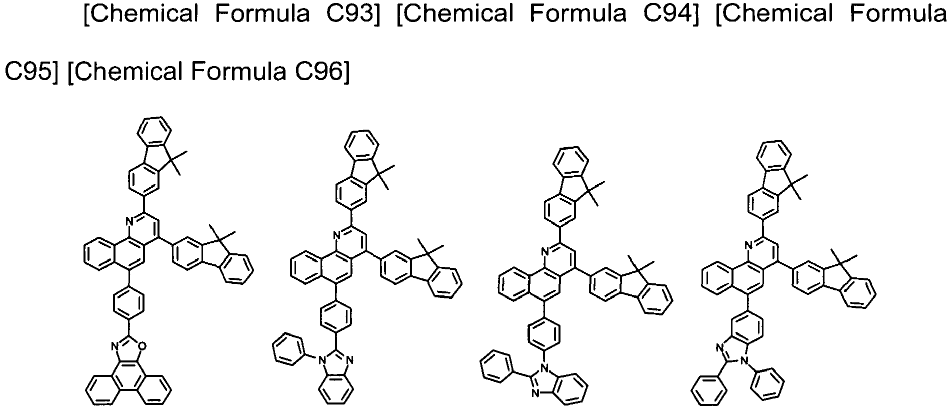

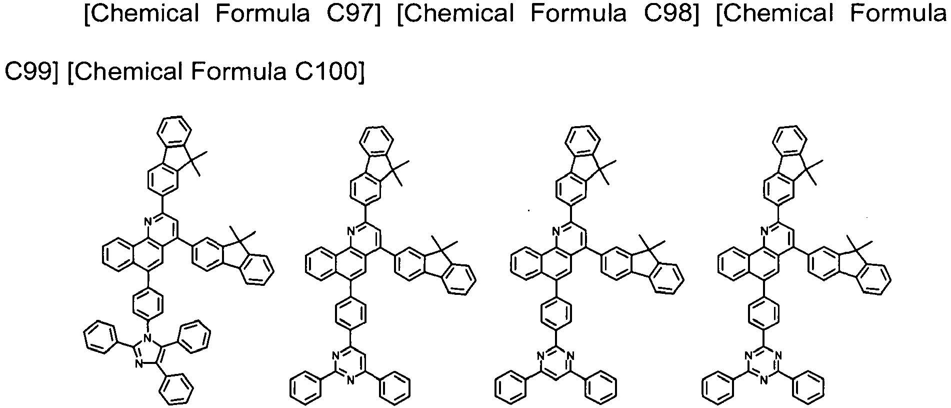

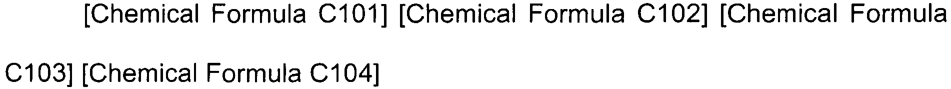

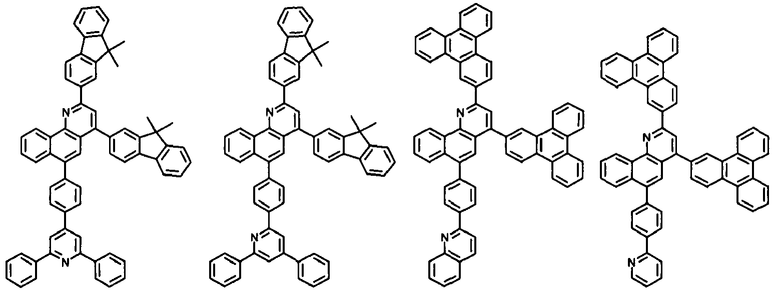

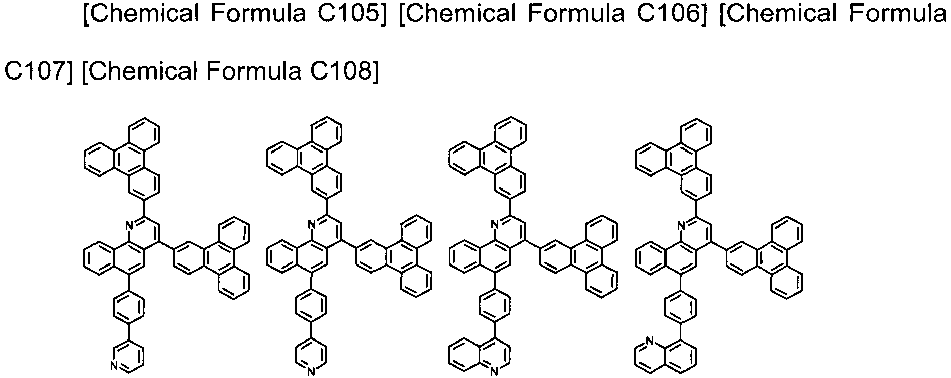

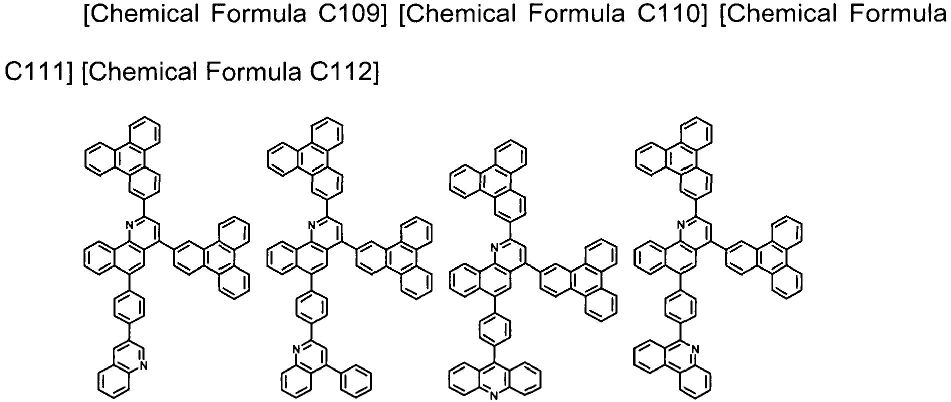

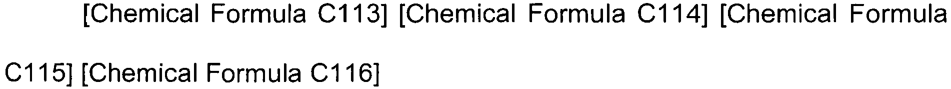

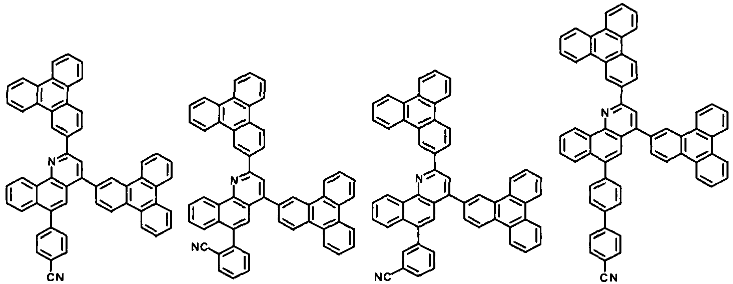

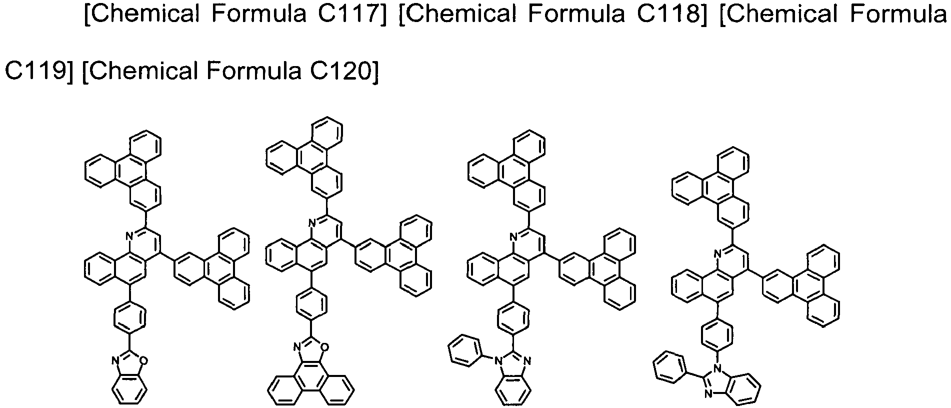

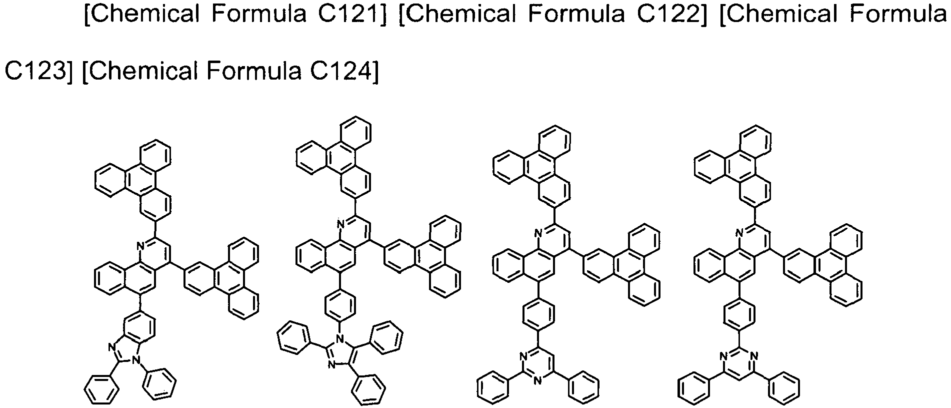

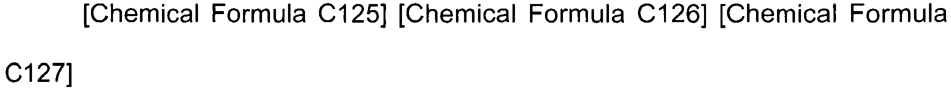

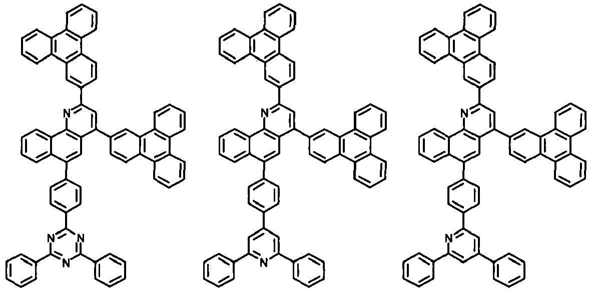

10. An organic light emitting diode, comprising
an anode, a cathode, and at least one or more organic thin layer between the anode and the cathode,
wherein at least one of the organic thin layer comprises the compound for an organic optoelectronic device the of any one of claim 1 to claim 9.

11. The organic light emitting diode of claim 10, wherein the organic thin layer is selected from the group consisting of an emission layer, a hole transport layer (HTL), a hole injection layer (HIL), an electron transport layer (ETL), an electron injection layer (EIL), a hole blocking layer, and a combination thereof.

12. The organic light emitting diode of claim 10, wherein the compound for an organic optoelectronic device is included in an electron transport layer (ETL) or an electron injection layer (EIL).

13. A display device including the organic light emitting diode according to claim 10.

## Patentansprüche

1. Verbindung für ein organisches optoelektronisches Bauelement, wobei die Verbindung durch die folgende chemische Formel 2 dargestellt ist: 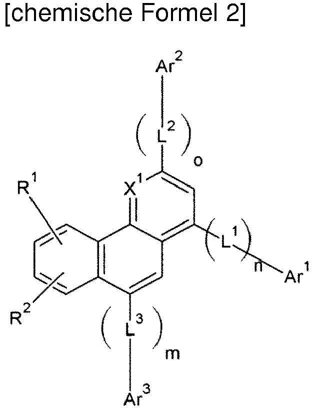 wobei in der chemischen Formel 2
X¹ -N- ist,
R¹ und R² gleich oder verschieden sind und unabhängig voneinander Wasserstoff, Deuterium, eine substituierte oder unsubstituierte C₁- bis C₂₀-Alkylgruppe, eine substituierte oder unsubstituierte C₆- bis C₃₀-Arylgruppe, eine substituierte oder unsubstituierte C₃- bis C₃₀-Heteroarylgruppe oder eine Kombination davon bezeichnen,
Ar¹ bis Ar³ gleich oder verschieden sind und unabhängig voneinander eine substituierte oder unsubstituierte C₆- bis C₃₀-Arylgruppe oder eine substituierte oder unsubstituierte C₃- bis C₃₀-Heteroarylgruppe bezeichnen,
L¹ bis L³ gleich oder verschieden sind und unabhängig voneinander eine Einfachbindung, eine substituierte oder unsubstituierte C₂- bis C₆-Alkenylgruppe, eine substituierte oder unsubstituierte C₂- bis C₆-Alkynylgruppe, eine substituierte oder unsubstituierte C₆- bis C₃₀-Arylengruppe, eine substituierte oder unsubstituierte C₃- bis C₃₀-Heteroarylengruppe oder eine Kombination davon bezeichnen, und
n, m oder o gleich oder verschieden sind und unabhängig voneinander 0 oder 1 betragen.

2. Verbindung nach Anspruch 1, wobei Ar¹ und/oder Ar² eine substituierte oder unsubstituierte C₃- bis C₃₀-Heteroarylgruppe ist.

3. Verbindung nach Anspruch 1, wobei Ar¹ eine substituierte oder unsubstituierte C₃- bis C₃₀-Heteroarylgruppe ist, und
wobei Ar² und Ar³ jeweils eine substituierte oder unsubstituierte C₆- bis C₃₀-Arylgruppe sind.

4. Verbindung nach Anspruch 1, wobei Ar² eine substituierte oder unsubstituierte C₃- bis C₃₀-Heteroarylgruppe ist, und
wobei Ar¹ und Ar³ jeweils eine substituierte oder unsubstituierte C₆- bis C₃₀-Arylgruppe sind.

5. Verbindung nach Anspruch 1,
wobei die substituierte oder unsubstituierte C₃- bis C₃₀-Heteroaryl-gruppe eine substituierte oder unsubstituierte Imidazolylgruppe, eine substituierte oder unsubstituierte Triazolylgruppe, eine substituierte oder unsubstituierte Tetrazolylgruppe, eine substituierte oder unsubstituierte Carbazolylgruppe, eine substituierte oder unsubstituierte Oxadiazolylgruppe, eine substituierte oder unsubstituierte Oxatriazolylgruppe, eine substituierte oder unsubstituierte Thiatriazolylgruppe, eine substituierte oder unsubstituierte Benzimidazolylgruppe, eine substituierte oder unsubstituierte Benzotriazolylgruppe, eine substituierte oder unsubstituierte Pyridinylgruppe, eine substituierte oder unsubstituierte Pyrimidinylgruppe, eine substituierte oder unsubstituierte Triazinylgruppe, eine substituierte oder unsubstituierte Pyrazinylgruppe, eine substituierte oder unsubstituierte Pyridazinylgruppe, eine substituierte oder unsubstituierte Purinylgruppe, eine substituierte oder unsubstituierte Chinolinylgruppe, eine substituierte oder unsubstituierte Isochinolinylgruppe, eine substituierte oder unsubstituierte Phthalazinylgruppe, eine substituierte oder unsubstituierte Naphpyridinylgruppe, eine substituierte oder unsubstituierte Chinoxalinylgruppe, eine substituierte oder unsubstituierte Chinazolinylgruppe, eine substituierte oder unsubstituierte Acridinylgruppe, eine substituierte oder unsubstituierte Phenanthrolinylgruppe, eine substituierte oder unsubstituierte Phenazinylgruppe oder eine Kombination davon ist.

6. Verbindung nach Anspruch 1, wobei die substituierte oder unsubstituierte C₆-bis C₃₀-Arylgruppe eine substituierte oder unsubstituierte Phenylgruppe, eine substituierte oder unsubstituierte Naphthylgruppe, eine substituierte oder unsubstituierte Triperylenylgruppe, eine substituierte oder unsubstituierte Fluorenylgruppe, eine substituierte oder unsubstituierte Spirofluorenylgruppe, eine substituierte oder unsubstituierte Biphenylgruppe, eine substituierte oder unsubstituierte Terphenylgruppe, eine substituierte oder unsubstituierte Pyrenylgruppe, eine substituierte oder unsubstituierte Perylenylgruppe, eine substituierte oder unsubstituierte Phenanthrenylgruppe, eine substituierte oder unsubstituierte Anthracenylgruppe oder eine Kombination davon ist.

7. Verbindung nach Anspruch 1, dargestellt durch die folgenden chemischen Formeln A1 bis A126:

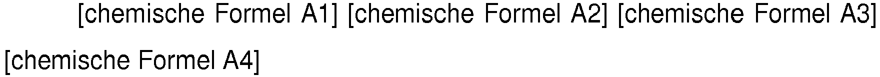

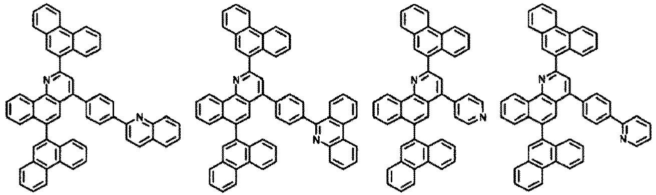

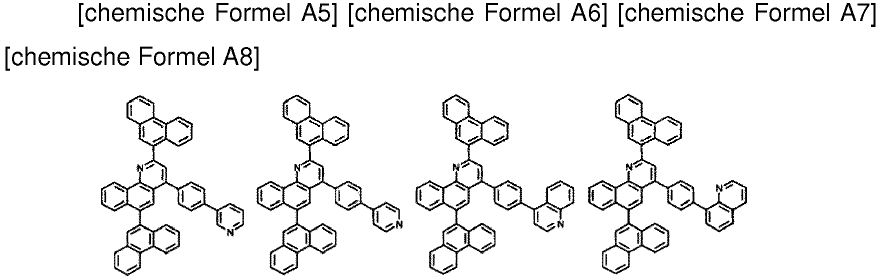

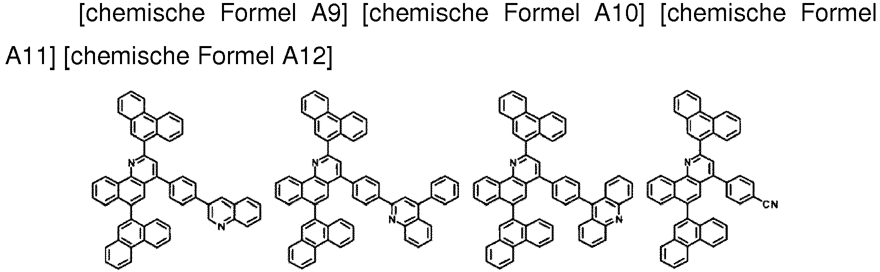

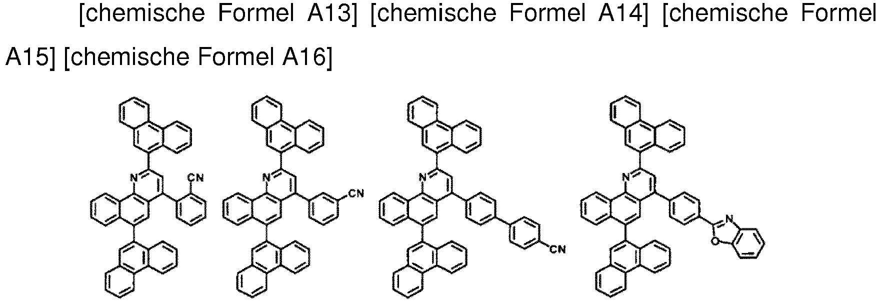

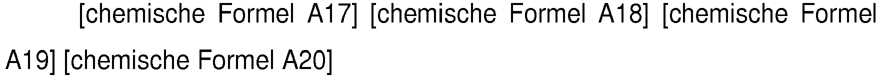

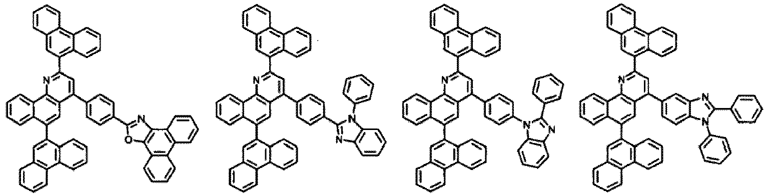

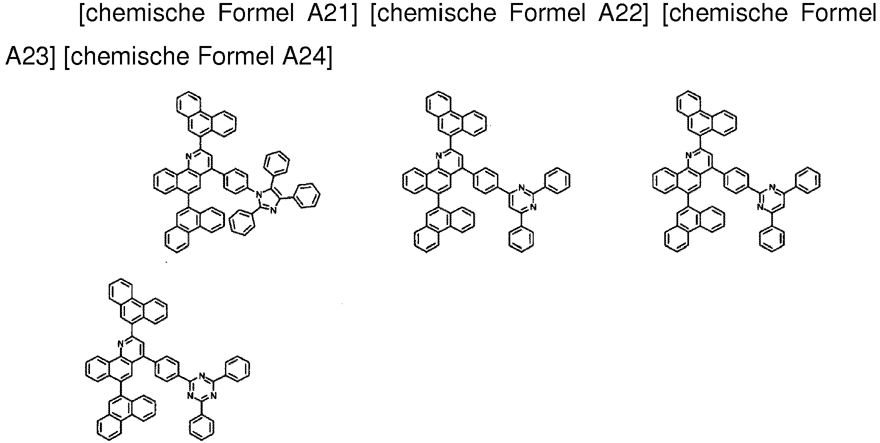

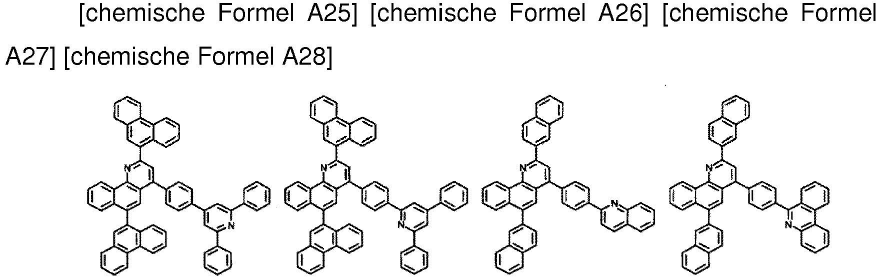

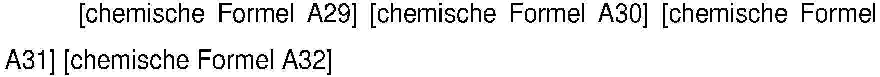

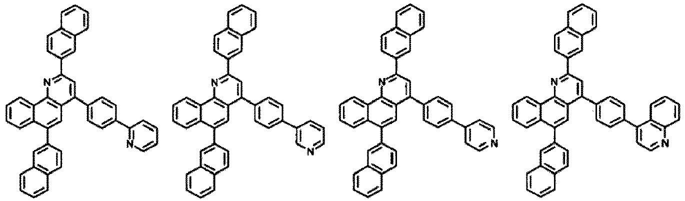

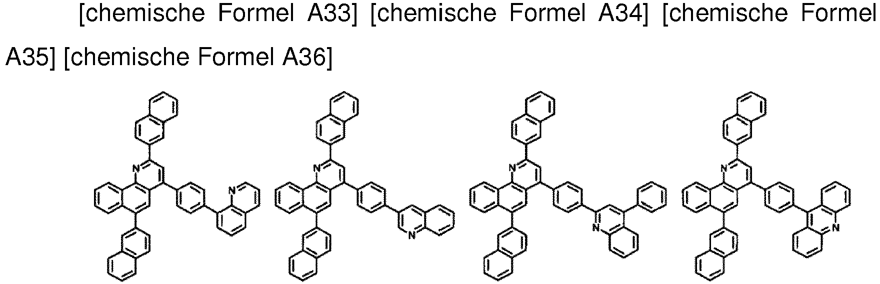

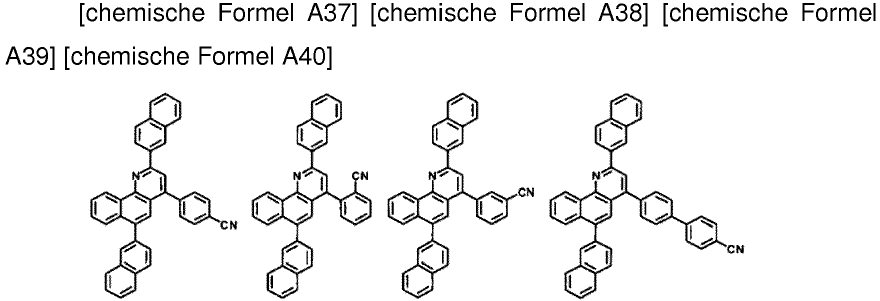

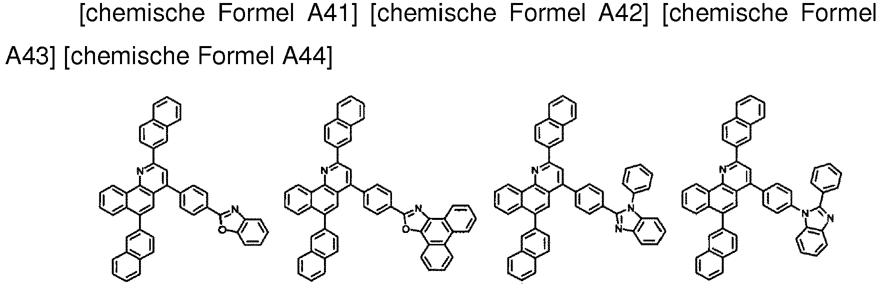

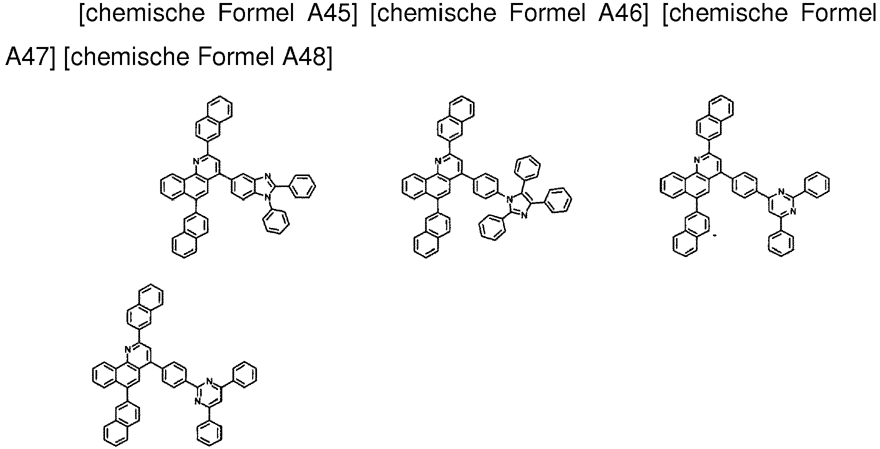

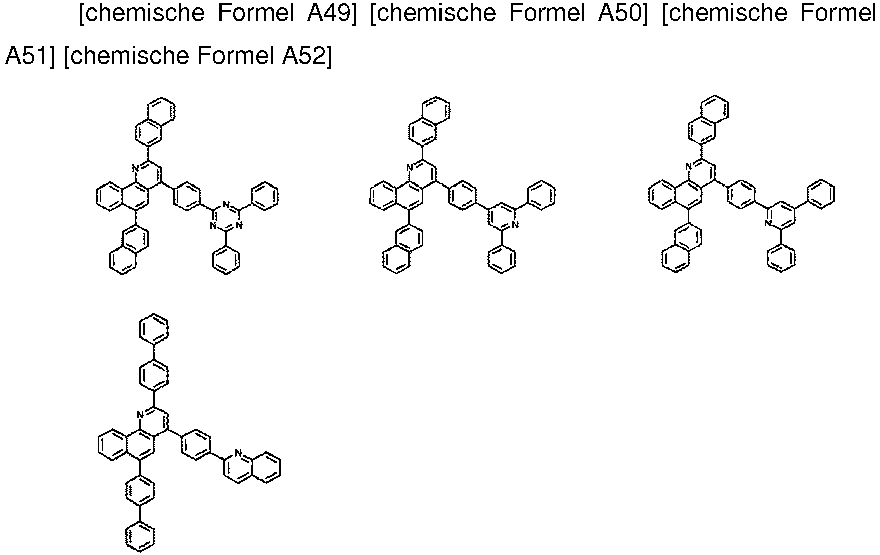

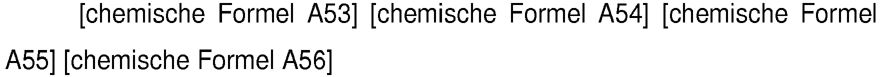

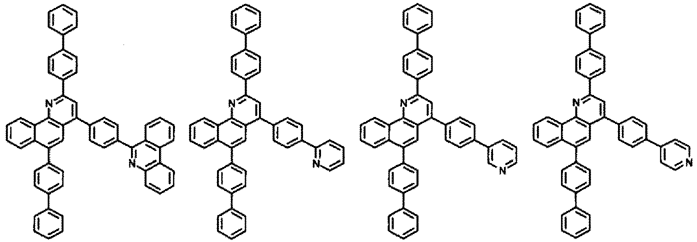

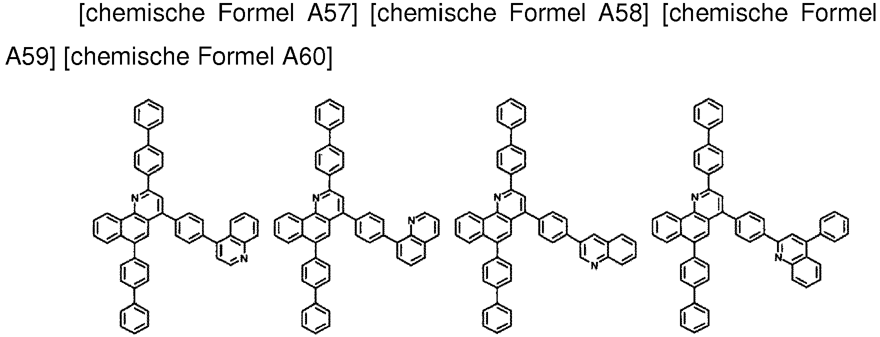

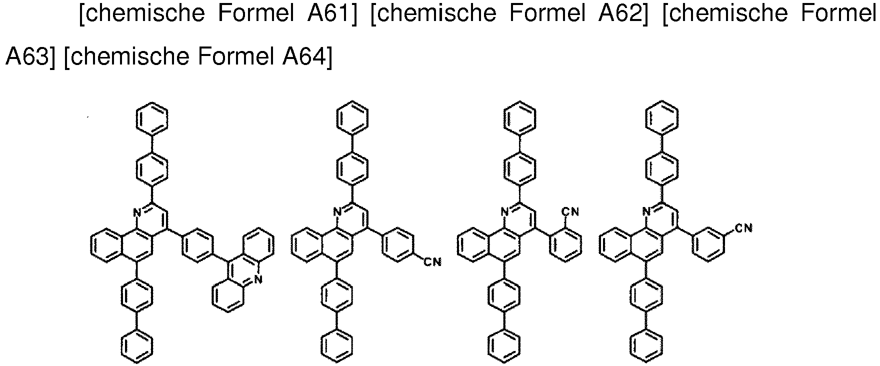

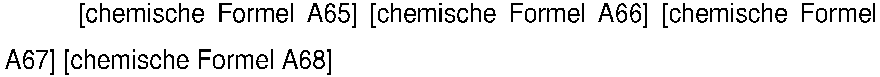

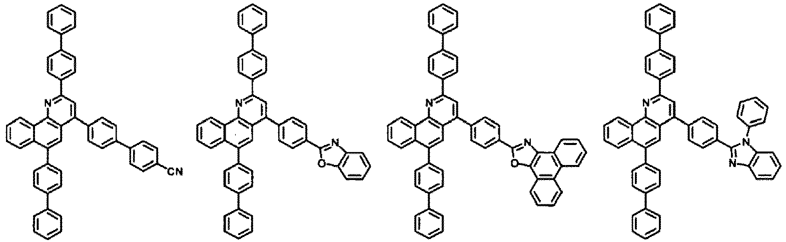

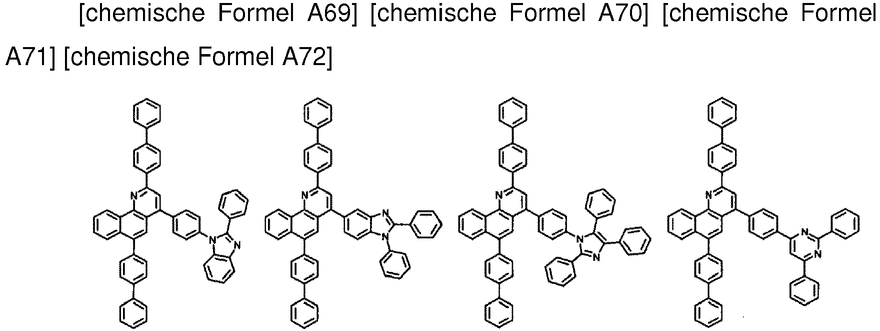

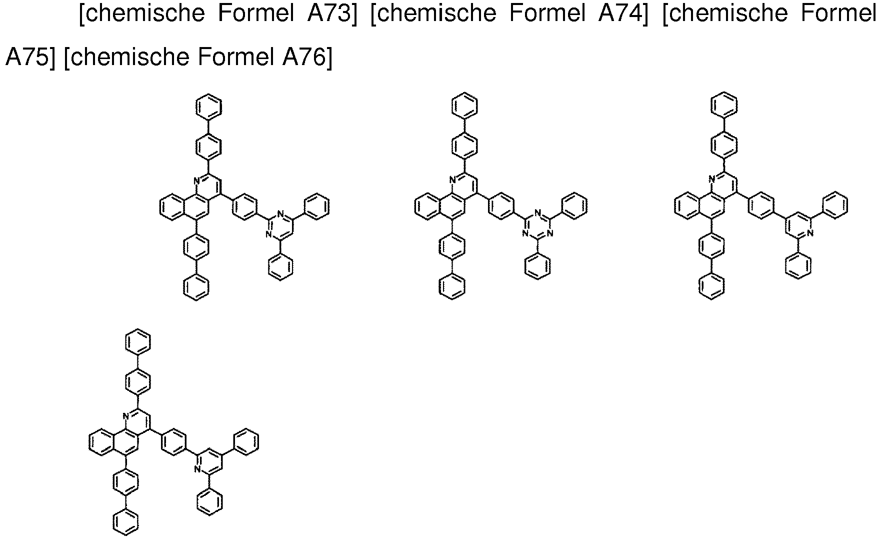

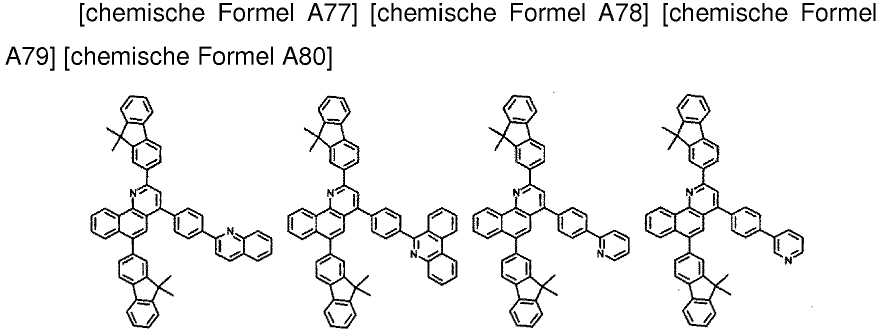

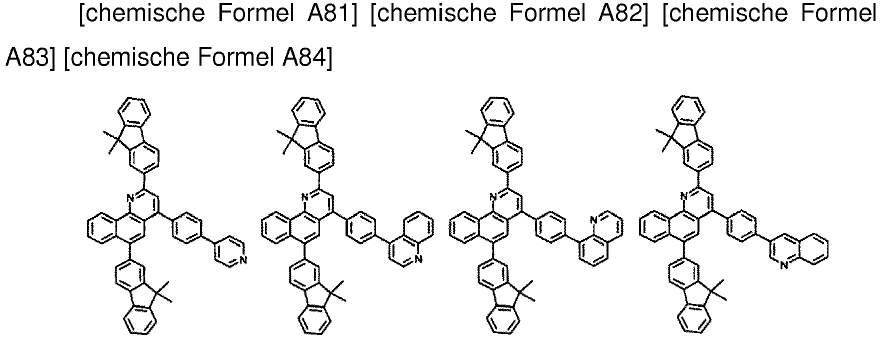

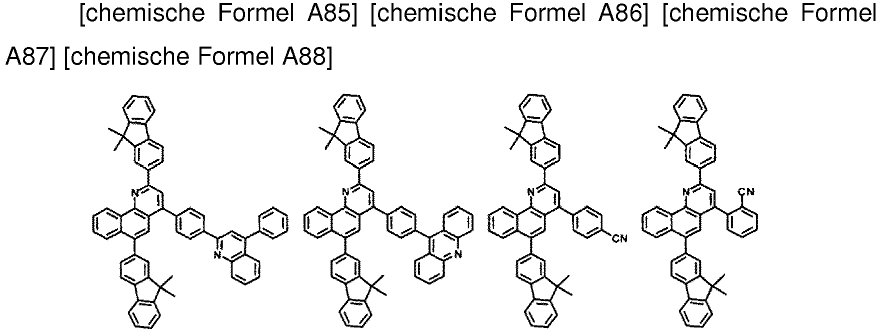

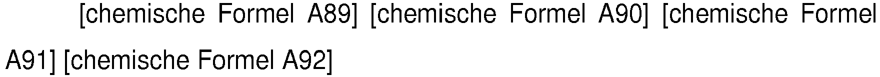

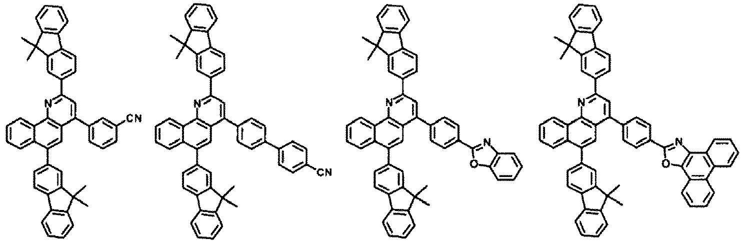

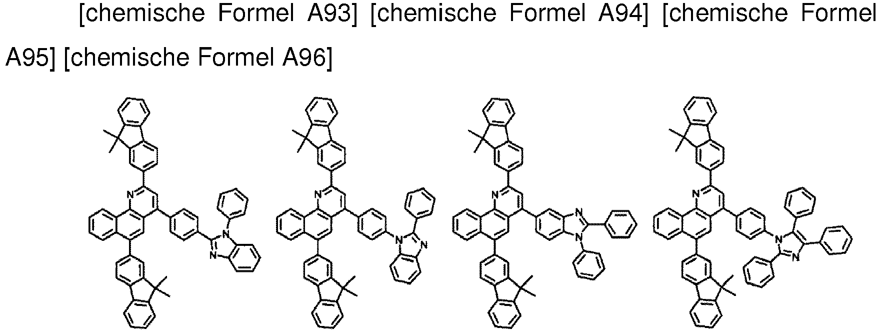

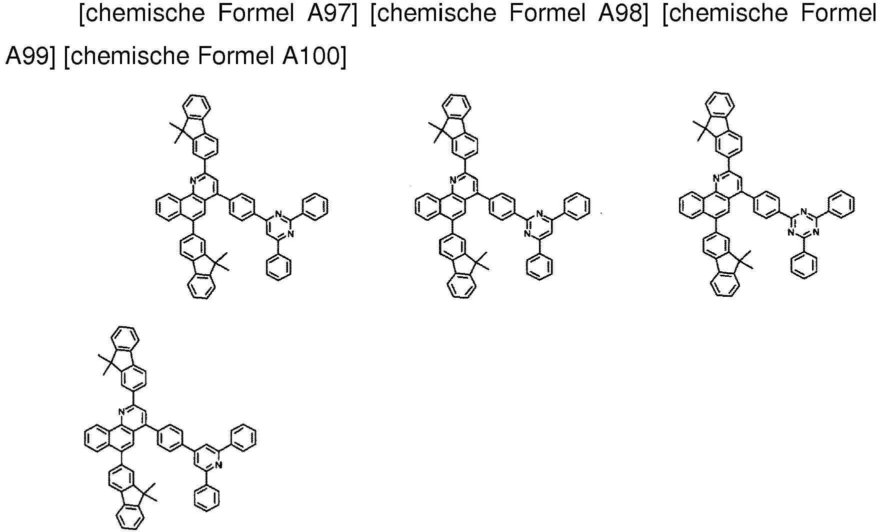

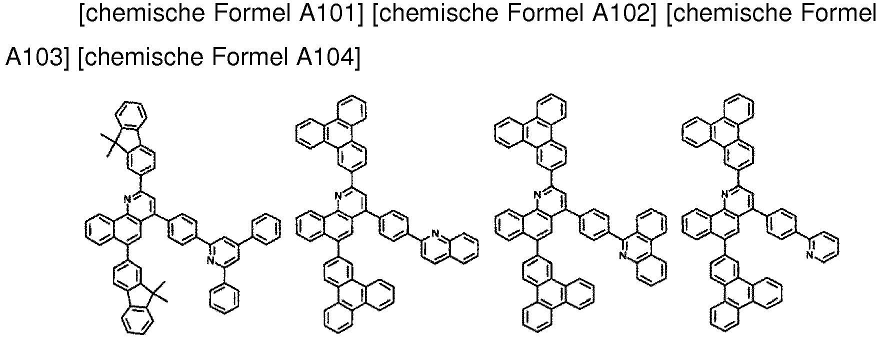

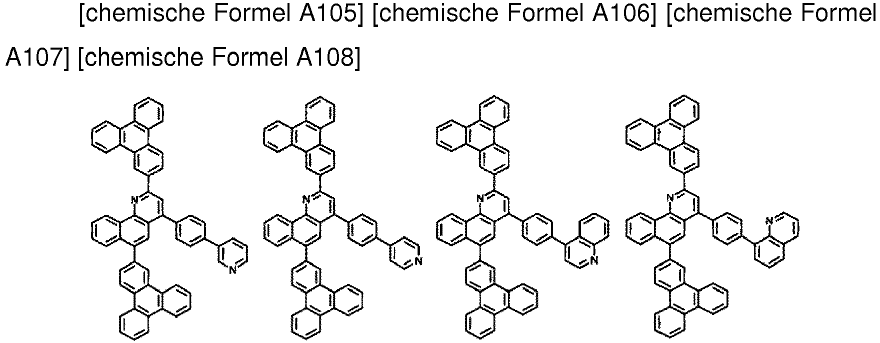

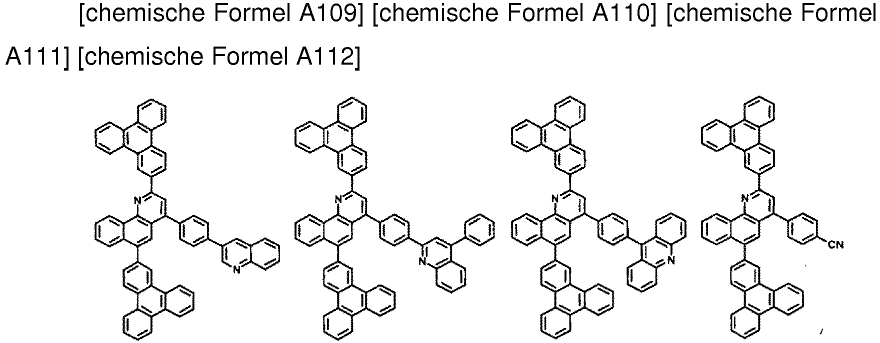

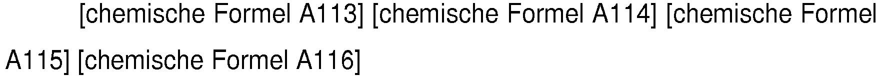

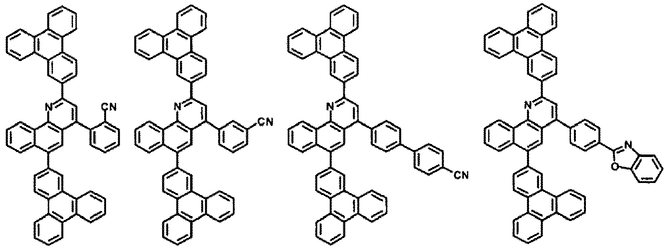

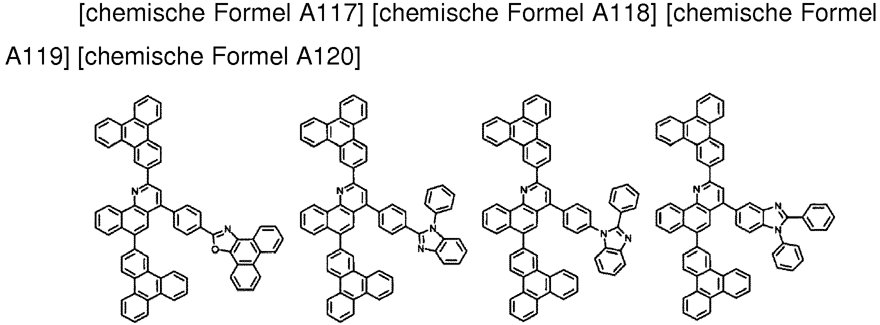

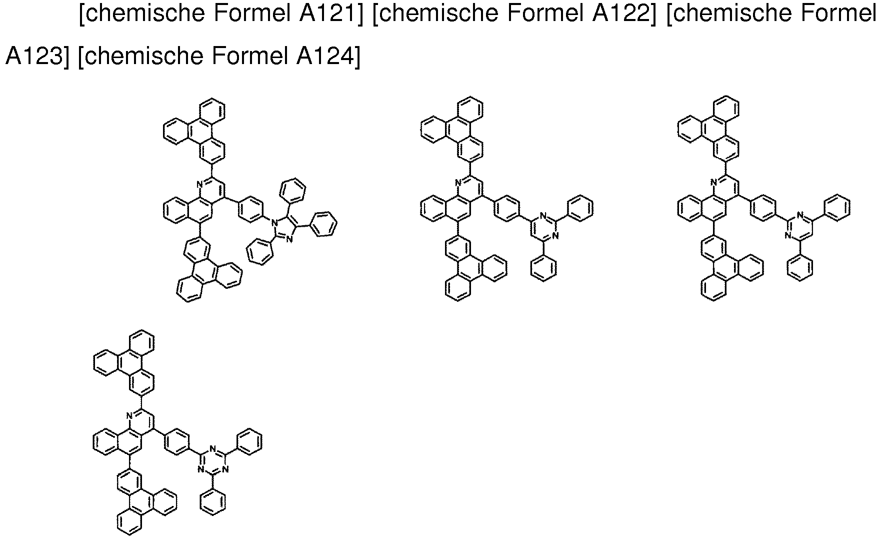

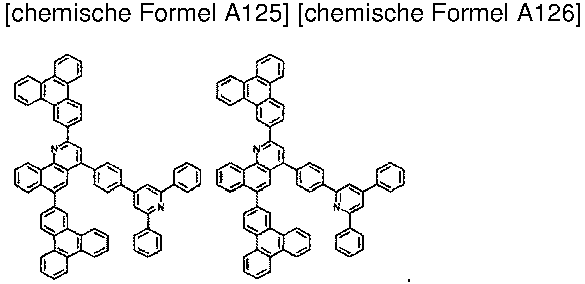

8. Verbindung nach Anspruch 1, dargestellt durch die folgenden chemischen Formeln B1 bis B125:

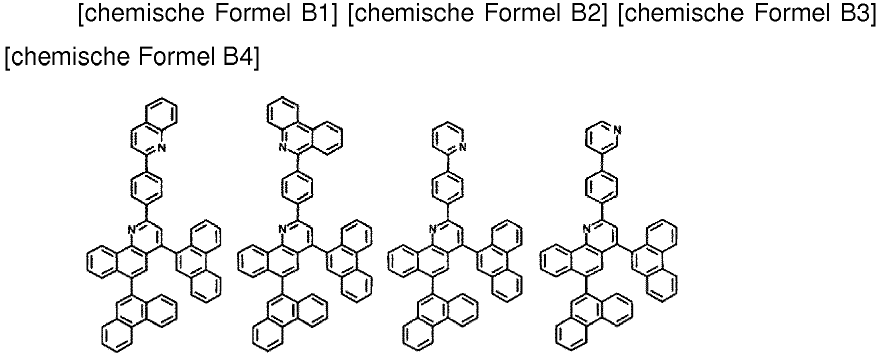

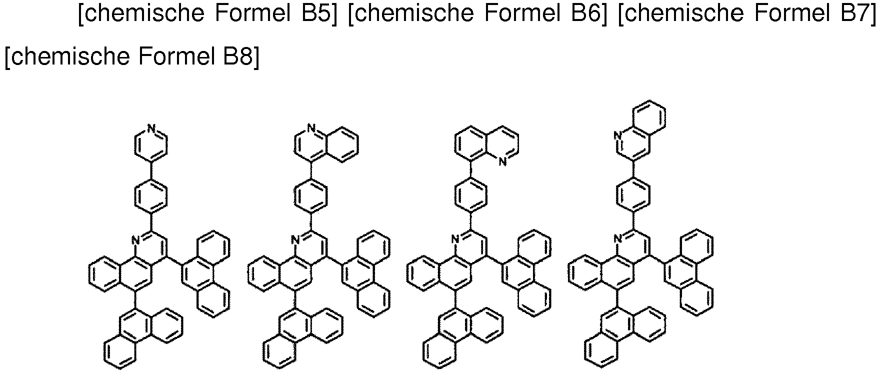

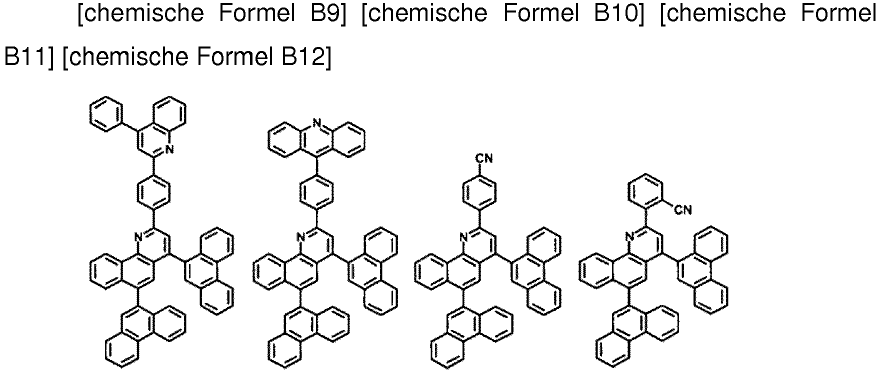

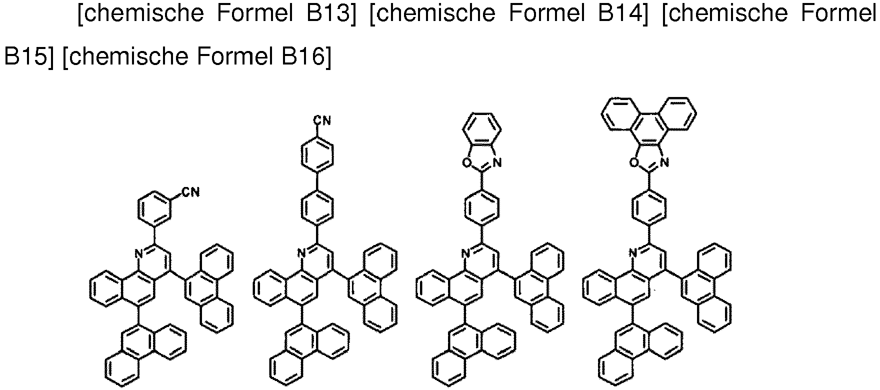

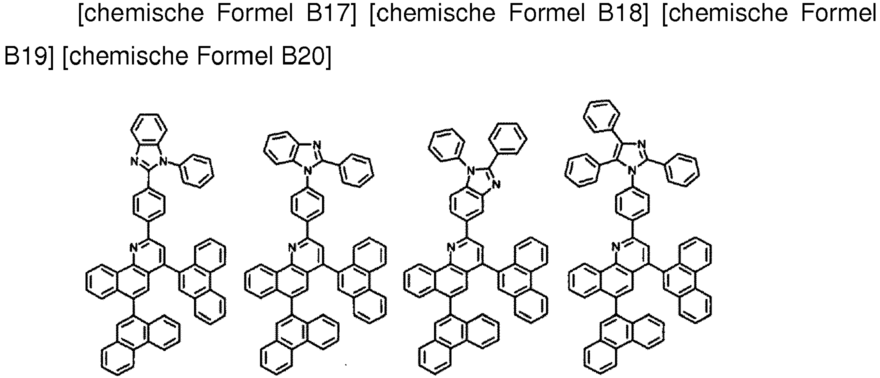

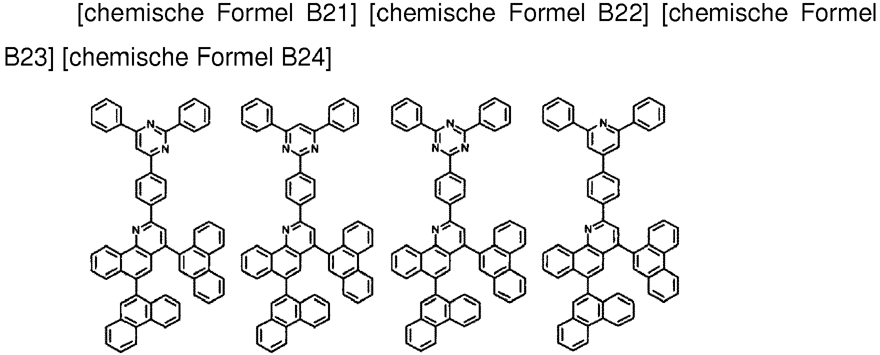

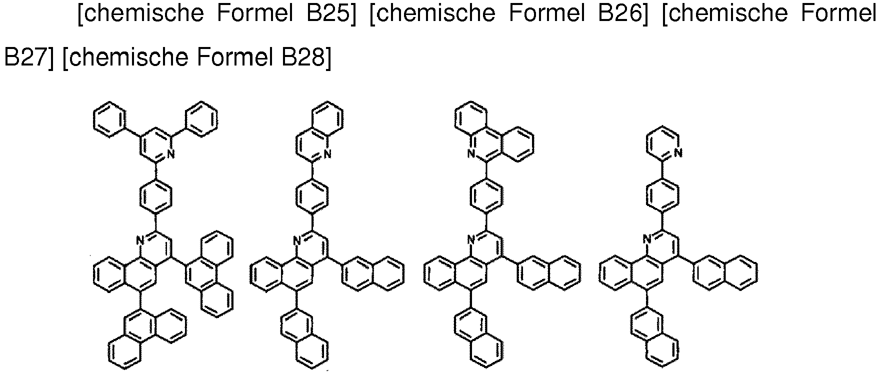

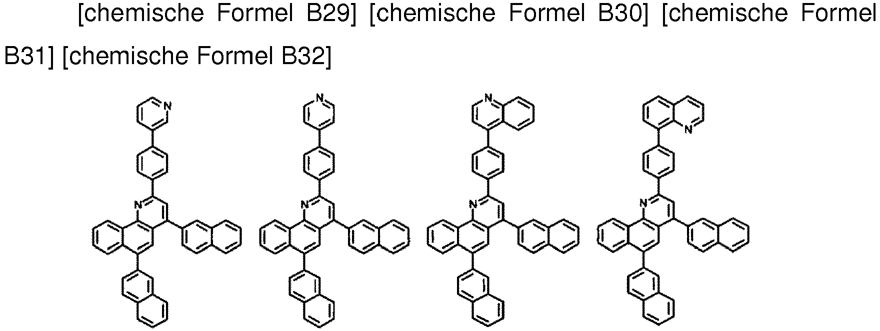

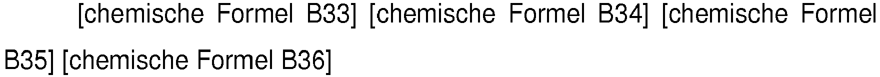

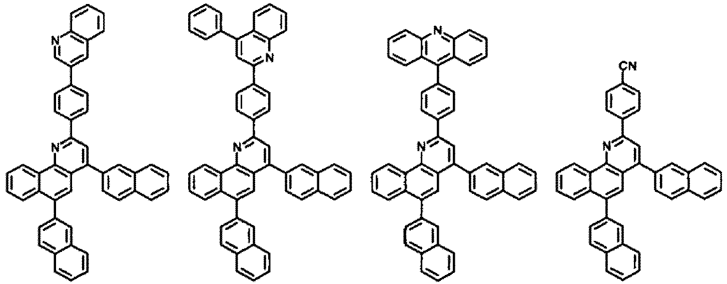

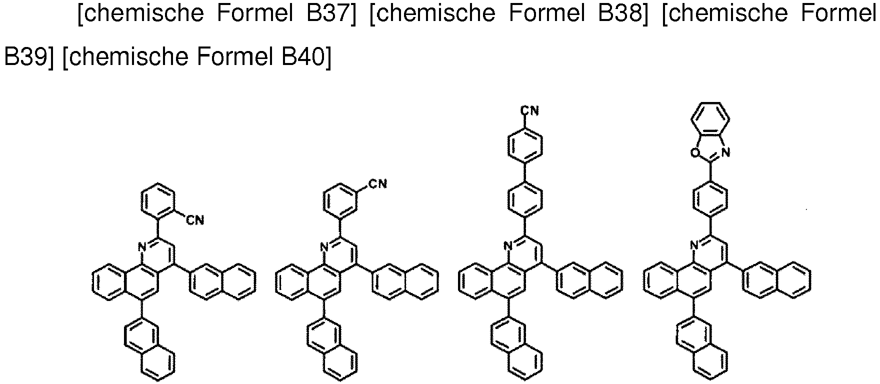

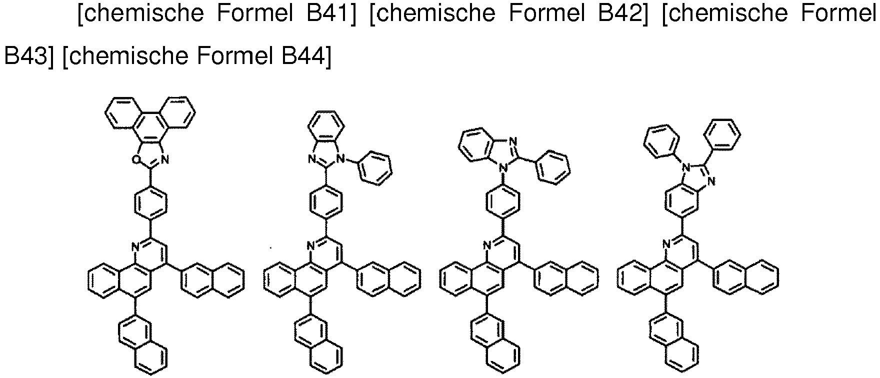

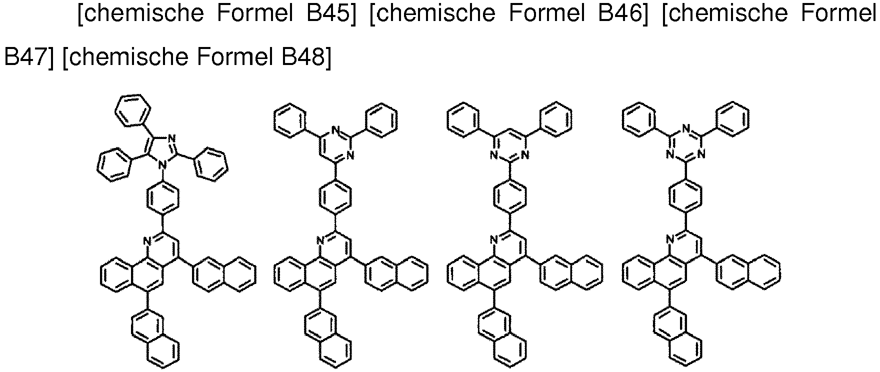

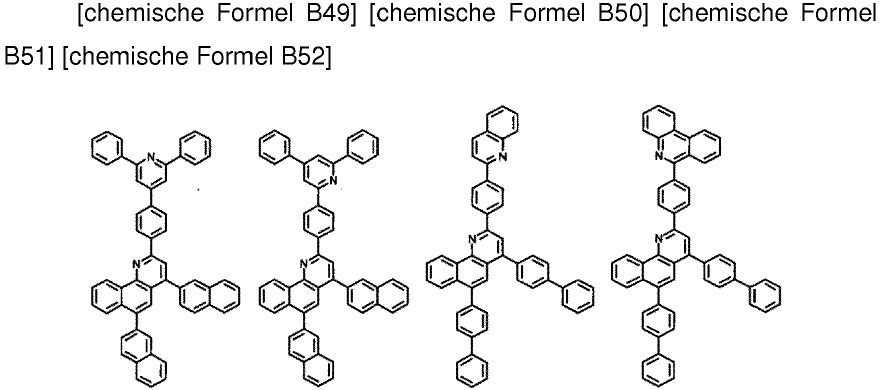

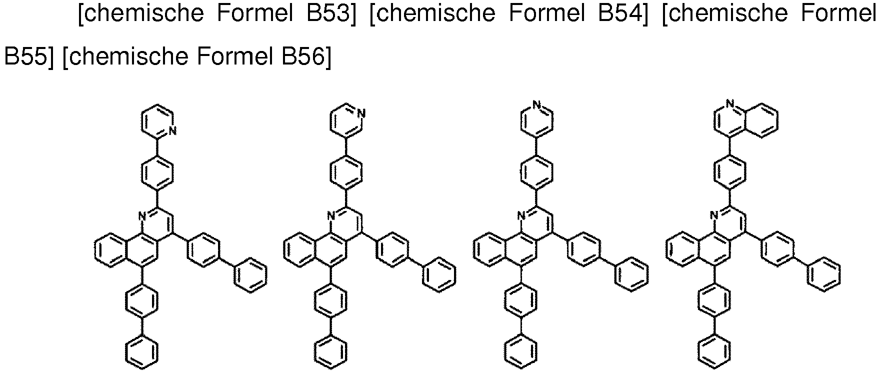

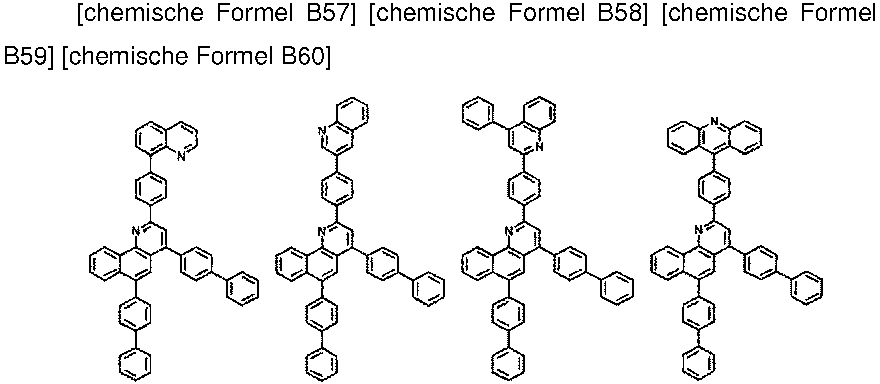

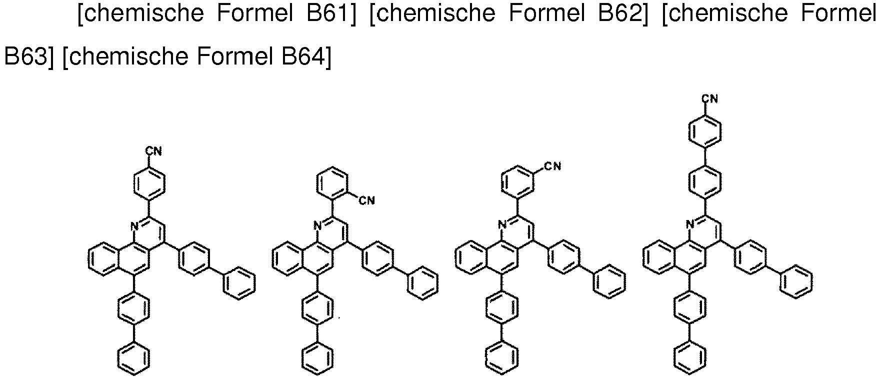

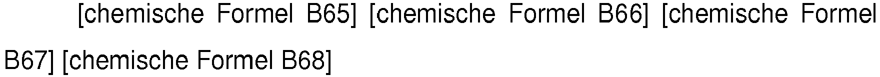

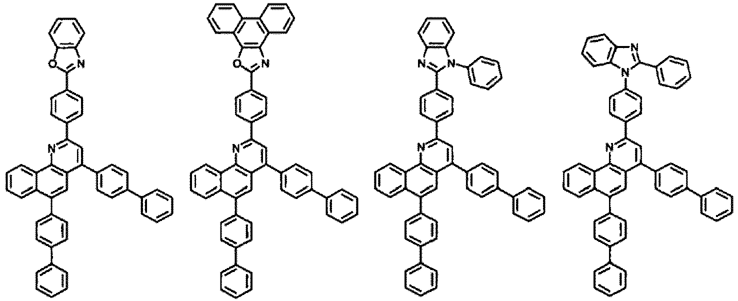

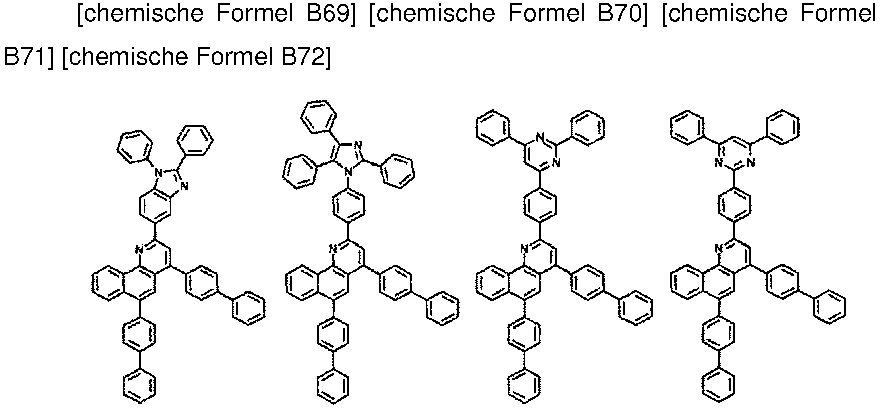

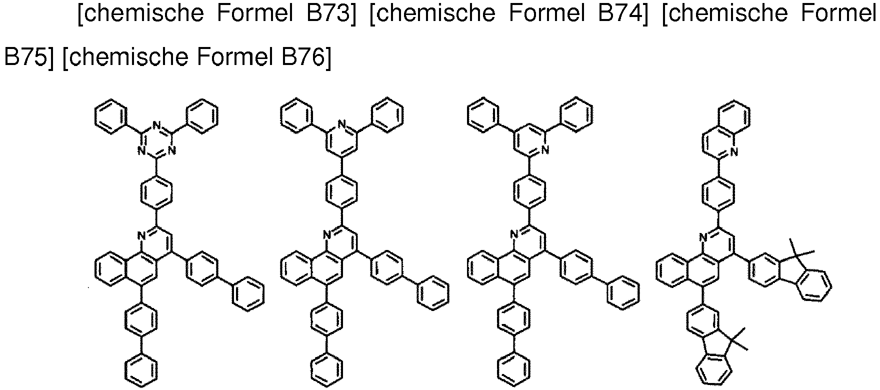

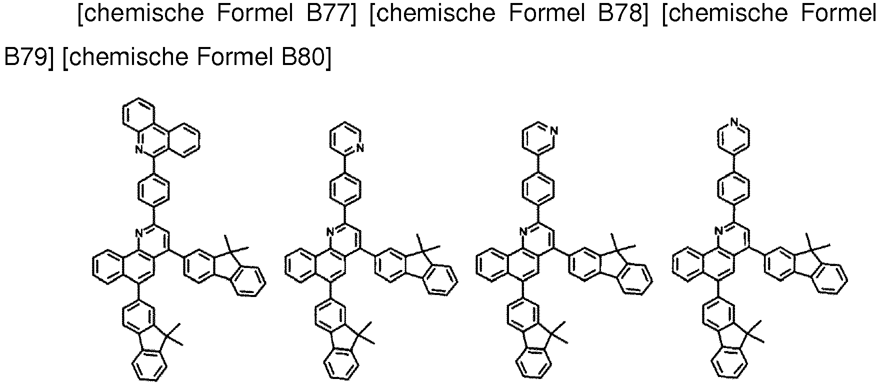

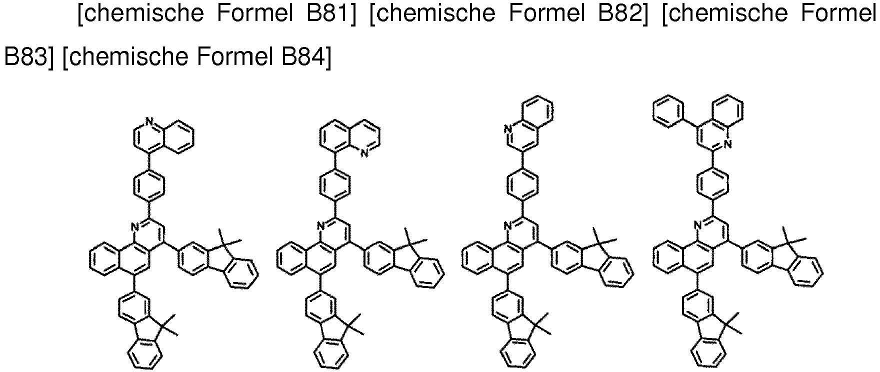

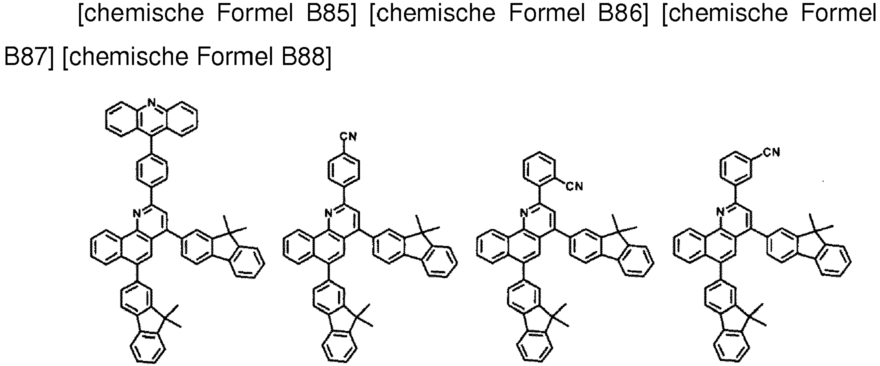

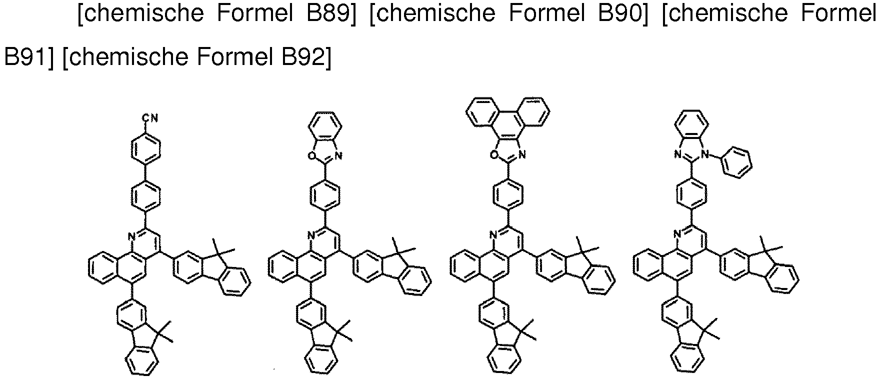

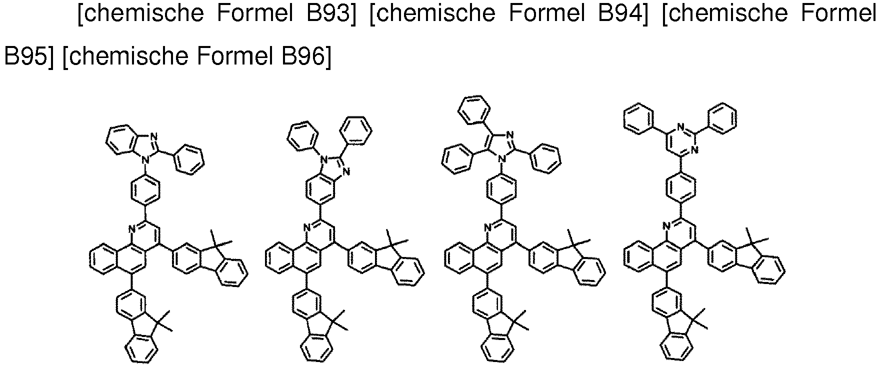

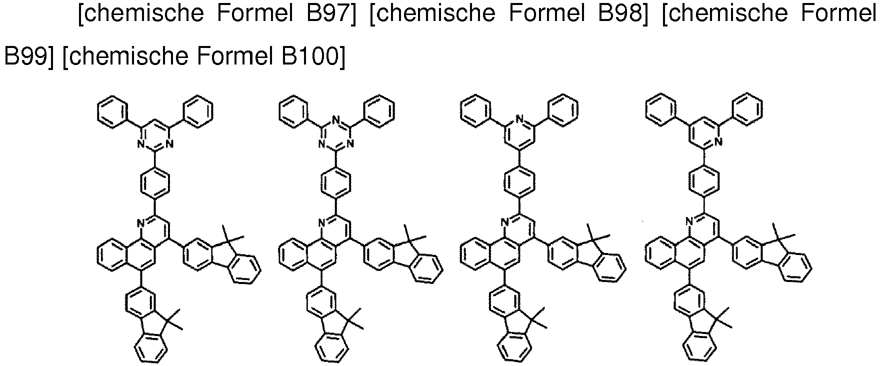

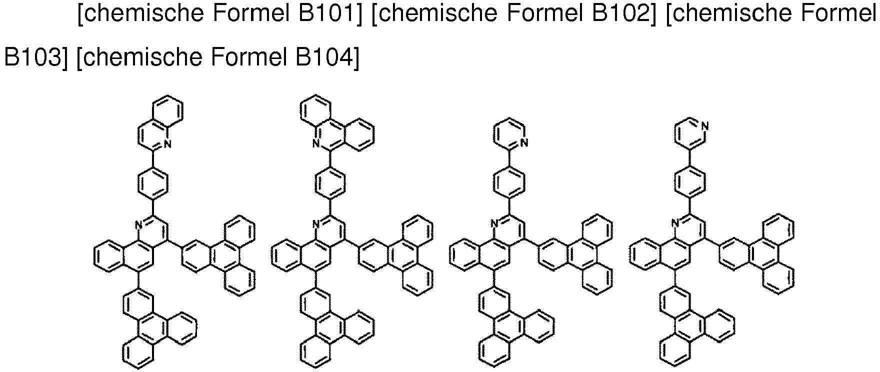

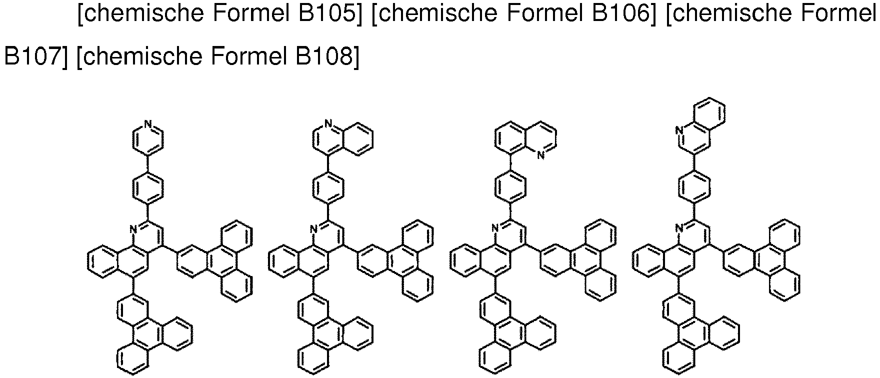

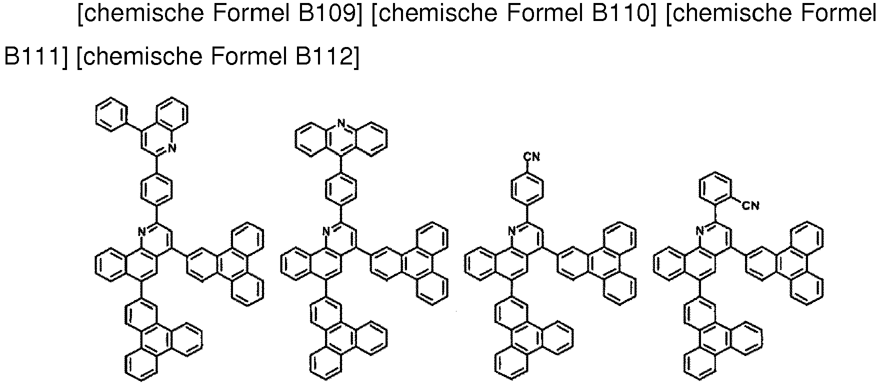

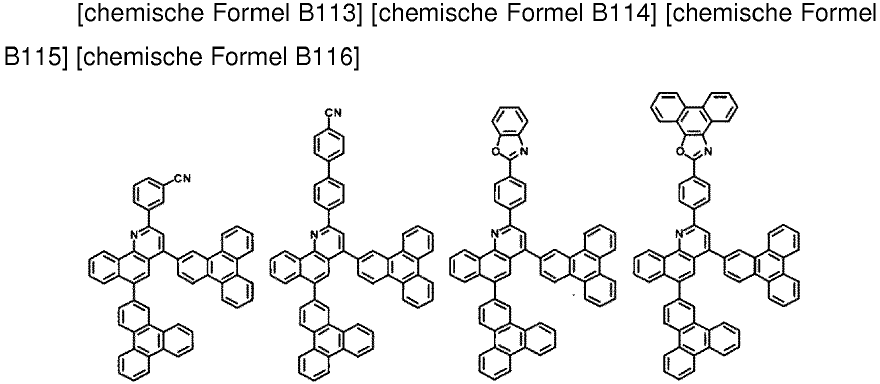

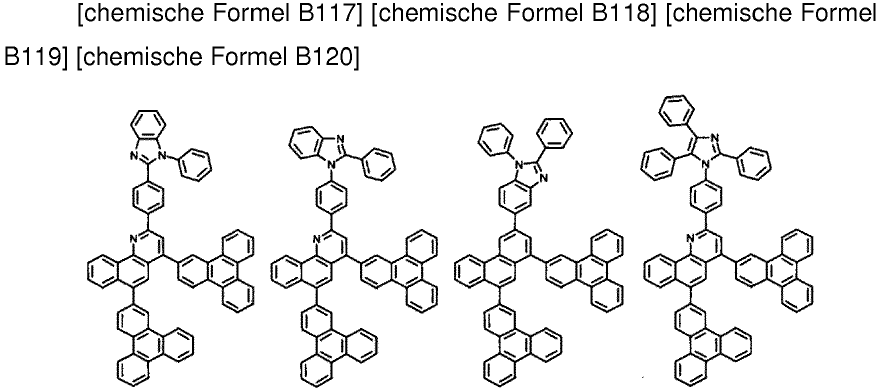

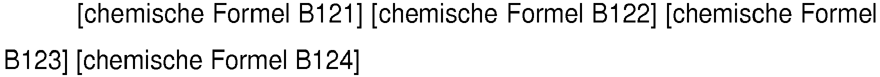

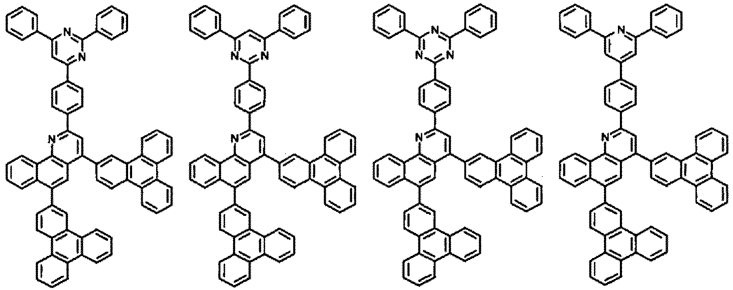

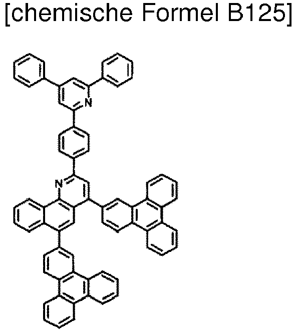

9. Verbindung nach Anspruch 1, dargestellt durch eine der folgenden chemischen Formeln C1 bis C127:

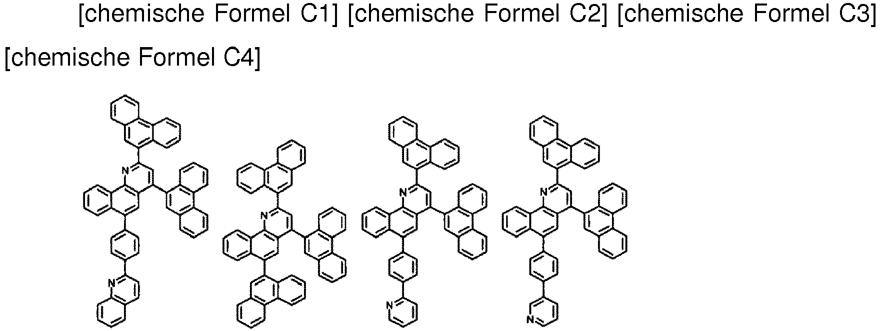

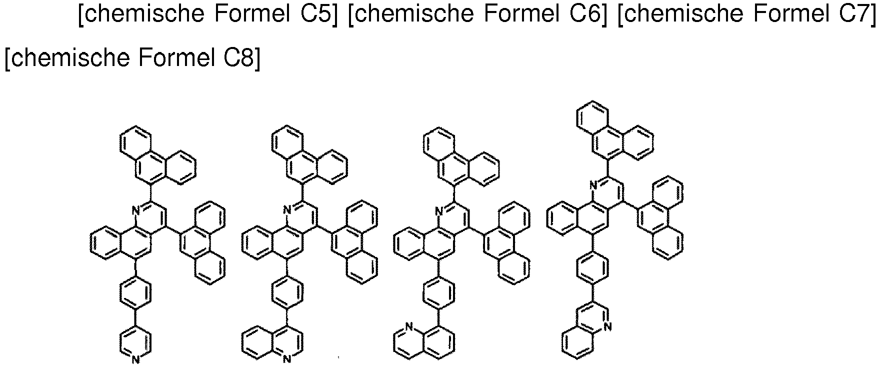

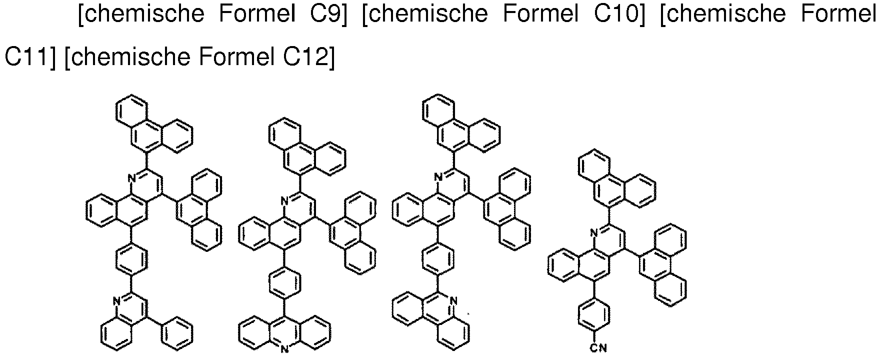

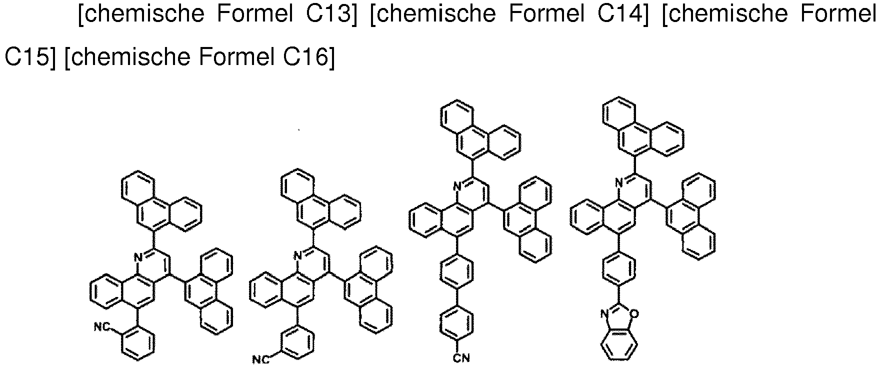

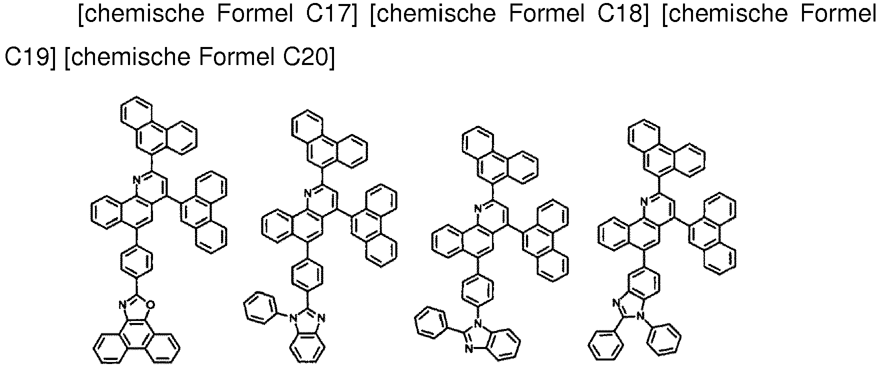

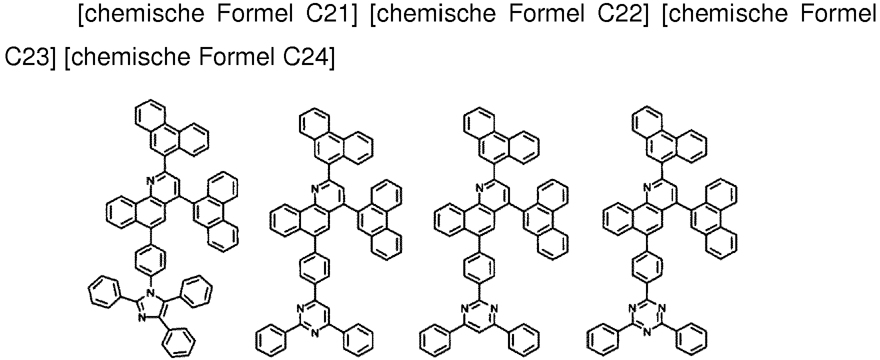

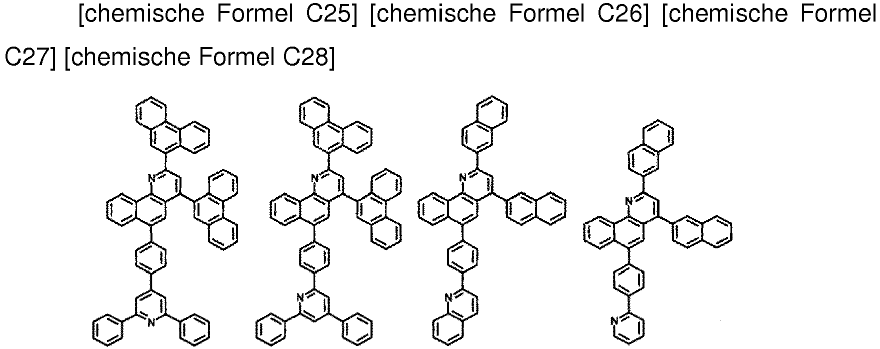

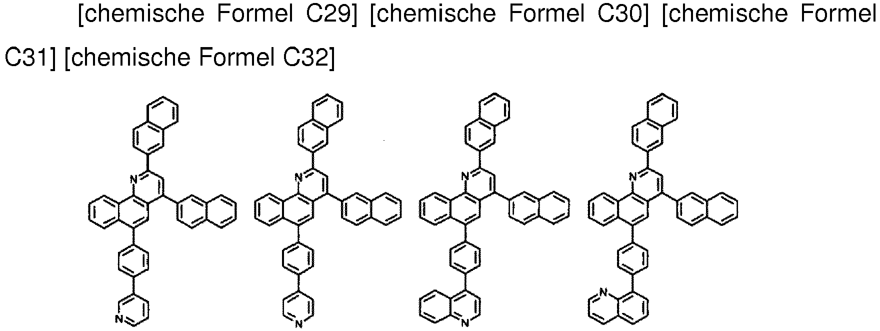

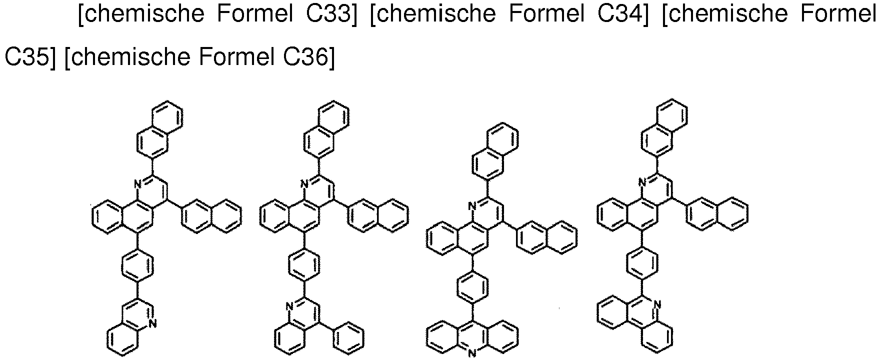

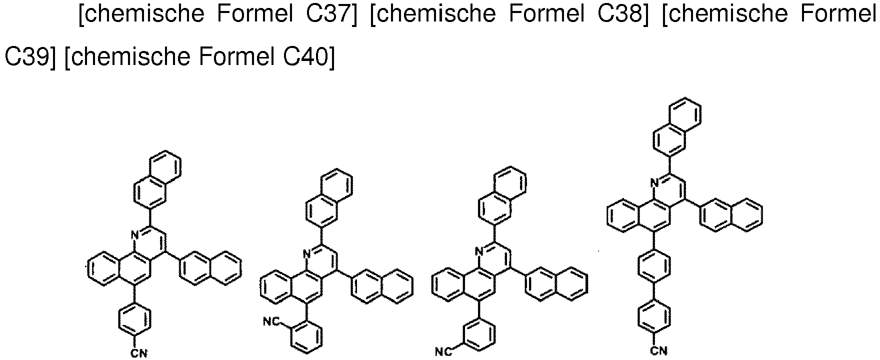

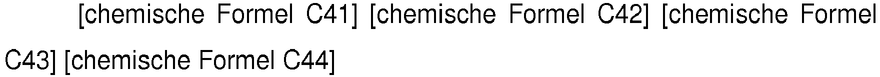

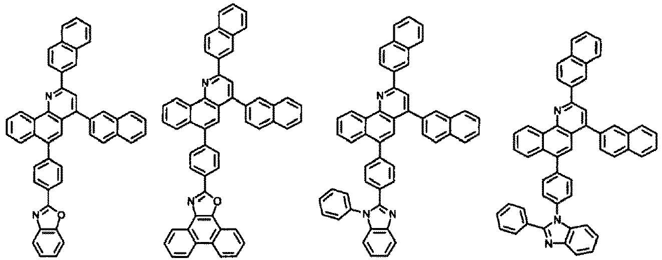

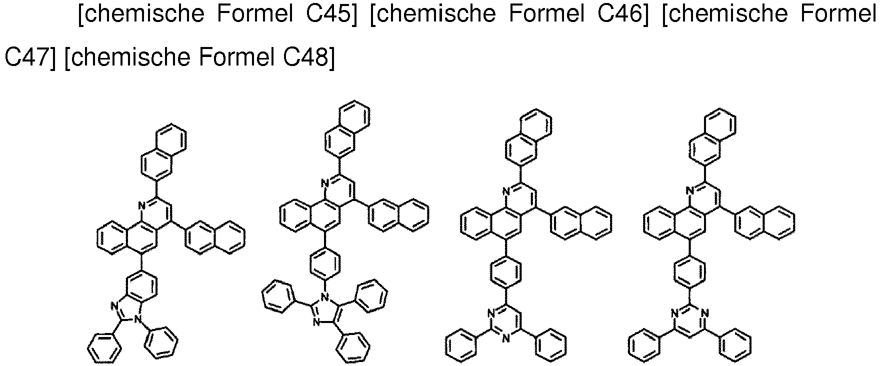

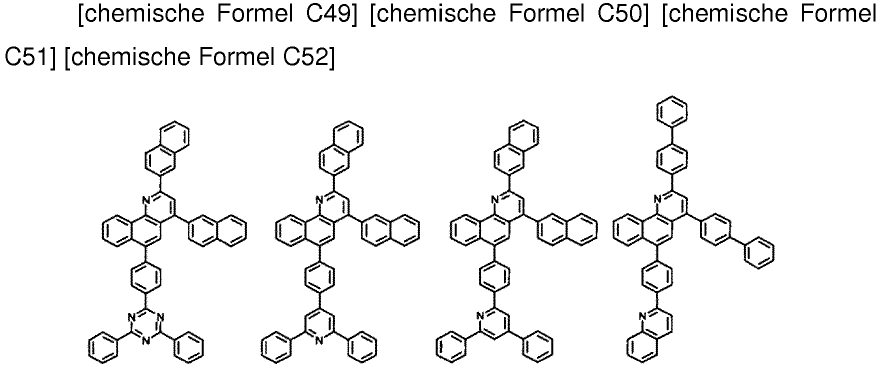

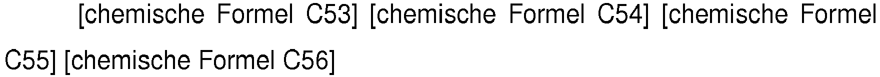

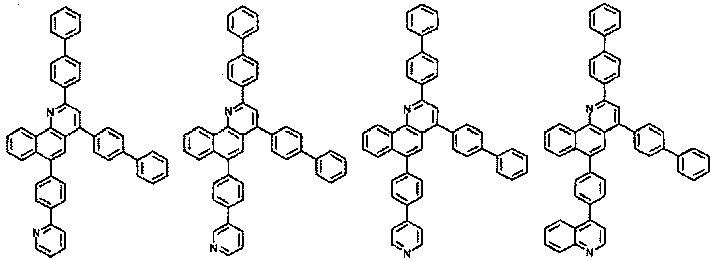

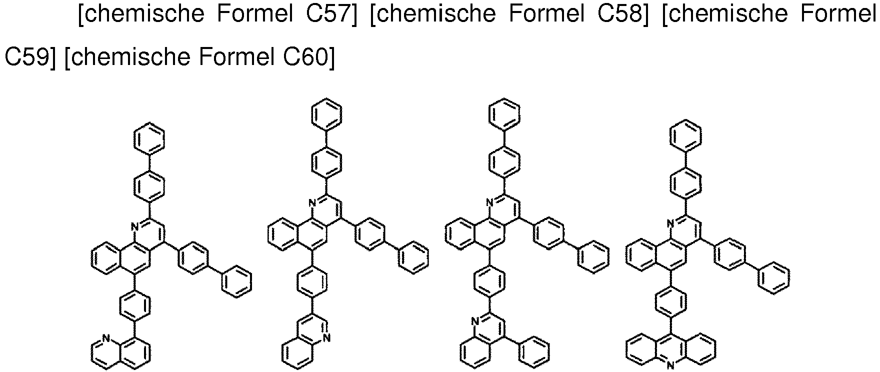

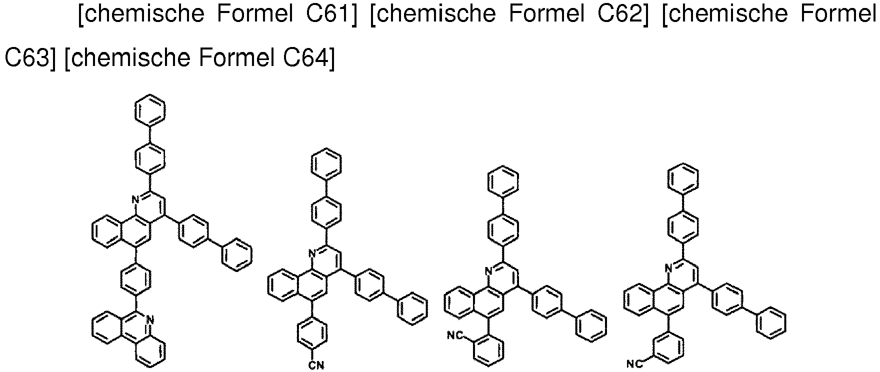

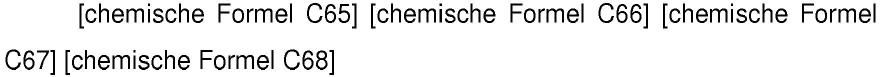

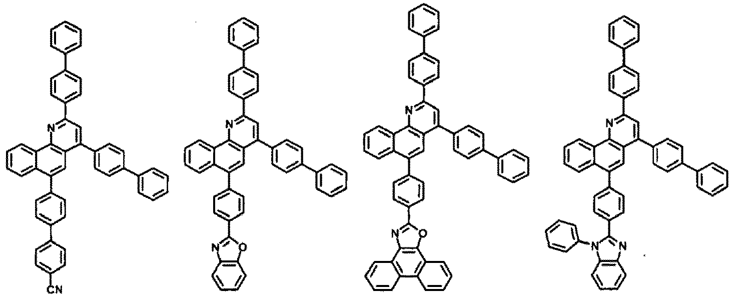

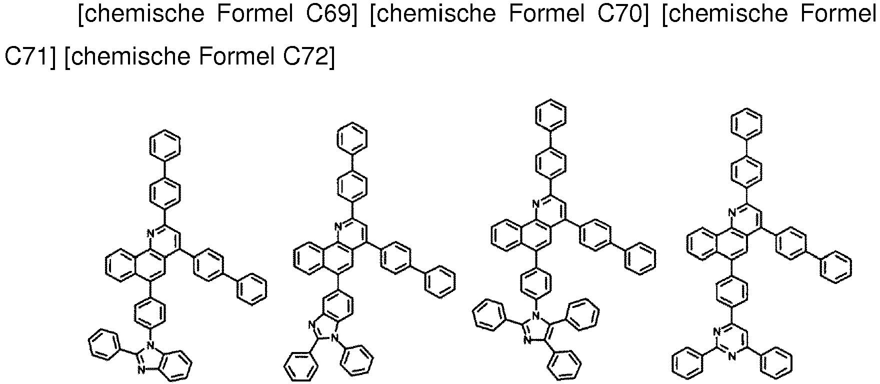

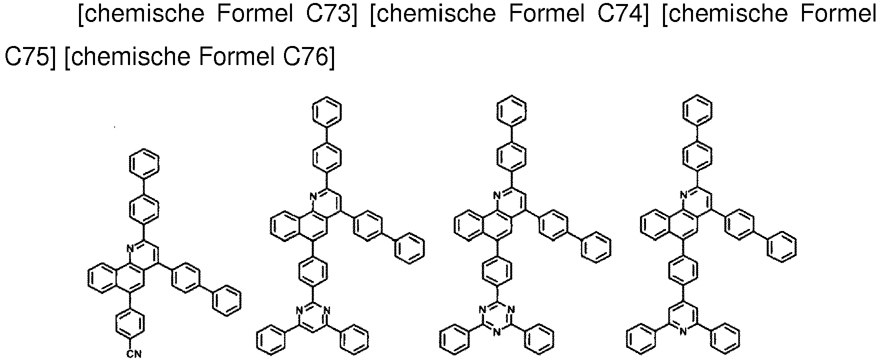

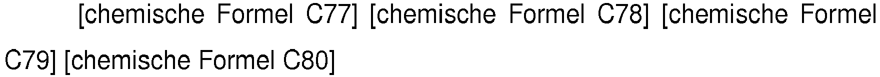

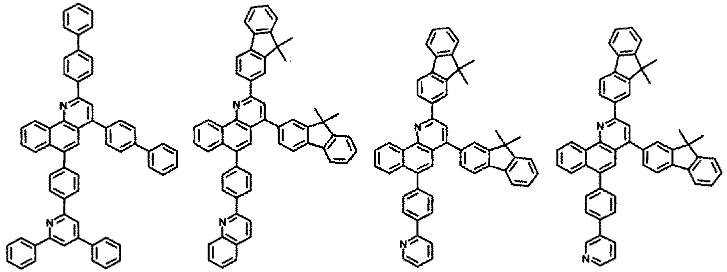

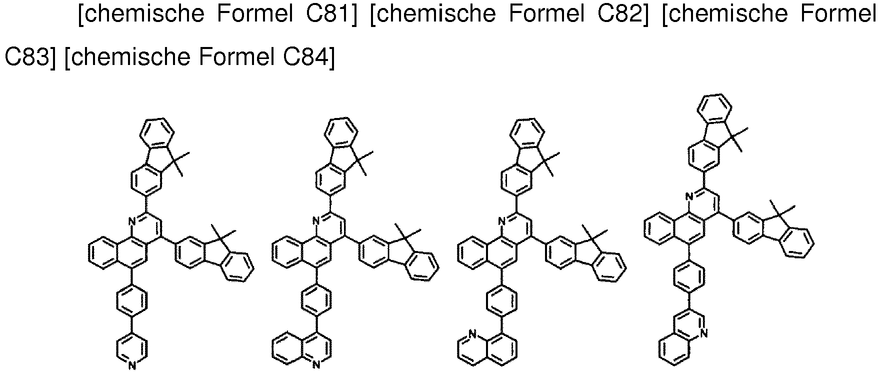

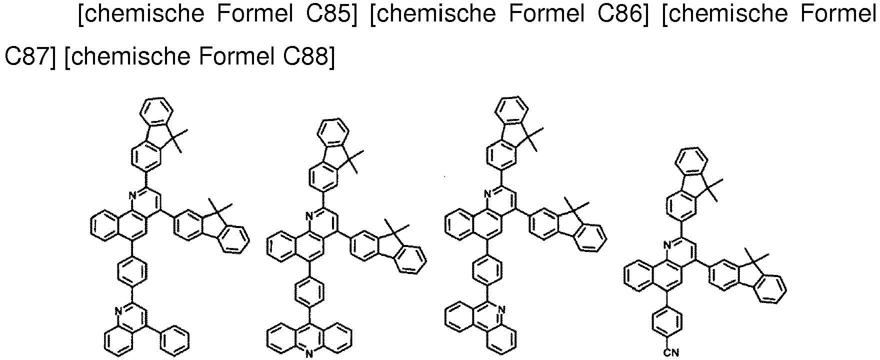

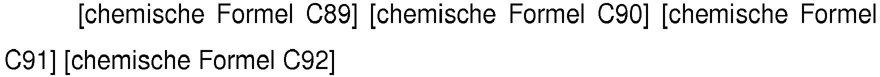

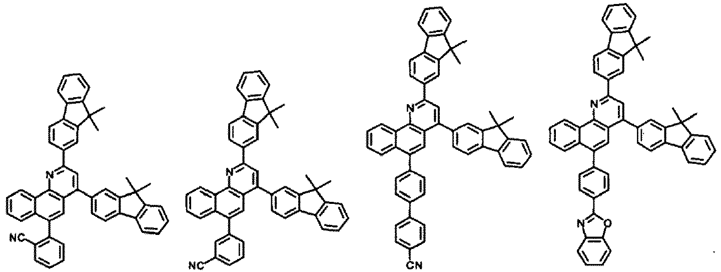

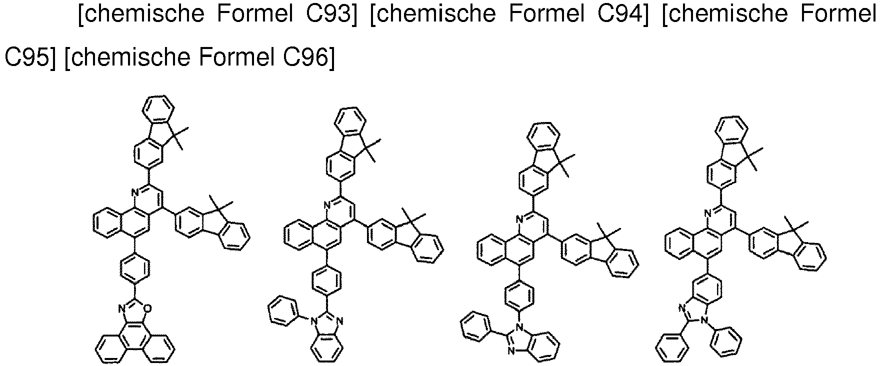

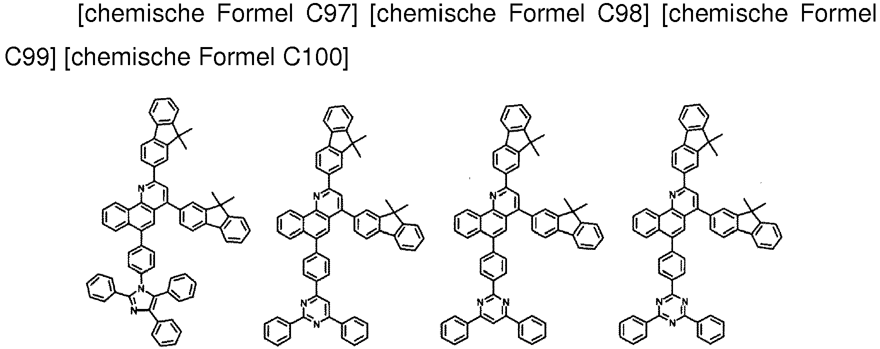

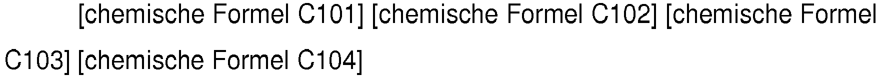

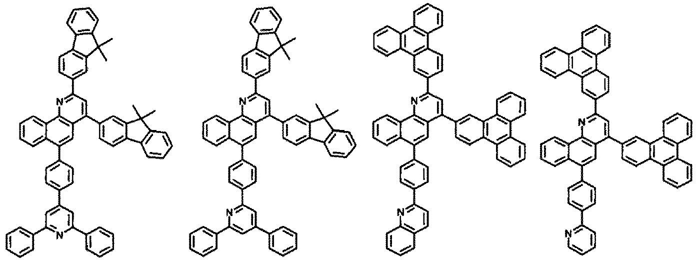

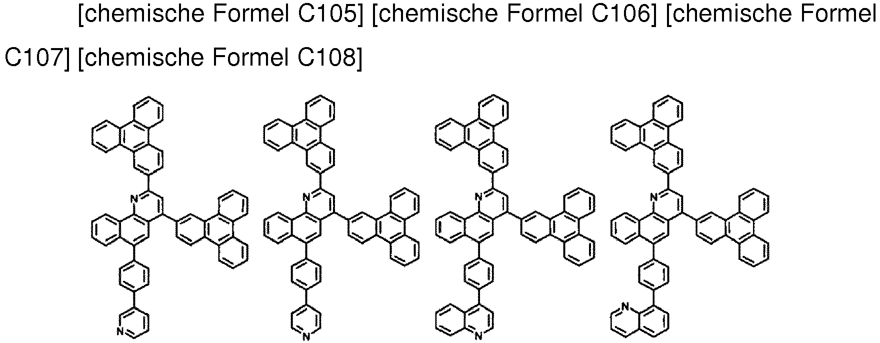

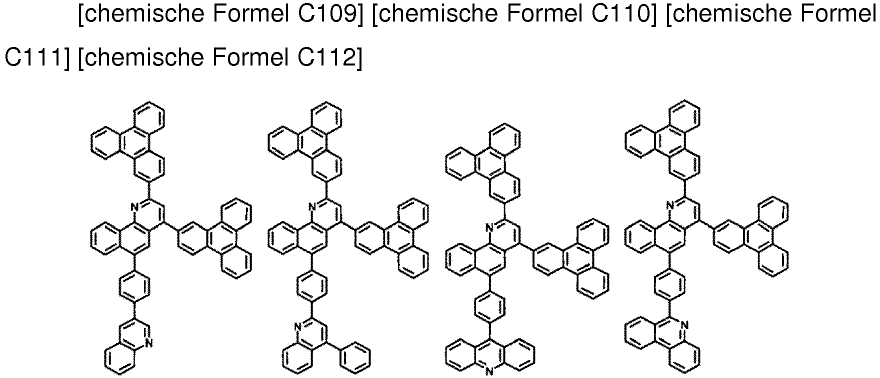

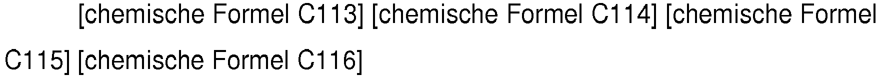

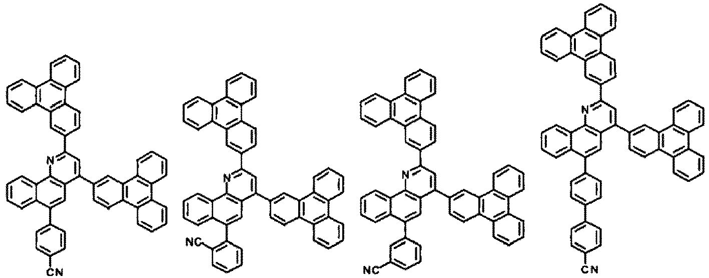

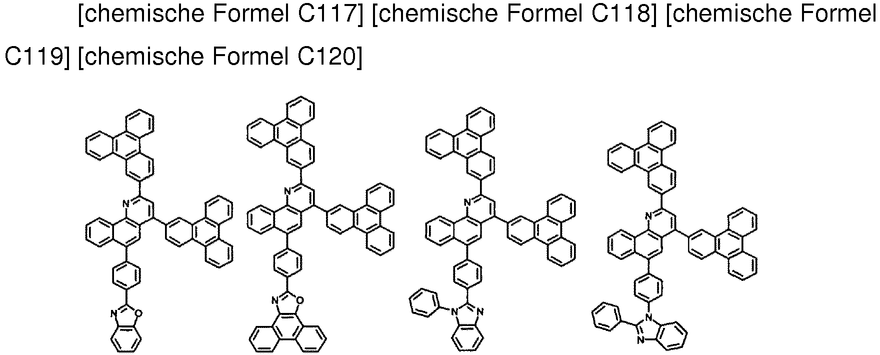

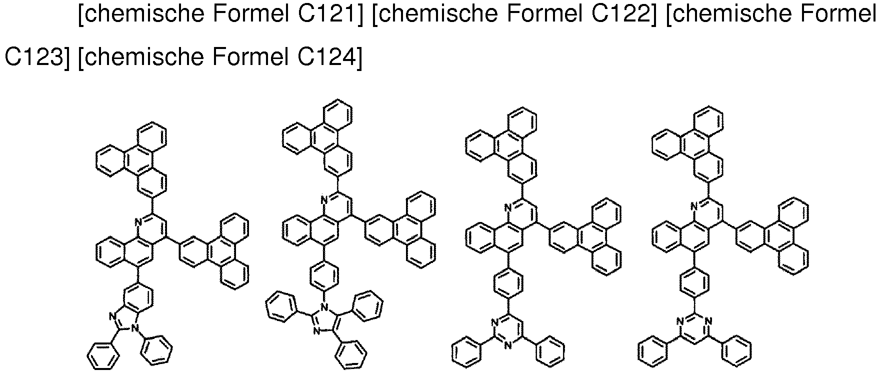

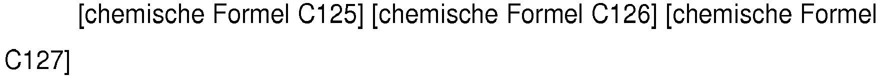

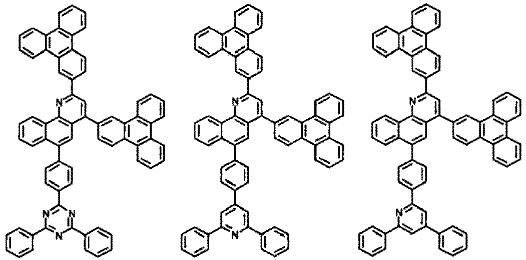

10. Organische Leuchtdiode, mit
einer Anode, einer Kathode und mindestens einer oder mehreren organischen Dünnschichten zwischen der Anode und der Kathode,
wobei mindestens eine organische Dünnschicht die Verbindung für ein optoelektronisches Bauelement nach einem der Ansprüche 1 bis 9 aufweist.

11. Organische Leuchtdiode nach Anspruch 10, wobei die organische Dünnschicht ausgewählt ist aus der Gruppe bestehend aus einer Emissionsschicht, einer Lochtransportschicht (HTL), einer Lochinjektionsschicht (HIL), einer Elektronentransportschicht (ETL), einer Elektroneninjektionsschicht (EIL), einer Lochsperrschicht und einer Kombination davon.

12. Organische Leuchtdiode nach Anspruch 10, wobei die Verbindung für ein organisches optoelektronisches Bauelement in einer Elektronentransportschicht (ETL) oder in einer Elektroneninjektionsschicht (EIL) enthalten ist.

13. Displayvorrichtung mit der organischen Leuchtdiode nach Anspruch 10.

## Revendications

1. Composé pour un dispositif optoélectronique organique représenté par la Formule Chimique 2 suivante : 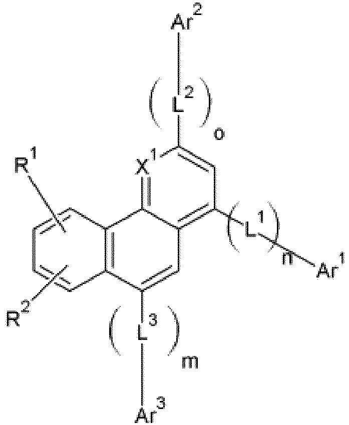 dans lequel, dans la Formule Chimique 2,
X¹ est -N-
R¹ et R² sont identiques ou différents et représentent indépendamment un atome d'hydrogène, un atome de deutérium, un groupe alkyle en C1 à C20 substitué ou non substitué, un groupe aryle en C6 à C30 substitué ou non substitué, un groupe hétéroaryle en C3 à C30 substitué ou non substitué, ou une combinaison de ceux-ci,
Ar¹ à Ar³ sont identiques ou différents et représentent indépendamment un groupe aryle en C6 à C30 substitué ou non substitué ; ou un groupe hétéroaryle en C3 à C30 substitué ou non substitué,
L¹ à L³ sont identiques ou différents et représentent indépendamment une liaison simple, un groupe alcényle en C2 à C6 substitué ou non substitué, un groupe alcynyle en C2 à C6 substitué ou non substitué, un groupe arylène en C6 à C30 substitué ou non substitué, un groupe hétéroarylène en C3 à C30 substitué ou non substitué, ou une combinaison de ceux-ci, et
n, m ou o sont identiques ou différents et représentent indépendamment 0 ou 1.

2. Composé pour un dispositif optoélectronique organique selon la Revendication 1, dans lequel au moins l'un de Ar¹ ou Ar² peut être un groupe hétéroaryle en C3 à C30 substitué ou non substitué.

3. Composé pour un dispositif optoélectronique organique selon la Revendication 1, dans lequel Ar¹ est un groupe hétéroaryle en C3 à C30 substitué ou non substitué, et Ar² et Ar³ sont un groupe aryle en C6 à C30 substitué ou non substitué.

4. Composé pour un dispositif optoélectronique organique selon la Revendication 1, dans lequel Ar² est un groupe hétéroaryle en C3 à C30 substitué ou non substitué, et Ar¹ et Ar³ sont un groupe aryle en C6 à C30 substitué ou non substitué.

5. Composé pour un dispositif optoélectronique organique selon la Revendication 1, dans lequel le groupe hétéroaryle en C3 à C30 substitué ou non substitué est un groupe imidazolyle substitué ou non substitué, un groupe triazolyle substitué ou non substitué, un groupe tétrazolyle substitué ou non substitué, un carbazolyle substitué ou non substitué, un groupe oxadiazolyle substitué ou non substitué, un groupe oxatriazolyle substitué ou non substitué, un groupe thiatriazolyle substitué ou non substitué, un groupe benzimidazolyle substitué ou non substitué, un groupe benzotriazolyle substitué ou non substitué, un groupe pyridinyle substitué ou non substitué, un groupe pyrimidinyle substitué ou non substitué, un groupe triazinyle substitué ou non substitué, un groupe pyrazinyle substitué ou non substitué, un groupe pyridazinyle substitué ou non substitué, un groupe purinyle substitué ou non substitué, un groupe quinolinyle substitué ou non substitué, un groupe isoquinolinyle substitué ou non substitué, un groupe phtalazinyle substitué ou non substitué, un groupe naphpyridinyle substitué ou non substitué, un groupe quinoxalinyle substitué ou non substitué, un groupe quinazolinyle substitué ou non substitué, un groupe acridinyle substitué ou non substitué, un groupe phénanthrolinyle substitué ou non substitué, un groupe phénazinyle substitué ou non substitué, ou une combinaison de ceux-ci.

6. Composé pour un dispositif optoélectronique organique selon la Revendication 1, dans lequel le groupe aryle en C6 à C30 substitué ou non substitué est un groupe phényle substitué ou non substitué, un groupe naphtyle substitué ou non substitué, un groupe triperylenyl substitué ou non substitué, un groupe fluorényle substitué ou non substitué groupe, un groupe spirofluorenyle substitué ou non substitué, un groupe biphényle substitué ou non substitué, un groupe terphényle substitué ou non substitué, un groupe pyrényle substitué ou non substitué, un groupe pérylényle substitué ou non substitué, un groupe phénanthrényle substitué ou non substitué, un groupe anthracényle substitué ou non substitué, ou une combinaison de ceux-ci.

7. Composé pour un dispositif optoélectronique organique selon la Revendication 1 représenté par les Formules Chimiques A1 à A126 suivantes :

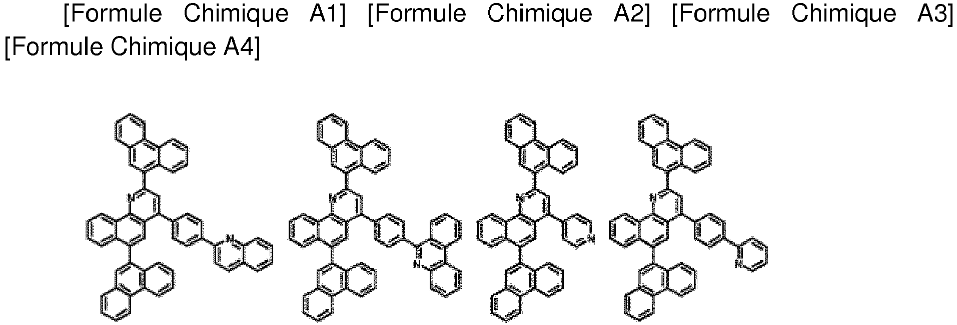

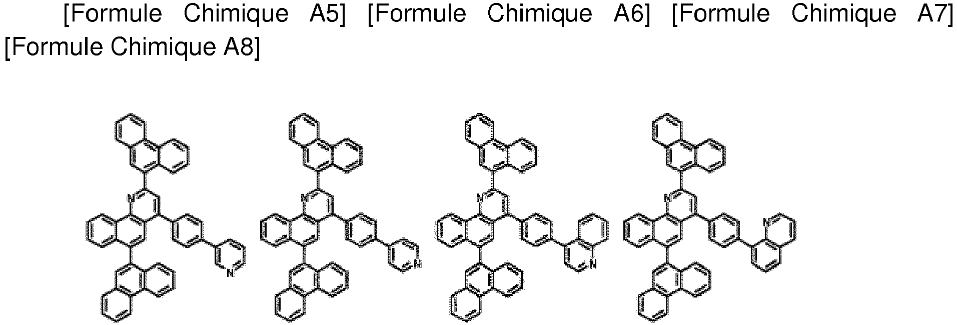

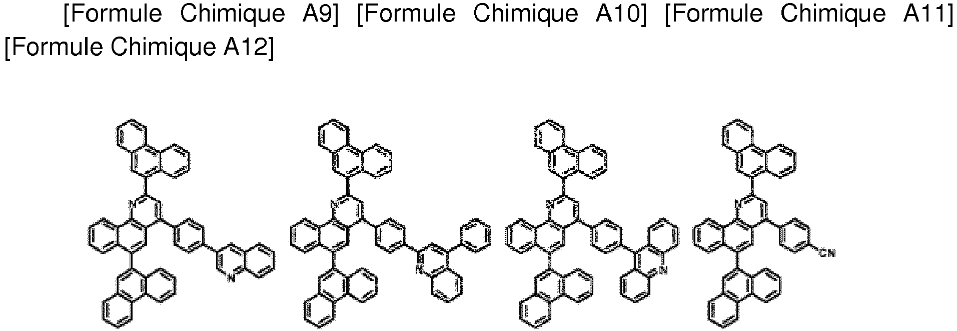

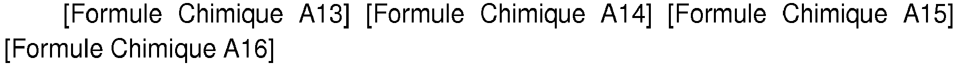

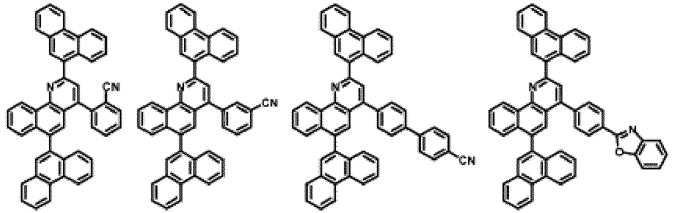

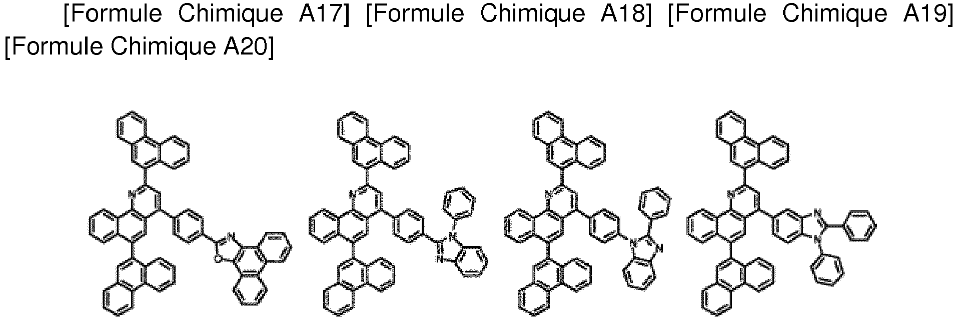

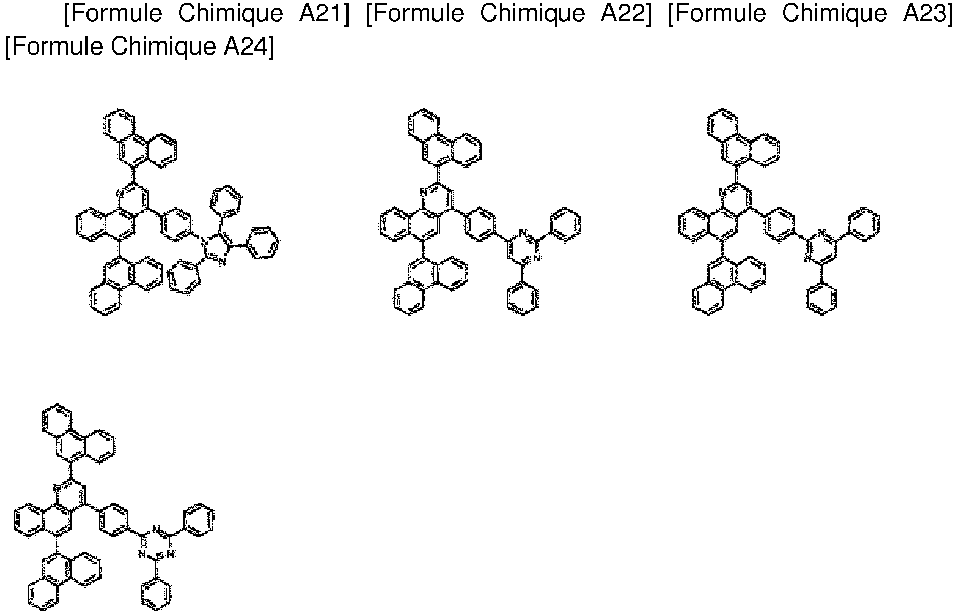

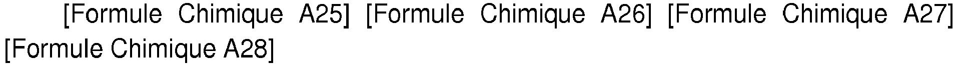

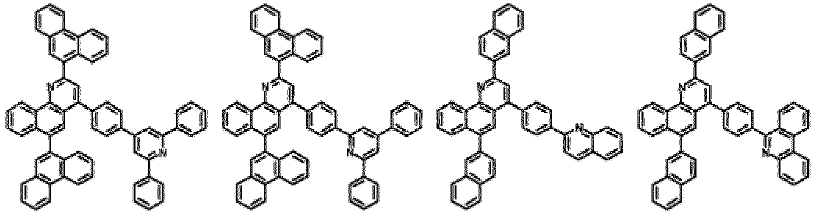

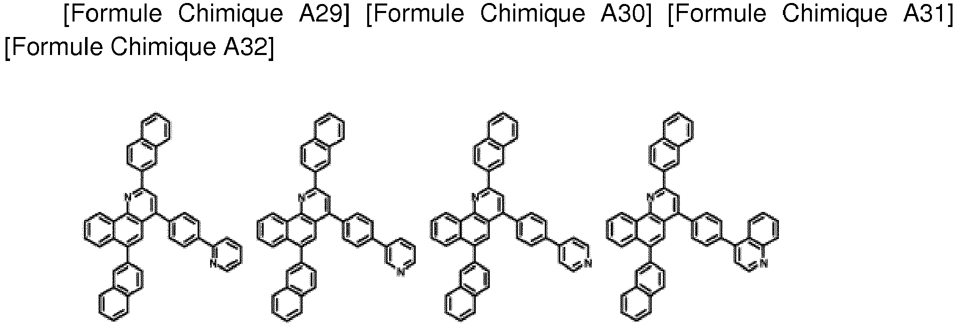

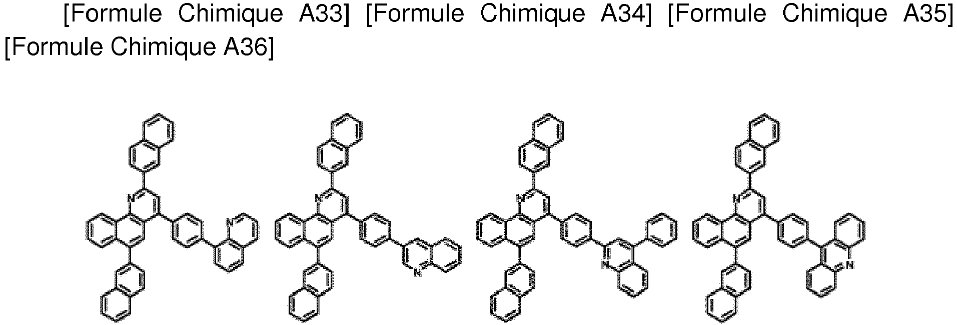

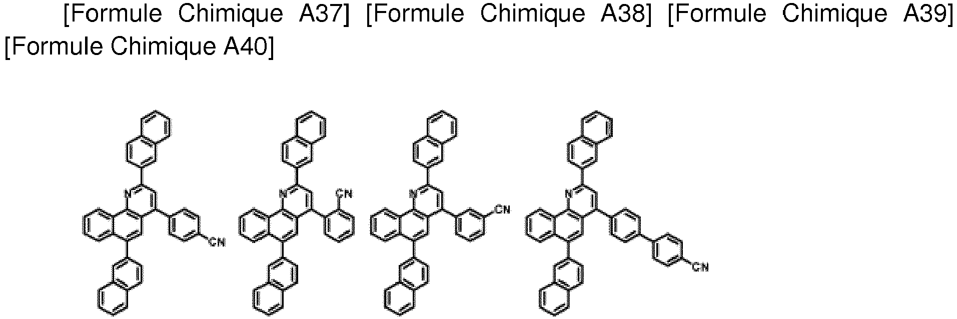

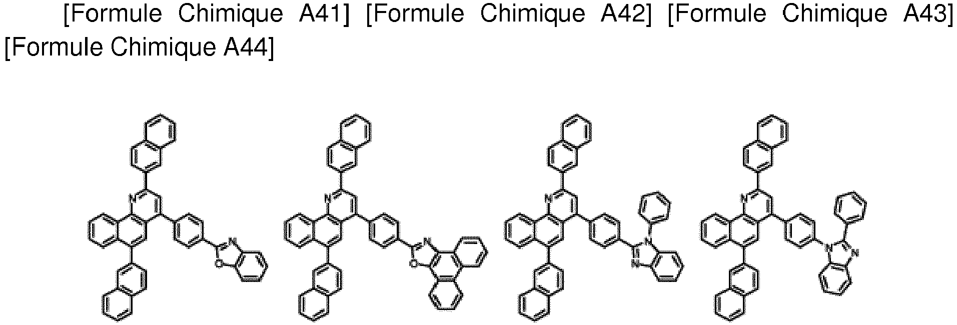

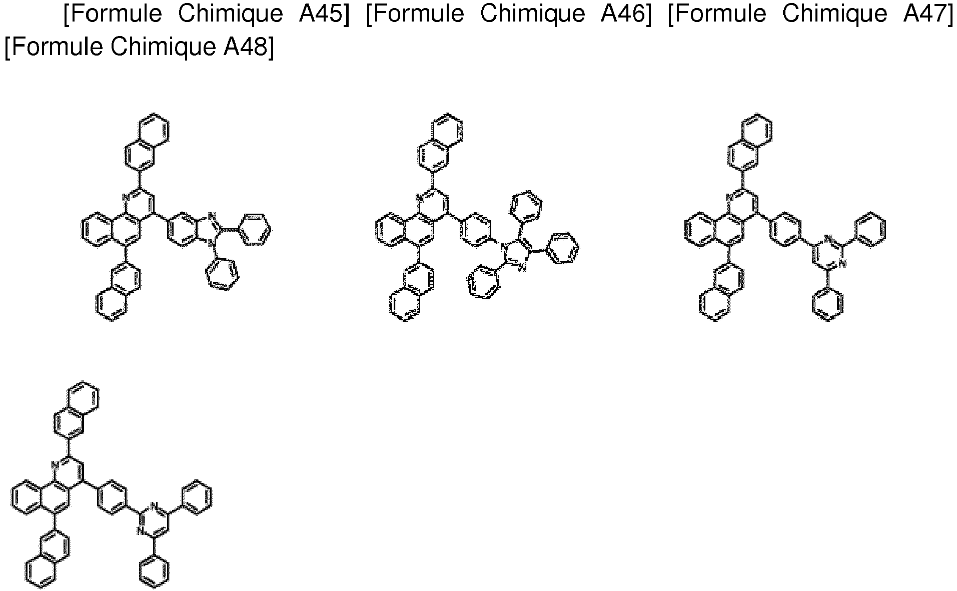

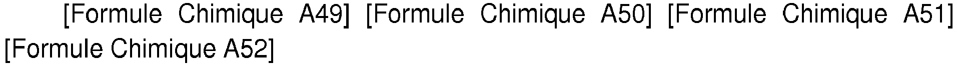

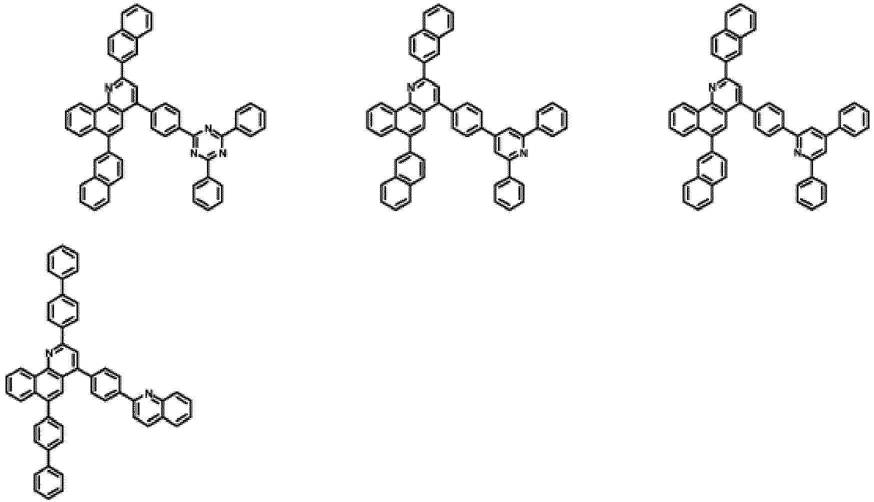

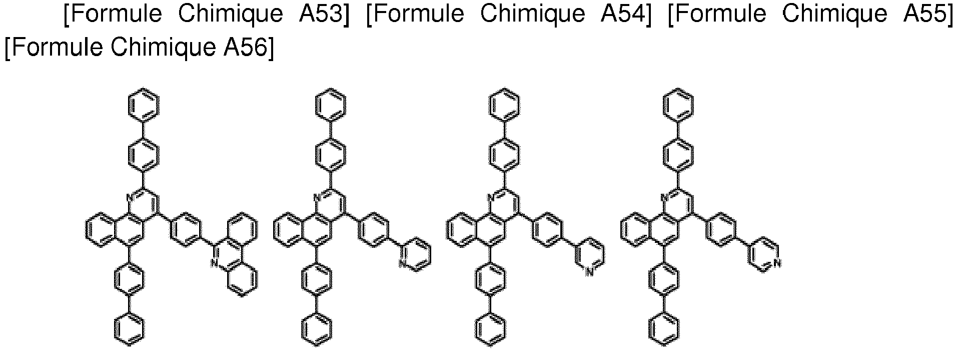

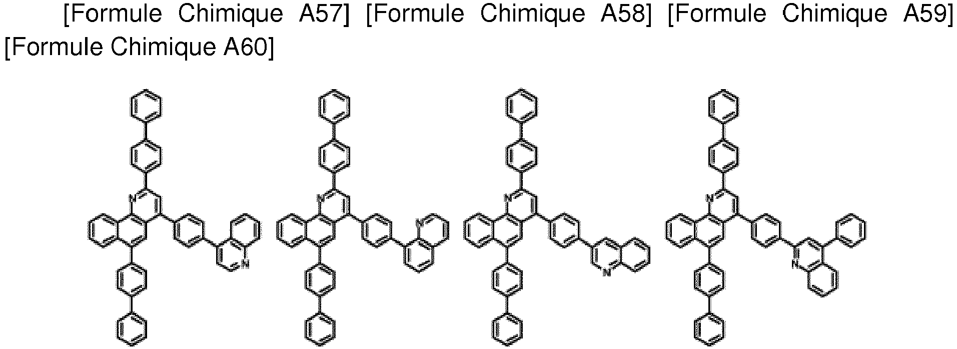

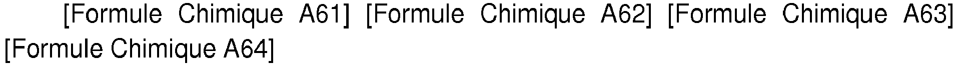

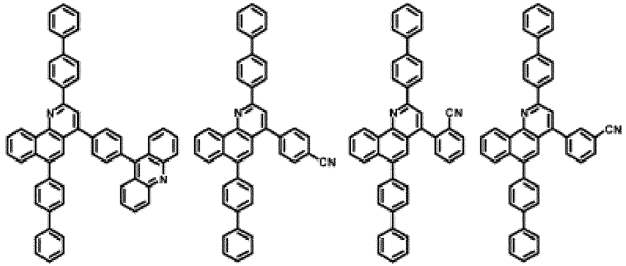

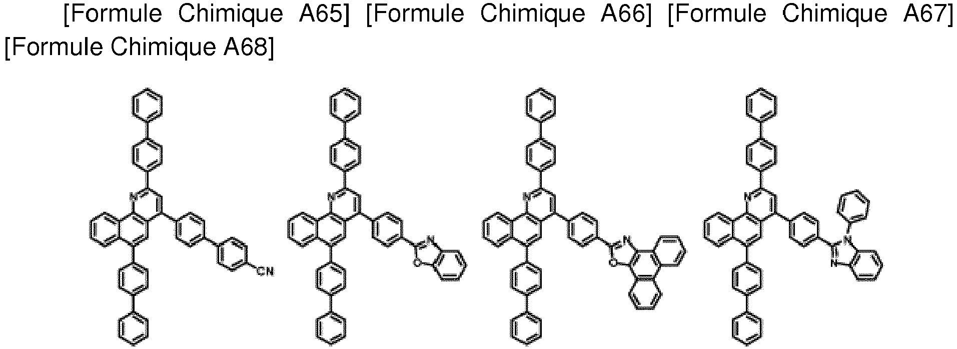

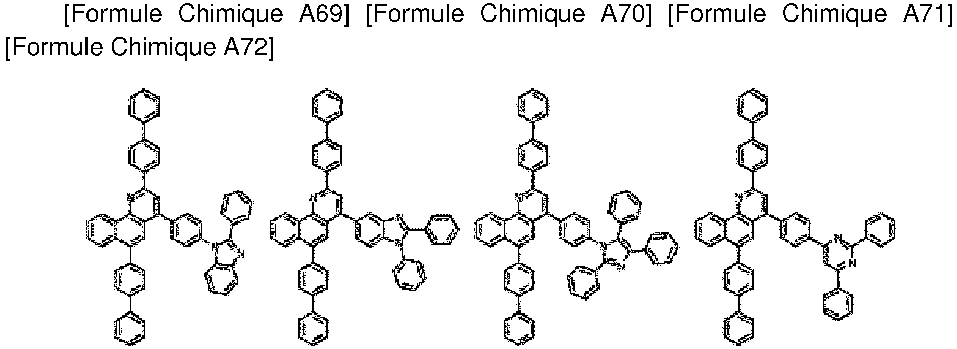

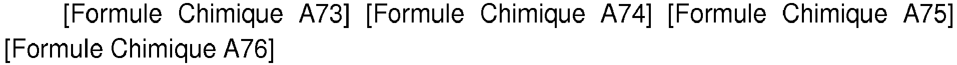

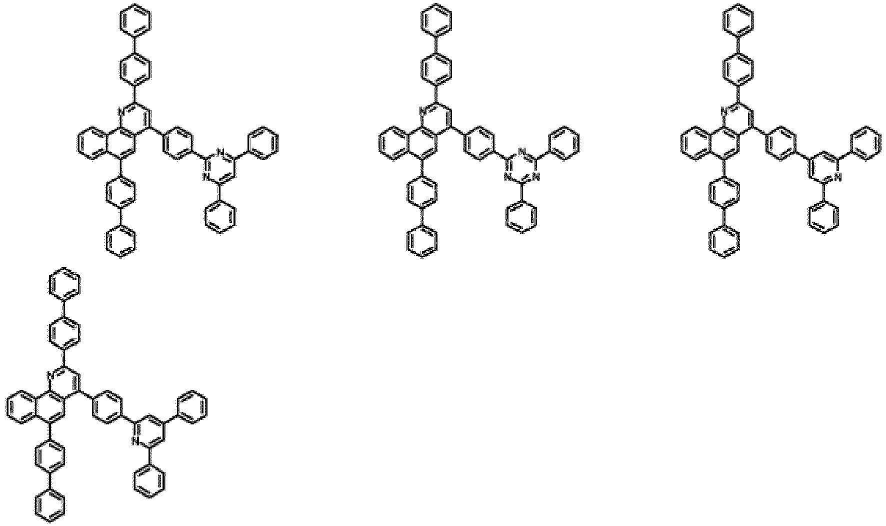

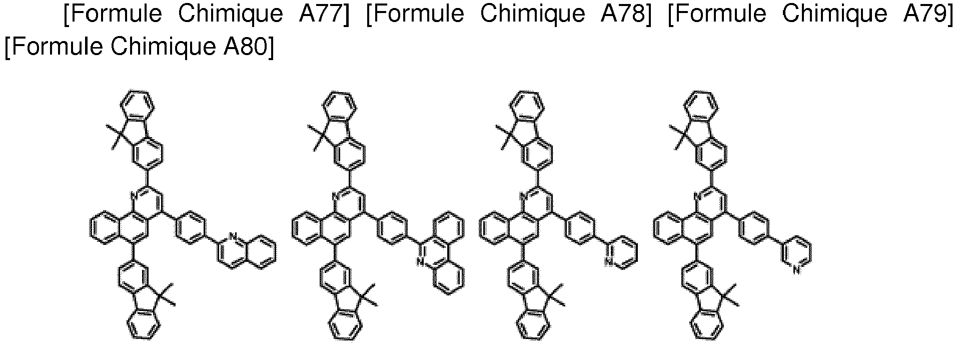

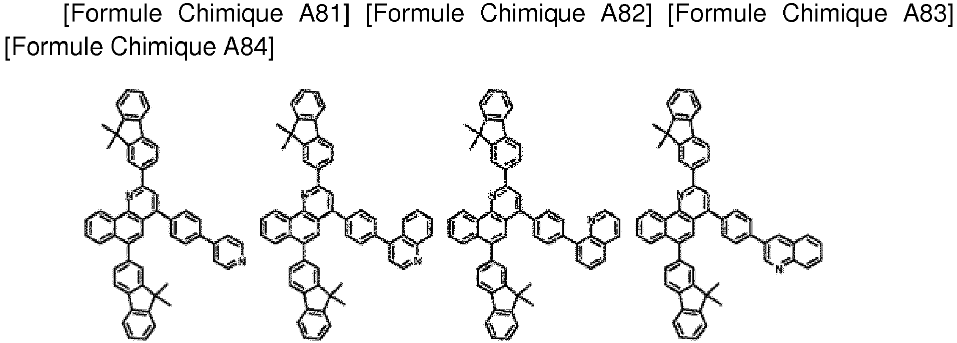

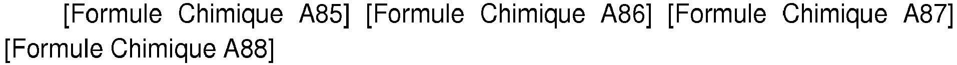

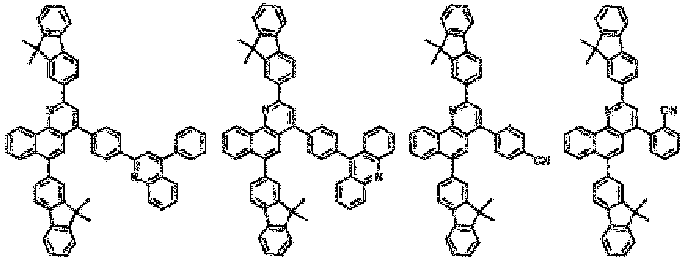

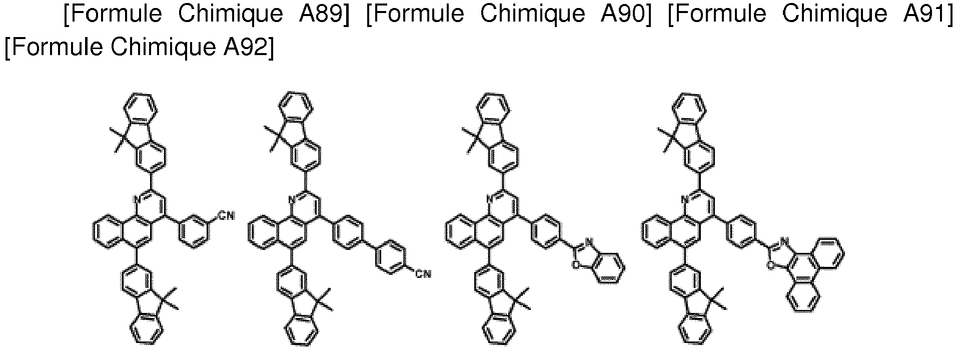

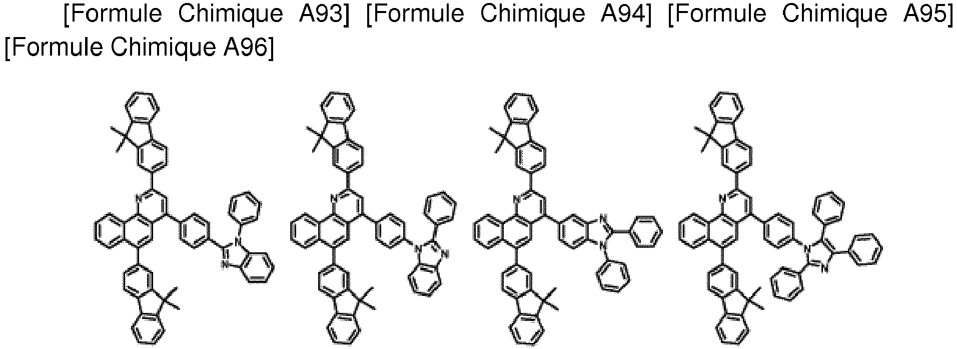

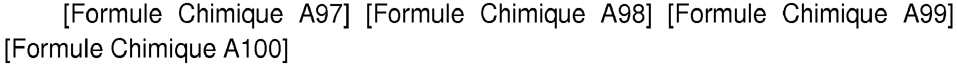

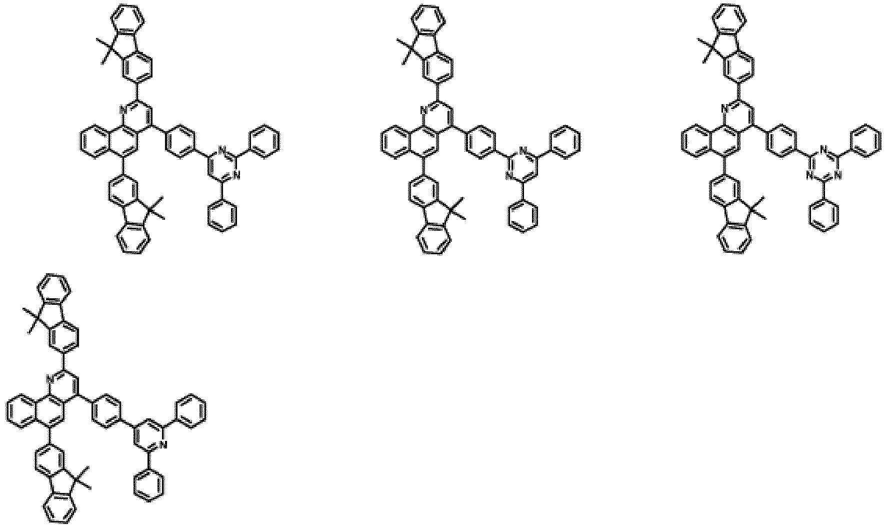

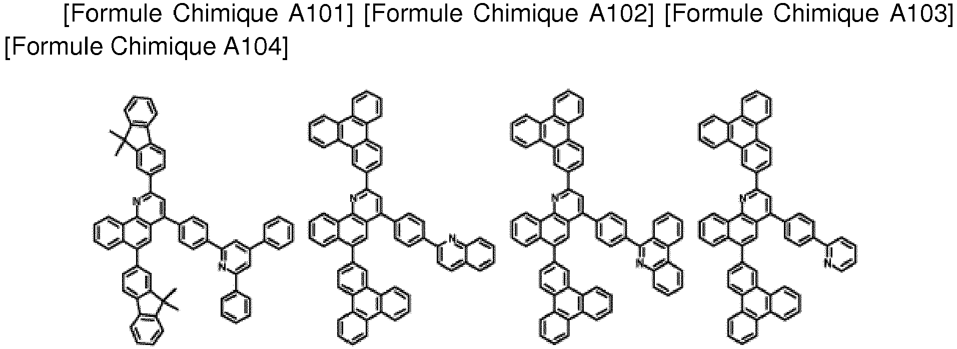

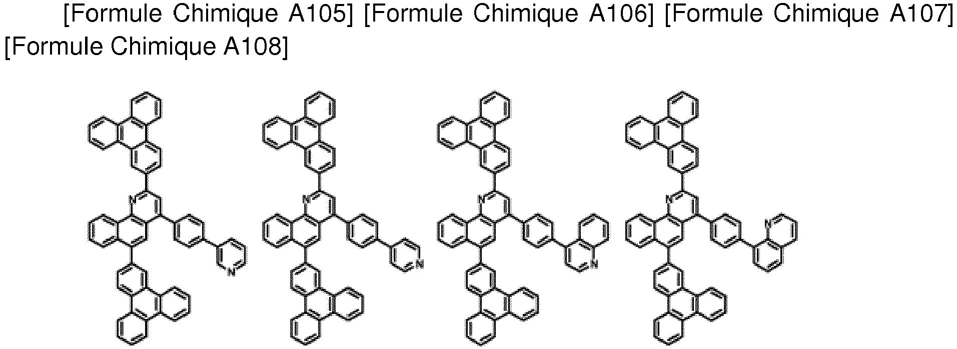

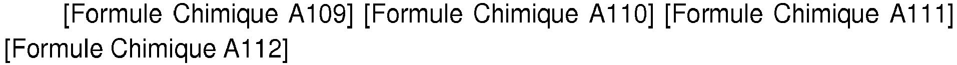

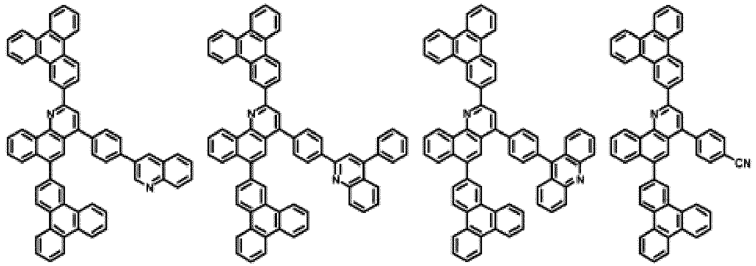

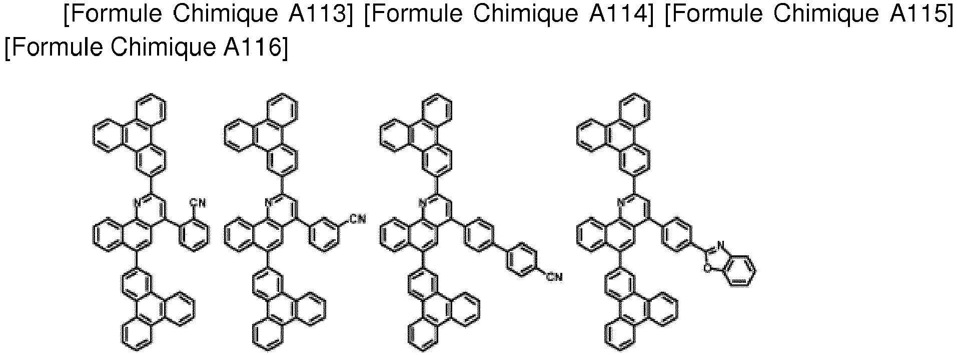

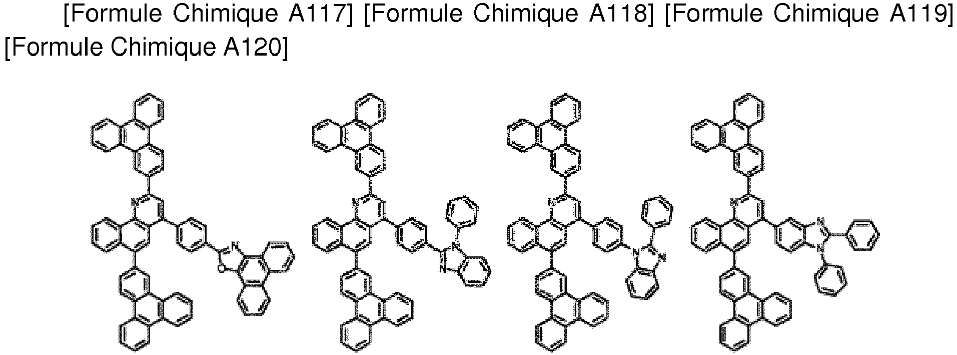

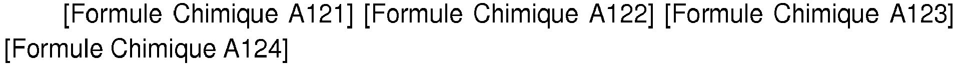

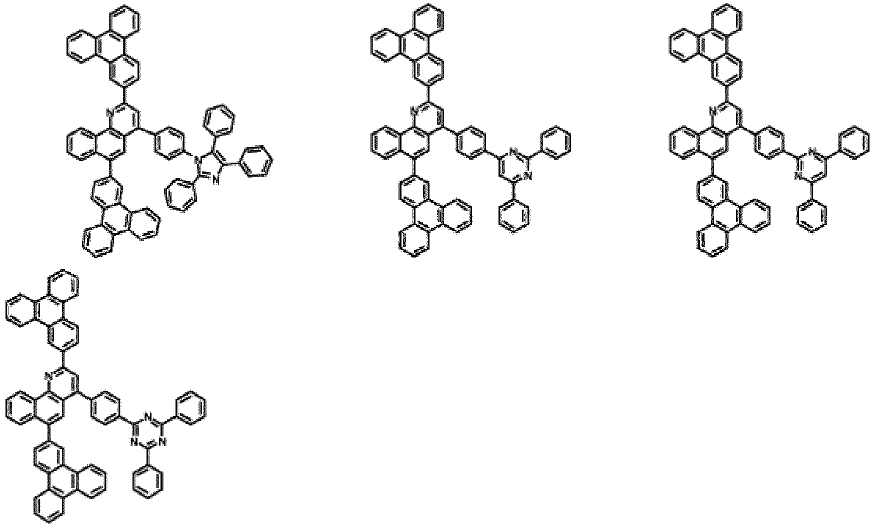

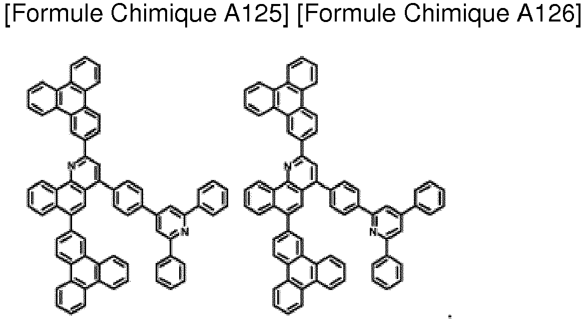

8. Composé pour un dispositif optoélectronique organique selon la Revendication 1 représenté par les Formules Chimiques B1 à B125 suivantes :

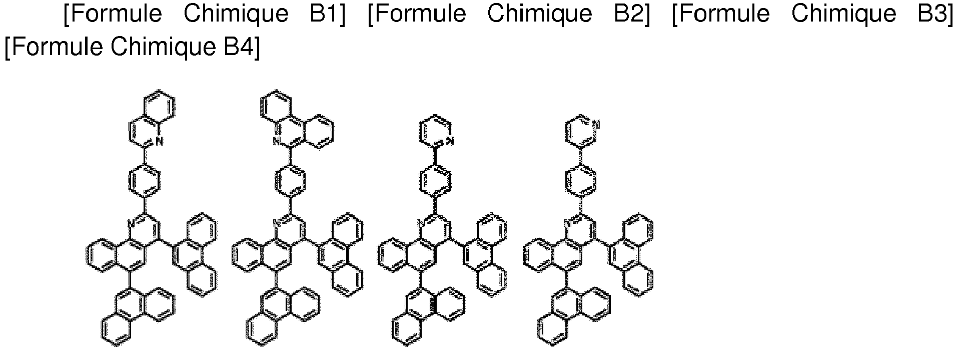

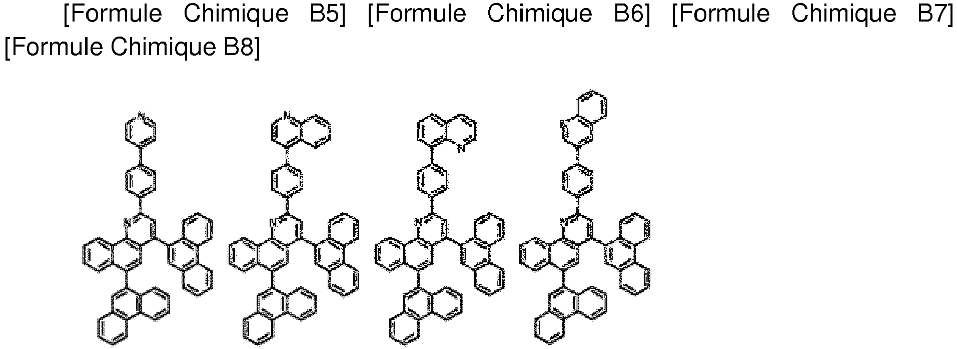

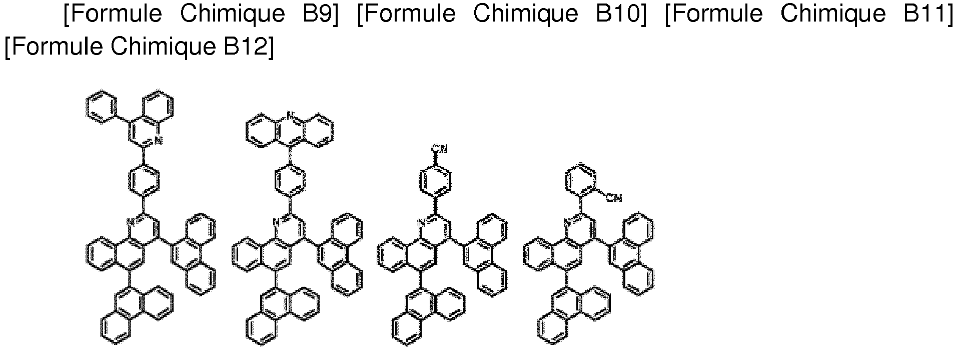

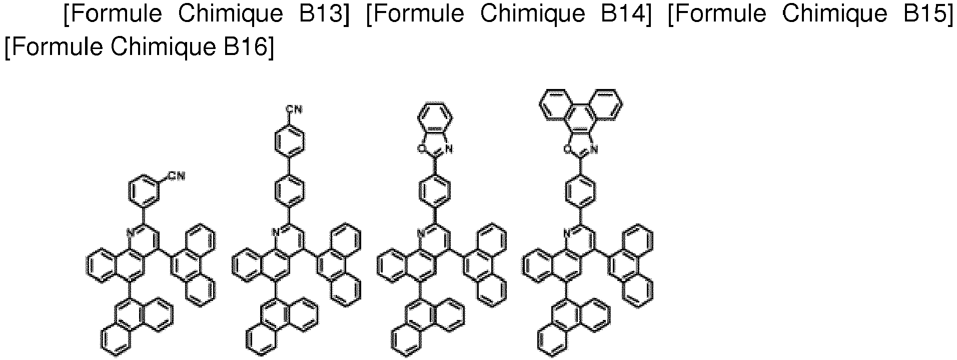

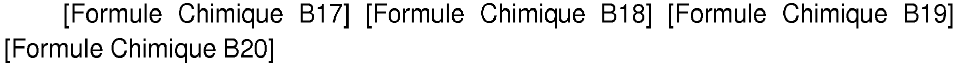

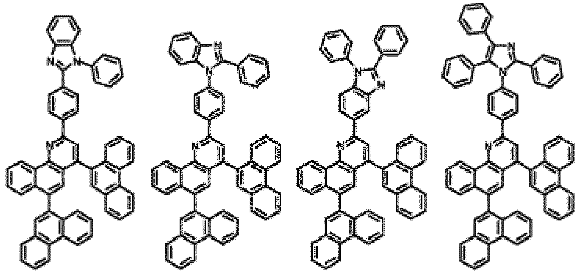

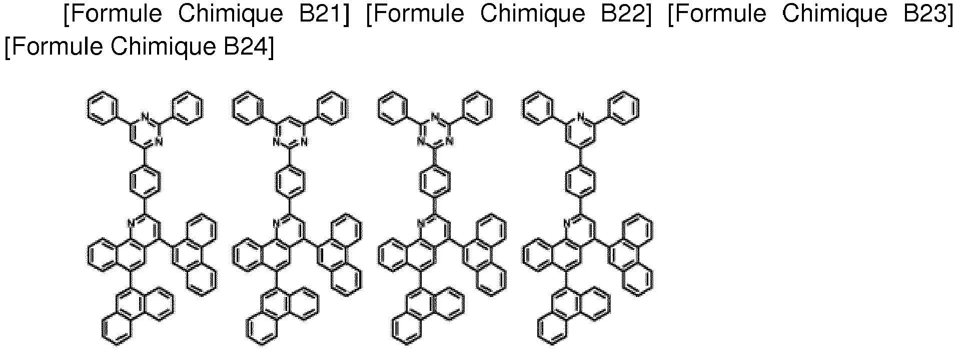

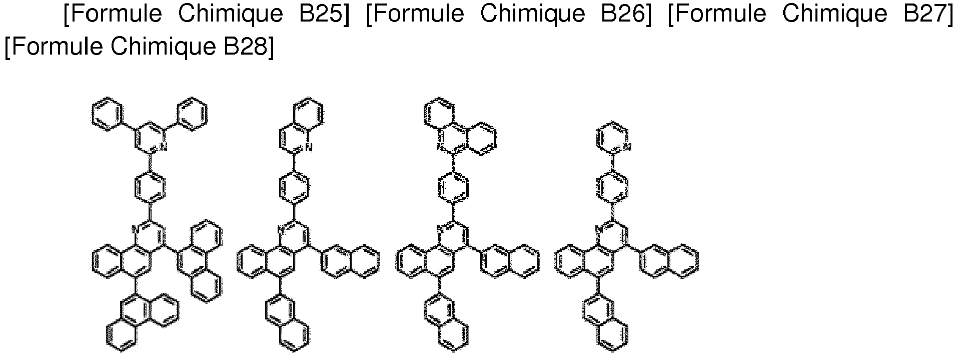

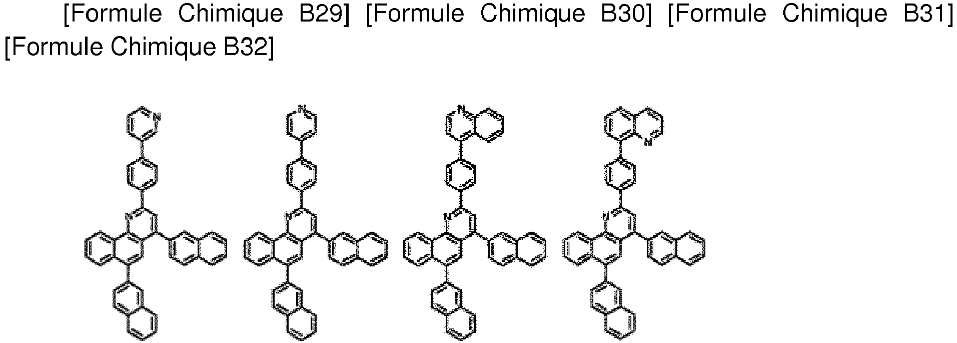

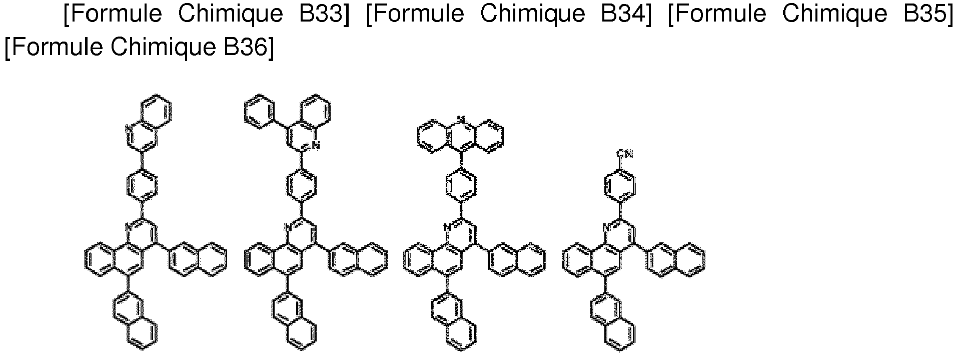

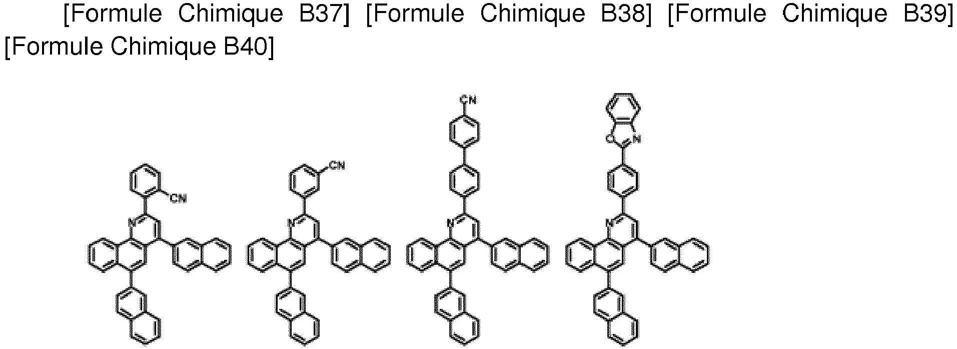

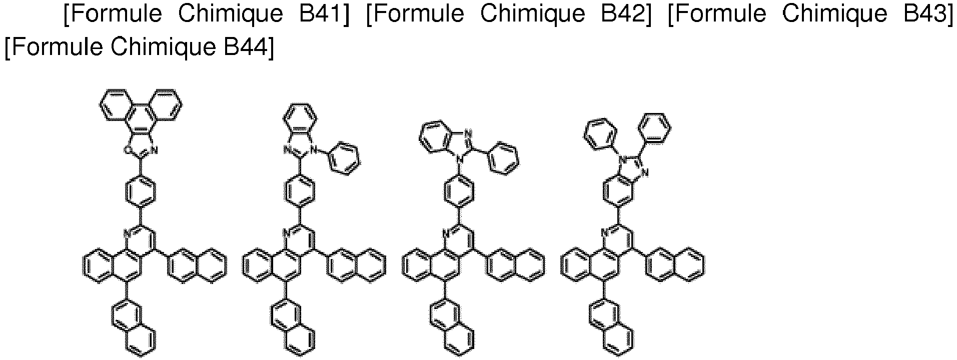

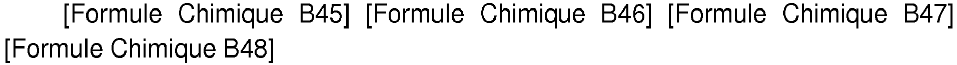

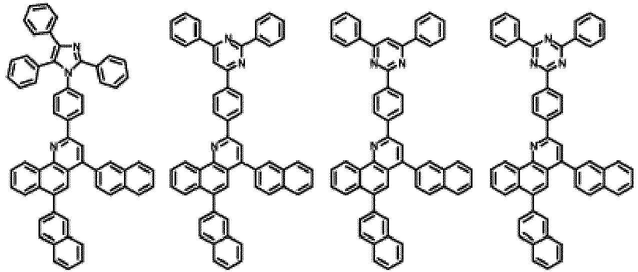

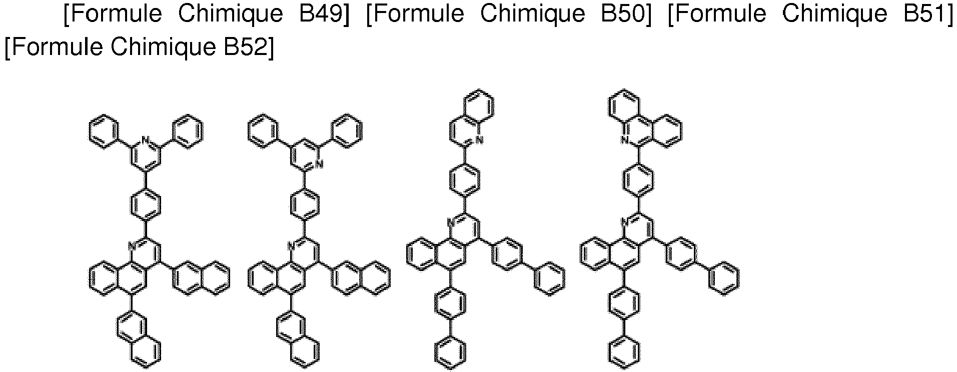

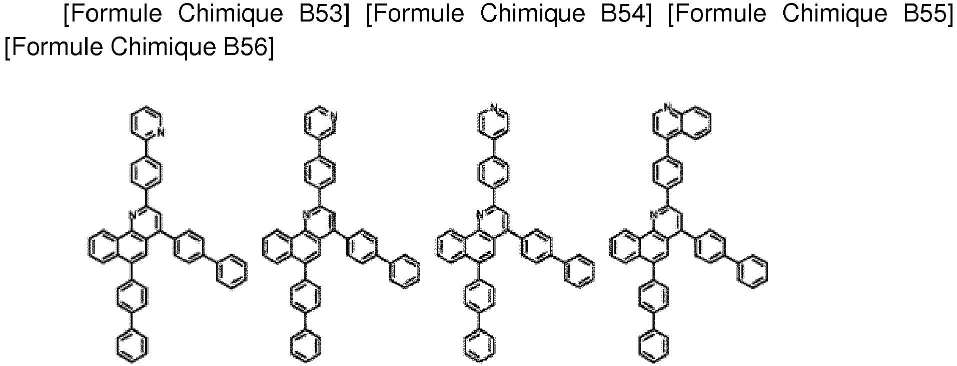

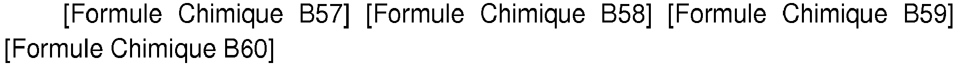

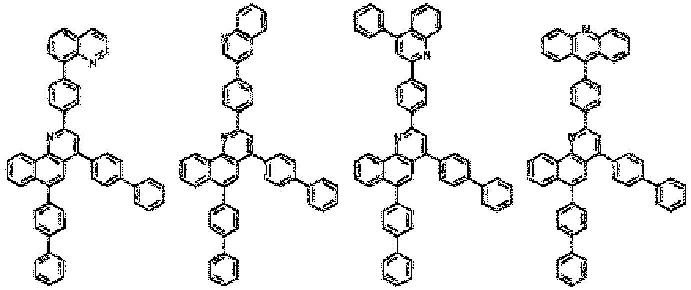

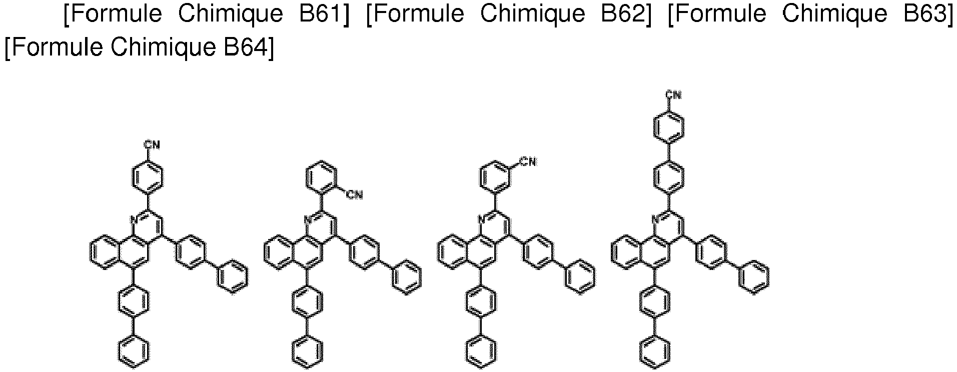

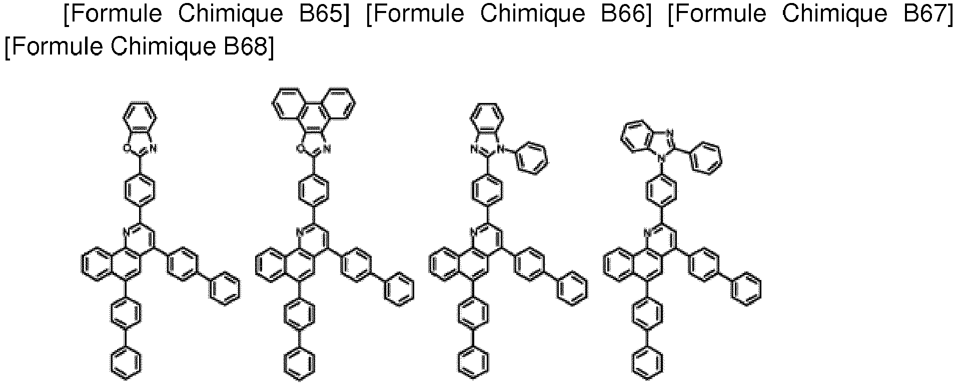

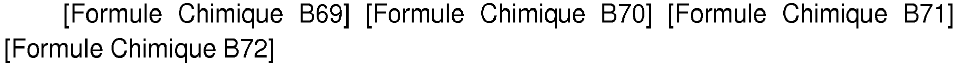

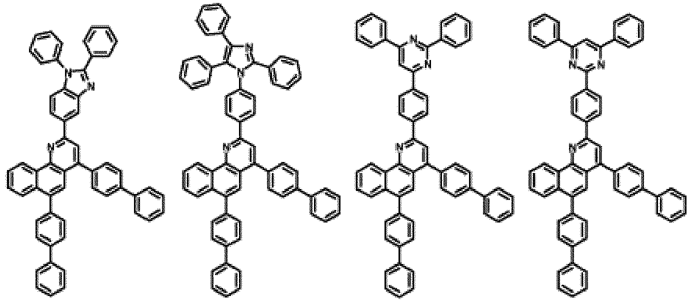

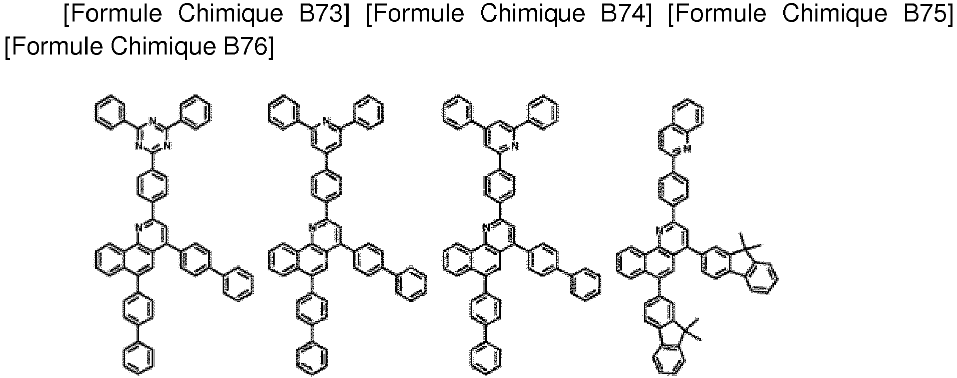

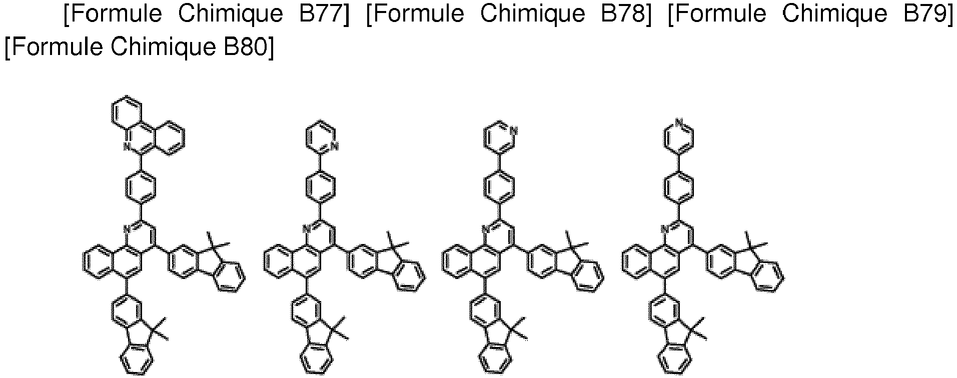

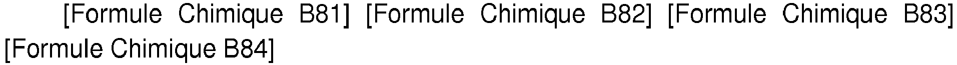

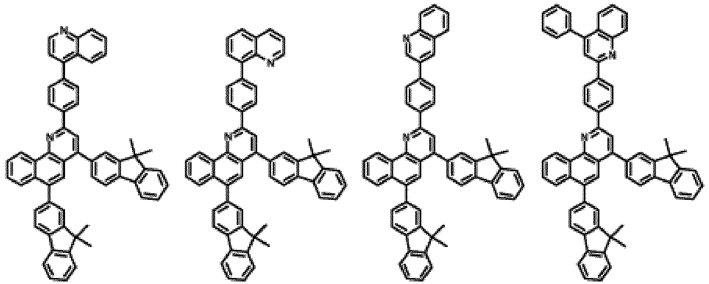

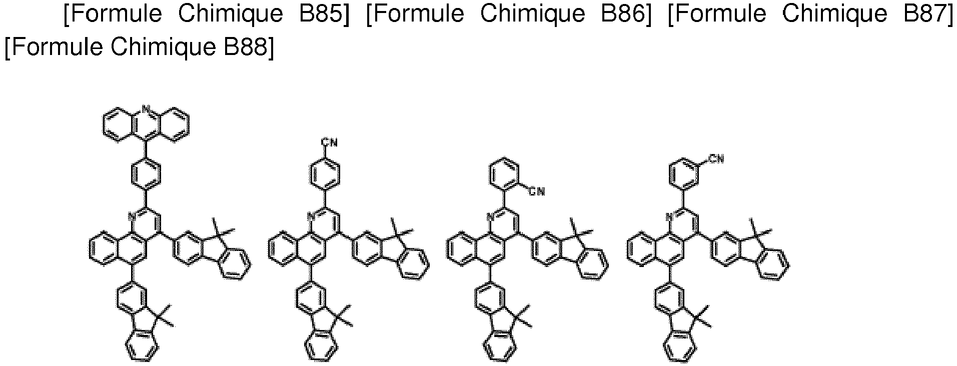

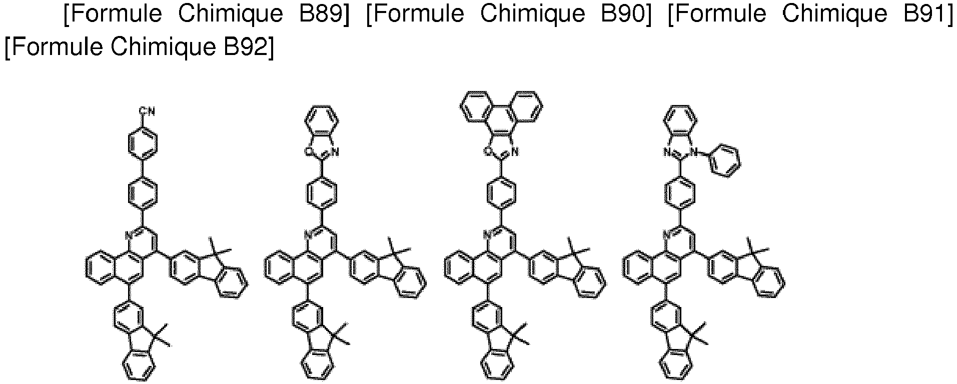

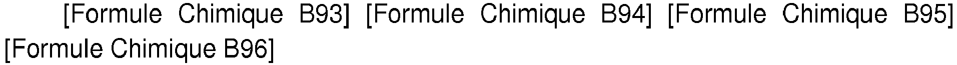

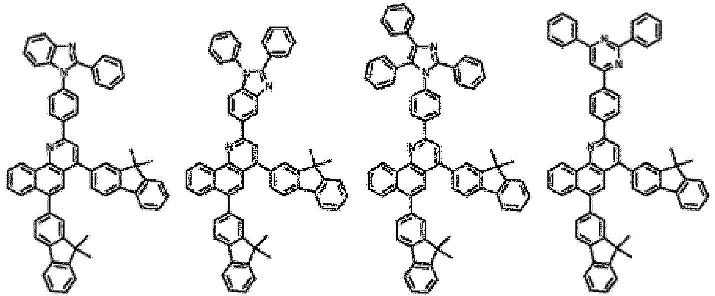

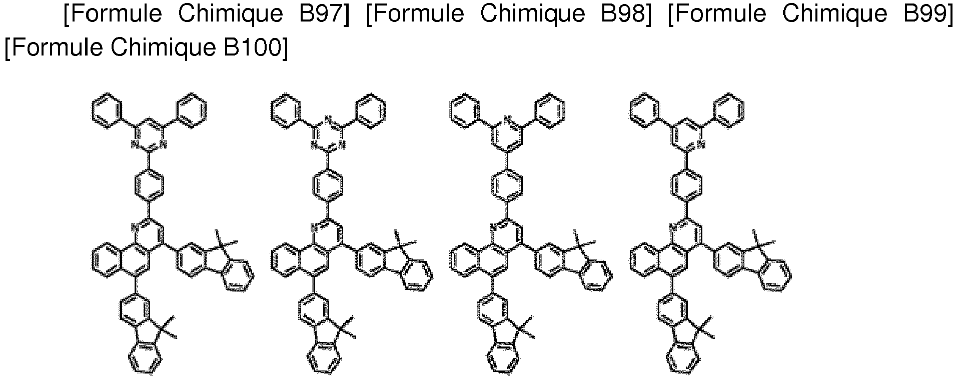

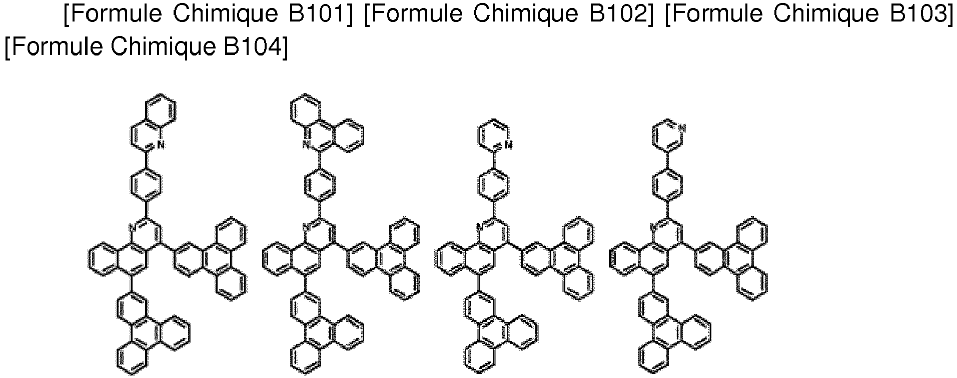

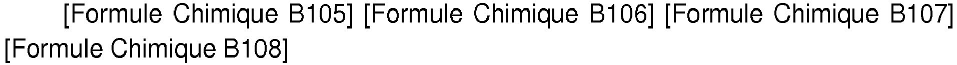

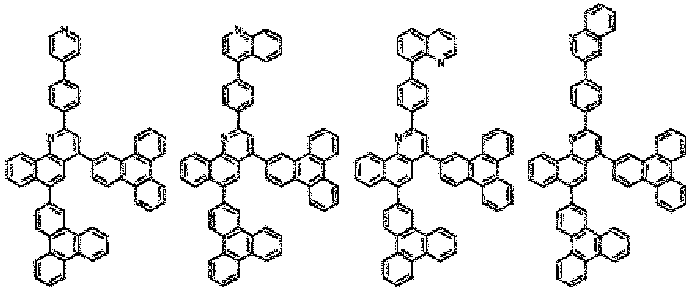

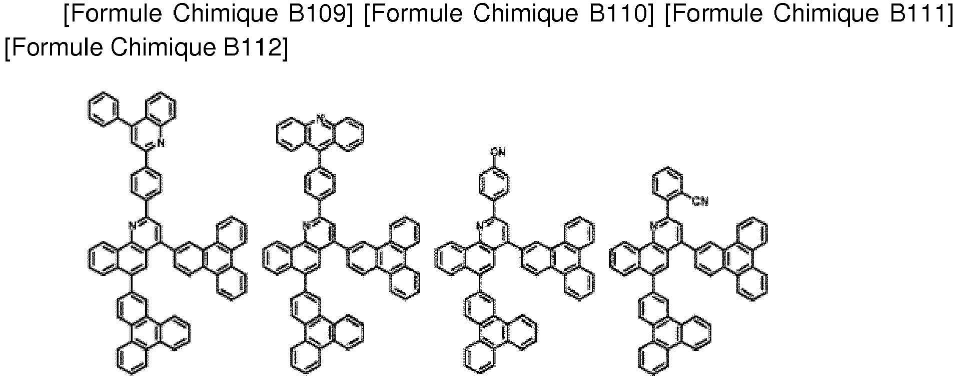

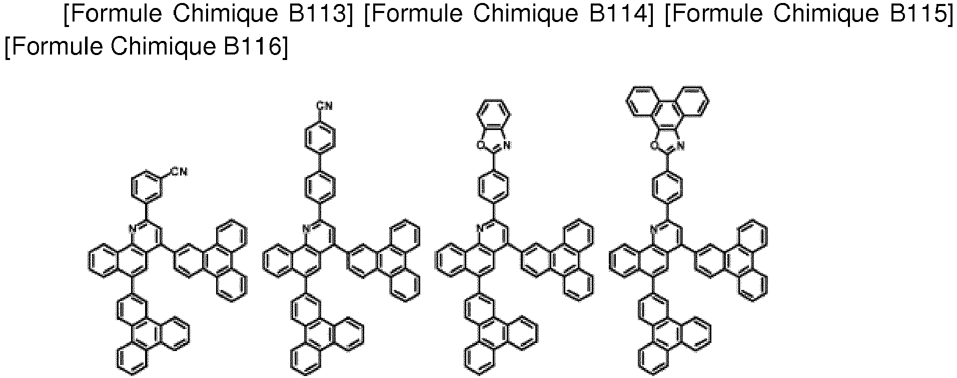

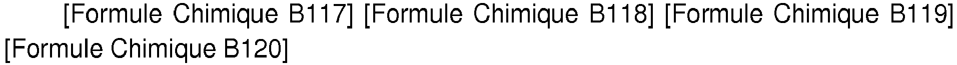

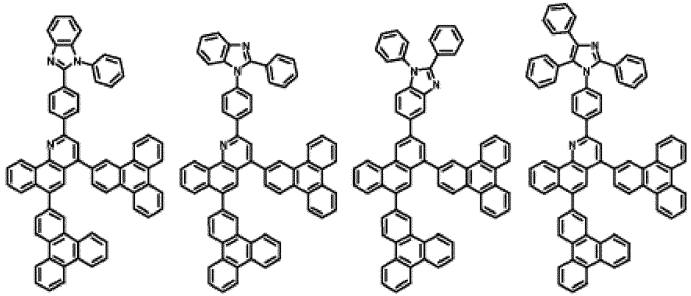

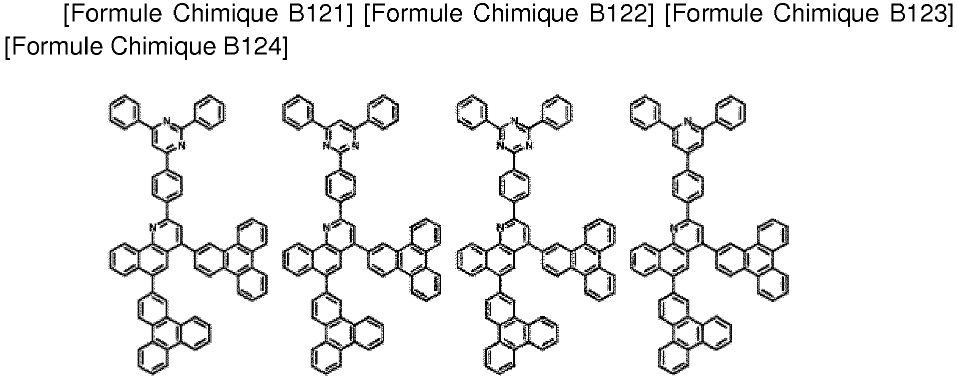

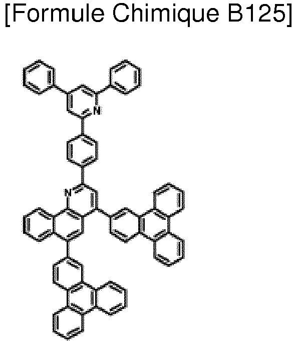

9. Composé pour un dispositif optoélectronique organique selon la Revendication 1 représenté par une des Formules Chimiques C1 à C127 suivantes :

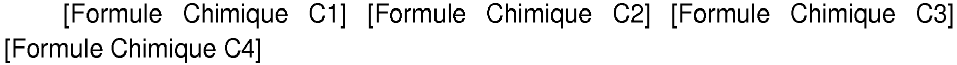

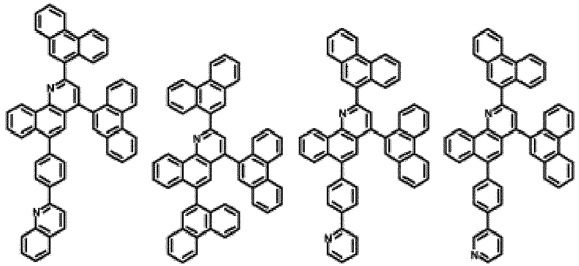

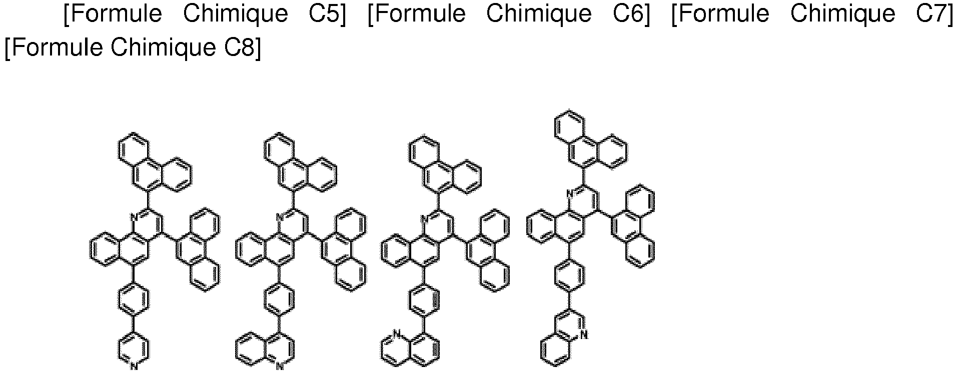

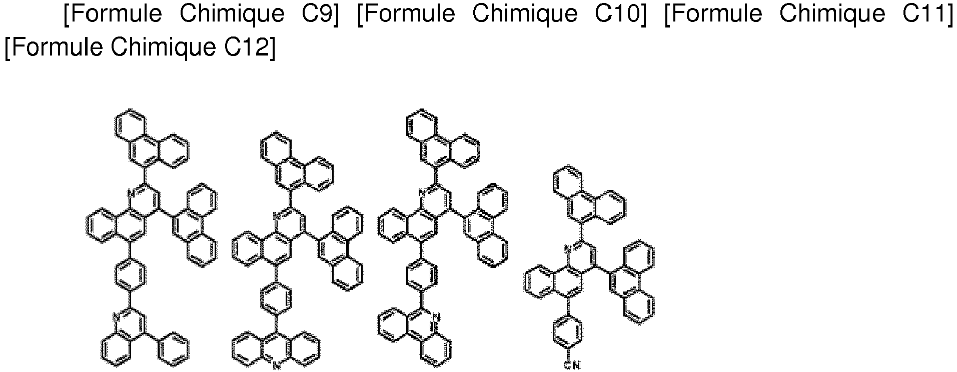

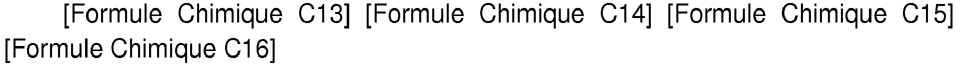

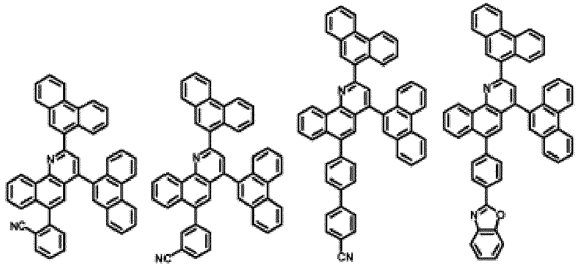

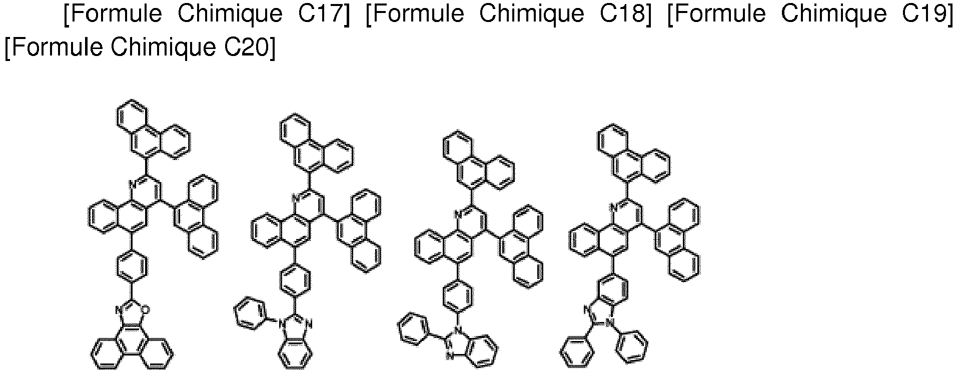

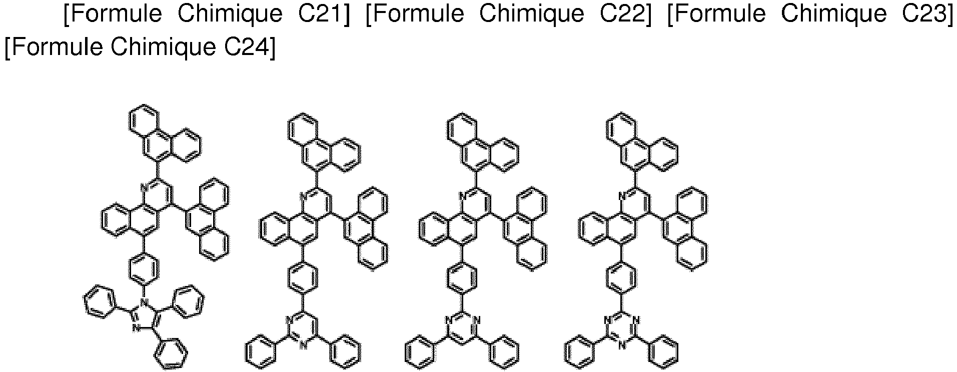

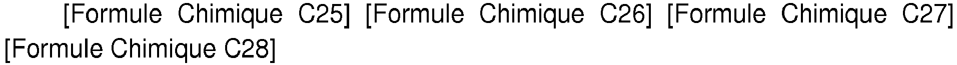

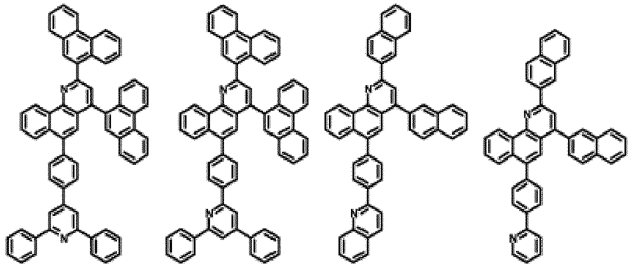

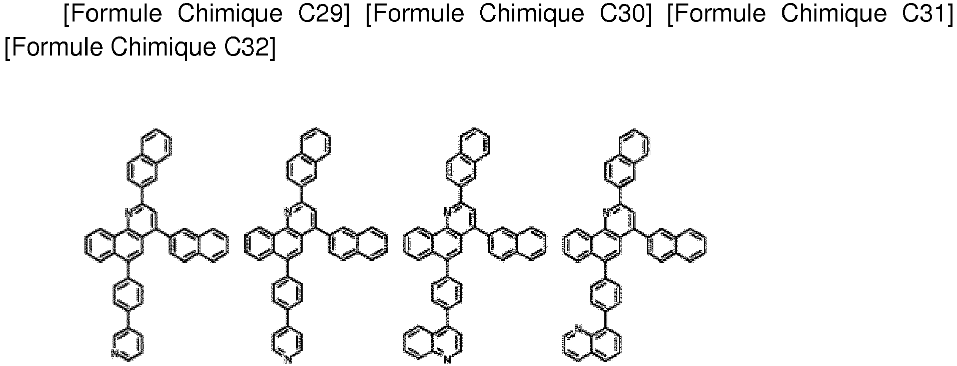

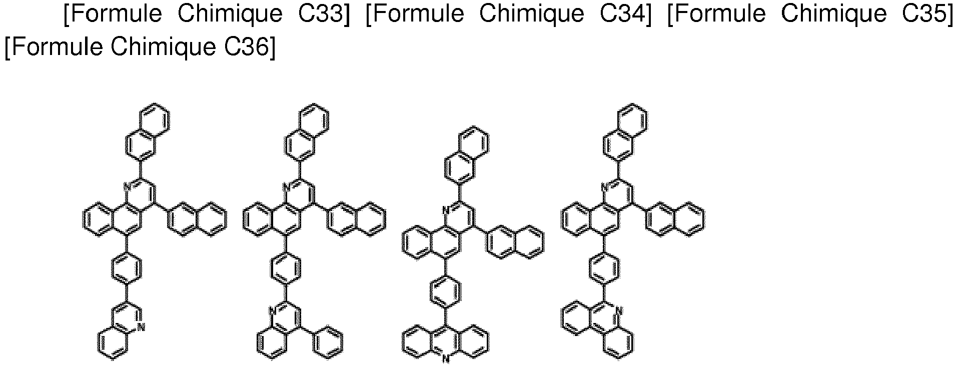

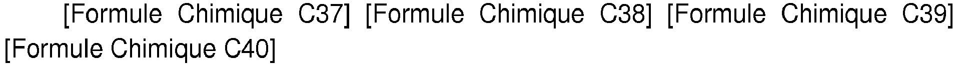

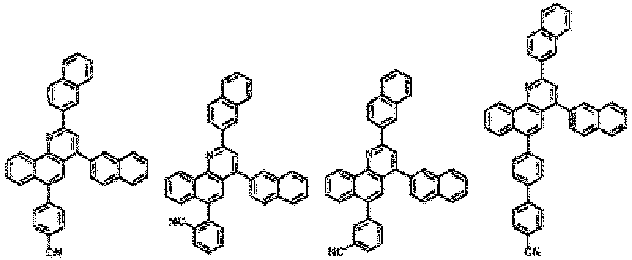

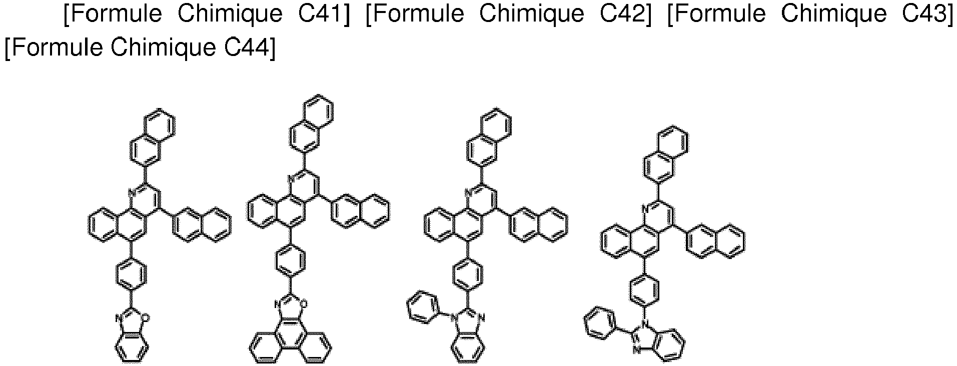

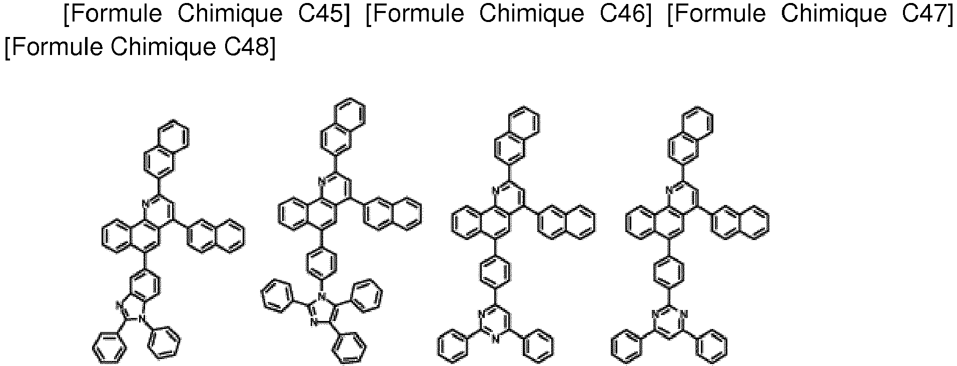

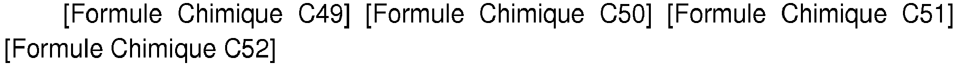

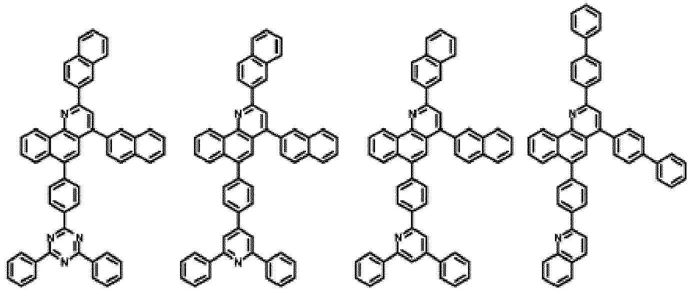

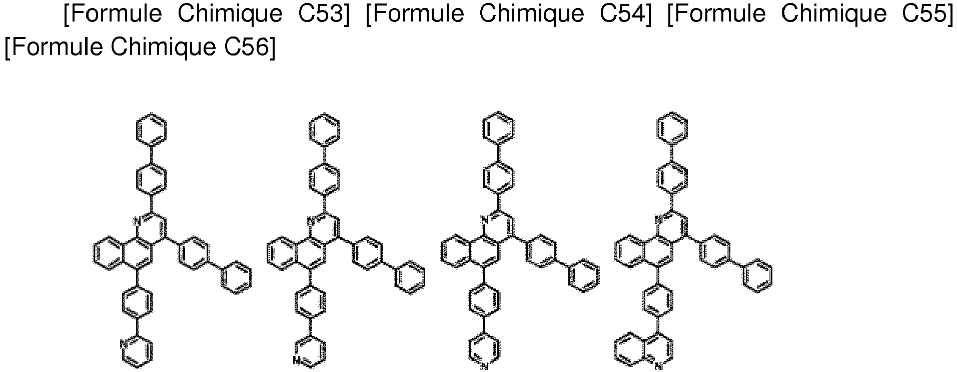

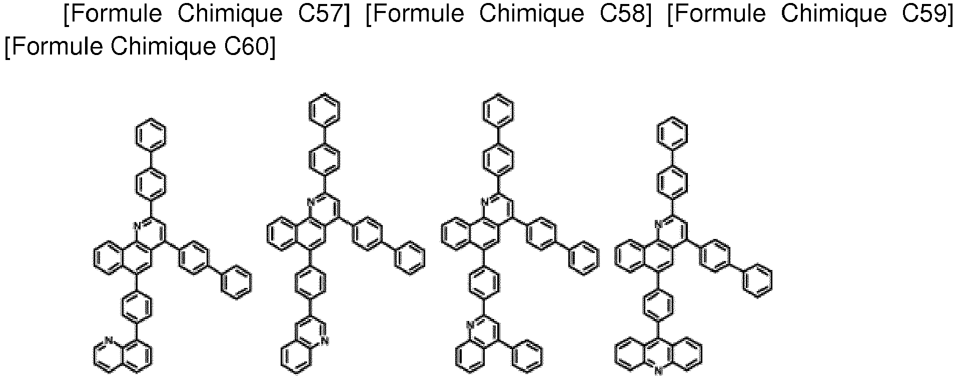

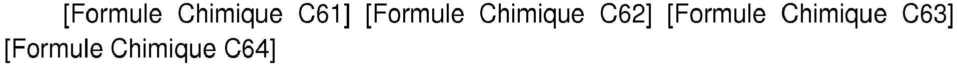

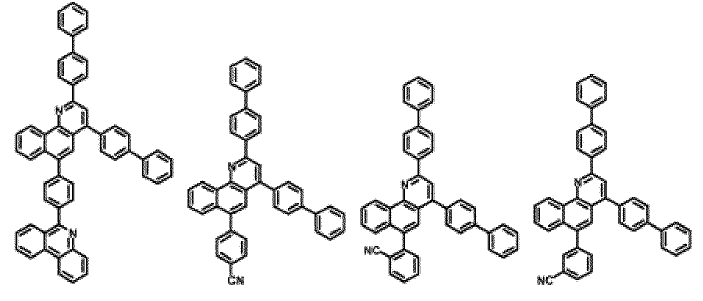

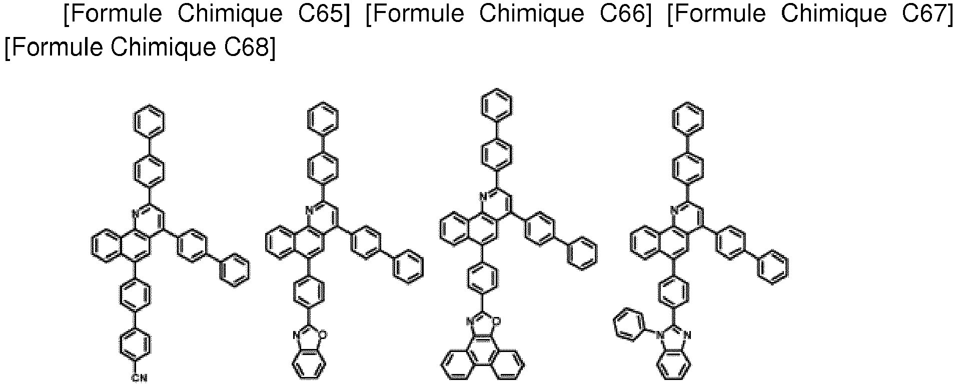

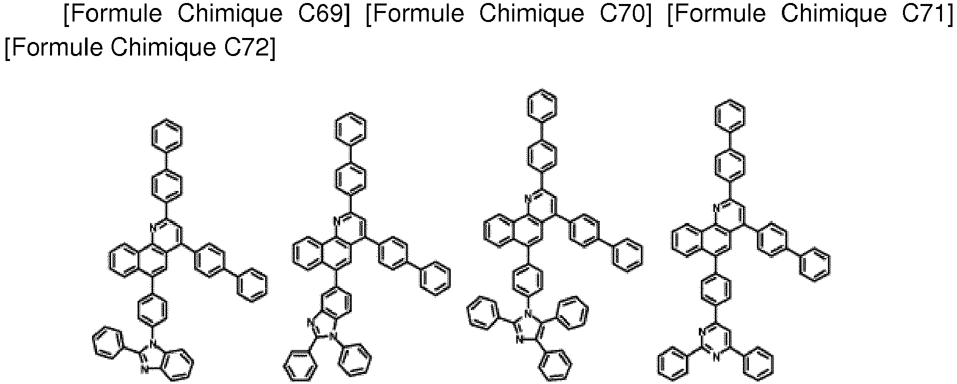

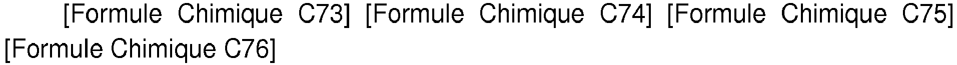

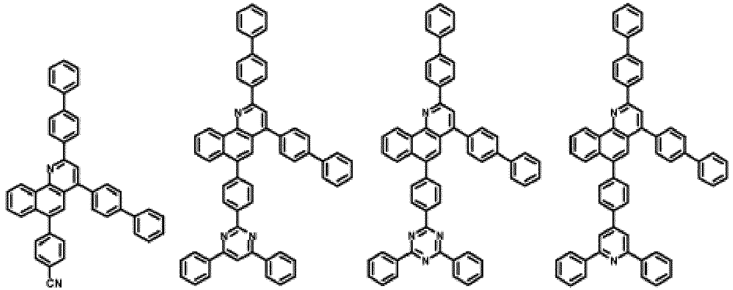

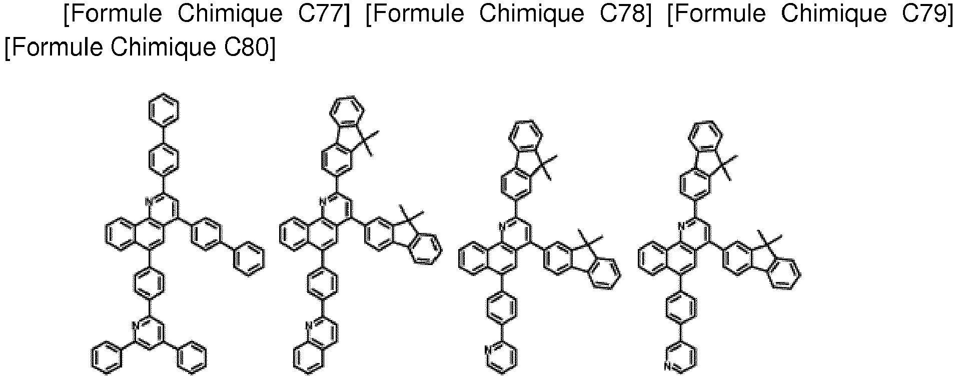

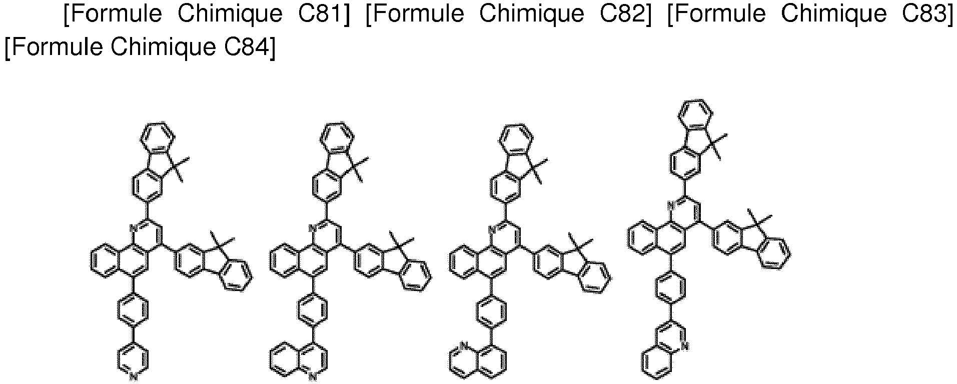

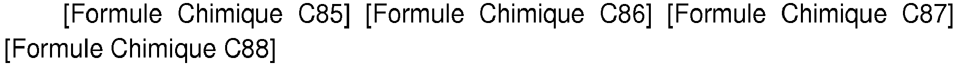

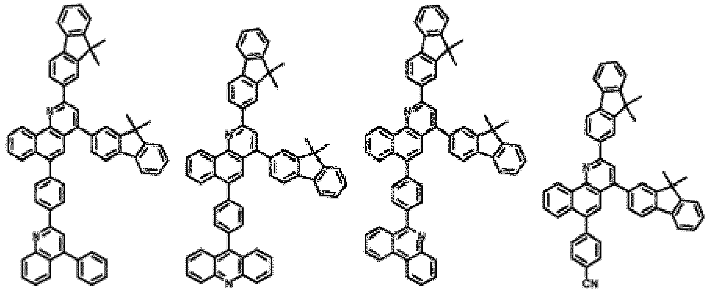

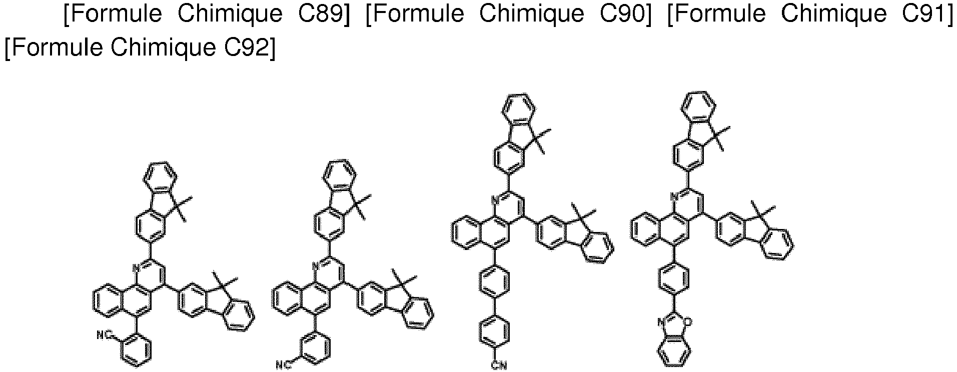

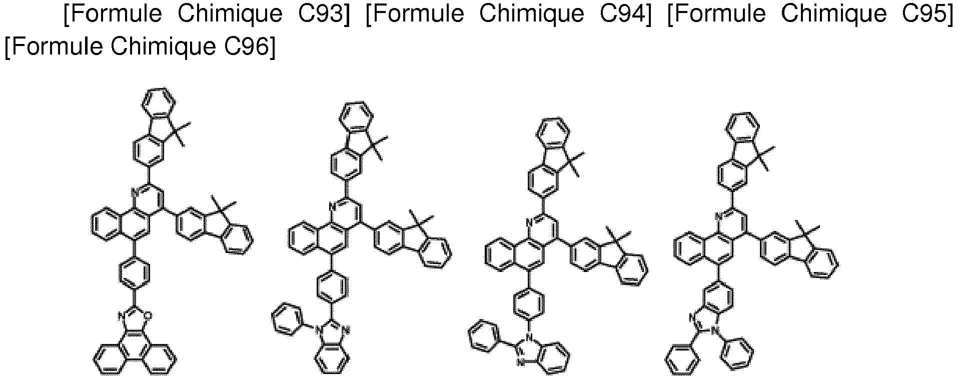

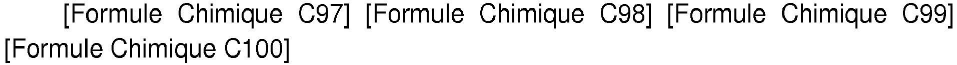

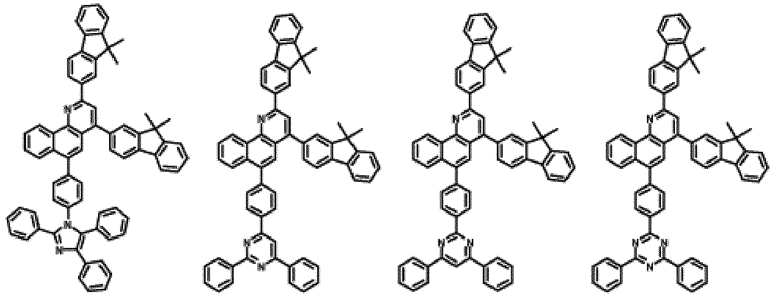

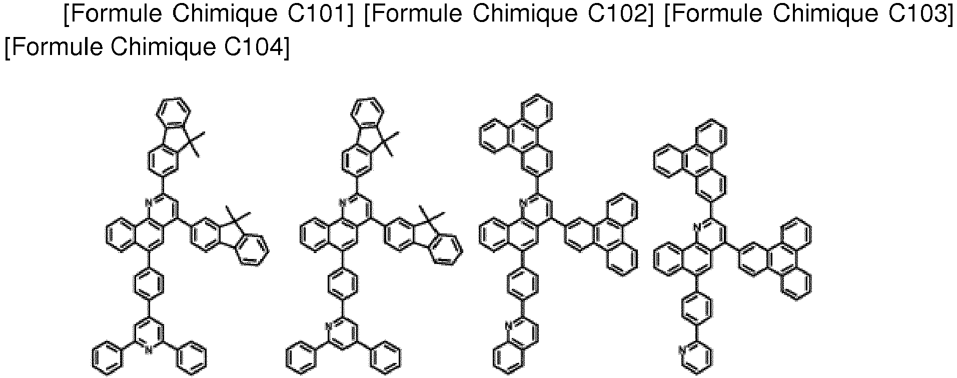

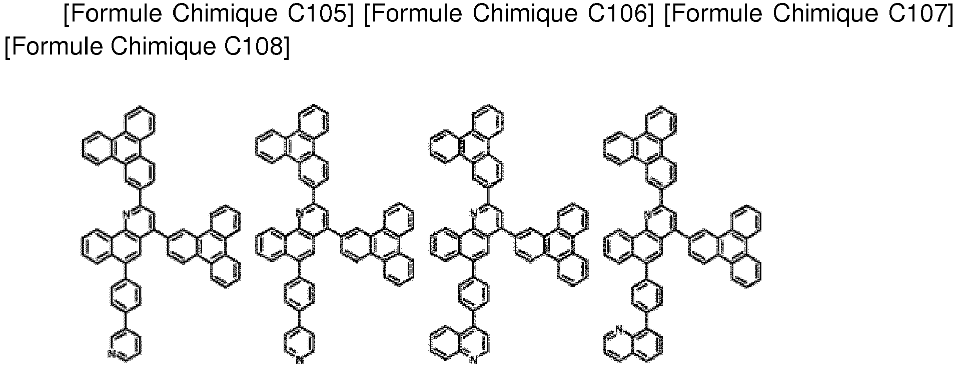

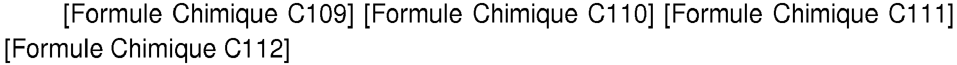

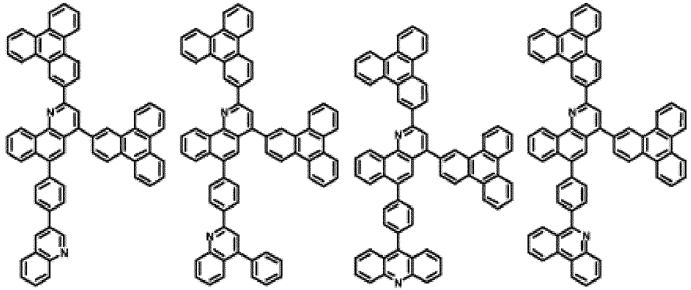

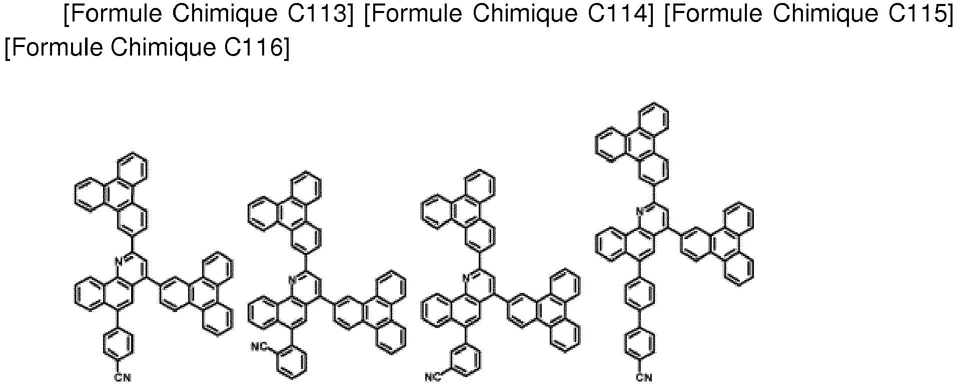

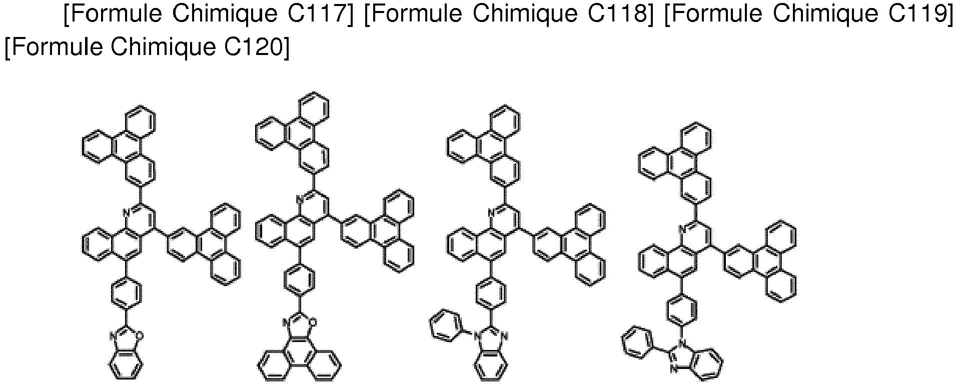

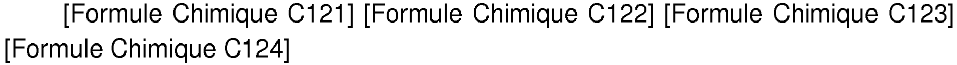

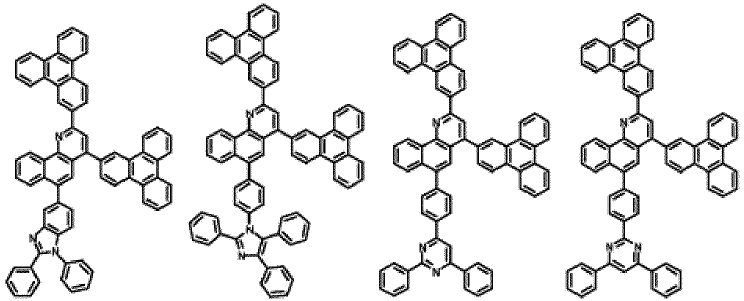

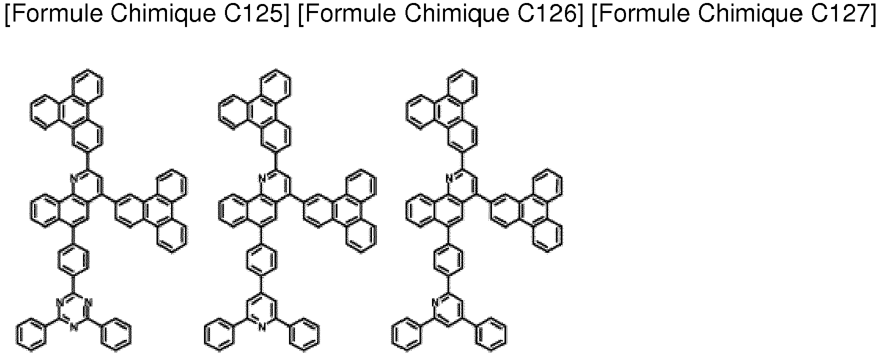

10. Diode électroluminescente organique, comprenant
une anode, une cathode et au moins une ou plusieurs couches minces organiques entre l'anode et la cathode,
dans laquelle au moins une des couches minces organiques comprend le composé pour un dispositif optoélectronique organique selon l'une quelconque des Revendications 1 à 9.

11. Diode électroluminescente organique selon la Revendication 10, dans laquelle la couche mince organique est choisie dans le groupe constitué par une couche d'émission, une couche de transport de trous (HTL), une couche d'injection de trous (HIL), une couche de transport d'électrons (ETL), une couche d'injection d'électrons (EIL), une couche de blocage de trous, et une combinaison de celles-ci.

12. Diode électroluminescente organique selon la Revendication 10, dans laquelle le composé pour un dispositif optoélectronique organique est inclus dans une couche de transport d'électrons (ETL) ou une couche d'injection d'électrons (EIL).

13. Dispositif d'affichage comprenant la diode électroluminescente organique selon la Revendication 10.
